(19) Europäisches Patentamt
European Patent Office
Office européen des brevets

(11) EP 4 570 807 A1

(12) **EUROPEAN PATENT APPLICATION**
published in accordance with Art. 153(4) EPC

(43) Date of publication:
**18.06.2025 Bulletin 2025/25**

(21) Application number: **23838385.5**

(22) Date of filing: **16.01.2023**

(51) International Patent Classification (IPC):
***C07D 519/00*** (2006.01) ***C07D 487/04*** (2006.01)
***C07D 487/12*** (2006.01) ***A61K 31/519*** (2006.01)
***A61P 29/00*** (2006.01) ***A61P 31/00*** (2006.01)
***A61P 35/00*** (2006.01) ***A61P 37/00*** (2006.01)

(52) Cooperative Patent Classification (CPC):
**A61K 31/519; A61K 31/5377; A61K 31/5383; A61K 31/5386; A61P 29/00; A61P 31/00; A61P 35/00; A61P 37/00; A61P 37/02; C07D 487/04; C07D 487/12; C07D 519/00**

(86) International application number:
**PCT/CN2023/072296**

(87) International publication number:
**WO 2024/011883 (18.01.2024 Gazette 2024/03)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **13.07.2022 CN 202210817976**

(71) Applicant: **Jiangsu Tasly Diyi Pharmaceutical Co., Ltd.**
**Huai'an, Jiangsu 223003 (CN)**

(72) Inventors:
- **SONG, Li**
  **Huaian, Jiangsu 223003 (CN)**
- **TANG, Hai**
  **Huaian, Jiangsu 223003 (CN)**
- **MA, Xiaohui**
  **Huaian, Jiangsu 223003 (CN)**
- **ZHOU, Shuiping**
  **Huaian, Jiangsu 223003 (CN)**
- **CAI, Jinyong**
  **Huaian, Jiangsu 223003 (CN)**
- **DONG, Liming**
  **Huaian, Jiangsu 223003 (CN)**
- **SONG, Zhuang**
  **Huaian, Jiangsu 223003 (CN)**

(74) Representative: **Gulde & Partner**
**Patent- und Rechtsanwaltskanzlei mbB**
**Berliner Freiheit 2**
**10785 Berlin (DE)**

# (54) WEE1 INHIBITOR, PREPARATION THEREFOR AND USE THEREOF

(57) The present invention relates to a WEE1 inhibitor, preparation therefor and a use thereof. The structure of the WEE1 inhibitor is represented by Formula I. The present invention relates to a compound of formula (I), or a stereoisomer thereof, or a pharmaceutically acceptable salt thereof, and a use thereof in the preparation of drugs for treating diseases related to WEE1 activity.

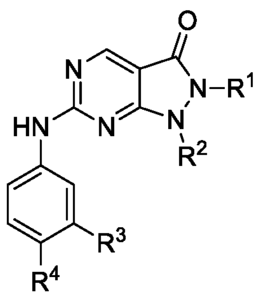

Formula I

EP 4 570 807 A1

## Description

### TECHNICAL FIELD

The present invention relates to compounds that inhibit WEE1 kinase activity, and the use thereof in the treatment of WEE1-mediated disease.

### BACKGROUND ART

Weel tyrosine kinase is the checkpoint of G2 phase of cell cycle. Cell cycle is tightly regulated and controlled. When the cellular DNA is not damaged, the checkpoints of Gl, S and G2 phases promote cells to enter division phase to ensure the successful completion of cell cycle (Clinical Cancer Research,2011,17(13):4200-4207). Cell cycle is regulated and controlled by CDKs (Cyclin-dependent kinases). CDKs family comprises 14 kinds of serine/threonine protein kinases. The activity of CDK is regulated and controlled by phosphorylation and the binding of different cyclins. The transition of cells from G2 phase to division phase is positively regulated by the phosphorylation of CDK1(also called CDC2) and its associated cyclin B. CDK1 is in an inactive state before division and is phosphorylated by WEE1 in tyrosine 15, and then phosphorylated by myelin transcription factor (MYT1) in threonine 14. Therefore, WEE1 is a negative regulator of cell cycle that negatively regulates the passage of cells from G2 phase to division phase by preventing cyclin B and activated CDK1 complexes from entering the nucleus. The expression and activity of WEE1 are both increased in S and G2 phases and decreased in the highly phosphorylated M phase. When cells enter G2 phase and no DNA damage occurs, polo-like protein kinase 1 (PLK1) phosphorylates WEE1, which is degraded by the ubiquitin ligase complex. PLK1 also phosphorylates and activates the protein phosphatase cell division cycle 25 analog (CDC25), which activates CDK1 by dephosphorylation. Active CDK1 binds to cyclin B and promotes cell entry into division phase (Molecular & Cellular Biology, 2012, 32(20):4226).

When a cell's DNA is damaged, the checkpoints of Gl, S, and G2 phases delay the cell's entry into division phase, buying time to repair the damaged DNA before the cell enters division, thus ensuring the integrity of the genome. The key regulator of the G1 phase checkpoint P53 is in a mutated form in many malignant cells (Proceedings of the National Academy of Sciences of the United States of America,2007,104(10):3753-3758 ). The tumor cells with defective P53 function fail to block the cell cycle in G1 phase when DNA is damaged, and are therefore more dependent on the G2 phase checkpoint. In response to DNA damage, the G2 phase checkpoint inhibits CDK1 phosphorylation through two parallel and interconnected pathways, thereby delaying cell entry into division phase. Depending on the type of DNA damage, ataxia telangiectasia mutated (ATM) protein kinase or ataxia telangiectasia-related (ATR) protein kinase is activated. (Oncotarget, 2016, 7 (31):49902-49916)

ATM is activated by ionizing radiation, radioactive agents, and agents that cause double-stranded DNA breaks. ATM phosphorylates and activates checkpoint kinase 2 (CHK2), CHK2 phosphorylates 5er216 of cell division cycle 25C phosphatase (CDC25C). This leads to a nuclear export and cytoplasmic segregation of CDC25C, thereby inhibiting its phosphorylation activity. Inhibition of CDC25C activity leads to inhibition of CDK1/CDK2 binding cyclin B complex phosphorylation, which puts CDK1 in an inactivated form and inhibits cell entry into division (Molecular Cancer, 2014, 13(1):72).

ATR is activated by a wide range of genotoxic stimuli that cause single-stranded DNA breaks.

ATR is the main kinase responsible for the phosphorylation and activation of CHK1. In contrast to CHK2, which can only be activated by ATM, CHK1 can be activated by both ATM and ATR. CHK1 phosphorylates both WEE1 and CDC25C, activates WEE1 kinase activity and inhibits CDC25C phosphatase activity. WEE1 phosphorylates CDK1-binding cyclin B, leading to cell cycle arrest in G2 phase and providing time for DNA repair (Drug News&Perspectives,2010,23(7):425).

WEE1 is overexpressed in many malignant tumors, such as hepatocellular carcinoma, breast cancer, malignant glioma, melanoma, adult and pediatric brain tumors. Part of these tumor cells have abnormal G1 checkpoints, and inhibition of WEE1 activity leads to G2 phase checkpoint malfunction, at this time cells with unrepaired damaged DNA will continue to divide and eventually divide to death (Molecular Cancer Therapeutics, 2013,12(12):2675-2684). Inhibition of WEE1 activity, whether by pyrimidine derivatives (PD0166285) or small interfering RNA knockdown, will make ovarian, colon, cervical, osteosarcoma, malignant glioma, and lung cancer cells more sensitive to DNA damage produced by radiation and topoisomerase inhibition. Therefore, WEE1 inhibitors have a wide scope for development both as single drug and concomitant drugs (Cancer Biology &Therapy, 2010, 9(7):523-525).

Small molecule compounds with WEE1 kinase inhibitory activity were disclosed in the patent applications of WO2007126122, WO2008133866, WO2013012681, WO2013126656, WO2014167347, WO2015092431, WO2018011569, WO2018011570, WO2018090939, WO2018133829, WO2018171633, etc. At present, the compound with the fastest development progress is AZD1775, which has entered the phase II clinical trial and shows favorable cancer treatment results.

## SUMMARY OF THE INVENTION

The present invention is aimed at providing a compound of Formula I, or a stereoisomer thereof, or a pharmaceutically acceptable salt thereof:

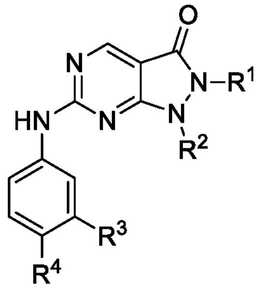

Formula I

wherein,

the $R^1$ is selected from a group consisting of $-C_{1\sim6}$ alkyl, $-C_{2\sim6}$ alkenyl, $-C_{2\sim6}$ alkynyl, $-C_{0\sim2}$ alkylene-CN, $-C_{0\sim2}$ alkylene-(3~10-membered cycloalkyl), $-C_{0\sim2}$ alkylene-(3~10-membered heterocycloalkyl):
$R^2$ is selected from a group consisting of

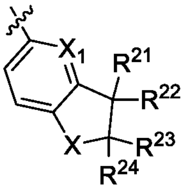 , 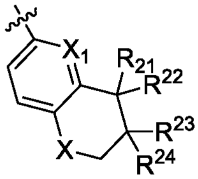 , 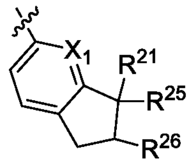 ,

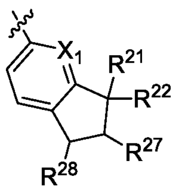 , 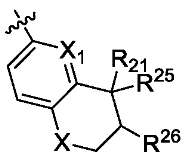 , 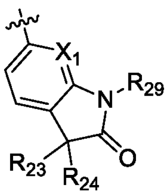 , 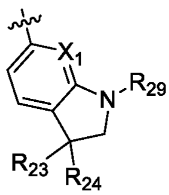 , 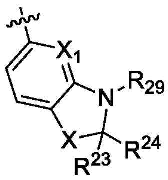 , 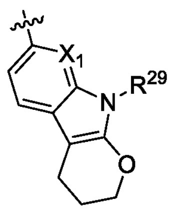 ,

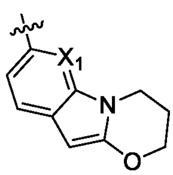 , 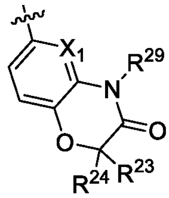 , 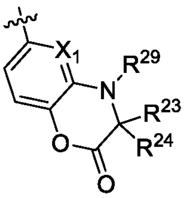 , 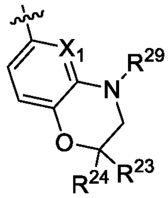 , 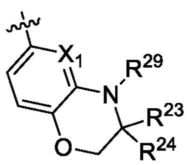 , 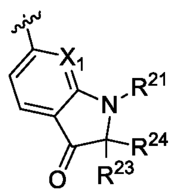 ,

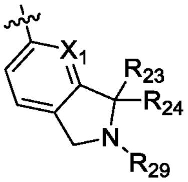 ;

the X is selected from a group consisting of O, NH or $CH_2$;
the $X_1$ is selected from a group consisting of CH or N;
$R^{21}$, $R^{22}$, $R^{29}$ are independently selected from a group consisting of hydrogen, deuterium, halogen, cyano, nitro, -OH, $-C_{1\sim6}$ alkyl, halogen-substituted $C_{1\sim6}$ alkyl, $-C_{0\sim2}$ alkylene-OH, $-O(C_{1\sim6}$ alkyl), -O(halogen-substituted $C_{1\sim6}$ alkyl),

-$NH_2$, -$C_{0\sim2}$ alkylene-NH($C_{1\sim6}$ alkyl), -$C_{0\sim2}$ alkylene-N($C_{1\sim6}$ alkyl) ($C_{1\sim6}$ alkyl), -$C_{0\sim2}$ alkylene-(3~10-membered cycloalkyl), -$C_{0\sim2}$ alkylene-(3~10-membered heterocycloalkyl);

the $R^{23}$, $R^{24}$ together with the atom adjacent therewith form 3~10-membered carbocyclyl, 3~10-membered heterocyclyl;

the $R^{25}$, $R^{26}$ together with the atom adjacent therewith form 3~10-membered carbocyclyl, 3~10-membered heterocyclyl;

the $R^{27}$, $R^{28}$ together with the atom adjacent therewith form 3~10-membered carbocyclyl, 3~10-membered heterocyclyl;

$R^3$ is selected from a group consisting of hydrogen, deuterium, halogen, cyano, nitro, -$C_{1\sim6}$ alkyl, halogen-substituted $C_{1\sim6}$ alkyl, -$C_{0\sim2}$ alkylene-OH, -O($C_{1\sim6}$ alkyl), -O(halogen-substituted $C_{1\sim6}$ alkyl), -$NH_2$, -$C_{0\sim2}$ alkylene-NH($C_{1\sim6}$ alkyl), -$C_{0\sim2}$ alkylene-N($C_{1\sim6}$ alkyl) ($C_{1\sim6}$ alkyl);

the $R^4$ is selected from a group consisting of 3~12-membered heterocycloalkyl; the heterocycloalkyl is optionally substituted by one, two, three or four independent $R^{41}$;

the $R^{41}$ is selected from a group consisting of hydrogen, halogen, cyano, nitro, -OH, -$C_{1\sim6}$ alkyl, halogen-substituted $C_{1\sim6}$ alkyl, -$C_{0\sim2}$ alkylene-OH, -O($C_{1\sim6}$ alkyl), -O(halogen-substituted $C_{1\sim6}$ alkyl), -$NH_2$, -$C_{0\sim2}$ alkylene-NH($C_{1\sim6}$ alkyl), -$C_{0\sim2}$ alkylene-N($C_{1\sim6}$ alkyl) ($C_{1\sim6}$ alkyl), -C(O)$C_{1\sim6}$ alkyl, 3~10-membered carbocyclyl, 3~10-membered heterocyclyl; the carbocyclyl, heterocyclyl are optionally substituted by one, two, three or four independent $R^{31}$;

or, the $R^3$, $R^4$ together with the atom adjacent therewith form 3~10-membered carbocyclyl, 3~10-membered heterocyclyl; said carbocyclyl, heterocycloalkyl is optionally substituted by one, two, three or four independent $R^{31}$;

the $R^{31}$ is selected from a group consisting of hydrogen, halogen, cyano, nitro, -OH, -$C_{1\sim6}$ alkyl, halogen-substituted $C_{1\sim6}$ alkyl, -$C_{0\sim2}$ alkylene-OH, -O($C_{1\sim6}$ alkyl), -O(halogen-substituted $C_{1\sim6}$ alkyl), -$NH_2$, -$C_{0\sim2}$ alkylene-NH($C_{1\sim6}$ alkyl), -$C_{0\sim2}$ alkylene-N($C_{1\sim6}$ alkyl) ($C_{1\sim6}$ alkyl).

Preferably, in the compound of the invention, or a stereoisomer thereof, or a pharmaceutically acceptable salt thereof, the $R^1$ is selected from a group consisting of

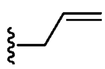
,

methyl, ethyl,

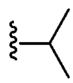
,

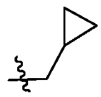
.

Preferably, in the compound of the invention, or a stereoisomer thereof, or a pharmaceutically acceptable salt thereof, $R^{21}$, $R^{22}$, $R^{29}$ are independently selected from a group consisting of hydrogen, deuterium, cyano, methyl, ethyl, -OH, trifluoromethyl, cyclopropyl, -$CH_2OH$, -$NH_2$.

Preferably, in the compound of the invention, or a stereoisomer thereof, or a pharmaceutically acceptable salt thereof, the $R^{23}$, $R^{24}$ together with the atom adjacent therewith form cyclopropyl, cyclobutyl, cyclopentyl;

the $R^{25}$, $R^{26}$ together with the atom adjacent therewith form cyclopropyl, cyclobutyl, cyclopentyl;

the $R^{27}$, $R^{28}$ together with the atom adjacent therewith form cyclopropyl, cyclobutyl, cyclopentyl.

Preferably, in the compound of the invention, or a stereoisomer thereof, or a pharmaceutically acceptable salt thereof, the $R^2$ is selected from a group consisting of

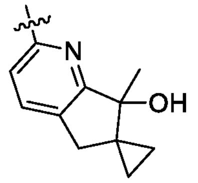,

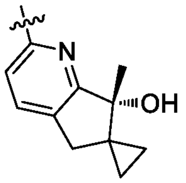, 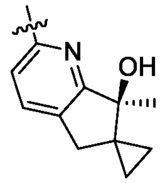, 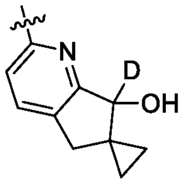, 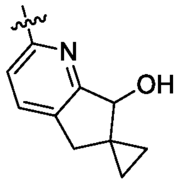, 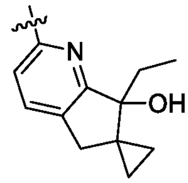, 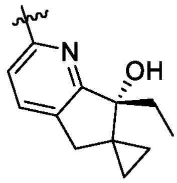,

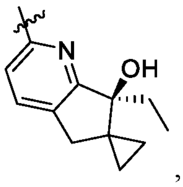, 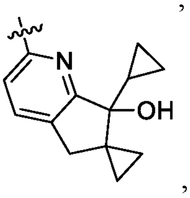, 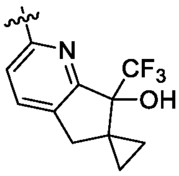, 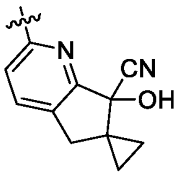, 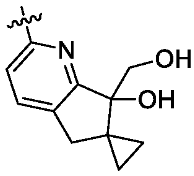, 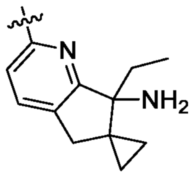,

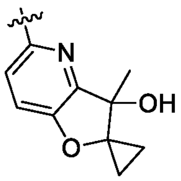, 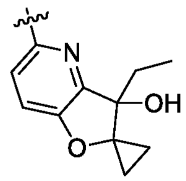, 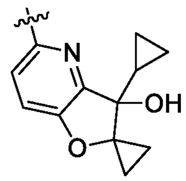, 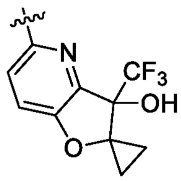, 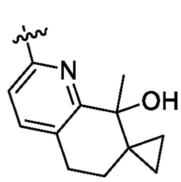, 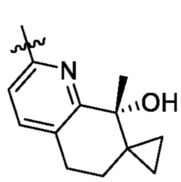,

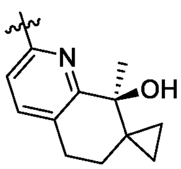, 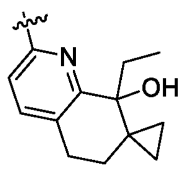, 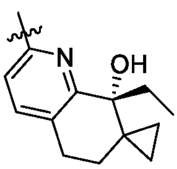, 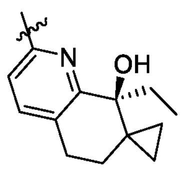, 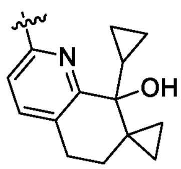, 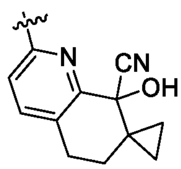,

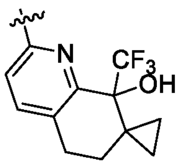, 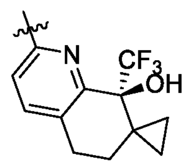, 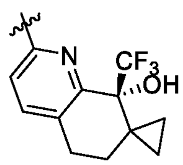, 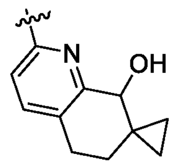, 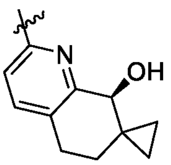, 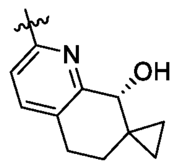,

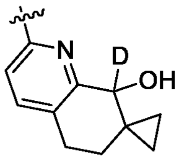, 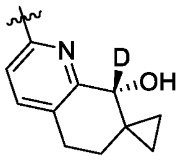, 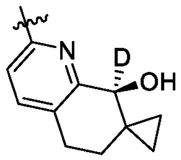, 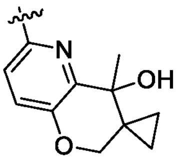, 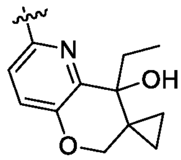, 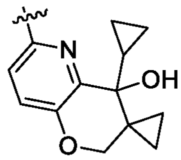,

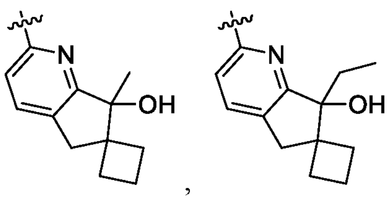, , 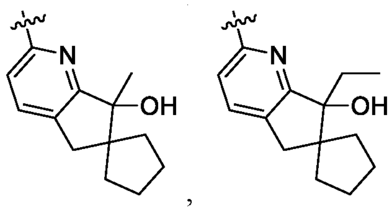, , 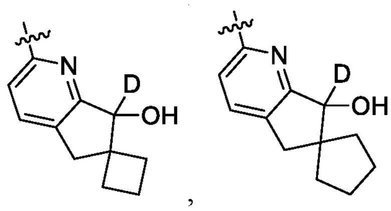, ,

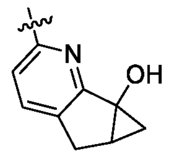 , 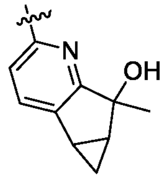 , 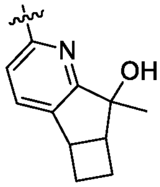 , 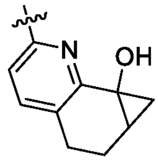 , 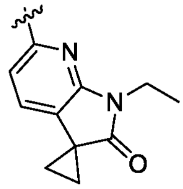 , 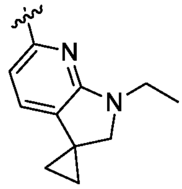 ,

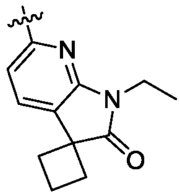 , 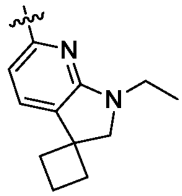 , 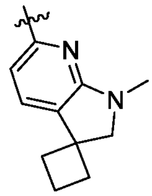 , 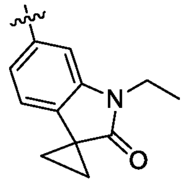 , 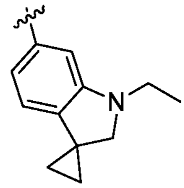 , 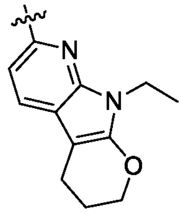 ,

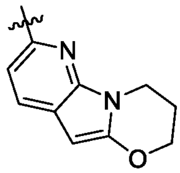 , 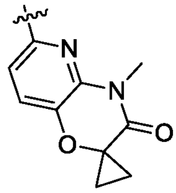 , 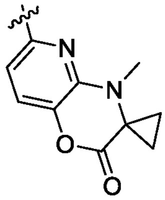 , 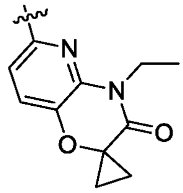 , 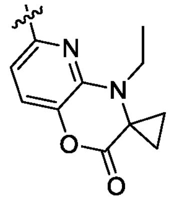 , 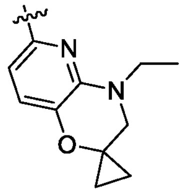 ,

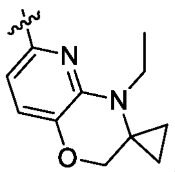 , 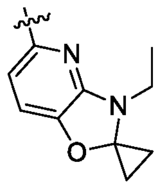 , 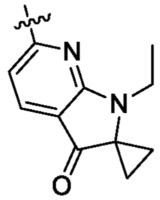 , 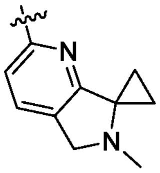 .

Preferably, in the compound of the invention, or a stereoisomer thereof, or a pharmaceutically acceptable salt thereof, the $R^3$ is selected from a group consisting of hydrogen, fluoro, methyl, $-CH_2OH$, methoxy.

Preferably, in the compound of the invention, or a stereoisomer thereof, or a pharmaceutically acceptable salt thereof, the $R^4$ is selected from a group consisting of nitrogen-containing 6-membered heterocyclyl, 7-membered nitrogen-containing bridged-ring, 8-membered nitrogen-containing bridged-ring, 9-membered nitrogen-containing heterospiro-ring, 11-membered nitrogen-containing heterospiro-ring.

Furthermore, the $R^4$ is selected from a group consisting of

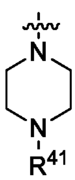 , 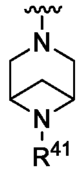 , 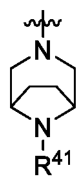 , 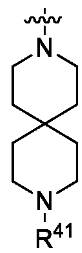 ,

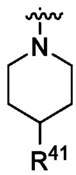 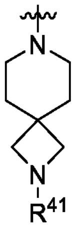 , 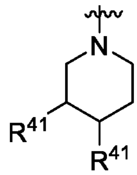 , 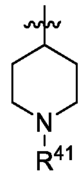 , 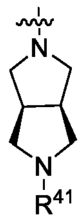 , 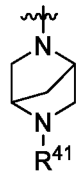 , 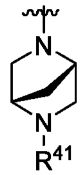 , 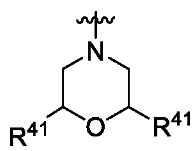 .

Furthermore, the $R^4$ is selected from a group consisting of

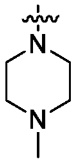, 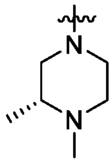, 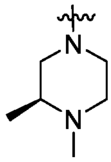, 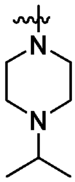,

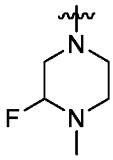, 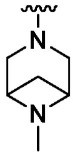, 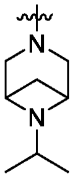, 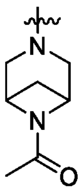, 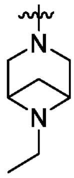, 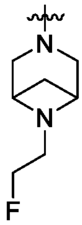, 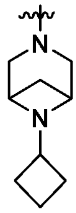, 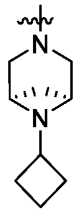, 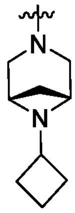, 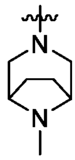, 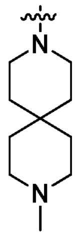, 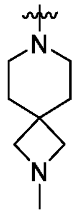,

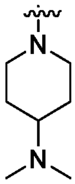, 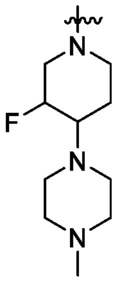, 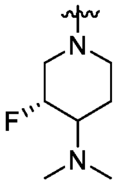, 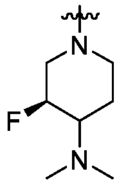, 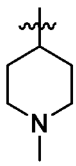, 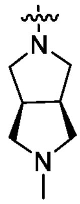, 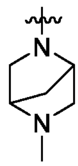, 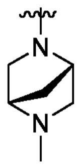, 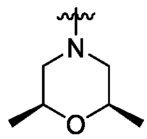, 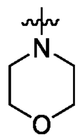.

Preferably, in the compound of the invention, or a stereoisomer thereof, or a pharmaceutically acceptable salt thereof, the $R^3$, $R^4$ together with the atom adjacent therewith form 6-membered nitrogen-containing heterocyclyl.

More preferably, in the compound of the invention, or a stereoisomer thereof, or a pharmaceutically acceptable salt thereof, the $R^3$, $R^4$ together with the atom adjacent therewith form

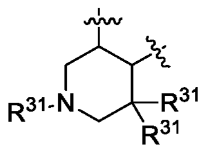, 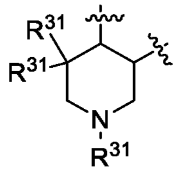.

Furthermore, the $R^{31}$ is selected from a group consisting of methyl.

Preferably, in the compound of the invention, or a stereoisomer thereof, or a pharmaceutically acceptable salt thereof, the compound of Formula I is specifically:

| No. | Compound Structure | No. | Compound Structure |
|---|---|---|---|
| WEE1-001 | 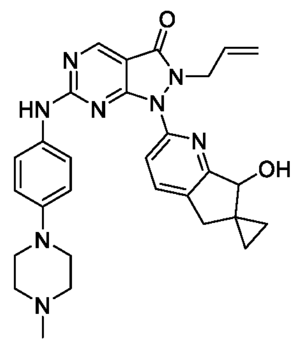 | WEE1-002 | 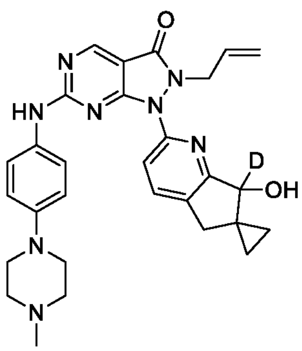 |

(continued)

| No. | Compound Structure | No. | Compound Structure |
|---|---|---|---|
| WEE1-003 | 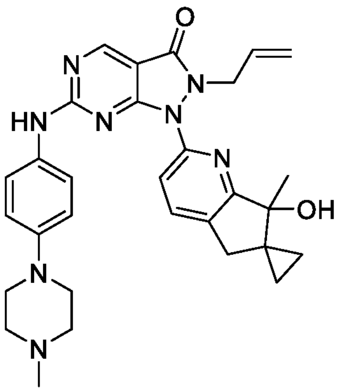 | WEE1-004 | 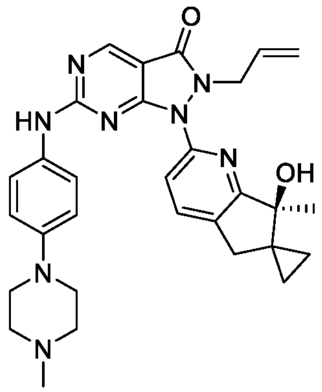 |
| WEE1-005 | 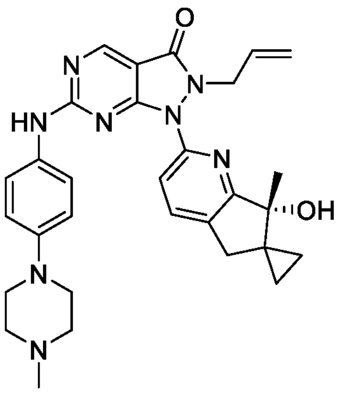 | WEE1-006 | 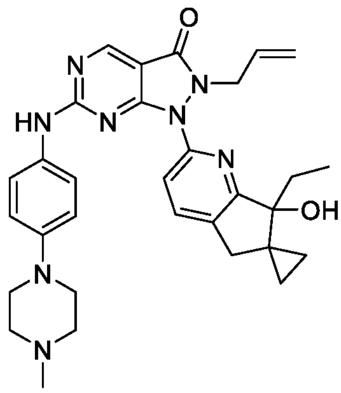 |
| WEE1-007 | 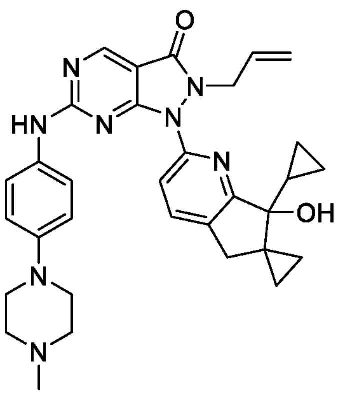 | WEE1-008 | 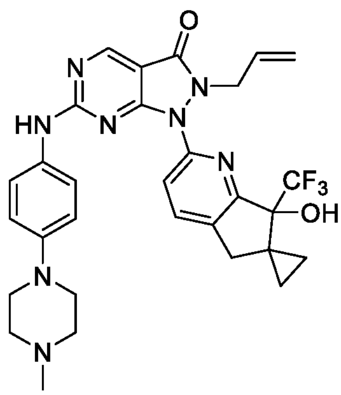 |
| WEE1-009 | 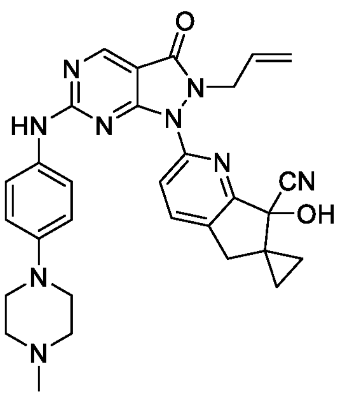 | WEE1-010 | 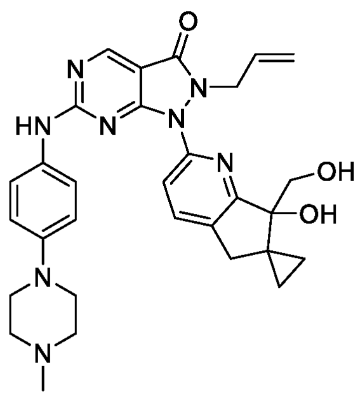 |

(continued)

| No. | Compound Structure | No. | Compound Structure |
|---|---|---|---|
| WEE1-011 | 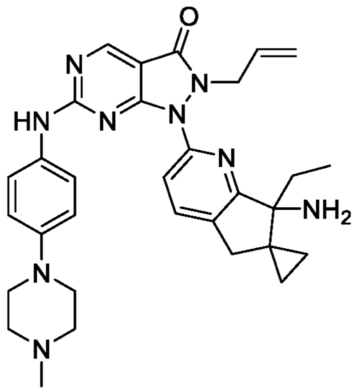 | WEE1-012 | 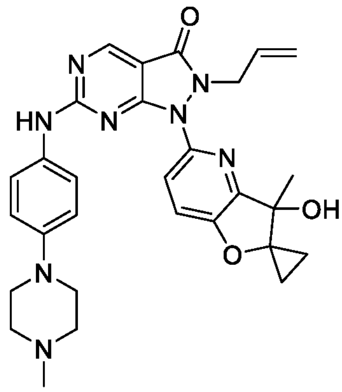 |
| WEE1-013 | 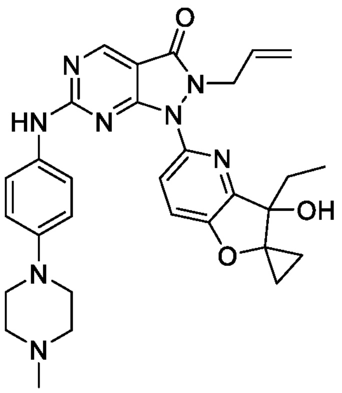 | WEE1-014 | 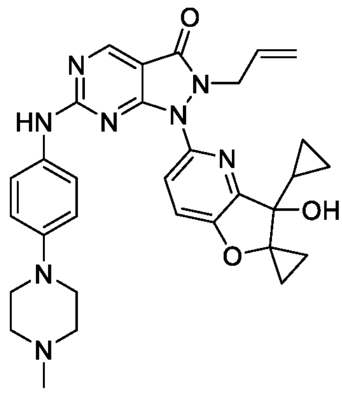 |
| WEE1-015 | 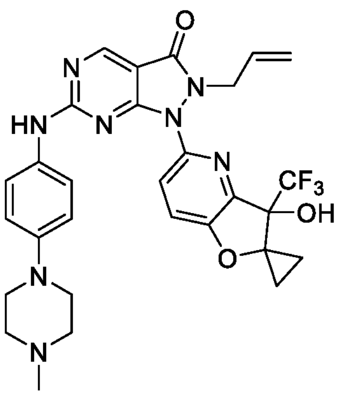 | WEE1-016 | 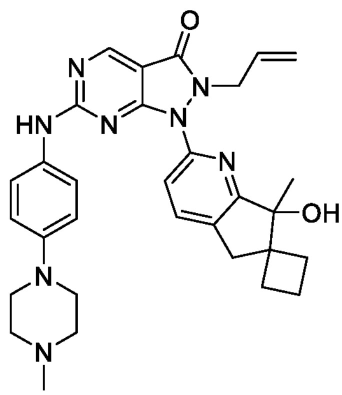 |
| WEE1-017 | 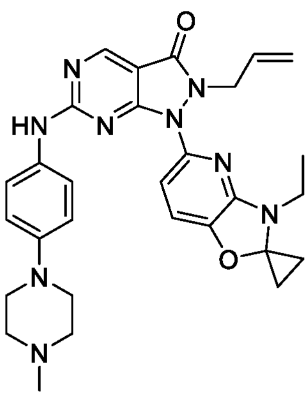 | WEE1-018 | 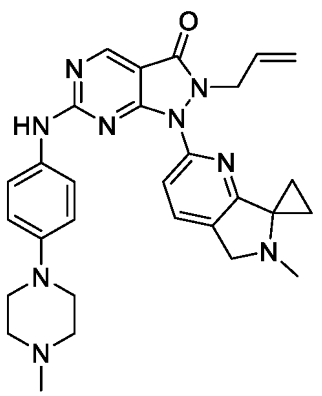 |

(continued)

| No. | Compound Structure | No. | Compound Structure |
|---|---|---|---|
| WEE1-019 | 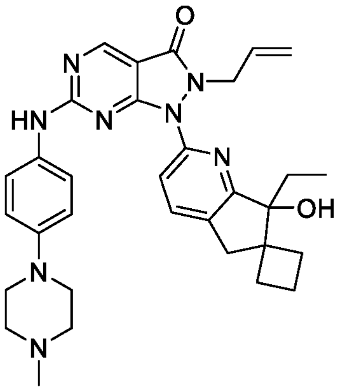 | WEE1-020 | 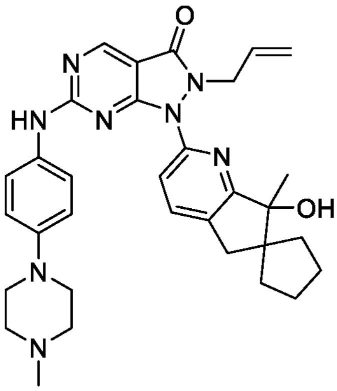 |
| WEE1-021 | 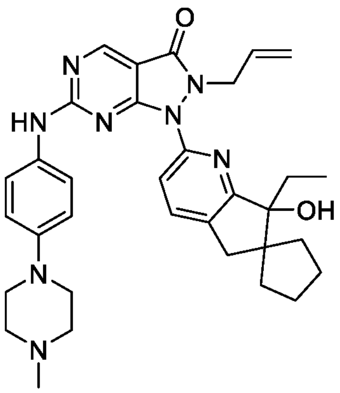 | WEE1-022 | 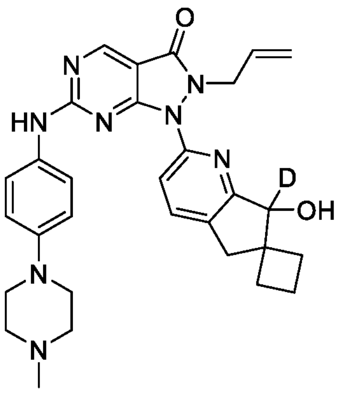 |
| WEE1-023 | 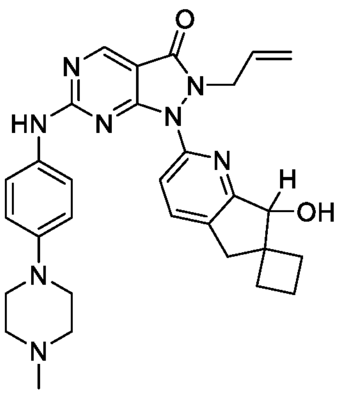 | WEE1-024 | 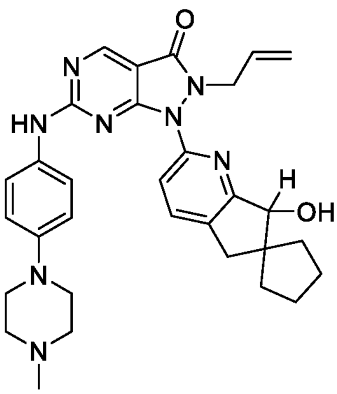 |
| WEE1-025 | 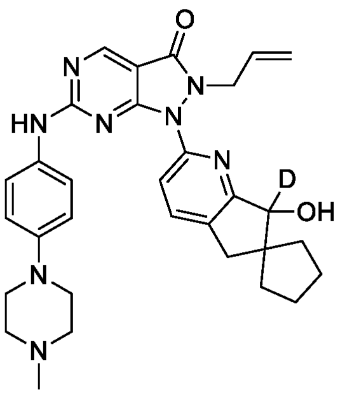 | WEE1-026 | 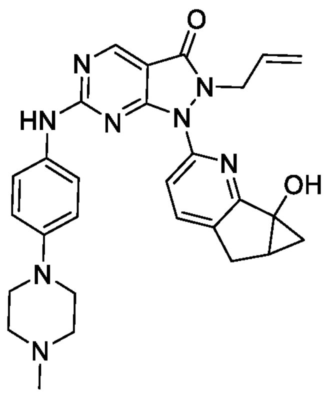 |

(continued)

| No. | Compound Structure | No. | Compound Structure |
|---|---|---|---|
| WEE1-027 | 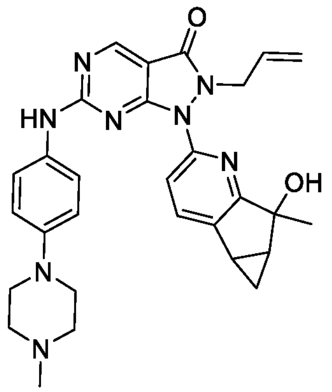 | WEE1-028 | 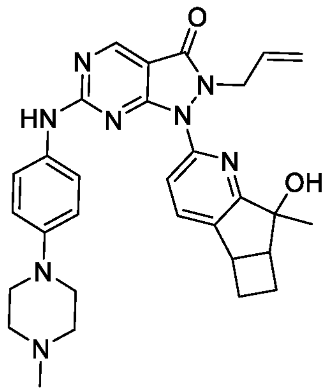 |
| WEE1-029 | 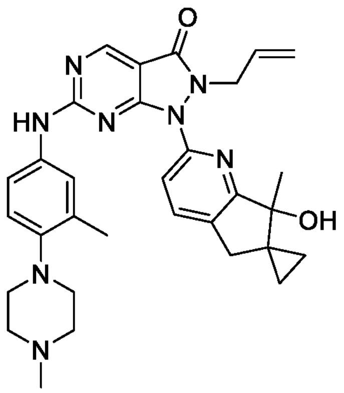 | WEE1-030 | 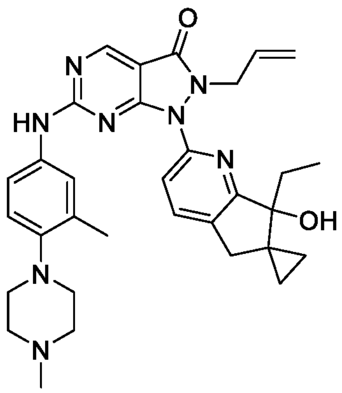 |
| WEE1-031 | 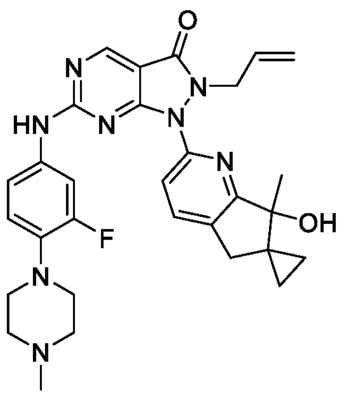 | WEE1-032 | 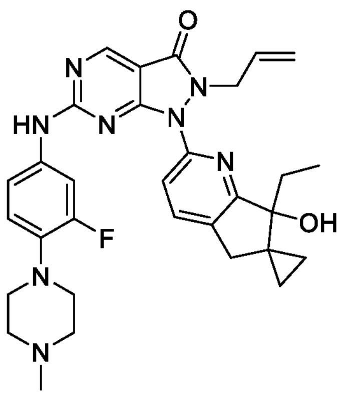 |
| WEE1-033 | 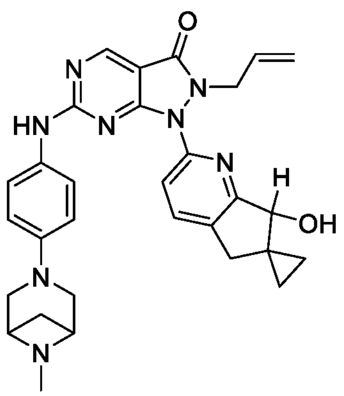 | WEE1-034 | 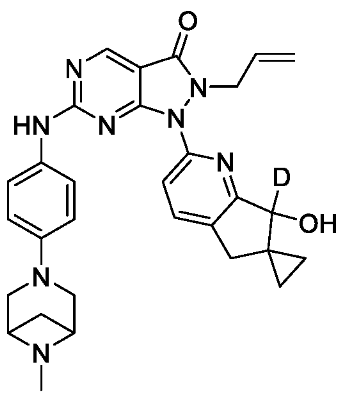 |

(continued)

| No. | Compound Structure | No. | Compound Structure |
|---|---|---|---|
| WEE1-035 | 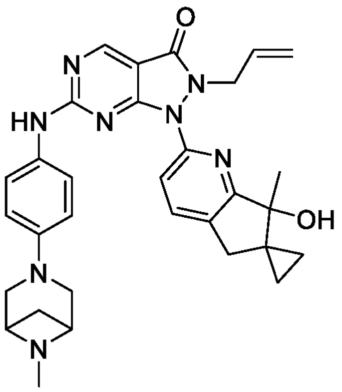 | WEE1-036 | 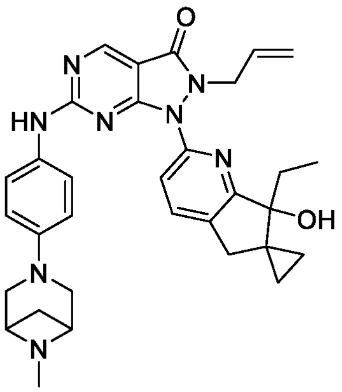 |
| WEE1-037 | 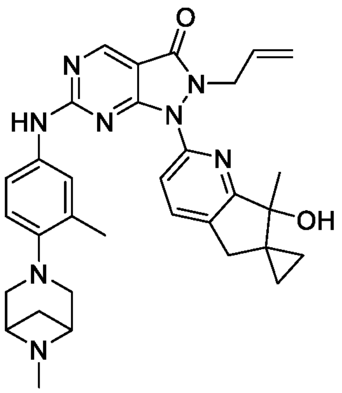 | WEE1-038 | 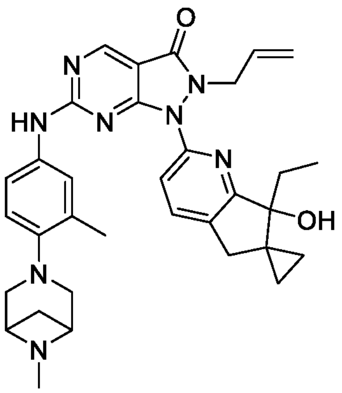 |
| WEE1-039 | 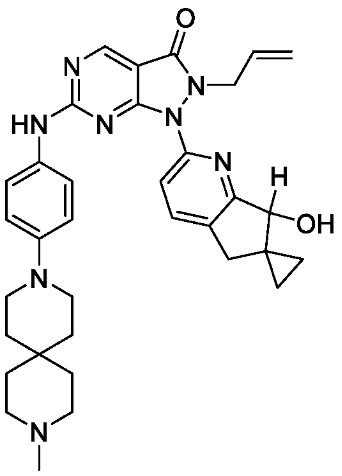 | WEE1-040 | 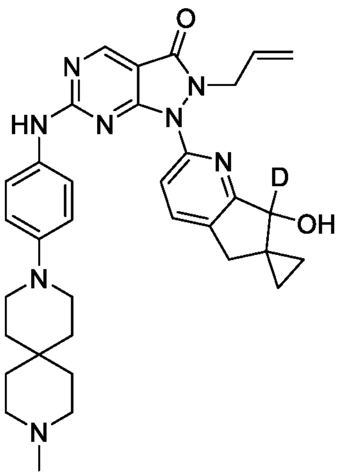 |

(continued)

| No. | Compound Structure | No. | Compound Structure |
|---|---|---|---|
| WEE1-041 | 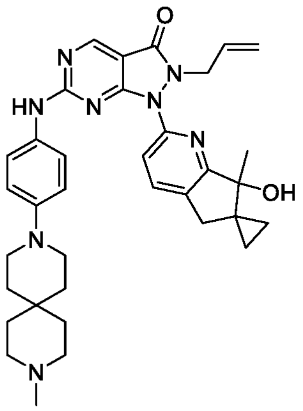 | WEE1-042 | 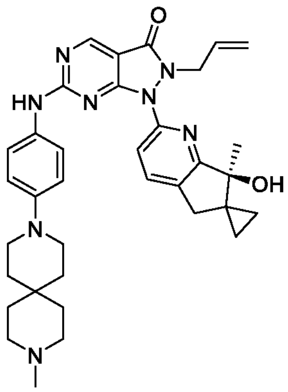 |
| WEE1-043 | 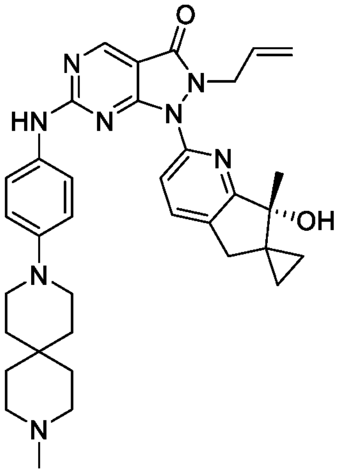 | WEE1-044 | 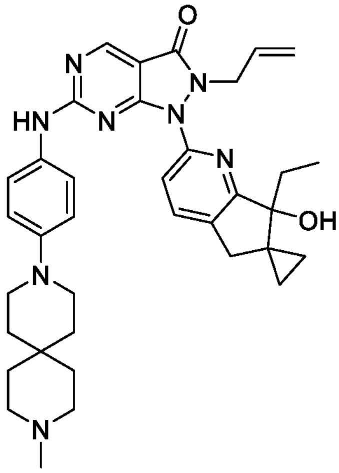 |
| WEE1-045 | 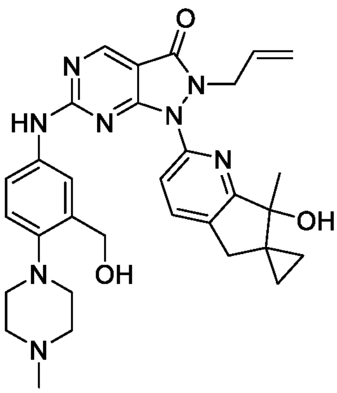 | WEE1-046 | 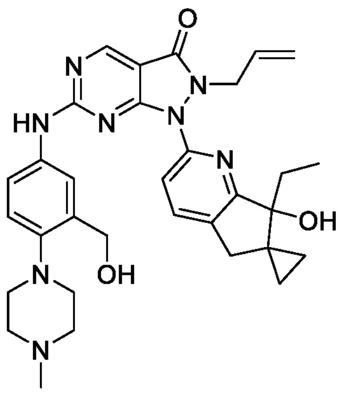 |

(continued)

| No. | Compound Structure | No. | Compound Structure |
|---|---|---|---|
| WEE1-047 | 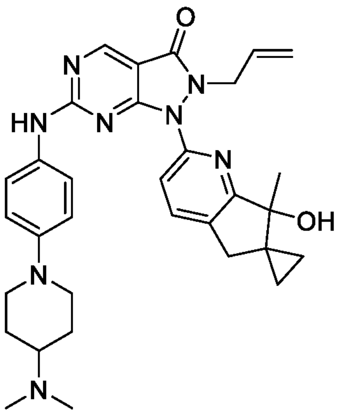 | WEE1-048 | 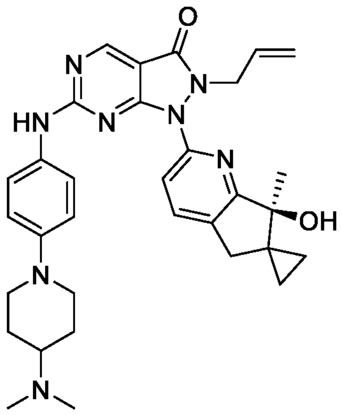 |
| WEE1-049 | 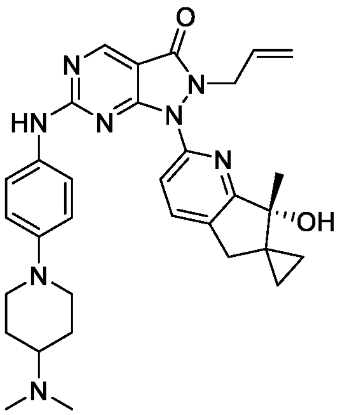 | WEE1-050 | 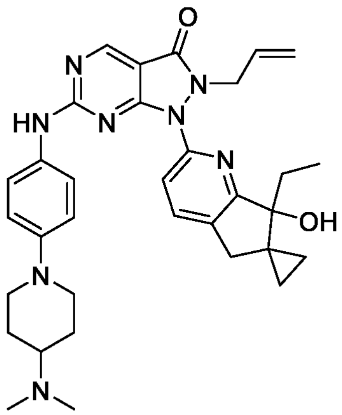 |
| WEE1-051 | 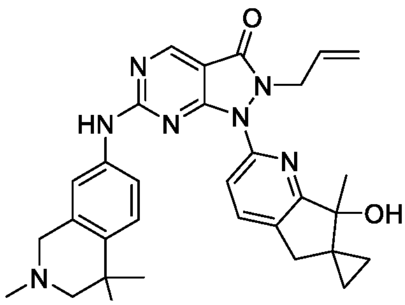 | WEE1-052 | 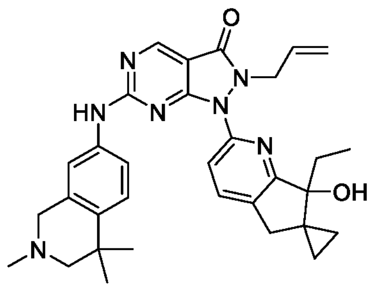 |
| WEE1-053 | 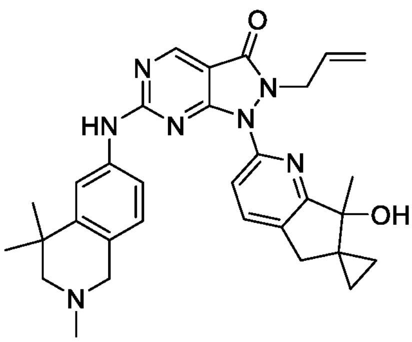 | WEE1-054 | 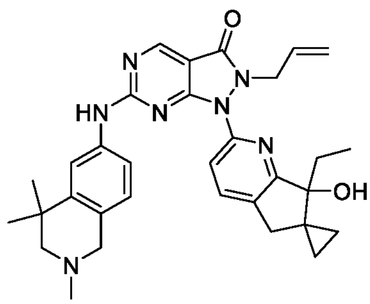 |

(continued)

| No. | Compound Structure | No. | Compound Structure |
|---|---|---|---|
| WEE1-055 | 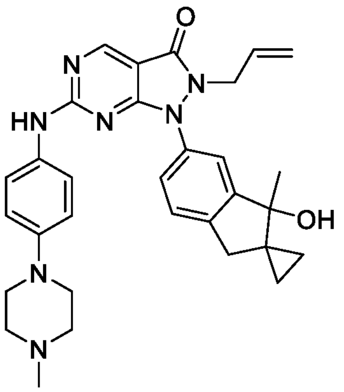 | WEE1-056 | 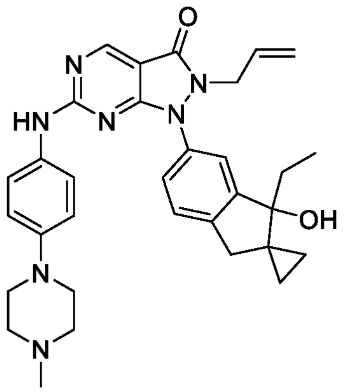 |
| WEE1-057 | 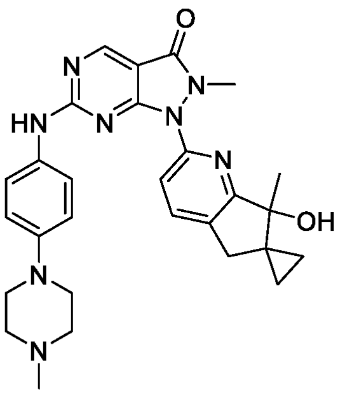 | WEE1-058 | 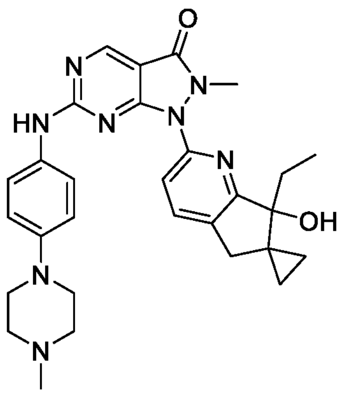 |
| WEE1-059 | 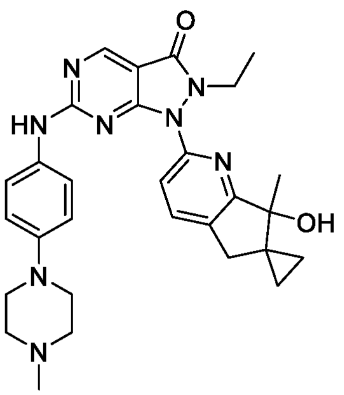 | WEE1-060 | 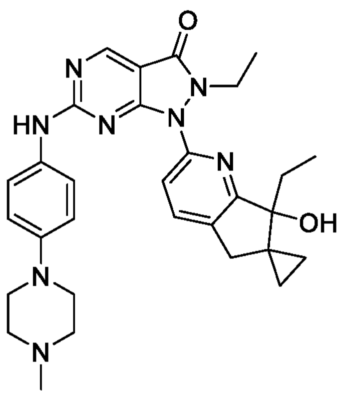 |
| WEE1-061 | 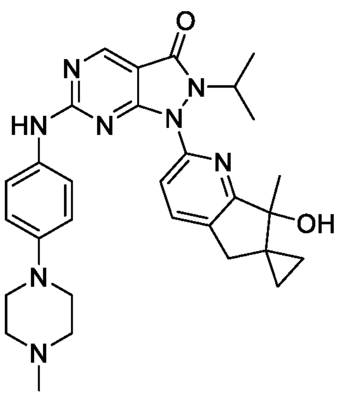 | WEE1-062 | 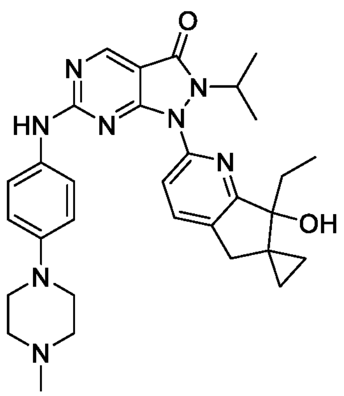 |

(continued)

| No. | Compound Structure | No. | Compound Structure |
|---|---|---|---|
| WEE1-063 | 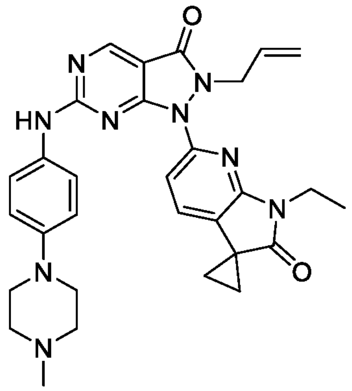 | WEE1-064 | 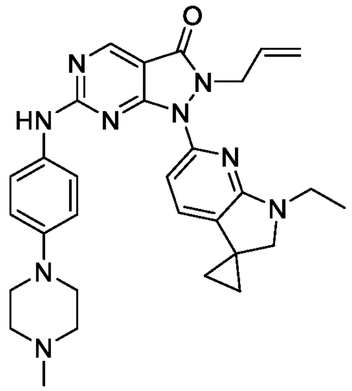 |
| WEE1-065 | 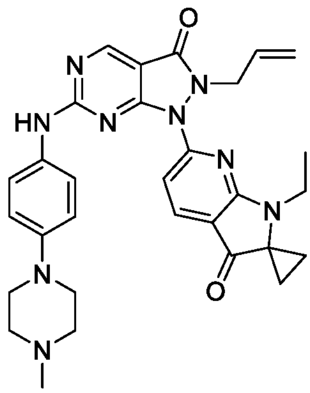 | WEE1-066 | 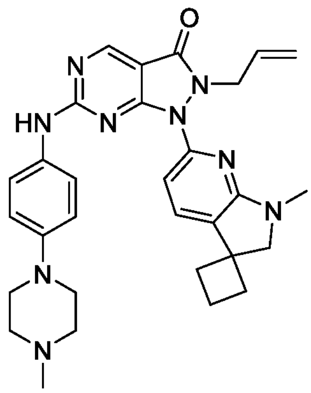 |
| WEE1-067 | 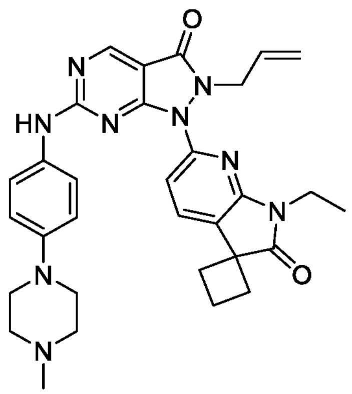 | WEE1-068 | 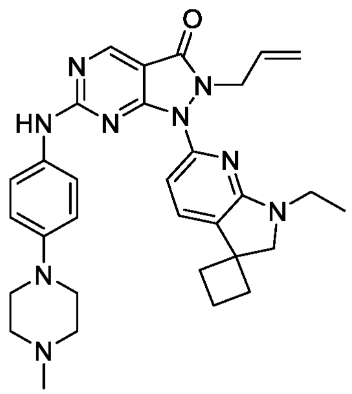 |
| WEE1-069 | 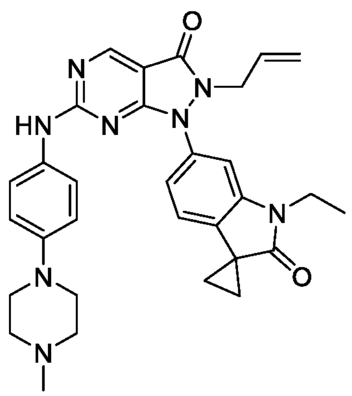 | WEE1-070 | 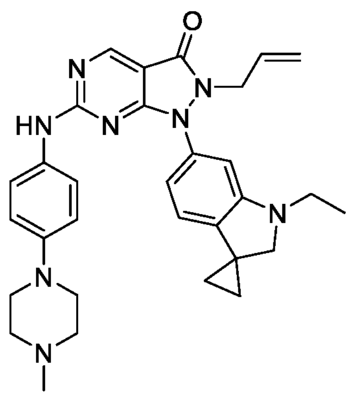 |

(continued)

| No. | Compound Structure | No. | Compound Structure |
|---|---|---|---|
| WEE1-071 | 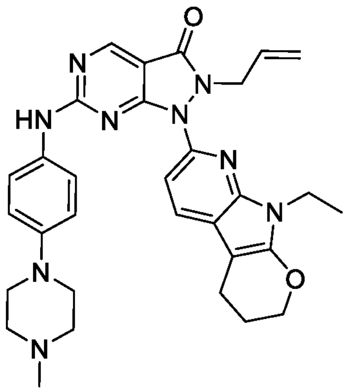 | WEE1-072 | 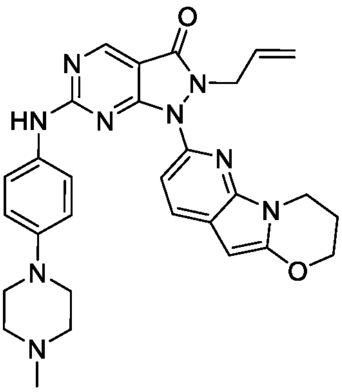 |
| WEE1-073 | 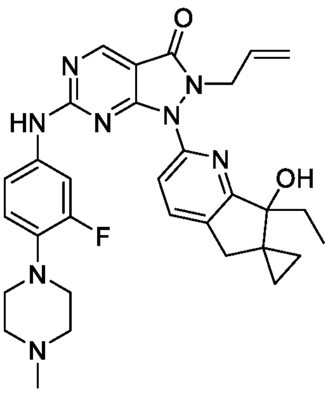 | WEE1-074 | 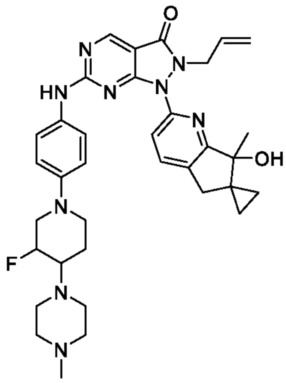 |
| WEE1-075 | 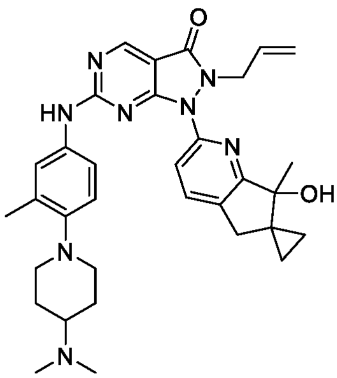 | WEE1-076 | 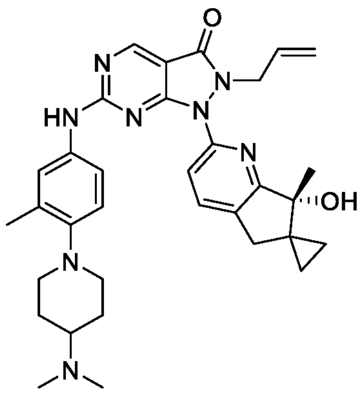 |
| WEE1-077 | 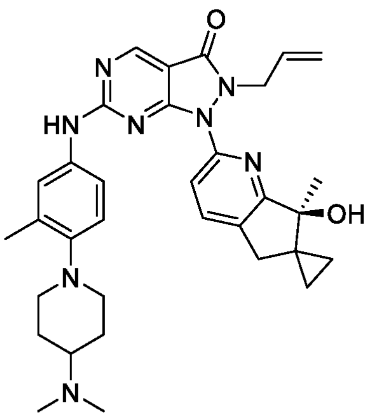 | WEE1-078 | 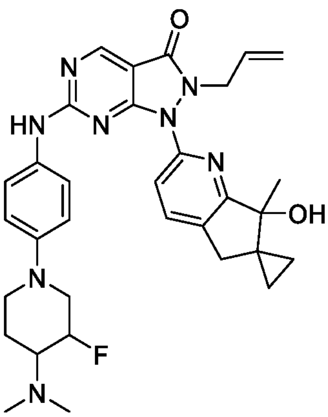 |

(continued)

| No. | Compound Structure | No. | Compound Structure |
|---|---|---|---|
| WEE1-079 | 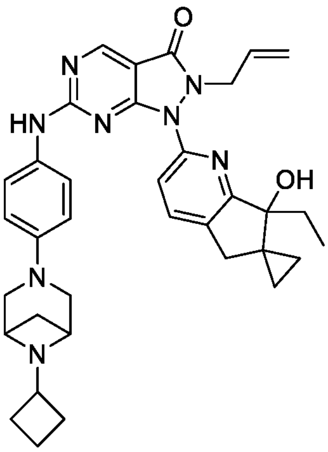 | WEE1-080 | 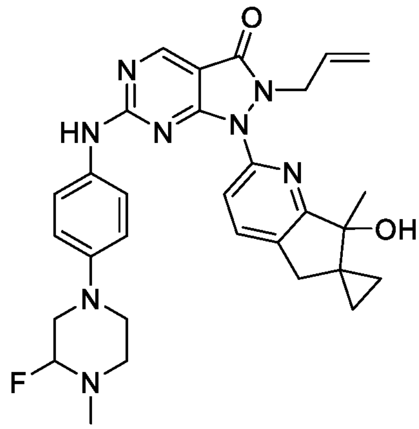 |
| WEE1-081 | 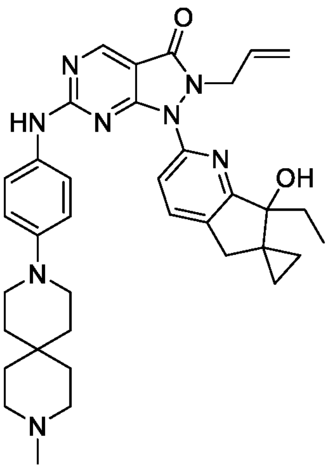 | WEE1-082 | 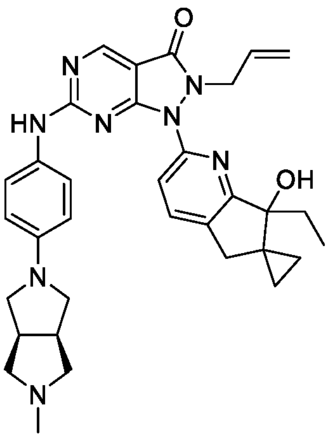 |
| WEE1-083 | 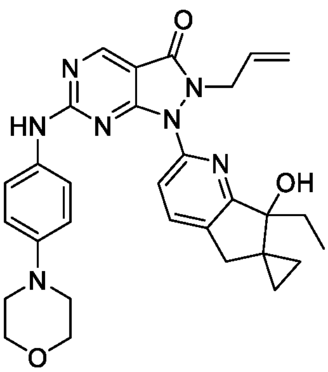 | WEE1-084 | 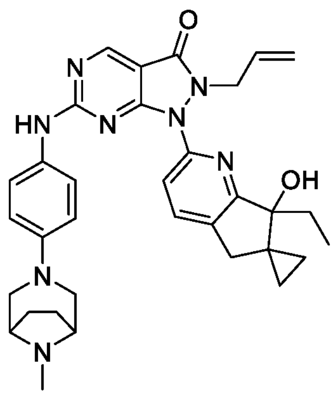 |

(continued)

| No. | Compound Structure | No. | Compound Structure |
|---|---|---|---|
| WEE1-085 | 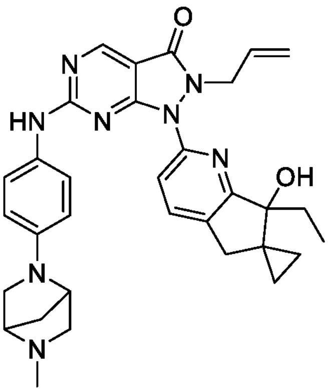 | WEE1-086 | 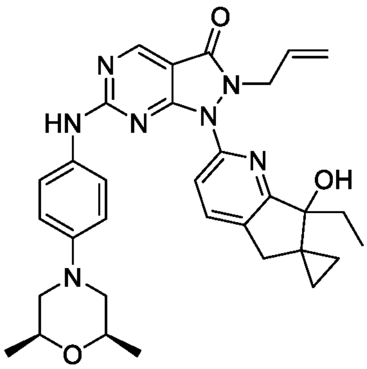 |
| WEE1-087 | 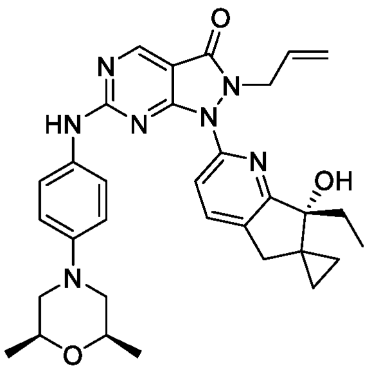 | WEE1-088 | 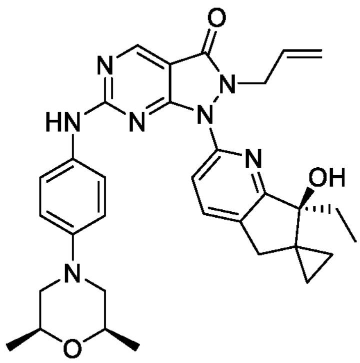 |
| WEE1-089 | 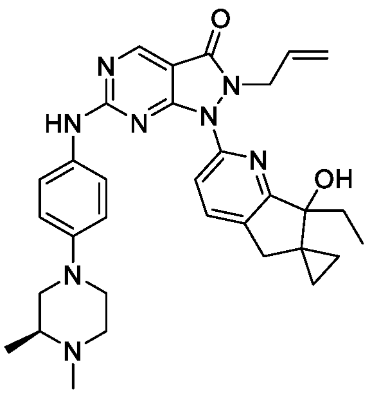 | WEE1-090 | 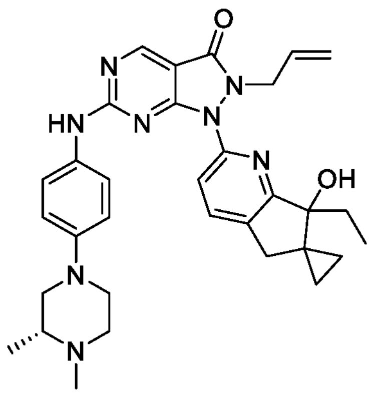 |
| WEE1-091 | 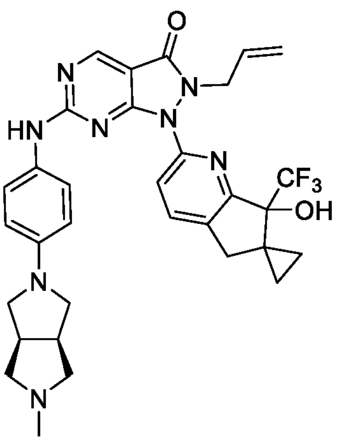 | WEE-092 | 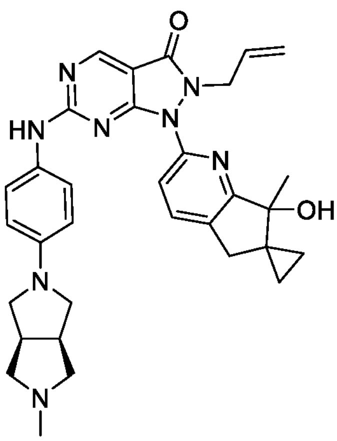 |

(continued)

| No. | Compound Structure | No. | Compound Structure |
|---|---|---|---|
| WEE1-093 | 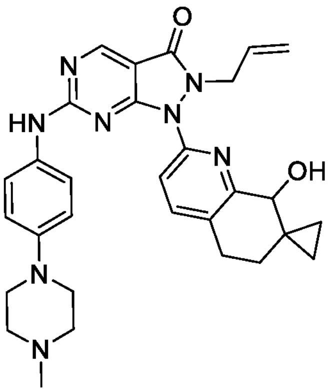 | WEE1-094 | 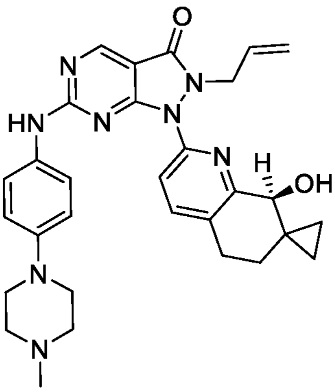 |
| WEE1-095 | 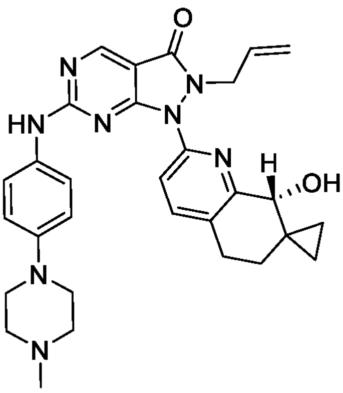 | WEE1-096 | 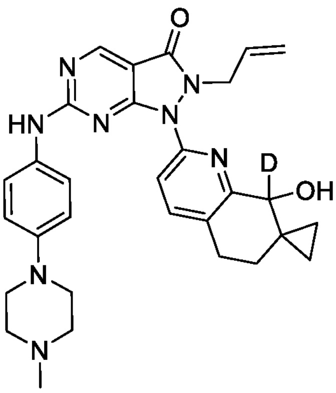 |
| WEE1-097 | 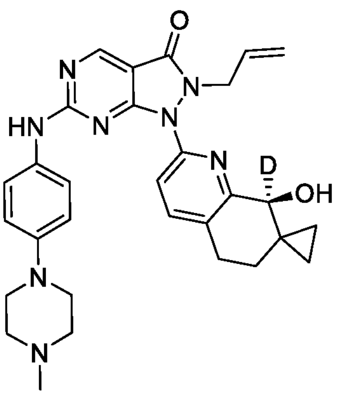 | WEE1-098 | 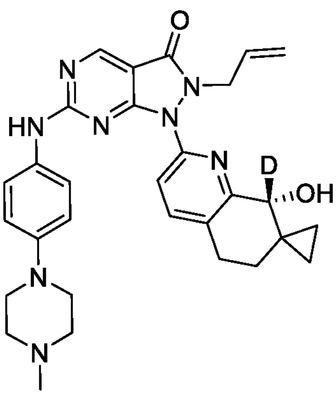 |
| WEE1-099 | 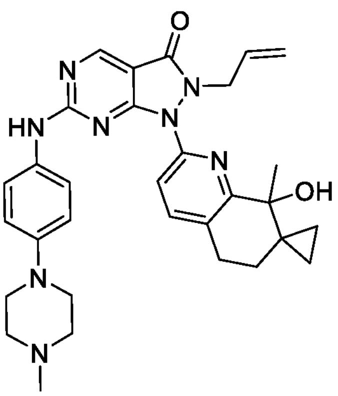 | WEE1-100 | 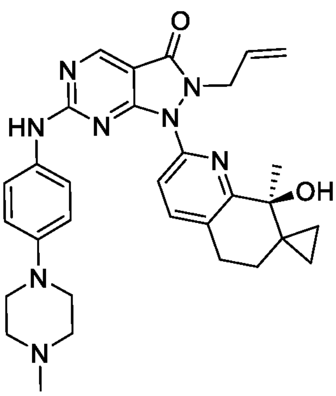 |

(continued)

| No. | Compound Structure | No. | Compound Structure |
|---|---|---|---|
| WEE1-101 | 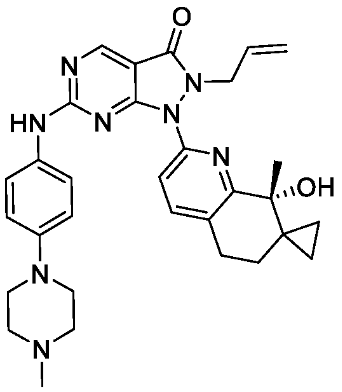 | WEE1-102 | 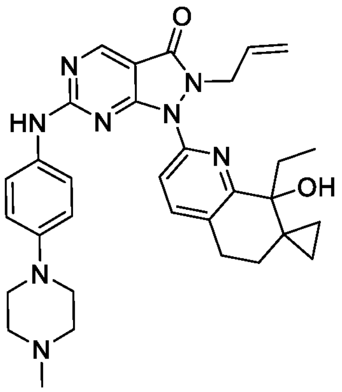 |
| WEE1-103 | 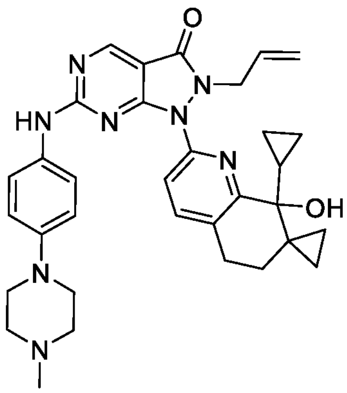 | WEE1-104 | 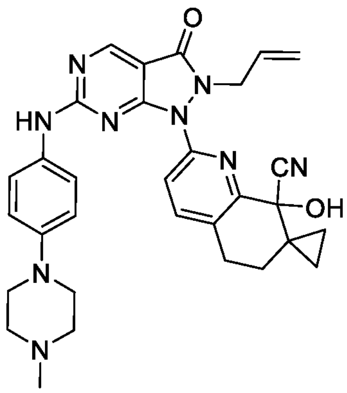 |
| WEE1-105 | 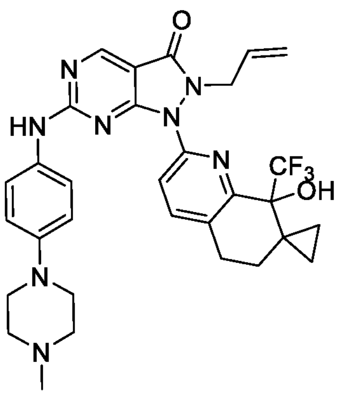 | WEE1-106 | 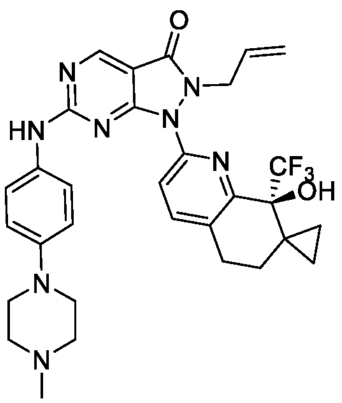 |
| WEE1-107 | 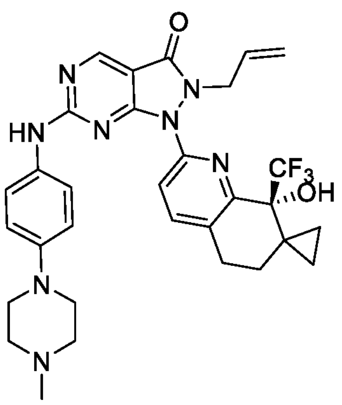 | WEE1-108 | 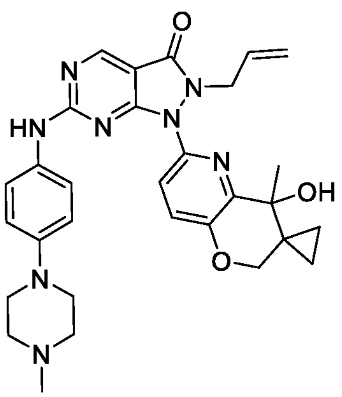 |

(continued)

| No. | Compound Structure | No. | Compound Structure |
|---|---|---|---|
| WEE1-109 | 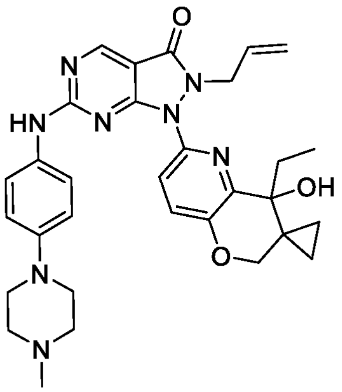 | WEE1-110 | 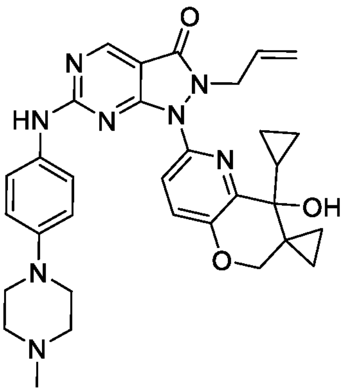 |
| WEE1-111 | 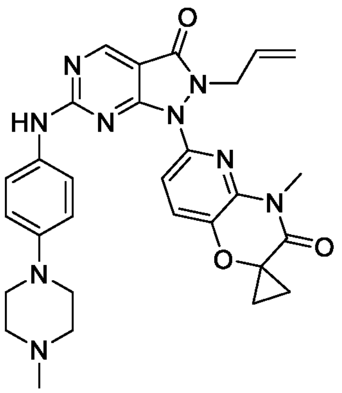 | WEE1-112 | 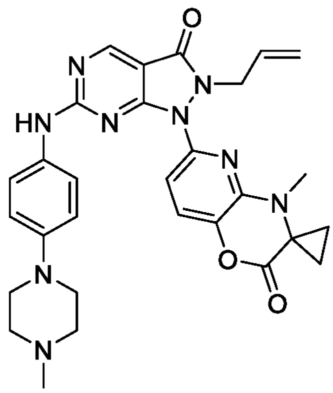 |
| WEE1-113 | 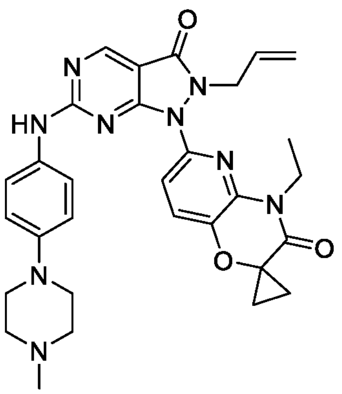 | WEE1-114 | 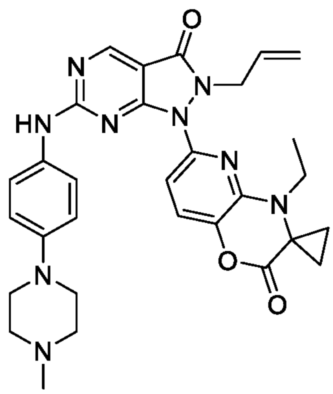 |
| WEE1-115 | 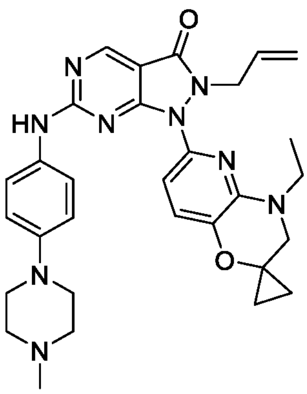 | WEE1-116 | 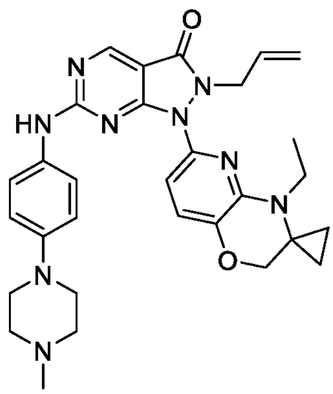 |

(continued)

| No. | Compound Structure | No. | Compound Structure |
|---|---|---|---|
| WEE1-117 | 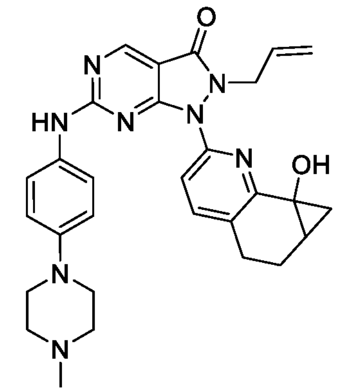 | WEE1-118 | 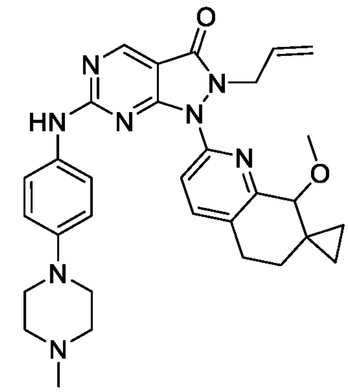 |
| WEE1-119 | 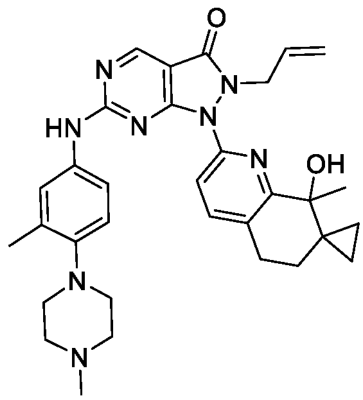 | WEE1-120 | 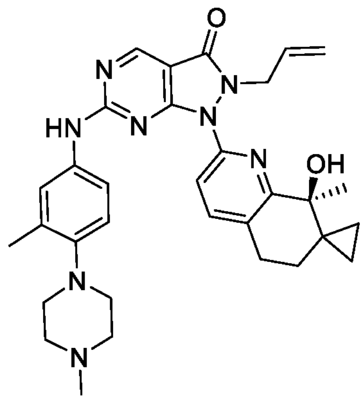 |
| WEE1-121 | 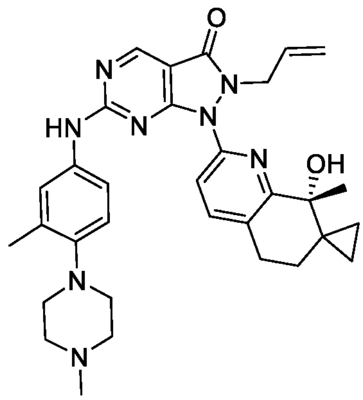 | WEE1-122 | 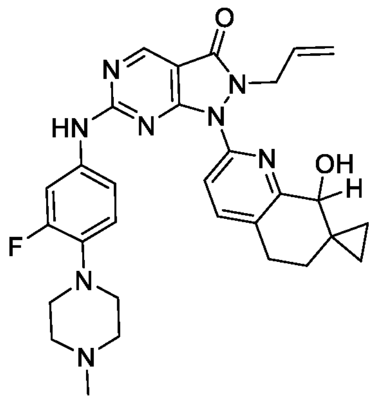 |
| WEE1-123 | 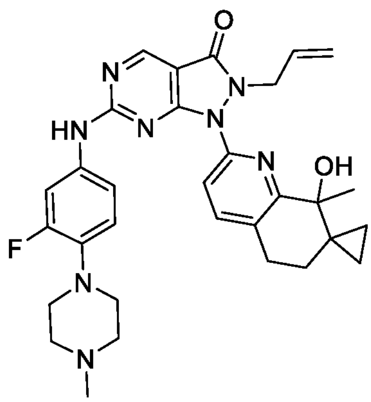 | WEE1-124 | 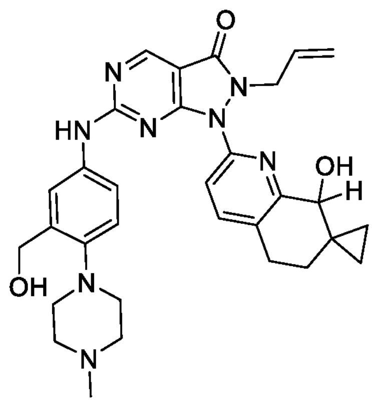 |

(continued)

| No. | Compound Structure | No. | Compound Structure |
|---|---|---|---|
| WEE1-125 | 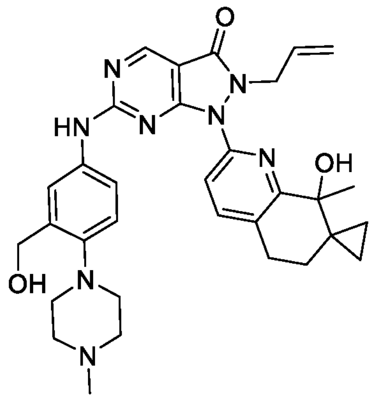 | WEE1-126 | 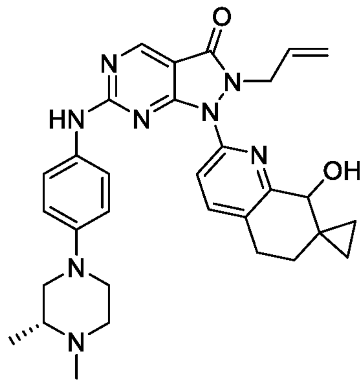 |
| WEE1-127 | 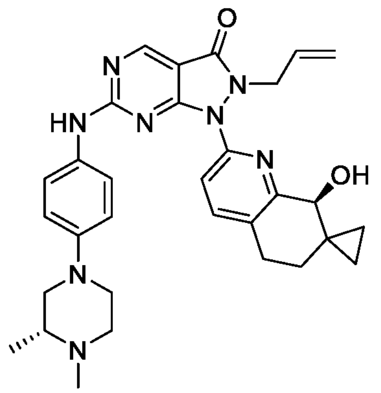 | WEE1-128 | 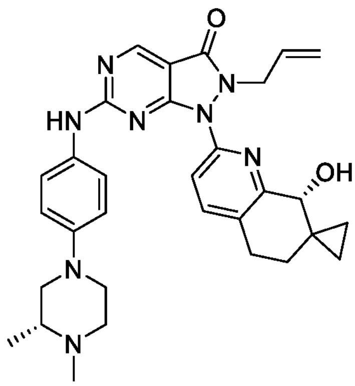 |
| WEE1-129 | 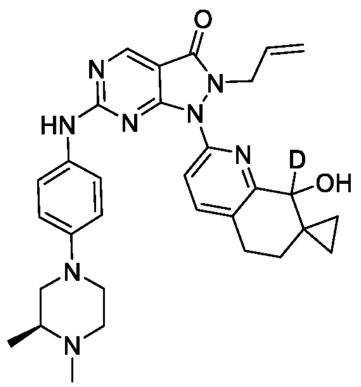 | WEE1-130 | 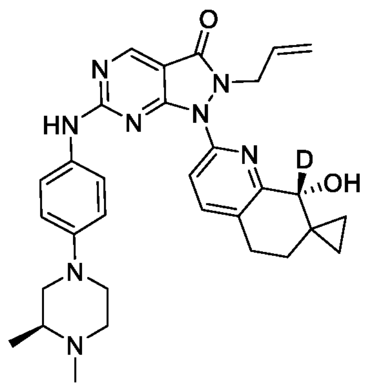 |
| WEE1-131 | 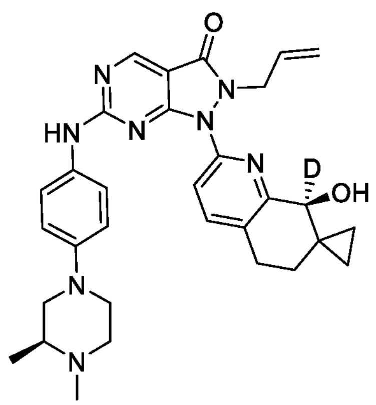 | WEE1-132 | 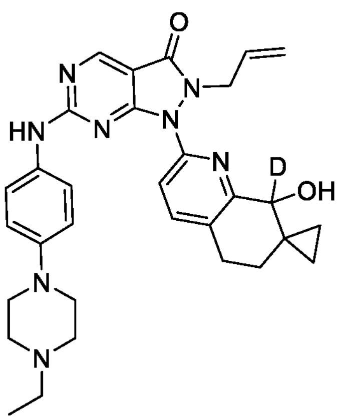 |

(continued)

| No. | Compound Structure | No. | Compound Structure |
|---|---|---|---|
| WEE1-133 | 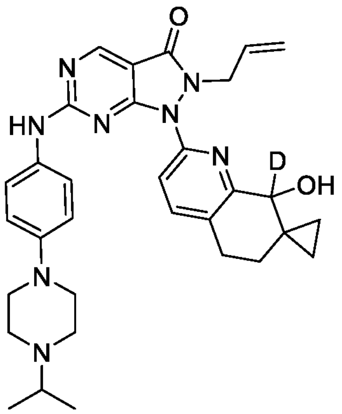 | WEE1-134 | 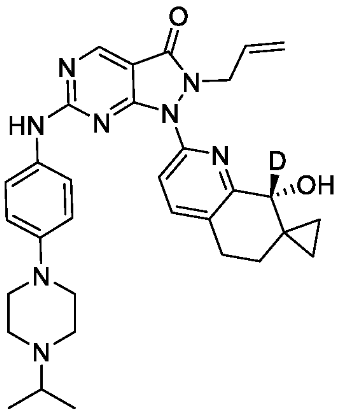 |
| WEE1-135 | 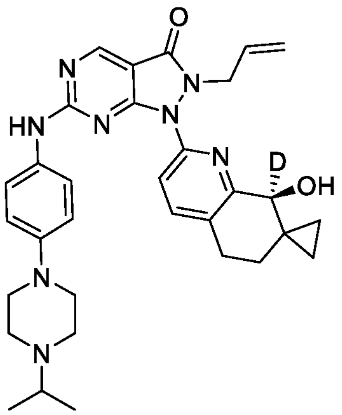 | WEE1-136 | 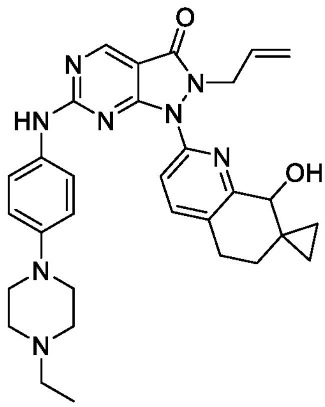 |
| WEE1-137 | 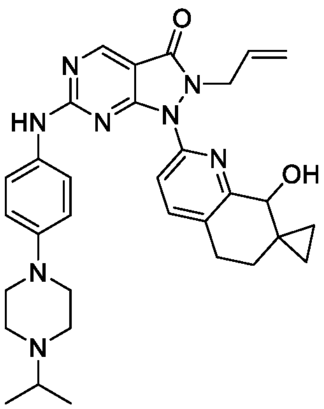 | WEE1-138 | 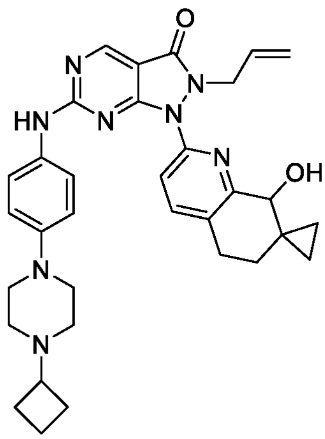 |

(continued)

| No. | Compound Structure | No. | Compound Structure |
|---|---|---|---|
| WEE1-139 | 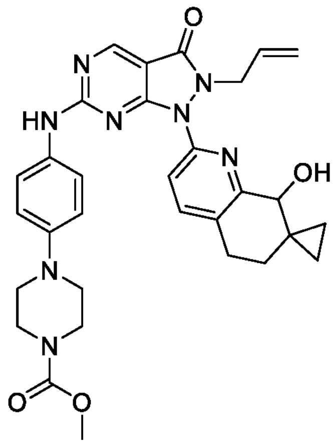 | WEE1-140 | 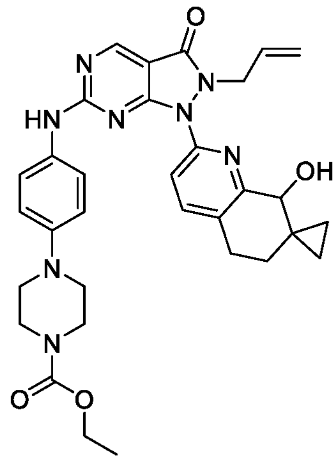 |
| WEE1-141 | 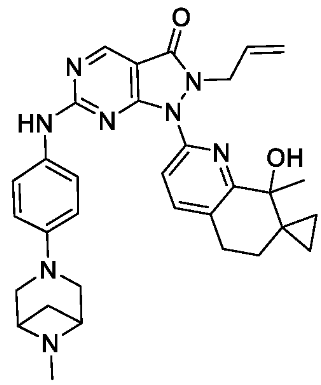 | WEE1-142 | 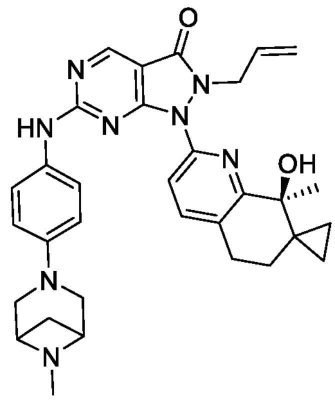 |
| WEE1-143 | 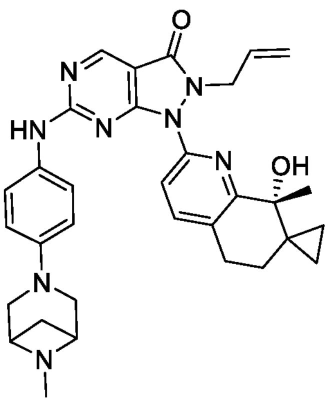 | WEE1-144 | 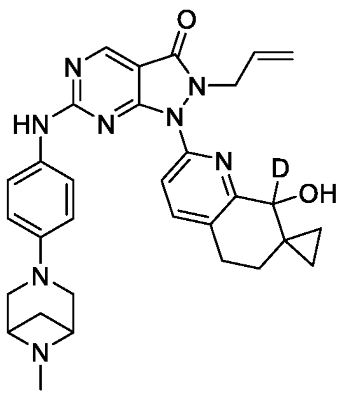 |
| WEE1-145 | 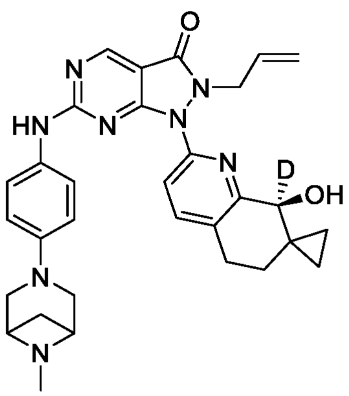 | WEE1-146 | 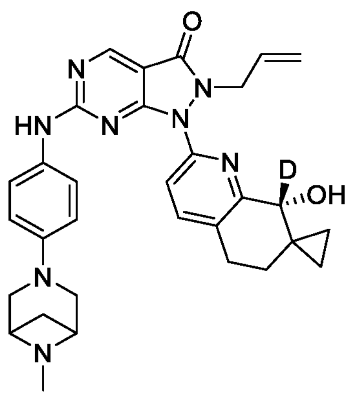 |

(continued)

| No. | Compound Structure | No. | Compound Structure |
|---|---|---|---|
| WEE1-147 | 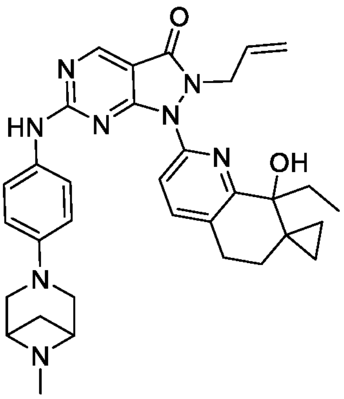 | WEE1-148 | 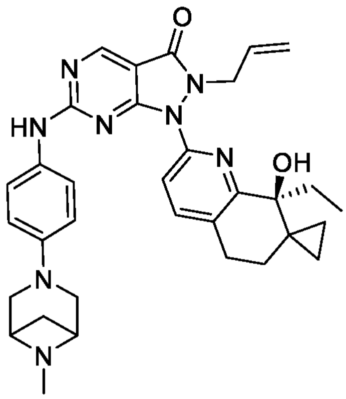 |
| WEE1-149 | 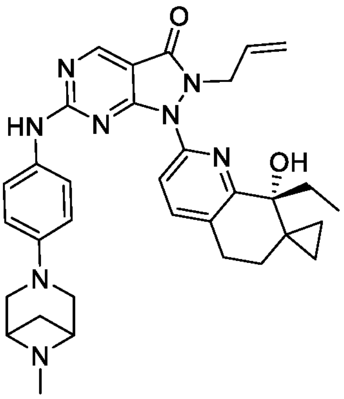 | WEE1-150 | 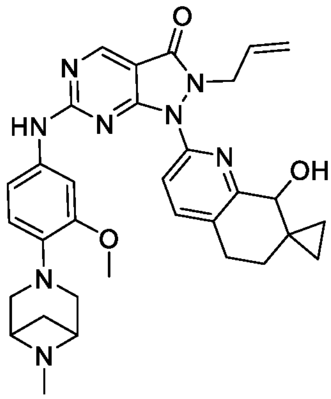 |
| WEE1-151 | 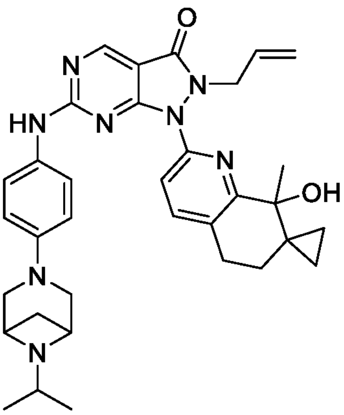 | WEE1-152 | 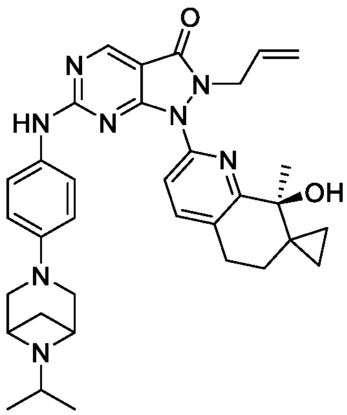 |
| WEE1-153 | 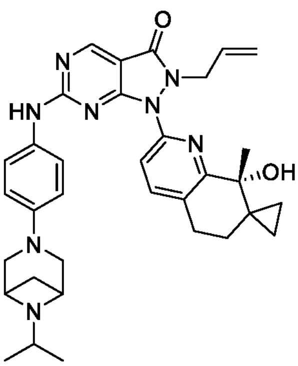 | WEE1-154 | 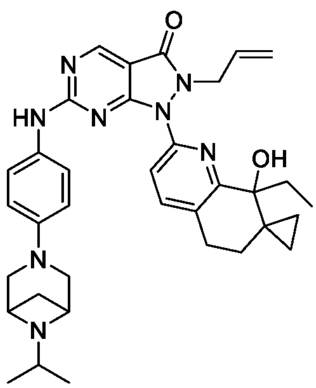 |

(continued)

| No. | Compound Structure | No. | Compound Structure |
|---|---|---|---|
| WEE1-155 | 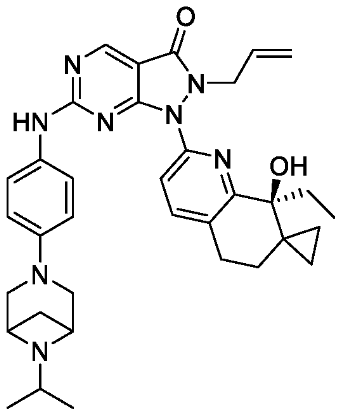 | WEE1-156 | 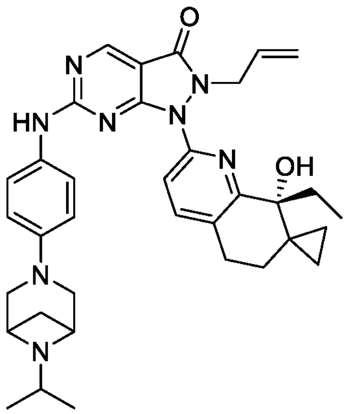 |
| WEE1-157 | 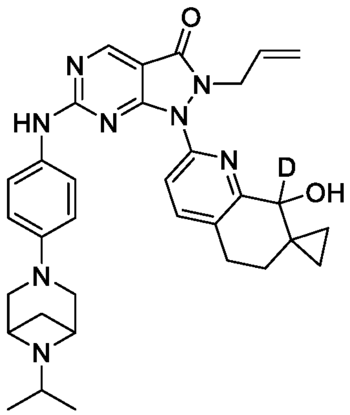 | WEE1-158 | 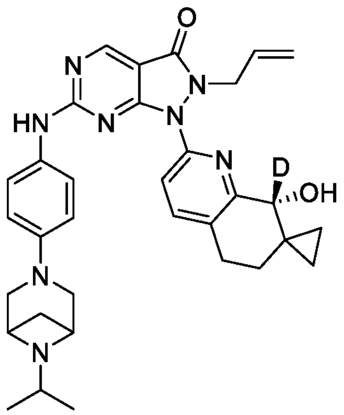 |
| WEE1-159 | 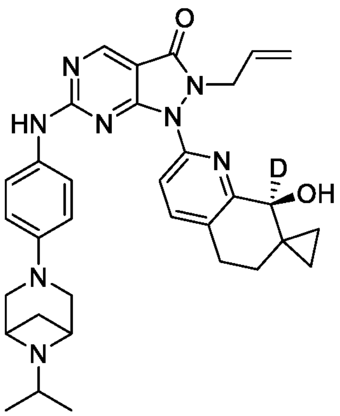 | WEE1-160 | 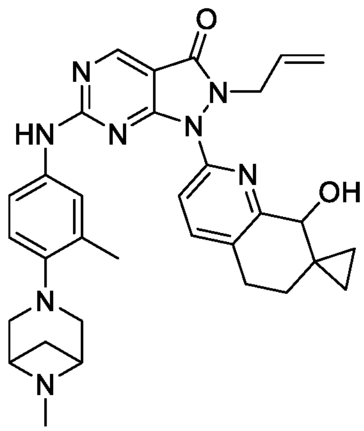 |

(continued)

| No. | Compound Structure | No. | Compound Structure |
|---|---|---|---|
| WEE1-161 | 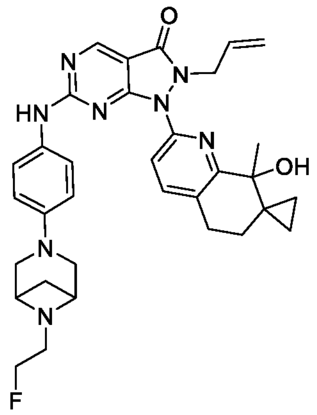 | WEE1-162 | 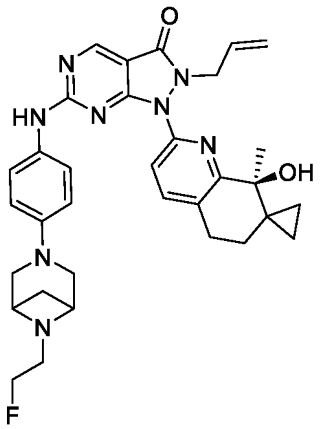 |
| WEE1-163 | 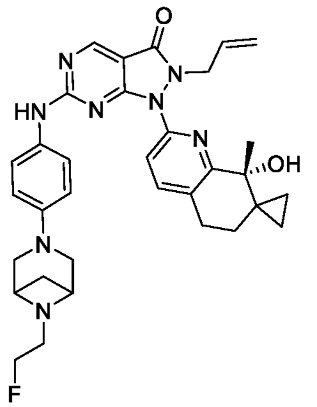 | WEE1-164 | 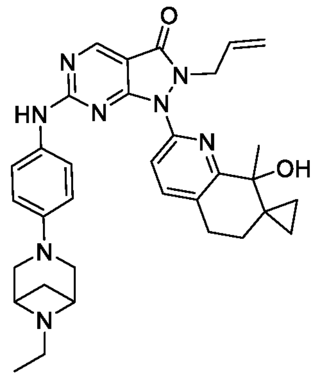 |
| WEE1-165 | 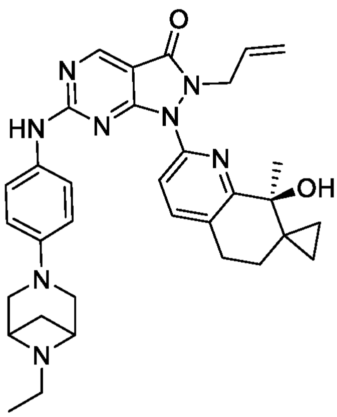 | WEE1-166 | 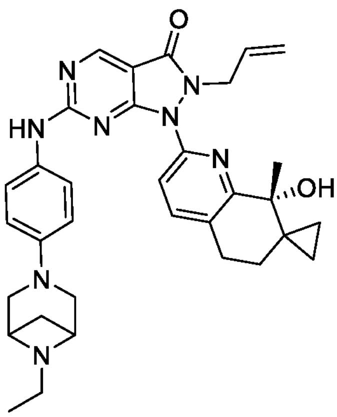 |

(continued)

| No. | Compound Structure | No. | Compound Structure |
|---|---|---|---|
| WEE1-167 | 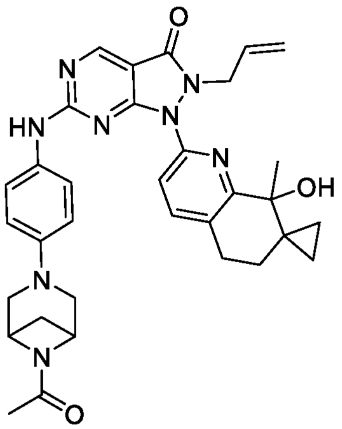 | WEE1-168 | 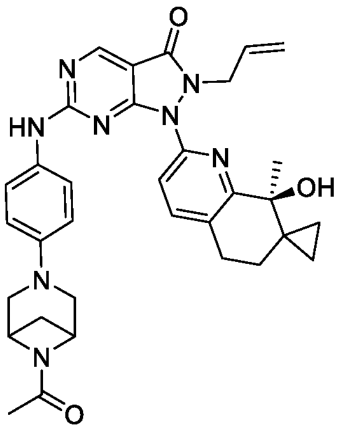 |
| WEE1-169 | 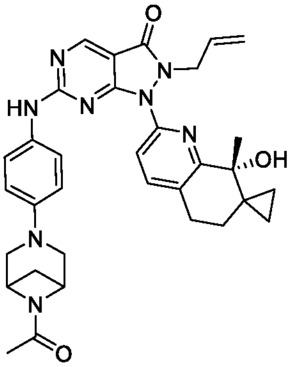 | WEE1-170 | 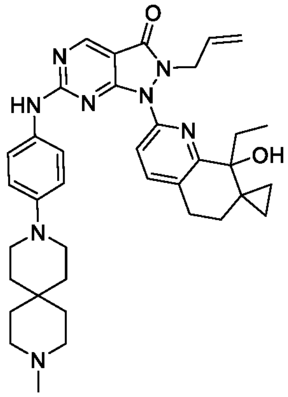 |
| WEE1-171 | 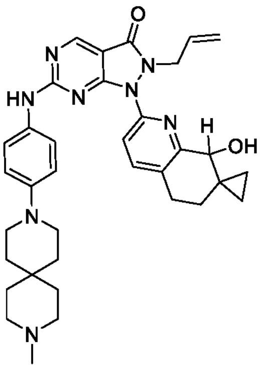 | WEE1-172 | 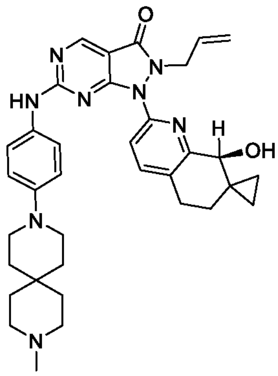 |
| WEE1-173 | 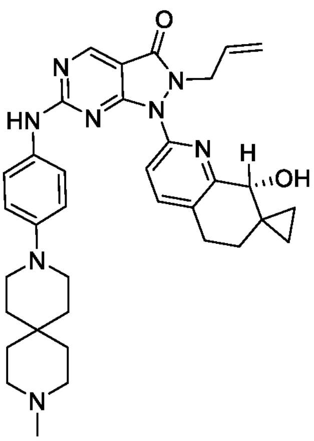 | WEE1-174 | 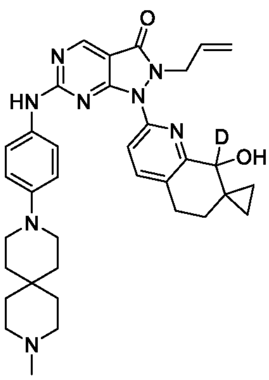 |

(continued)

| No. | Compound Structure | No. | Compound Structure |
|---|---|---|---|
| WEE1-175 | 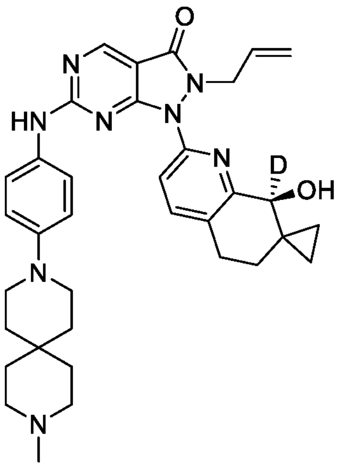 | WEE1-176 | 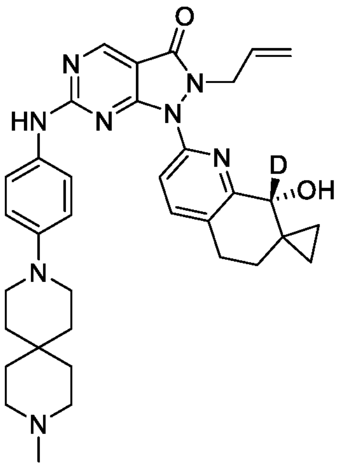 |
| WEE1-177 | 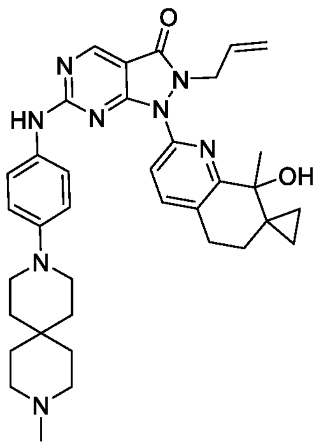 | WEE1-178 | 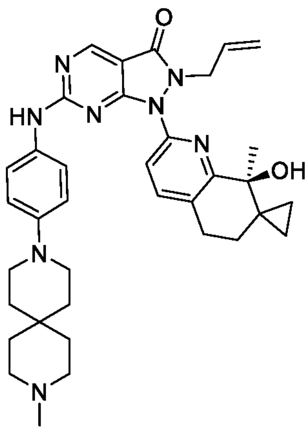 |
| WEE1-179 | 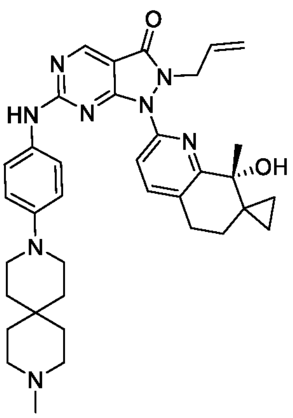 | WEE1-180 | 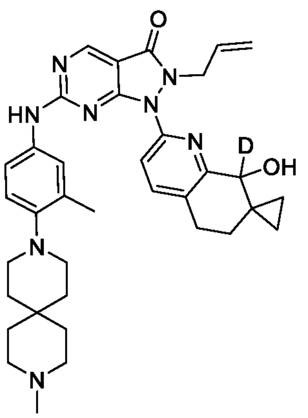 |

(continued)

| No. | Compound Structure | No. | Compound Structure |
|---|---|---|---|
| WEE1-181 | 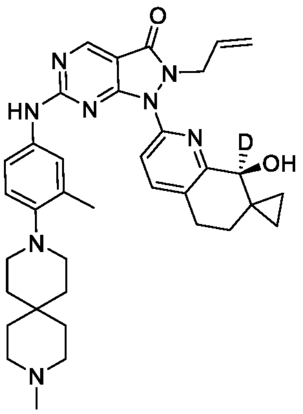 | WEE1-182 | 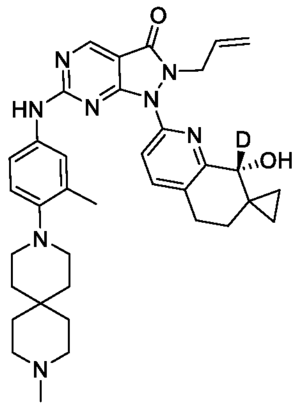 |
| WEE1-183 | 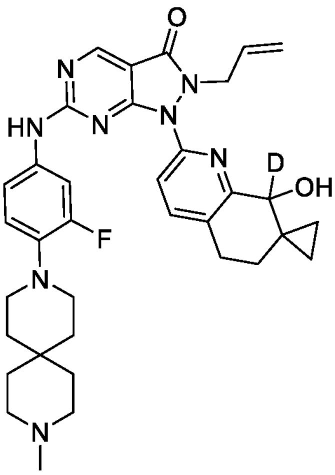 | WEE1-184 | 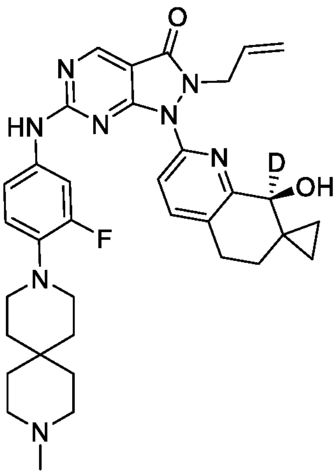 |
| WEE1-185 | 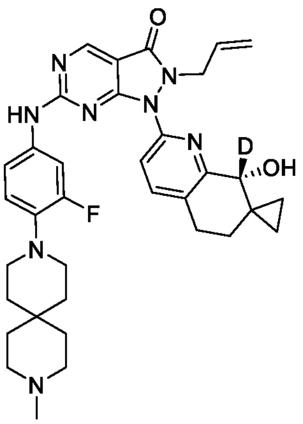 | WEE1-186 | 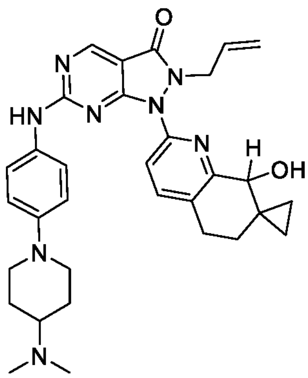 |

(continued)

| No. | Compound Structure | No. | Compound Structure |
|---|---|---|---|
| WEE1-187 | 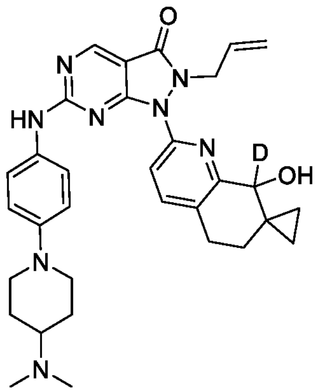 | WEE1-188 | 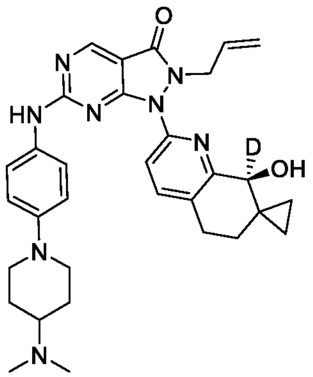 |
| WEE1-189 | 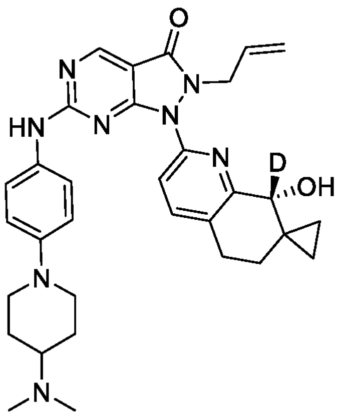 | WEE1-190 | 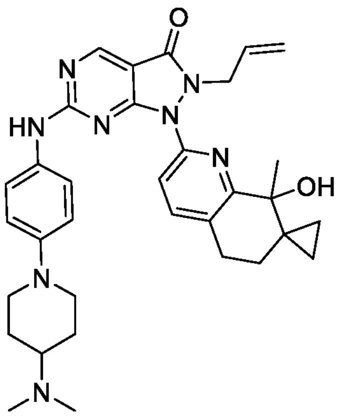 |
| WEE1-191 | 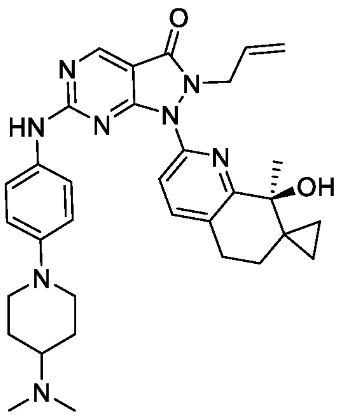 | WEE1-192 | 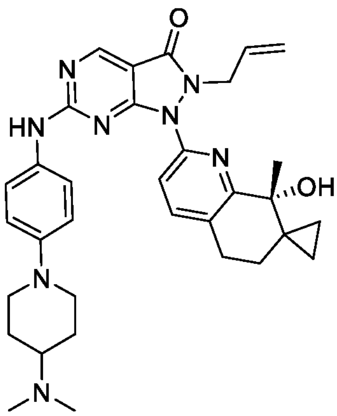 |
| WEE1-193 | 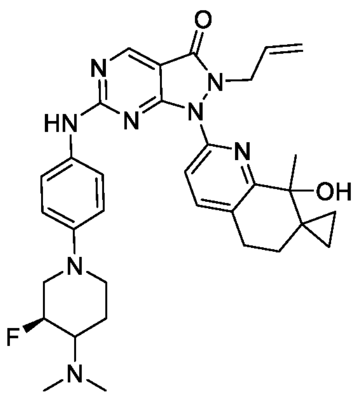 | WEE1-194 | 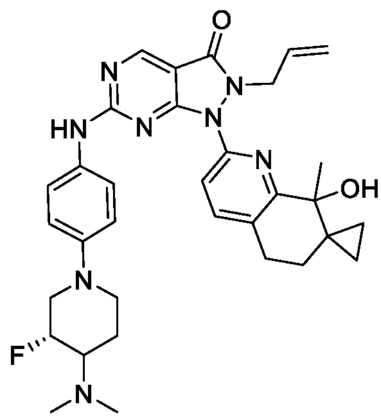 |

(continued)

| No. | Compound Structure | No. | Compound Structure |
|---|---|---|---|
| WEE1-195 | 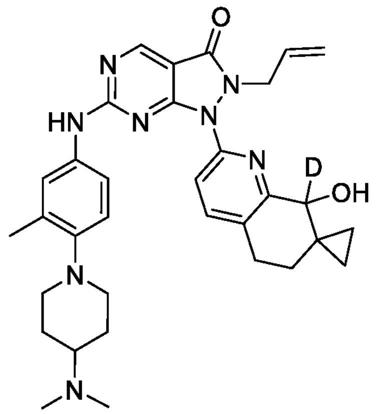 | WEE1-196 | 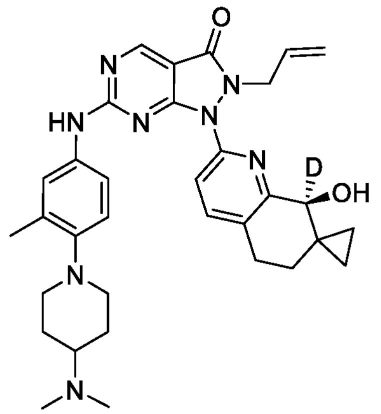 |
| WEE1-197 | 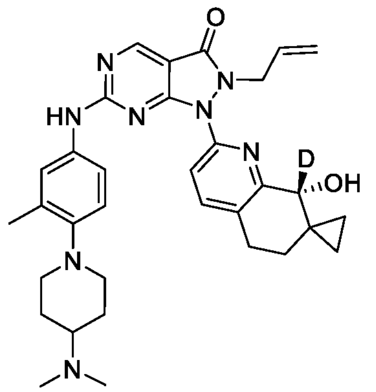 | WEE1-198 | 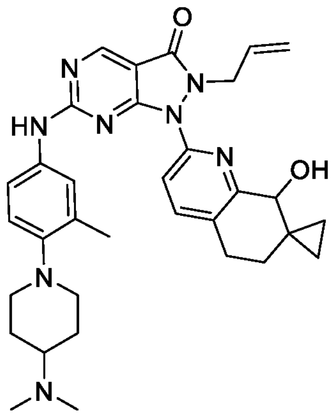 |
| WEE1-199 | 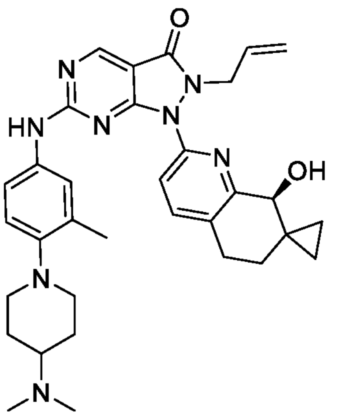 | WEE1-200 | 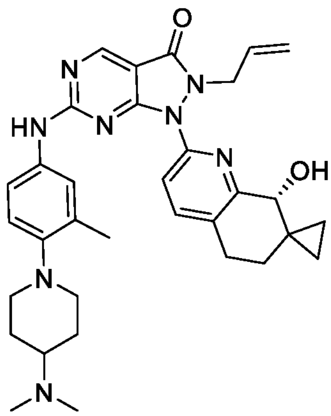 |
| WEE1-201 | 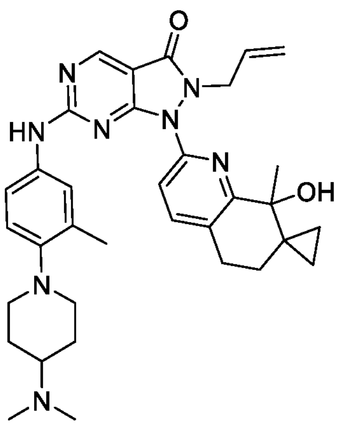 | WEE1-202 | 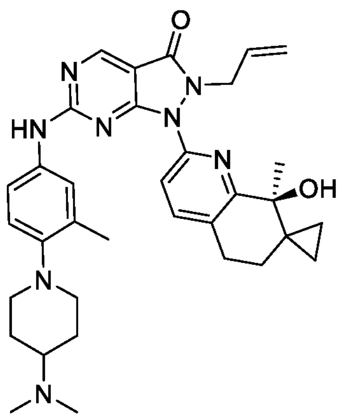 |

(continued)

| No. | Compound Structure | No. | Compound Structure |
|---|---|---|---|
| WEE1-203 | 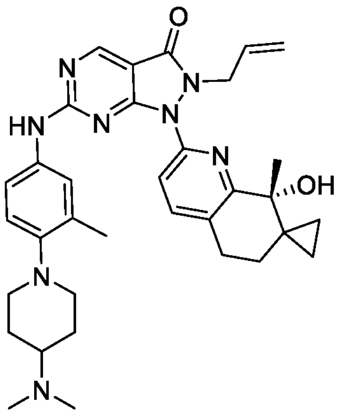 | WEE1-204 | 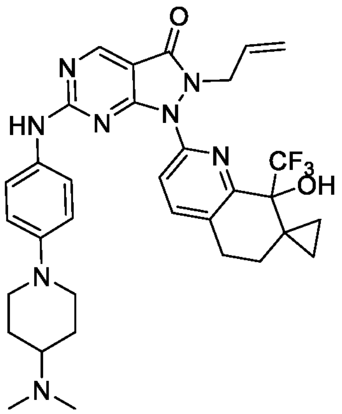 |
| WEE1-205 | 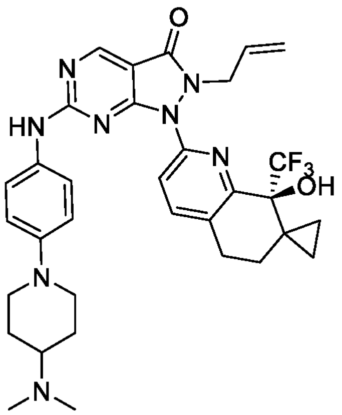 | WEE1-206 | 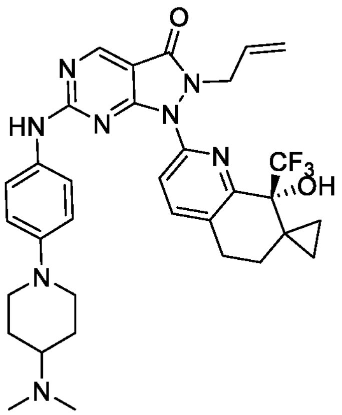 |
| WEE1-207 | 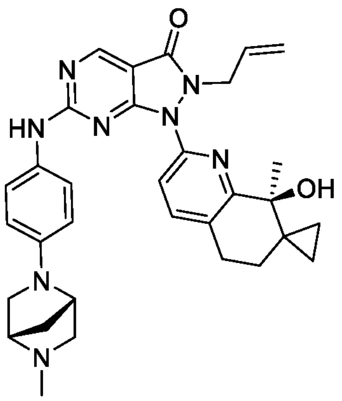 | WEE1-208 | 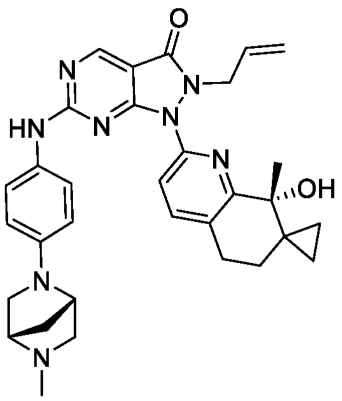 |
| WEE1-209 | 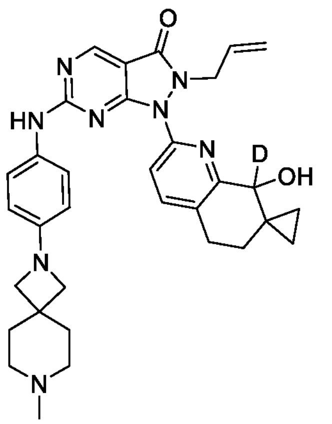 | WEE1-210 | 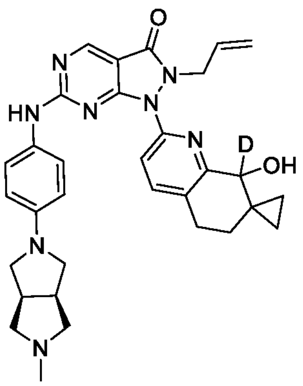 |

(continued)

| No. | Compound Structure | No. | Compound Structure |
|---|---|---|---|
| WEE1-211 | 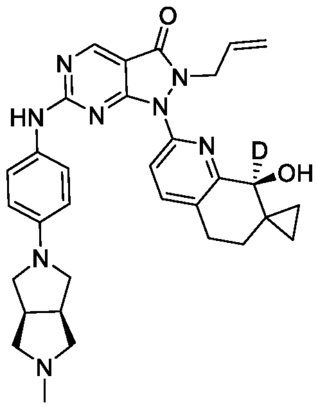 | WEE1-212 | 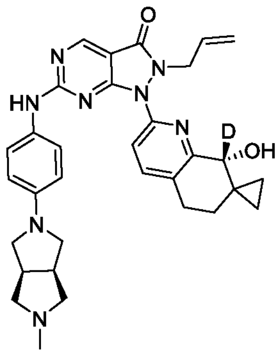 |
| WEE1-213 | 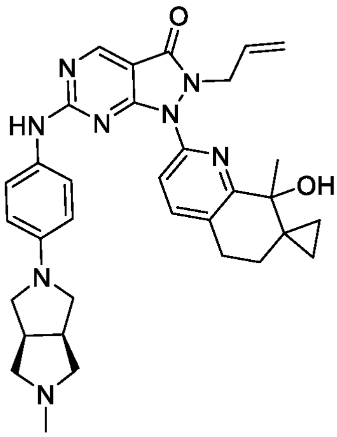 | WEE1-214 | 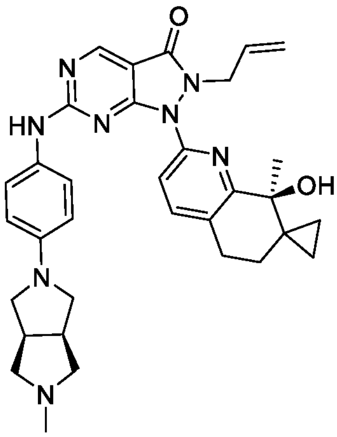 |
| WEE1-215 | 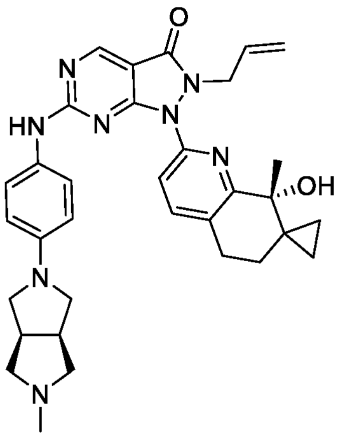 | WEE1-216 | 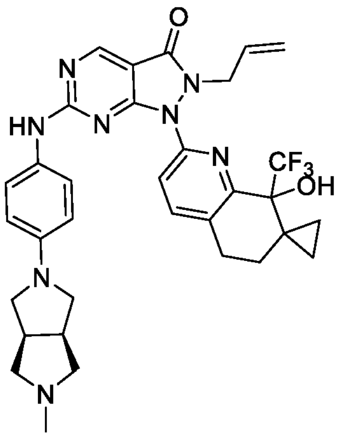 |

(continued)

| No. | Compound Structure | No. | Compound Structure |
|---|---|---|---|
| WEE1-217 | 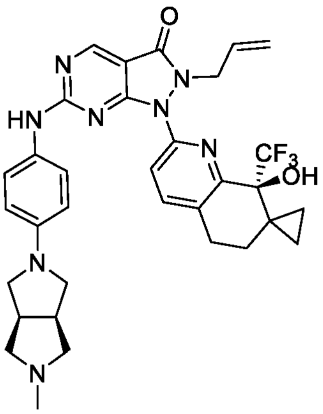 | WEE1-218 | 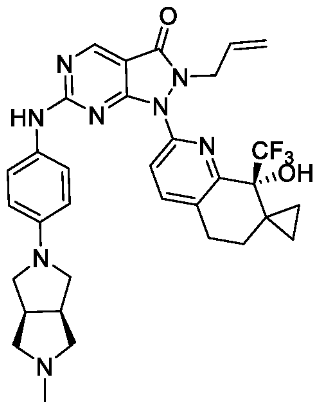 |
| WEE1-219 | 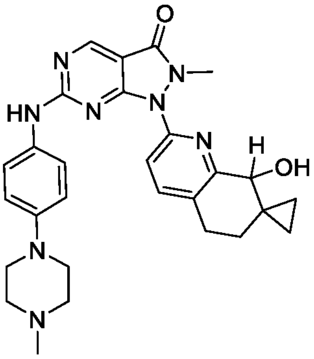 | WEE1-220 | 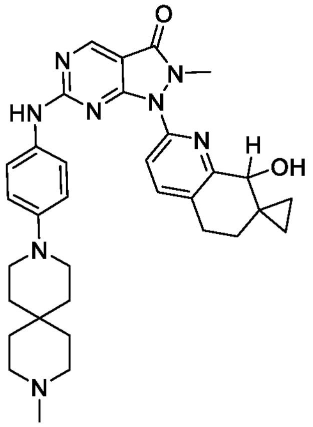 |
| WEE1-221 | 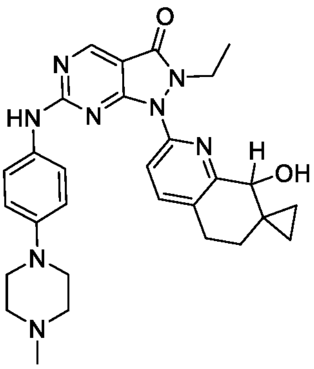 | WEE1-222 | 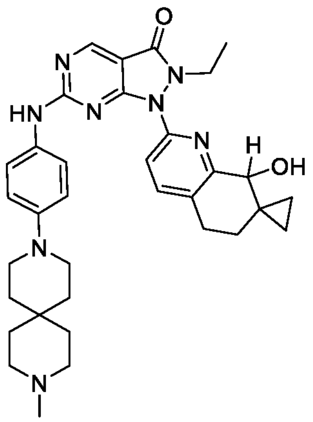 |

(continued)

| No. | Compound Structure | No. | Compound Structure |
|---|---|---|---|
| WEE1-223 | 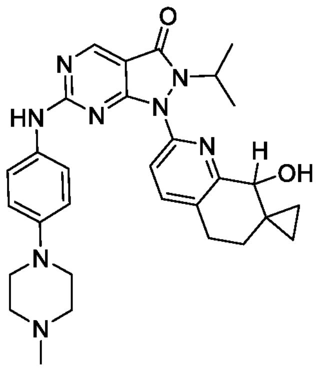 | WEE1-224 | 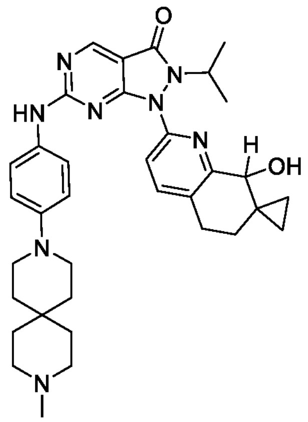 |
| WEE1-225 | 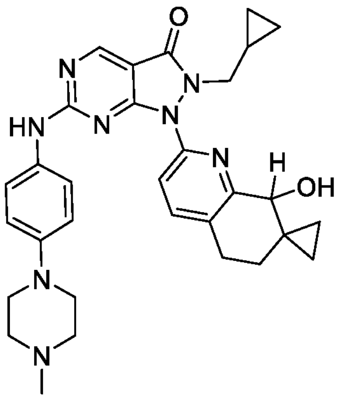 | WEE1-226 | 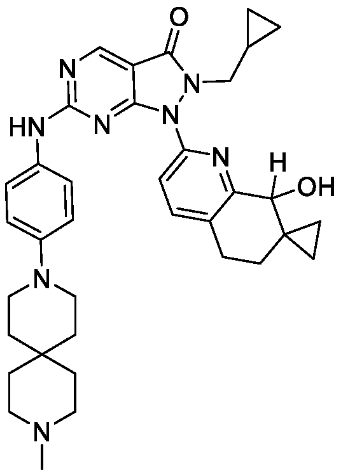 |
| WEE1-227 | 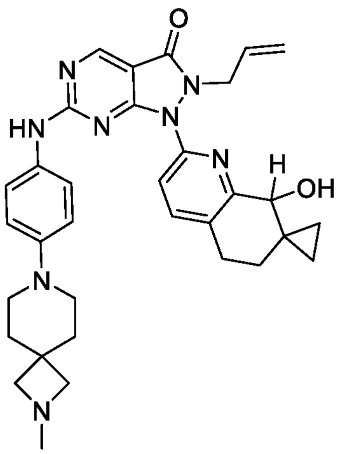 | WEE1-228 | 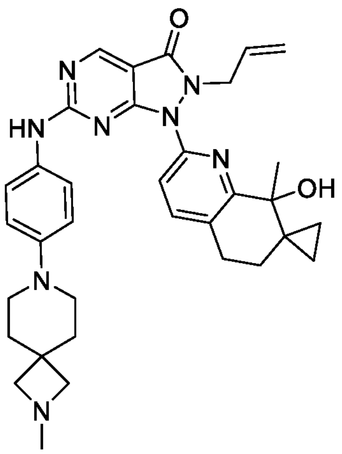 |

(continued)

| No. | Compound Structure | No. | Compound Structure |
|---|---|---|---|
| WEE1-229 | 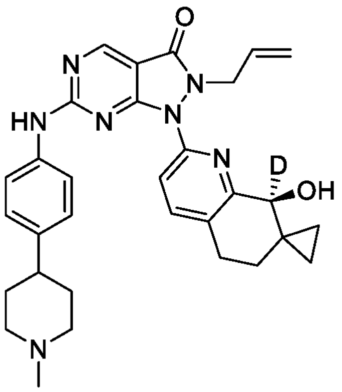 | WEE1-230 | 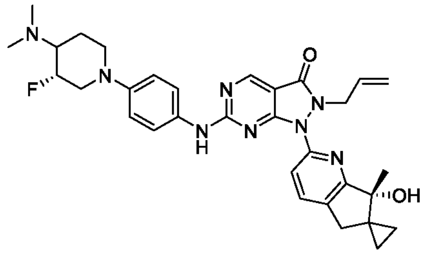 |
| WEE1-231 | 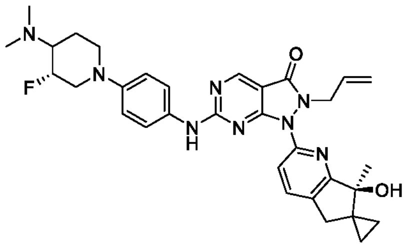 | WEE1-232 | 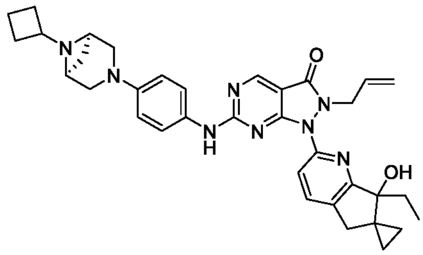 |
| WEE1-233 | 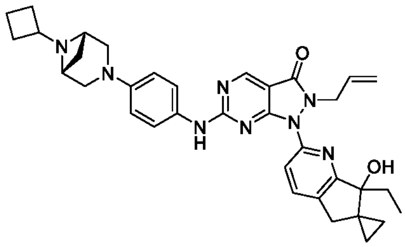 | WEE1-234 | 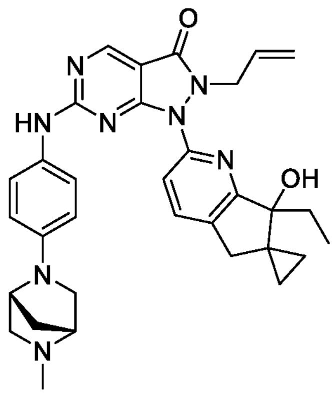 |
| WEE1-235 | 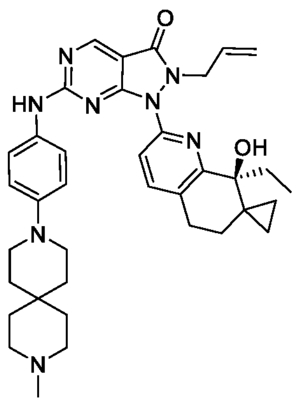 | WEE1-236 | 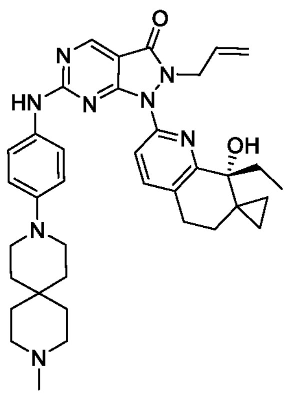 |

(continued)

| No. | Compound Structure | No. | Compound Structure |
|---|---|---|---|
| WEE1-237 | 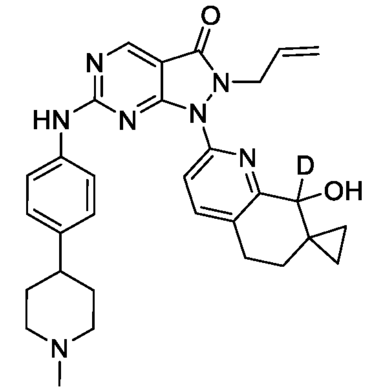 | | |

More preferably, in the compound of the invention, or a stereoisomer thereof, or a pharmaceutically acceptable salt thereof, the compound of Formula I is specifically:

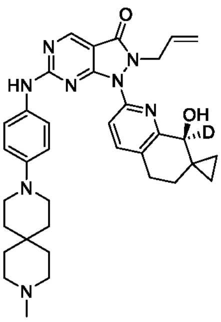

,

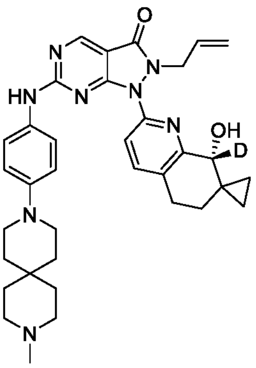

.

The present invention further provides the use of any of the abovementioned compounds, or a stereoisomer thereof, or a pharmaceutically acceptable salt thereof in the preparation of a medicament for treatment of WEE1-mediated disease.

The WEE1-mediated disease is one or more selected from diseases related to inflammation, autoimmune disease, infectious disease, cancer, precancer syndrome.

The present invention further provides a pharmaceutical composition which is prepared with the compound of any of the abovementioned compound or a stereoisomer thereof, or a pharmaceutically acceptable salt thereof, as the pharmaceutically active ingredient, together with pharmaceutically acceptable excipients.

The following is an illustration and explanation of the terminology of the present invention:

"Cancer" or "malignant neoplasm" means any of a number of diseases characterized by the uncontrolled abnormal cell proliferation which includes the spread of affected cells locally or through the bloodstream and lymphatic system to other parts of the body (i.e., metastasis) and any of many characteristic structural and/or molecular features. "Cancer cell" means a cell that undergoes multiple steps of tumor progression in the early, intermediate, or advanced phases. Cancers include sarcoma, breast cancer, lung cancer, brain cancer, cancer of bone, liver cancer, renal cancer, colon cancer and prostatic cancer. In some embodiments, compounds of Formula I are used to treat a cancer selected from colon cancer, brain cancer, breast cancer, fibrosarcoma, and squamous cell carcinoma. In some embodiments, the cancer is selected from melanoma, breast cancer, colon cancer, lung cancer, and ovarian cancer. In some embodiments, the cancer treated is a metastatic cancer.

Autoimmune diseases are caused by the body's immune response to substances and tissues normally present in the body. Examples of autoimmune diseases include myocarditis, lupus nephritis, primary biliary cirrhosis, psoriasis, type I diabetes mellitus, Grave's disease, celiac disease, Crohn's disease, autoimmune neutropenia, juvenile arthritis, rheumatoid arthritis, fibromyalgia, Guillain-Barre syndrome, multiple sclerosis and autoimmune retinopathy. Some embodiments of the present invention relate to the treatment of autoimmune diseases such as psoriasis or multiple sclerosis.

Inflammatory diseases include a wide range of conditions characterized by pathologic inflammation of tissues. Examples of inflammatory diseases include acne vulgaris, asthma, celiac disease, chronic prostatitis, glomerulonephritis,

inflammatory bowel disease, pelvic inflammatory disease, reperfusion injury, rheumatoid arthritis, sarcoidosis, vasculitis, airway inflammation due to house dust mites, and interstitial cystitis. There is significant overlap between inflammatory and autoimmune diseases. Some embodiments of the present invention relate to the treatment of the inflammatory disease asthma. The immune system is usually involved in inflammatory disease and is manifested in allergic reactions and some myopathies. Many immune system diseases result in abnormal inflammation. IL-17A-mediated diseases also include autoimmune inflammatory diseases.

Compounds and derivatives provided in the present invention can be named according to IUPAC (International Union of Pure and Applied Chemistry) or CAS (Chemical Abstracts Service, Columbus, OH) nomenclature system.

Definition of terms used in the present invention: Unless otherwise specified, the initial definition provided by the group or term herein is applicable to the group or term in the whole specification. For terms that are not specifically defined herein, they should be given meanings that can be given by those skilled in the art according to the disclosure and context.

"Substitution" means that the hydrogen atom in the molecule is replaced by other different atoms or molecules.

The minimum and maximum values of the carbon atom content in the hydrocarbon group are indicated by prefixes. For example, the prefix Cab alkyl indicates any alkyl group containing "a" to "b" carbon atoms. Therefore, for example, $C_{1\sim4}$ alkyl refers to alkyl groups containing 1 to 4 carbon atoms.

"Alkyl" refers to a saturated hydrocarbon chain with a specified number of member atoms. For example, $C_{1\sim6}$ alkyl refers to any alkyl group containing 1 to 6 member atoms, such as alkyl group containing 1 to 4 member atoms. Alkyl groups can be linear or branched. A representative branched alkyl group has one, two or three branches. Alkyl groups can be optionally substituted by one or more substituents as defined herein. Alkyl includes methyl, ethyl, propyl (n-propyl and isopropyl), butyl (n-butyl, isobutyl and tert-butyl), pentyl (n-pentyl, isopentyl and neopentyl) and hexyl. Alkyl group can also be a part of other groups, wherein said other group is for example $C_1\sim C_6$ alkoxyl.

"Cycloalkyl", "cycloalkane" means a saturated or partially saturated cyclic group with carbon atoms and no cyclic heteroatoms, and with a single ring or multiple rings (including fused, combined,bridle ring). For polycyclic systems having aromatic and non-aromatic cyclyls without ring heteroatoms, the term "cycloalkyl" applies when the connection point is at a non-aromatic carbon atom(for example, 5,6,7,8,- tetrahydronaphthalen-5-y1). The term "cycloalkyl" includes cycloalkenyl groups such as cyclohexenyl. Examples of cycloalkyl groups include, for example, adamantyl, cyclopropyl, cyclobutyl, cyclohexyl, cyclopentyl, cyclooctyl, cyclopentenyl, and cyclohexenyl. Examples of cycloalkyl groups including polybicycloalkyl ring systems are -bicyclohexyl, bicyclopentyl, bicyclooctyl, etc., such as

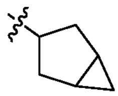, 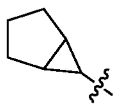, 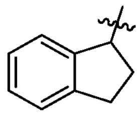.

"Alkenyl" means a straight or branched chain hydrocarbon groups having from 2 to 10 carbon atoms and in some embodiments from 2 to 6 carbon atoms or from 2 to 4 carbon atoms and having at least 1 vinyl unsaturated site (>C=C<). For example, $(C_a\text{-}C_b)$alkenyl refers to an alkenyl group having a to b carbon atoms and is intended to include, for example, vinyl, propenyl, isopropenyl, 1 ,3-butadienyl, etc.

"Alkynyl" means a straight chain monovalent hydrocarbon group or a branched chain monovalent hydrocarbon group containing at least one triple bond. The term "alkynyl" is also intended to include those hydrocarbon groups having a triple bond and a double bond. For example, $(C_2\text{-}C_6)$ alkynyl are intended to include ethynyl, propynyl, etc.

"Halogen" is fluorine, chlorine, bromine or iodine.

"Halogen substituted alkyl" refers to an alkyl wherein one or more hydrogen atoms are replaced by halogen; for example Halogen substituted $C_{1\sim4}$ halogen alkyl refers to an alkyl containing 1 to 4 carbon atoms wherein one or more hydrogen atoms are replaced by halogen.

"Heterocyclic group", "heterocycloalkyl", "heterocycloalkane" refers to a saturated or non-aromatic unsaturated ring containing at least one heteroatom; wherein a heteroatom refers to a nitrogen atom, an oxygen atom, or a sulfur atom.

"Aromatic heterocyclyl" means an aromatic unsaturated ring containing at least one heteroatom; wherein heteroatom means a nitrogen atom, an oxygen atom, a sulfur atom.

"Stereoisomer" includes both enantiomers and diastereomers.

The compounds of the present invention may contain asymmetric or chiral centers, and thus different stereoisomers exist. All stereoisomeric forms of the compounds of the present invention include but not limited to diastereomers, enantiomers, atropisomerism, and mixtures thereof, such as racemic mixtures. They form part of the present invention. Many organic compounds exist in optically active forms, i.e., they have the ability to rotate planes of plane-polarized light. **In** describing optically active compounds, the prefixes D,L or R,S are used to indicate the absolute configuration of the chiral center of the molecule. These stereoisomers have the same chemical structure, but their stereo structures are different. Specific stereoisomers may be enantiomers, and mixtures of isomers are often referred to as enantiomeric mixtures. A 50:50 mixture of enantiomers is referred to as a racemic mixture or racemate, which may result in a chemical

reaction process that is not stereoselective or stereotactic. The terms "racemic mixture" and "racemate" refer to an equimolar mixture of two enantiomers lacking optical activity.

The term "pharmaceutically acceptable" refers to a medium, carrier, diluent, excipient, and/or a salt formed thereby chemically or physically compatible with other components constituting a pharmaceutical dosage form and physiologically compatible with the receptor.

The pharmaceutical compositions of the present invention may be in any one of a number of compoundable pharmaceutical dosage forms, e.g., oral, injectable, topical, etc.; the oral dosage forms include, but are not limited to: tablets, capsules, oral liquids, granules, pills, suspension; the injectable dosage forms are selected from point injection, powder injection; the topical dosage forms are selected from patches, creams. All dosage forms can be prepared according to common pharmaceutical techniques, such as using any of the compounds of the present invention, or stereoisomers thereof, or pharmaceutically acceptable salts thereof, as the active pharmaceutical ingredient, and, if necessary, incorporating pharmaceutically acceptable carriers, to form the above pharmaceutical dosage forms suitable for administration; wherein, the unit dose of the pharmaceutically active ingredient may be 0.1 mg-1000 mg, e.g. tablets containing 0.1 mg-1000 mg, preferably 5-500 mg of the pharmaceutically active ingredient per tablet.

The term "salt" and "pharmaceutically acceptable salt" refers to an acidic and/or basic salt formed by the abovementioned compound or a stereoisomer thereof, and inorganic and/or organic acid and base, also including zwitterionic salts (internal salts), also including quaternary ammonium salt, for example alkylammonium salt. These salts can be directly obtained in the final separation and purification of the compound. Alternatively, they can be obtained by mixing the abovementioned compound, or a stereoisomer thereof, and a certain amount of acid or base appropriately (for example in same equivalence). These salts may form precipitates in the solution and be collected by filtration, or be recovered after solvent evaporation, or be prepared by freeze-drying after the reaction in water medium. Said salt in the present invention can be compound hydrochloride salt, sulfate salt, citrate salt, benzene sulfonate salt, hydrobromide salt, hydrofluoride salt, phosphorate salt, acetate salt, propionate salt, succinate salt, oxalate salt, malate salt, succinate salt, fumarate salt, maleate salt, tartarate salt or trifluoroacetate salt.

In some embodiments, one or more compounds of the present invention can be used in combination with each other. Alternatively, the compound of the present invention can be used in combination with any other active agents. It is used to prepare drugs or pharmaceutical compositions for regulating cell functions or treating diseases. If a group of compounds are used, these compounds can be administered to the subjects simultaneously, separately and orderly.

The preparation method of the compound of the present invention is described, and the specific steps are as follows:

Step 1:

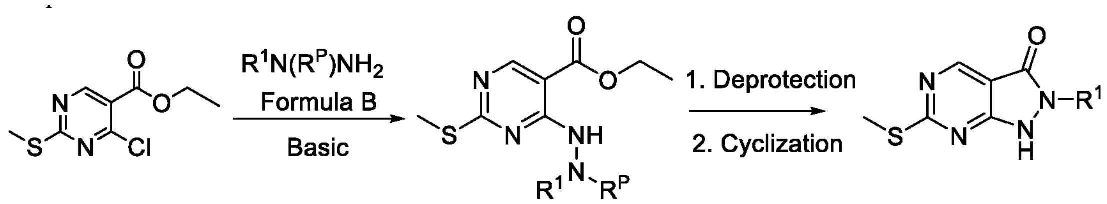


Formula A  Formula C  Formula D

In Formula B and Formula C, $R^P$ represents hydrogen atom or a protecting group of imino. As $R^P$, the protecting group of imino is preferably benzyl, p-methoxy benzyl, tert-butoxy carbonyl, benzyloxy carbonyl and the like. In Formula B, Formula C and Formula D, the definition of $R^1$ group can be referred to above, which is identical to the definition as described above.

In Preparation Method 1, the compound of Formula A and the hydrazine derivative of Formula B are reacted in the presence of base, in order to give the compound of Formula C. The reaction is generally conducted in the presence of organic base such as triethylamine, diisopropyl ethyl amine DIPEA, pyridine, 4-dimethylamino pyridine, or inorganic base such as sodium hydroxide, potassium hydroxide, sodium carbonate, potassium carbonate, sodium bicarbonate; for example in an inert solvent such as dichloromethane, chloroform, tetrahydrofuran, diethyl ether, benzene, toluene, xylene, dimethyl formamide or the mixture solvent thereof. Then, the compound is subjected to deprotection reaction, and the compound is cyclized to give the compound of Formula D. The abovementioned base is used in the amount of preferably equivalent mole to excessive mole, more preferably 1 mole to 5 mole, most preferably 1 mole to 3 mole in respect to 1 mole of the compound of Formula A. Moreover, when the base is a liquid, the base can also act as a solvent and a base. The reaction temperature is usually -78°C-200°C, preferably 20-100°C. The reaction time is usually 5 minutes-7 days,

preferably 8 hours-96 hours.

In Preparation Method 1, the compound of Formula C is subjected to deprotection and cyclization reaction to give the compound of Formula D. In the deprotection reaction, the reaction reagent is selected from a group consisting of trifluoroacetic acid, hydrochloric acid solution and the like, and the solvent is selected from methanol, dichloromethane or 1,4-dioxane and the like. Preferably, the protecting group is removed by the method of TFA/$CH_2Cl_2$. If Boc is used as the protecting group, the deprotection reaction can be conducted under standard condition, for example, conducted in the dichloromethane/trifluoroacetic acid system, saturated hydrogen chloride dioxane solution. In the cyclization reaction, the reaction condition is a basic condition, and the basic condition is selected from a group consisting of sodium hydroxide solution, potassium hydroxide solution, sodium carbonate solution, potassium carbonate solution or sodium bicarbonate solution and the like, preferably sodium hydroxide solution, with a certain concentration. The reaction temperature for deprotection and cyclization is usually -78°C-200°C, preferably 20-100°C, and the reaction time is usually 5 minutes-7 days, preferably 8 hours-96 hours.

Step 2:

The compound of Formula G can be prepared by the methods of Step 2-1 or Step 2-2, and the preparation method in Step 2-1 or Step 2-2 is shown as follows.

Step 2-1:

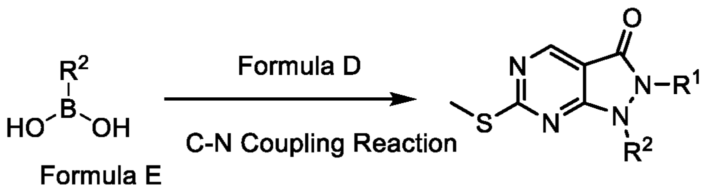


Formula E

Formula G

In the compounds of Formula E and Formula G, the substituents $R^1$ and $R^2$ has the definition as shown above, identical to the abovementioned definition. In the reaction, by C-N coupling reaction, the compounds of Formula E and Formula D are reacted to give the compound of Formula G. The reaction is conducted in a solvent of 1,4-dioxane, tetrahydrofuran, diethyl ether, benzene, toluene, xylene and the like or the mixture solvent. The reaction temperature is 0-200°C, preferably 20-150°C.

Step 2-2:

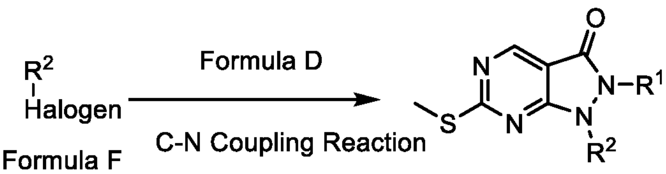


Formula F

Formula G

In the compounds of Formula E and Formula G, the substituents $R^1$ and $R^2$ has the definition as shown above, identical to the abovementioned definition. The halogen atom is F, Cl, Br, or I. The compound of Formula F and the compound of Formula D are subjected to C-N coupling reaction to give the compound of Formula G. The reaction is conducted in a solvent of 1,4-dioxane, tetrahydrofuran, diethyl ether, benzene, toluene, xylene and the like or the mixture solvent. The reaction temperature is 0-200°C, preferably 20-150°C. In this step, the C-N coupling reaction is a coupling method to constitute C-N bond described as routine in the field, such as Ullmann reaction, Buchwald reaction, preferably Ullmann reaction, more preferably under the reaction condition of copper (I) iodide/potassium carbonate/N ,N-diisopropyl ethyl amine DMEDA/1,4-dioxane (the coupling reaction condition is CuI, DMEDA, $K_2CO_3$, 1,4-dioxane), or more preferably CuI /$K_2CO_3$ /N,N'-dimethyl-1,2-cyclohexylene diamine, anisole/NaI/microwave, or more preferably CuI /$K_2CO_3$ /anisole/NaI/microwave.

The compounds $R^2B(OH)_2$ and $R^2$-haloge atom in the Steps 2-1 and 2-2 can be prepared from readily available starting materials by routine synthesis method in the organic chemistry field.

Step 3:

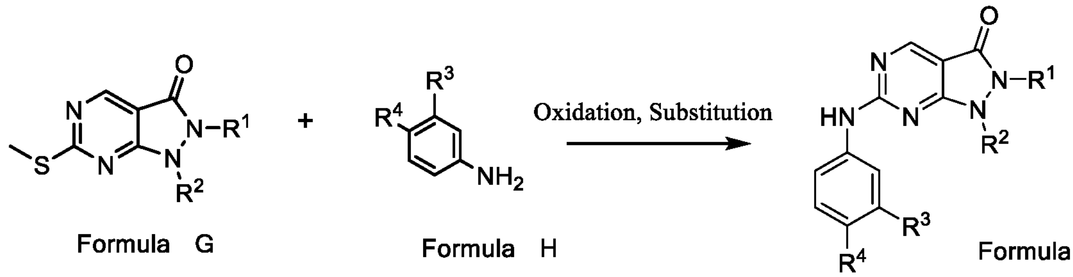


Formula G Formula H Formula I

In the compounds of Formula H and Formula G, the substituents $R^1$, $R^2$, $R^3$, $R^4$ has the definition as shown above, identical to the abovementioned definition. The compound of Formula G first form highly active intermediate sulfoxide in the presence of oxidant, and then is subjected to substitution reaction with the compound of Formula H to give the compound of Formula I. Among others, the reaction solvent is selected from a group consisting of dichloromethane, chloroform, tetrahydrofuran, diethyl ether, benzene, toluene, xylene, dimethyl formamide and the like or the mixture solvent thereof. In the oxidation reaction, the oxidant is preferably m-chloro-peroxybenzoic acid m-CPBA. The substitution reaction conditioni is the reaction conduction routine for substitution in the field, for example, a basic condition or an acidic condition. The basic condition is preferably diisopropyl ethyl amine DIPEA, and the acidic condition is preferably trifluoroacetic acid. The reaction temperature is -20-200°C, preferably 20-150°C, most preferably room temperature.
In the Step 3, the substituted aniline compound of Formula H can be prepared from readily available starting materials by routine synthesis method in the organic chemistry field.
Step 4: If the compound of Formula I as prepared in the Step 3 possess chiral center, those skilled in the art can obtain pure chiral compounds by chromatography or other resolution methods combined with known separation techniques. For example, two chiral compounds with one chiral center can be obtained by SFC resolution. If the compound of Formula I obtained in the Step 3 does not contain a chiral center, there is no need to carry out the resolution process in Step 4.
Obviously, according to the above contents of the present invention, according to the common technical knowledge and routine technique in the field, and without departing from the above basic technical ideas of the present invention, other various forms of modifications can be made to replace or changes.
In the following, the above content of the present invention will be further explained in detail through the concrete implementation in the form of examples. However, it should not be understood that the scope of the above theme of the present invention is limited to the following examples. All technologies realized based on the above content of the present invention belong to the scope of the present invention.

## DETAILED DESCRIPTION OF THE INVENTION

The structure of the compound was determined by nuclear magnetic resonance (NMR) and mass spectrometry (MS). The NMR shift (δ) was given in units of $10^{-6}$(ppm). The NMR was measured by (Bruker Avance III 400 and Bruker Avance 300) nuclear magnetic apparatus. Deuterated methyl sulfoxide (DMSO-d6), deuterated chloroform (CDC13) and deuterated methanol (CD3OD) were used as the characterization solvents, and tetramethylsilane (TMS) was used as the internal standard.
The LC-MS was determined by Shimadzu LC-MS 2020 (ESI). The HPLC was determined by Shimadzu LC-20A. MPLC (medium performance liquid chromatography) was conducted by Gilson GX-281 reverse phase preparative chromatography. Yantai Huanghai HSGF254 or Qingdao GF254 silica gel plate was used as the silica gel plate for thin-layer chromatography, and the specification of thin-layer chromatography separation and purification products was 0.4 mm-0.5 mm. Column chromatography generally used Yantai Huanghai silica gel 200-300 mesh silica gel as carrier.
The known starting materials of the present invention can be synthesized by or according to the methods known in the field, or can be purchased from Anneiji Chemical, Chengdu Kelon Chemical, Shaoyuan Chemical Technology, Bailingwei Technology etc.
Unless otherwise specified in Examples, the reaction was carried out under nitrogen atmosphere. Unless otherwise specified in the examples, the solution refers to an aqueous solution. Unless otherwise specified in Examples, the reaction temperature was room temperature. Unless otherwise specified in Examples, M refers to mole per liter.

THF: tetrahydrofuran; DIPEA: N,N-diisopropyl ethyl amine;

DCM: dichloromethane; TFA: trifluoroacetic acid; *m*-CPBA: m-chloroperoxybenzoic acid;
DMF: dimethyl formamide; PTSA: p-toluenesulfonamide;
DMSO: dimethyl sulfoxide; NBS: N-BROMOSUCCINIMIDE; AIBN: azodiisobutyronitrile;
DMP: dimethyl phthalate; TBAHS: tetrabutylammonium hydrogen sulfate;
BMS: 4-benzoyl-4"-methyl-diphenyl sulfide;

Unless otherwise specified in Examples, the HPLC test conditions were as follows:

**Method A:**

Column: Boston Green C18 150 mm*4.6 mm 5 um; Mobile Phase: A: 0.05% TFA Water B:0.05% TFA Acétonitrile; Gradient: B from 5% to 95% in 10.0 min and hold 95% for 5.0 min; Flow Rate: 1.5 mL/min; Column Temperature: 40 °C.

**Method B:**

Column: Boston Green ODS 150 mm*4.6 mm 5 um; Mobile Phase: A: 0.01M $NH_4HCO_3$ Water B: Acétonitrile; Gradient: B from 5% to 95% in 10.0 min and hold 95% for 5.0 min; Flow Rate: 1.5 mL/min; Column Temperature: 40 °C.

Unless otherwise specified, the SFC splitting condition is as follows:

Column: 3um, 150mm*3mm; Mobile Phase: A: $CO_2$, Mobile Phase B: Alcohols solvent; Flow Rate: 1 mL/min Column Temperature: 40°C

**Intermediate Example 1: Synthesis of Intermediate IM-1**

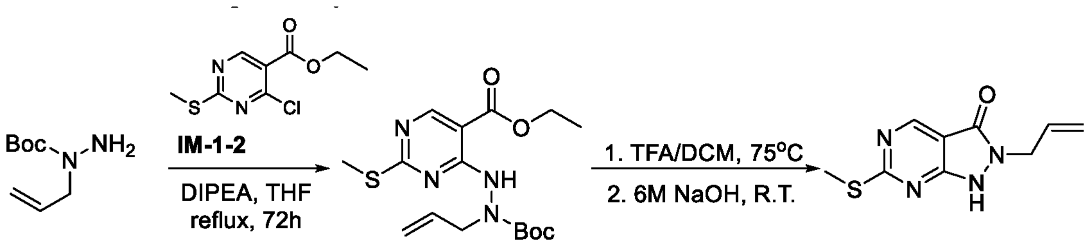


**IM-1-1** **IM-1-3** **IM-1**

**Step 1: Synthesis of Compound IM-1-3:**

A dry single-necked flask was added with Substrate **IM-1-2** (8.15 g, 35 mmol) and THF(50 mL). The mixture was stirred to dissolved, and then added with **IM-1-1** (6.3 g, 37 mol) and DIPEA (15 mL, 75 mol), heated to 110°C to react with LC-MS monitoring. After the reaction was completed, the system was recovered to room temperature, the solid was precipitated and then filtered, dried with oven, to give crude product **IM-1-3** (9.87 g, 76.6% yield), LCMS ($ESI^+$) m/z: 369.2 $[M+H]^+$.

**Step 2: Synthesis of Compound IM-1:**

A dry single-necked flask was added with Substrate **IM-1-3** (9.16 g, 27.5 mmol) and DCM(18 mL). The mixture was stirred to dissolved, and then slowly added with TFA(18 mL), heated to 75°C to react with LC-MS monitoring. After the reaction was completed, the organic solvent was concentrated under reduced pressure. The mixture was dissolved with ethanol, and added with 6M NaOH solution, stirred under room temperature with LC-MS monitoring. After the reaction was completed, the mixture was concentrated under reduced pressure, and then solid was precipitated. The solid was filtered, washed with water for three times, washed with cold ethanol for three times, dried under room temperature, to give crude product **IM-1** (5 g, 81.7% yield), LCMS ($ESI^+$) m/z: 223.1 $[M+H]^+$.

**Example 1: Synthesis of Compound 1**

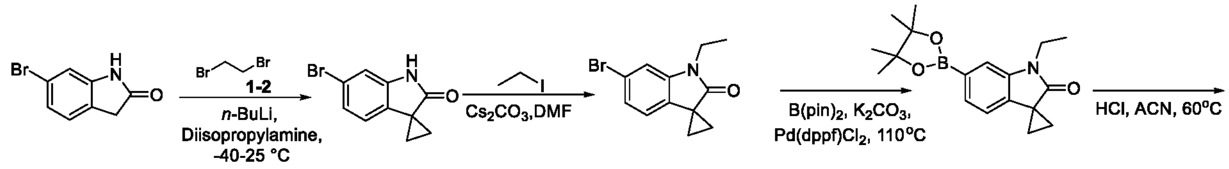


1-1 1-3 1-4 1-5

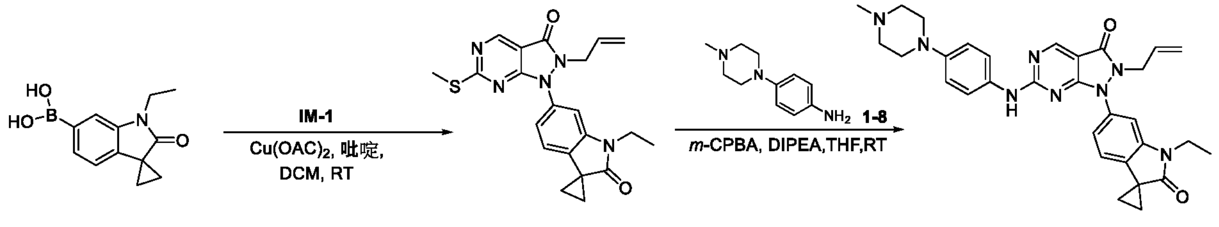


1-6 1-7 1

**Step 1: Synthesis of Compound 1-3:**

A dry single-necked flask was added with Substrate **1-1** (1 g, 4.72 mmol) and THF(10 mL). The mixture was stirred to dissolved, and then at -40°C and under the protection of nitrogen atmosphere slowly dropwise added with n-BuLi (2.5 M, 4.15 mL). The reaction was stirred at -40°C for 1 hour, and dropwise added with **1-2** (2.66 g, 14.15 mmol), after the dropwise addition was completed, recovered to room temperature and reacted for 12 hours with LC-MS monitoring. After the reaction was completed, the mixture was added with 4 N HCl (5 mL), after the solvent was evaporated, added with water and extracted with EA for three times. The organic phase was combined, treated with anhydrous sodium sulfate, and then filtered and dried under evaporation, to give the product **1-3** (1.2 g, crude), LCMS ($ESI^+$) m/z: 238/240 $[M+H]^+$.

**Step 2: Synthesis of Compound 1-4:**

A dry single-necked flask was added with Substrate **1-3** (700 mg, 2.94 mmol) and DMF (3 mL). The mixture was stirred to dissolved, and then added with iodoethane (458.57 mg, 2.94 mmol) and cesium carbonate (1.05 g, 3.23 mmol). At 75°C, the reaction was stirred for 8 hours with LC-MS monitoring. After the reaction was completed, the mixture was added with water and extracted with EA for three times. The organic phase was combined, treated with anhydrous sodium sulfate, and then filtered and dried under evaporation, purified by reverse phase column chromatography to give the product 1-4 (285 mg, 1.07 mmol, 36.42% yield), LCMS ($ESI^+$) m/z: 266 $[M+H]^+$.

**Step 3: Synthesis of Compound 1-5:**

A dry single-necked flask was added with Substrate **1-4** (155 mg, 582.41 μmol), $B(pin)_2$ (325.38 mg, 1.28 mmol), $Pd(dppf)Cl_2$ (42.73 mg, 58.24 μmol) and potassium carbonate (241.47 mg, 1.75 mmol) in 1,4-dioxane (5 mL). Under the protection of nitrogen atmosphere, the reaction was heated to 110°C, and was stirred for 12 hours with LC-MS monitoring. After the reaction was completed, the solvent was evaporated, added with water and extracted with EA for three times. The organic phase was combined, treated with anhydrous sodium sulfate, and then filtered and dried under evaporation, purified by reverse phase column chromatography to give the product **1-5** (80 mg, 255.43 μmol, 43.86% yield), LCMS ($ESI^+$) m/z: 314 $[M+H]^+$.

**Step 4: Synthesis of Compound 1-6:**

A dry single-necked flask was added with Substrate **1-5** (57 mg, 181.99 μmol). The mixture was dissolved with ACN (5 mL), added with HCL (6 M, 1 mL), heated to 60°C. The reaction was stirred for 6 hours with LC-MS monitoring. After the reaction was completed, the solvent was evaporated, added with water and extracted with EA for three times. The organic phase was combined, treated with anhydrous sodium sulfate, and then filtered and dried under evaporation, purified by reverse phase column chromatography to give the product **1-6** (24 mg, 103.87 μmol, 57.07% yield), LCMS ($ESI^+$) m/z: 232 $[M+H]^+$.

**Step 5: Synthesis of Compound 1-7:**

A dry single-necked flask was added with Substrate **1-6** (24 mg, 103.87 μmol). The mixture was dissolved with

DCM (3 mL), added with **IM-1** (26.93 mg, 121.18 μmol), copper acetate (4.52 mg, 24.86 μmol) and pyridine (9.59 mg, 121.18 μmol, 9.76 μL). The reaction was stirred under room temperature for 72 hours with LC-MS monitoring. After the reaction was completed, the solvent was evaporated, added with water and extracted with EA for three times. The organic phase was combined, treated with anhydrous sodium sulfate, and then filtered and dried under evaporation, purified by reverse phase column chromatography to give the product 1-7 (19 mg, 46.63 μmol, 38.48% yield), LCMS ($ESI^+$) m/z: 408 $[M+H]^+$.

**Step 6: Synthesis of Compound 1:**

A dry single-necked flask was added with Substrate **1-7** (19 mg, 46.63 μmol). The mixture was dissolved with THF (1.5 mL), added with m-CPBA (14.48 mg, 83.93 μmol). The reaction was stirred under room temperature for 0.5 hours, and then added with DIPEA (30.13 mg, 233.14 μmol, 40.61 μL), and **1-8** (10.70 mg, 55.95 μmol), and continued to stir under room temperature for 12 hours with LC-MS monitoring. After the reaction was completed, the solvent was evaporated. The mixture was purified by reverse phase column chromatography to give the product 1 (3.1 mg, 5.63 μmol, 12.07% yield). **$^1$H NMR** (600 MHz, DMSO-$d_6$) δ10.03 (s, 1H), 8.81 (s, 1H), 7.52 (s, 2H), 7.21 (s, 2H), 7.11 - 7.09 (m, 1H), 6.83 (d, *J* = 6.0 Hz, 2H), 5.70 - 5.67 (m, 1H), 5.09 (d, *J* =10.2 Hz, 1H), 4.97 (d, *J* = 17.4 Hz, 1H), 4.28 (s, 2H), 3.82 - 3.78 (m, 2H), 3.06 (s, 4H), 2.47 (s, 4H), 2.23 (s, 3H), 1.69 (d, *J* = 3.6 Hz, 2H), 1.58 (d, *J* = 3.0 Hz, 2H), 1.15 (t, *J* = 6.6 Hz, 3H). LCMS ($ESI^+$) m/z: 551.2 $[M+H]^+$, HPLC Method B: $R_T$=5.65 min, purity:100%.

**Example 2: Synthesis of Compound 2**

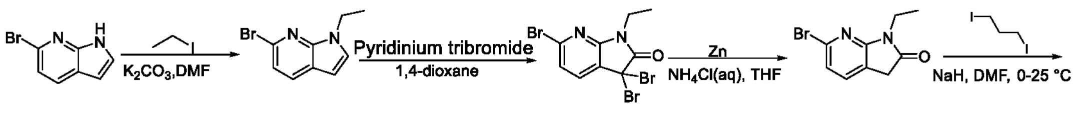


2-1 2-2 2-3 2-4

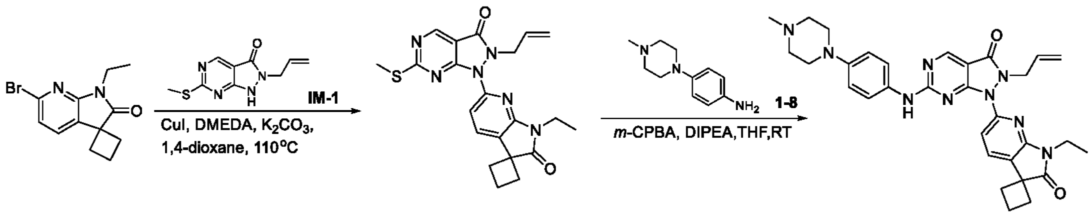


2-5 2-6 2

**Step 1: Synthesis of Compound 2-2:**

A dry single-necked flask was added with Substrate **2-1** (870 mg, 4.42 mmol) and DMF (8 mL). The mixture was stirred to dissolved, and then added with iodoethane (1.38 g, 8.83 mmol) and potassium carbonate (1.22 g, 8.83 mmol). At 75°C, the reaction was stirred for 4 hours with LC-MS monitoring. After the reaction was completed, the mixture was added with water and extracted with EA for three times. The organic phase was combined, treated with anhydrous sodium sulfate, and then filtered and dried under evaporation, purified by reverse phase column chromatography to give the product **2-2** (600 mg, 2.67 mmol, 60.37% yield), LCMS ($ESI^+$) m/z: 225 $[M+H]^+$.

**Step 2: Synthesis of Compound 2-3:**

A dry single-necked flask was added with Substrate **2-2** (600 mg, 2.67 mmol) and 1,4-dioxane (10 mL). The mixture was stirred to dissolved, and then added with tribromopyridine (3.41 g, 10.66 mmol). Under room temperature, the reaction was stirred for 16 hours with LC-MS monitoring. After the reaction was completed, the solvent was evaporated, added with water and extracted with DCM for three times. The organic phase was combined, treated with anhydrous sodium sulfate, and then filtered and dried under evaporation, purified by reverse phase column chromatography to give the product **2-3** (230 mg, 576.62 μmol, 21.63% yield), LCMS ($ESI^+$) m/z: 399 $[M+H]^+$.

**Step 3: Synthesis of Compound 2-4:**

A dry single-necked flask was added with Substrate **2-3** (230 mg, 576.62 μmol) and THF (1.5 mL). The mixture was stirred to dissolved, and then added with saturated ammonium chloride solution (1.5 mL) and zinc powder (753.99 mg, 11.53 mmol). Under room temperature, the reaction was stirred for 10 minutes with LC-MS monitoring. After the reaction was completed, the mixture was filtrated. The solvent was evaporated. The mixture was purified by reverse phase column chromatography to give the product **2-4** (44 mg, 182.51 μmol, 31.65% yield), LCMS ($ESI^+$) m/z: 241 $[M+H]^+$.

**Step 4: Synthesis of Compound 2-5:**

A dry single-necked flask was added with Substrate **2-4** (20 mg, 82.96 μmol) and DMF (1 mL). The mixture was stirred to dissolved, and then added with NaH (11.95 mg, 497.75 μmol). At 0°C, the reaction was stirred for 0.5 hours, and then added with 1,3-diiodopropane (73.64 mg, 248.88 μmol). Under room temperature, the reaction was stirred for 0.5 hours with LC-MS monitoring. After the reaction was completed, the mixture was added with water to quench the reaction, and extracted with EA for three times. The organic phase was combined, treated with anhydrous sodium sulfate, and then filtered and dried under evaporation, purified by reverse phase column chromatography to give the product **2-5** (10 mg, 35.57 μmol, 42.87% yield), LCMS ($ESI^+$) m/z: 281 $[M+H]^+$.

**Step 5: Synthesis of Compound 2-6:**

A dry single-necked flask was added with Substrate **2-5** (10 mg, 35.57 μmol) and 1,4-dioxane (1 mL). The mixture was stirred to dissolved, and then added with **IM-1** (10 mg, 44.99 μmol), CuI (15.58 mg, 81.80 μmol), $K_2CO_3$ (7.91 mg, 57.26 μmol) and DMEDA (14.42 mg, 163.60 μmol). Under the protection of nitrogen atmosphere, the reaction was heated to 110°C. The reaction was stirred for 3 hours with LC-MS monitoring. After the reaction was completed, the mixture was added with water and extracted with EA for three times. The organic phase was combined, treated with anhydrous sodium sulfate, and then filtered and dried under evaporation, purified by reverse phase column chromatography to give the product **2-6** (5 mg, 11.83 μmol, 28.93% yield), LCMS ($ESI^+$) m/z: 423 $[M+H]^+$.

**Step 6: Synthesis of Compound 2:**

A dry single-necked flask was added with Substrate **2-6** (5.0 mg, 11.86 μmol). The mixture was dissolved with THF (1 mL), added with m-CPBA (3.68 mg, 21.35 μmol). The reaction was stirred under room temperature for 0.5 hours, and then added with DIPEA (7.67 mg, 59.31 μmol, 10.33 μL), and **1-8** (2.72 mg, 14.23 μmol), and continued to stir under room temperature for 8 hours with LC-MS monitoring. After the reaction was completed, the solvent was evaporated. The mixture was purified by reverse phase column chromatography to give the product **2** (2.1 mg, 3.71 μmol, 31.30% yield). **$^1H$ NMR** (600 MHz, $CDCL_3$) δ 8.77 (s, 1H), 7.77 (d, *J* = 7.8 Hz, 1H), 7.44 (d, *J* = 4.8 Hz, 2H), 7.36 (d, *J* = 7.8 Hz, 1H), 6.86 (d, *J* = 9.0 Hz, 2H), 5.67 - 5.62 (m, 1H), 5.00 (d, *J* = 9.6 Hz, 1H), 4.92 (d, *J* = 16.8 Hz, 1H), 4.65 (d, *J* = 5.4 Hz, 2H), 3.75 (q, *J* = 14.4 Hz, 2H), 3.40 (s, 4H), 2.93 - 2.87 (m, 2H), 2.64 - 2.61 (m, 4H), 2.37 - 2.28 (m, 4H), 2.23 - 2.18 (m, 1H), 1.57 (s, 2H), 1.21 (t, *J* =7.2 Hz, 3H). LCMS ($ESI^+$) m/z: 566.2 $[M+H]^+$, HPLC Method B: $R_T$=6.03 min, purity:85.9%.

**Example 3: Synthesis of Compound 3**

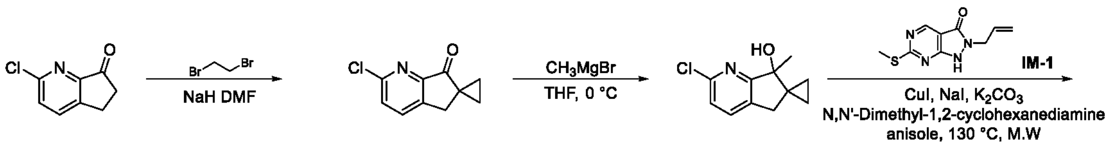


3-1 3-2 3-3

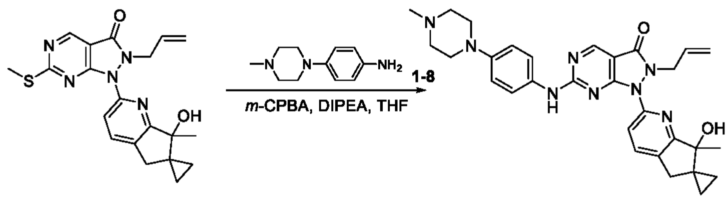


3-4 3

**Step 1: Synthesis of Compound 3-2:**

A dry single-necked flask was added with Substrate NaH (166.08 mg, 4.15 mmol, 60% purity) and DMF (20 mL). The mixture was stirred to dissolved, and then at 0°C and under the protection of nitrogen atmosphere, added with a solution of 1,2-dibromoethane (780 mg, 4.15 mmol) and **3-1** (200 mg, 1.19 mmol) in DMF (10 mL). At 0°C, the reaction was stirred for 1 hours, and then reacted under room temperature for 2 hours with LC-MS monitoring. After the reaction was completed, the mixture was added with saturated ammonium chloride solution and extracted with EA for three times. The organic phase was combined, treated with anhydrous sodium sulfate, and then filtered and dried under evaporation, purified by reverse phase column chromatography to give the product **3-2** (35 mg, 180.76 μmol, 15.24% yield), LCMS ($ESI^+$) m/z: 194.2 $[M+H]^+$.

**Step 2: Synthesis of Compound 3-3:**

A dry single-necked flask was added with Substrate **3-2** (33 mg, 170.43 μmol) and THF (5 mL). The mixture was stirred to dissolved, and then at 0°C and under the protection of nitrogen atmosphere dropwise added with methylmagnesium bromide (2 M, 340.86 μL). At 0°C, the reaction was stirred for 1 hours with LC-MS monitoring. After the reaction was completed, the mixture was added with saturated ammonium chloride solution and extracted with EA for three times. The organic phase was combined, treated with anhydrous sodium sulfate, and then filtered and dried under evaporation, purified by reverse phase column chromatography to give the product **3-3** (33 mg, 157.39 μmol, 92.35% yield), LCMS ($ESI^+$) m/z: 210.1 $[M+H]^+$.

**Step 3: Synthesis of Compound 3-4:**

A dry single-necked flask was added with Substrate **3-3** (33 mg, 157.39 μmol) and anisole (2 mL). The mixture was stirred to dissolved, and then added with **IM-1** (34.98 mg, 157.39 μmol), CuI (59.95 mg, 314.78 μmol), $K_2CO_3$ (54.38 mg, 393.47 μmol), N,N'-dimethyl-1,2-cyclohexylene diamine (89.55 mg, 629.56 μmol) and NaI (47.18 mg, 314.78 μmol), and subjected to microwave at 130°C. The reaction was stirred for 4 hours with LC-MS monitoring. After the reaction was completed, the mixture was added with water and extracted with EA for three times. The organic phase was combined, treated with anhydrous sodium sulfate, and then filtered and dried under evaporation, purified by reverse phase column chromatography to give the product **3-4** (38 mg, 96.09 μmol, 61.05% yield), LCMS ($ESI^+$) m/z: 396.1 $[M+H]^+$.

**Step 4: Synthesis of Compound 3:**

A dry single-necked flask was added with Substrate **3-4** (38 mg, 96.09 μmol). The mixture was dissolved with THF (4 mL), added with m-CPBA (35.11 mg, 172.96 μmol, 85% purity). The reaction was stirred under room temperature for 1 hours, and then added with DIPEA (124.18 mg, 960.86 μmol, 167.36 μL), and **1-8** (22.05 mg, 115.30 μmol), heated to 50°C and stirred for 5 hours with LC-MS monitoring. After the reaction was completed, the solvent was evaporated. The mixture was purified by reverse phase column chromatography to give the product **3** (22.6 mg, 40.24 μmol, 41.88% yield). **$^1$H NMR** (600 MHz, DMSO-$d_6$) δ 10.14 (s, 1H), 8.82 (s, 1H), 7.92 (s, 1H), 7.70 (d, $J$ = 8.4 Hz, 1H), 7.59 (s, 2H), 6.92 (d, $J$ = 8.4 Hz, 2H), 5.72 - 5.76 (m, 1H), 5.02 (s, 1H), 5.01 (d, $J$ = 10.2 Hz, 1H), 4.88 (d, $J$ = 17.4 Hz, 1H), 4.82 - 4.68 (m, 1H), 4.66 - 4.52 (m, 1H), 3.09 (t, $J$ = 4.8 Hz, 4H), 2.93 - 2.86 (m, 2H), 2.46 (t, $J$ = 4.8 Hz, 4H), 2.23 (s, 3H), 1.22 (s, 3H), 0.94 - 0.91 (m, 1H), 0.70 - 0.67 (m, 1H), 0.60 - 0.56 (m, 1H), 0.48 - 0.45 (m, 1H). LCMS ($ESI^+$) m/z: 539.3 $[M+H]^+$, HPLC Method B: $R_T$=6.92 min,

purity:95.9%.

Compound **3** was resolved by SFC to give the following two compounds: **3a** $R_T$= 2.51 min; **3b** $R_T$ = 3.51 min (Unless otherwise specified, the chirality of compound is only distinguished by molecules here, which does not represent the exact absolute configuration, the same below.).

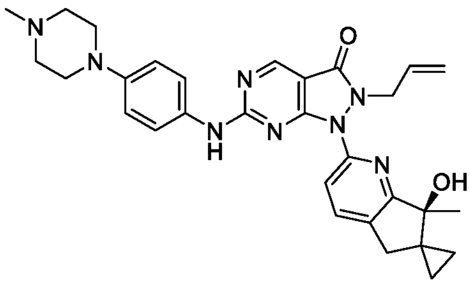

**3a** ,

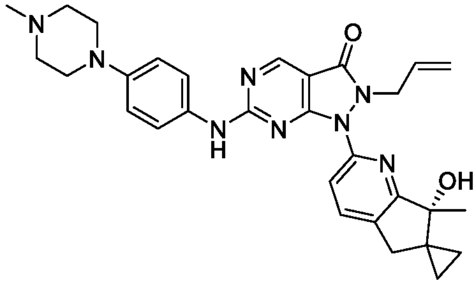

**3b**

**3a** structure characterization: **$^1$H NMR** (400 MHz, DMSO-*d*$_6$) δ 10.13 (s, 1H), 8.83 (s, 1H), 7.92 (d, *J* = 8.0 Hz, 1H), 7.70 (d, *J* = 8.0 Hz, 1H), 7.66 - 7.46 (m, 2H), 6.92 (d, *J* = 8.8 Hz, 2H), 5.74 - 5.67 (m, 1H), 5.10 - 4.98 (m, 2H), 4.90 - 4.86 (m, 1H), 4.83 - 4.67 (m, 1H), 4.60 (d, *J* = 14.8 Hz, 1H), 3.11 (t, *J* = 5.2 Hz, 4H), 2.90 (d, *J* = 2.4 Hz, 2H), 2.50 -2.44 (m, 4H), 2.26 (s, 3H), 1.22 (s, 3H), 0.95 - 0.90 (m, 1H), 0.71 - 0.66 (m, 1H), 0.61 - 0.56 (m, 1H), 0.48 - 0.45 (m, Hz, 1H). LCMS (ESI$^+$) m/z: 539.3 [M+H]$^+$, HPLC Method B: $R_T$=6.80 min, purity:97.8%.

**3b** structure characterization: **$^1$H NMR** (400 MHz, DMSO-*d*$_6$) δ 10.13 (s, 1H), 8.82 (s, 1H), 7.92 (d, *J* = 8.0 Hz, 1H), 7.70 (d, *J* = 8.0 Hz, 1H), 7.59 (s, 2H), 7.02 - 6.83 (m, 2H), 5.74 - 5.67 (m, 1H), 5.06 - 4.97 (m, 2H), 4.90 - 4.86 (m, 1H), 4.80 - 4.66 (m, 1H), 4.60 (d, *J* = 13.6 Hz, 1H), 3.09 (t, *J* = 5.2 Hz, 4H), 2.90 (d, *J* = 2.4 Hz, 2H), 2.45 (t, *J* = 5.2 Hz, 4H), 2.22 (s, 3H), 1.22 (s, 3H), 0.95 - 0.90 (m, 1H), 0.71 - 0.66 (m, 1H), 0.61 - 0.56 (m, 1H), 0.48 - 0.45 (m, Hz, 1H). LCMS (ESI$^+$) m/z: 539.3 [M+H]$^+$, HPLC Method B: $R_T$=6.80 min, purity:98.1%.

**Example 4: Synthesis of Compound 4**

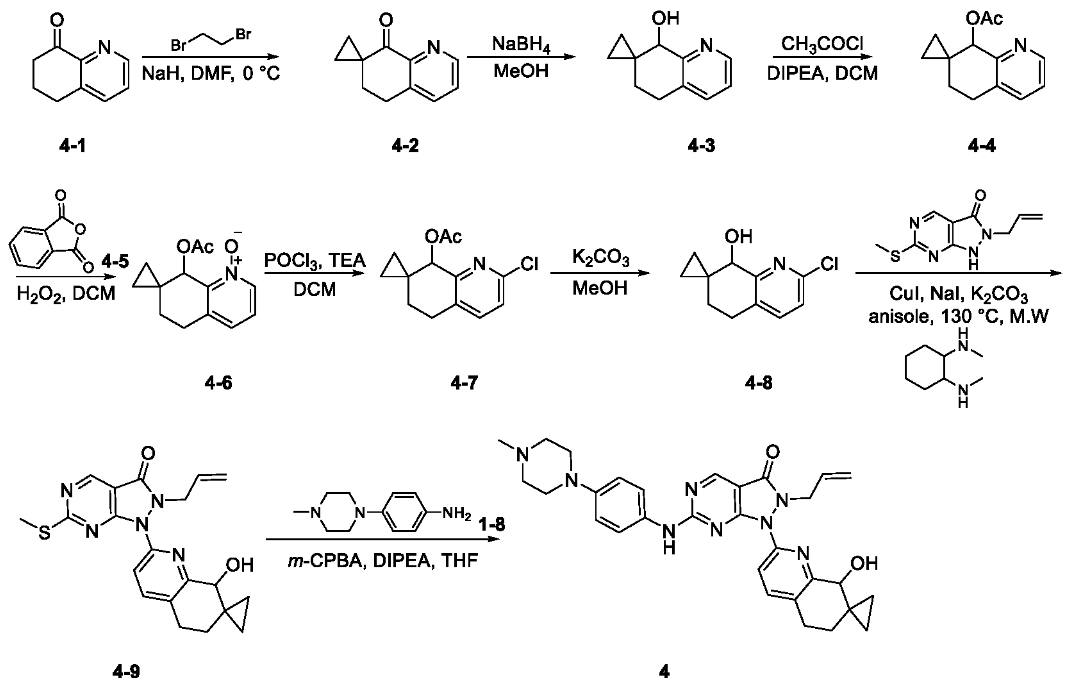


4-1 4-2 4-3 4-4 4-5 4-6 4-7 4-8 4-9 1-8 4

**Step 1: Synthesis of Compound 4-2:**

A dry single-necked flask was added with Substrate NaH (1.40 g, 34.96 mmol, 60% purity) and DMF (150 mL). The mixture was stirred to dissolved, and then at 0°C and under the protection of nitrogen atmosphere, added with a solution of 1,2-dibromoethane (6.57 g, 34.96 mmol) and **4-1** (1.47 g, 9.99 mmol) in DMF (30 mL). At 0°C, the reaction was stirred for 3 hours with LC-MS monitoring. After the reaction was completed, the mixture was added with saturated ammonium chloride solution and extracted with EA for three times. The organic phase was combined, treated with anhydrous sodium sulfate, and then filtered and dried under evaporation, purified by reverse phase column chromatography to give the product **4-2** (0.55 g, 3.18 mmol, 31.79% yield), LCMS ($ESI^+$) m/z: 174.0 $[M+H]^+$.

**Step 2: Synthesis of Compound 4-3:**

A dry single-necked flask was added with Substrate **4-2** (470 mg, 2.71 mmol) and methanol (20 mL). The mixture was stirred to dissolved, and then at 0°C added with sodium borohydride (205.30 mg, 5.43 mmol). At 0°C, the reaction was stirred for 1 hours with LC-MS monitoring. After the reaction was completed, the mixture was added with saturated sodium bicarbonate solution and extracted with DCM for three times. The organic phase was combined, treated with anhydrous sodium sulfate, and then filtered and dried under evaporation, purified by reverse phase column chromatography to give the product **4-3** (470 mg, 2.68 mmol, 98.95% yield), LCMS ($ESI^+$) m/z: 176.2 $[M+H]^+$.

**Step 3: Synthesis of Compound 4-4:**

A dry single-necked flask was added with Substrate **4-3** (470 mg, 2.68 mmol) and DCM (20 mL). The mixture was stirred to dissolved, and then at 0°C added with DIPEA (1.39 g, 10.73 mmol, 1.87 mL), at 0°C slowly dropwise added with acetyl chloride (421.10 mg, 5.36 mmol, 381.43 μL). At 0°C, the reaction was stirred for 1 hours with LC-MS monitoring. After the reaction was completed, the solvent was evaporated. The mixture was purified by reverse phase column chromatography to give the product **4-4** (540 mg, 2.49 mmol, 92.66% yield), LCMS ($ESI^+$) m/z: 218.2 $[M+H]^+$.

**Step 4: Synthesis of Compound 4-6:**

A dry single-necked flask was added with Substrate **4-4** (540 mg, 2.49 mmol) and DCM (5 mL). The mixture was stirred to dissolved, and then added with **4-5** (736.29 mg, 4.97 mmol) and $H_2O_2$ (845.31 mg, 7.46 mmol, 761.54 μL, 30% purity), heated to 40°C. The reaction was stirred for 16 hours with LC-MS monitoring. After the reaction was completed, the mixture was added with saturated sodium bicarbonate solution and extracted with DCM for three times. The organic phase was combined, treated with anhydrous sodium sulfate, and then filtered and dried under evaporation, purified by reverse phase column chromatography to give the product **4-6** (560 mg, 2.40 mmol, 96.59% yield), LCMS ($ESI^+$) m/z: 234.0 $[M+H]^+$.

**Step 5: Synthesis of Compound 4-7:**

A dry single-necked flask was added with Substrate **4-6** (560 mg, 2.40 mmol) and DCM (20 mL). The mixture was stirred to dissolved, and then at 0°C and under the protection of nitrogen atmosphere dropwise added with triethylamine (1.28 g, 12.65 mmol, 1.76 mL) and $POCl_3$ (775.65 mg, 5.06 mmol, 471.52 μL). At 0°C, the reaction was stirred for 3 hours with LC-MS monitoring. After the reaction was completed, the mixture was added with saturated sodium bicarbonate solution and extracted with DCM for three times. The organic phase was combined, treated with anhydrous sodium sulfate, and then filtered and dried under evaporation to give crude product **4-7** (650 mg, crude), LCMS ($ESI^+$) m/z: 252.0 $[M+H]^+$.

**Step 6: Synthesis of Compound 4-8:**

A dry single-necked flask was added with Substrate **4-7** (650 mg, 2.58 mmol) and methanol (20 mL). The mixture was stirred to dissolved, and then added with potassium carbonate (1.78 g, 12.91 mmol). Under room temperature, the reaction was stirred for 2 hours with LC-MS monitoring. After the reaction was completed, the mixture was filtrated, and the filtrate was dried under evaporation, purified by reverse phase column chromatography to give the product **4-8** (197 mg, 939.56 μmol, 36.38% yield), LCMS ($ESI^+$) m/z: 210.1 $[M+H]^+$.

**Step 7: Synthesis of Compound 4-9:**

Following the synthesis method of Example **3** Step **3,** the same synthesis method was performed, expect that in Step **3, 3-3** was replaced with **4-8** (40 mg, 190.77 μmol), to give Compound **4-9** (39 mg, 98.62 μmol, 51.69% yield), LCMS

(ESI$^+$) m/z: 396.0 [M+H]$^+$.

**Step 8: Synthesis of Compound 4:**

Following the synthesis method of Example **3** Step **4,** the same synthesis method was performed, expect that in Step **4, 3-4** was replaced with **4-9** (39 mg, 98.62 μmol), to give Compound **4** (24.4 mg, 45.30 μmol, 45.93% yield). **$^1$H NMR** (600 MHz, DMSO-$d_6$) δ 10.13 (s, 1H), 8.81 (s, 1H), 7.84 (d, $J$ = 8.4 Hz, 1H), 7.70 (d, $J$ = 8.4 Hz, 1H), 7.66 - 7.50 (m, 2H), 6.92 (d, $J$ = 8.4 Hz, 2H), 5.70 - 5.64 (m, 1H), 5.31 (d, $J$ = 5.4 Hz, 1H), 5.00 (d, $J$ = 10.2 Hz, 1H), 4.89 (d, $J$ = 17.4 Hz, 1H), 4.78 - 4.54 (m, 2H), 3.75 (d, $J$ = 5.4 Hz, 1H), 3.10 (t, $J$ = 4.8 Hz, 4H), 2.88 - 2.79 (m, 2H), 2.46 (t, $J$ = 4.8 Hz, 4H), 2.28 - 2.19 (m, 4H), 1.13 - 1.11 (m, 1H), 0.69 - 0.66 (m, 1H), 0.45 - 0.42 (m, 1H), 0.41 - 0.31 (m, 2H). LCMS (ESI$^+$) m/z: 539.2 [M+H]$^+$, HPLC Method B: $R_T$=6.98 min, purity:98.9%.

Compound **4** was resolved by SFC to give the following two compounds: **4a** $R_T$ = 3.12 min; **4b** $R_T$ = 5.44 min.

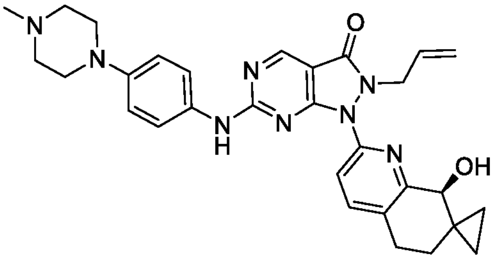

**4a** ,

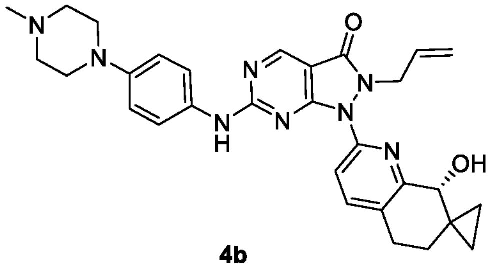

**4b**

**4a** structure characterization: **$^1$H NMR** (400 MHz, DMSO-$d_6$) δ 10.12 (s, 1H), 8.81 (s, 1H), 7.84 (d, $J$ = 8.4 Hz, 1H), 7.70 (d, $J$ = 8.4 Hz, 1H), 7.59 (s, 2H), 6.92 (d, $J$ = 9.2 Hz, 2H), 5.74 - 5.61 (m, 1H), 5.31 (d, $J$ = 5.2 Hz, 1H), 5.02 - 4.99 (m, 1H), 4.91 - 4.87 (m, 1H), 4.77 - 4.57 (m, 2H), 3.75 (d, $J$ = 5.2 Hz, 1H), 3.10 (t, $J$ = 5.2 Hz, 4H), 2.94 - 2.75 (m, 2H), 2.46 (t, $J$ = 5.2 Hz, 4H), 2.31 - 2.18 (m, 4H), 1.13 - 1.10 (m, 1H), 0.69 - 0.66 (m, 1H), 0.45 - 0.42 (m, 1H), 0.41 - 0.31 (m, 2H). LCMS (ESI$^+$) m/z: 539.2 [M+H]$^+$, HPLC Method B: $R_T$ =6.91 min, purity:98.7%.

**4b** structure characterization: **$^1$H NMR** (400 MHz, DMSO-$d_6$) δ 10.12 (s, 1H), 8.81 (s, 1H), 7.84 (d, $J$ = 8.4 Hz, 1H), 7.70 (d, $J$ = 8.4 Hz, 1H), 7.59 (s, 2H), 6.97 - 6.87 (m, 2H), 5.74 - 5.61 (m, 1H), 5.31 (d, $J$ = 5.6 Hz, 1H), 5.02 - 4.99 (m, 1H), 4.91 - 4.87 (m, 1H), 4.76 - 4.56 (m, 2H), 3.75 (d, $J$ = 5.2 Hz, 1H), 3.10 (t, $J$ = 4.8 Hz, 4H), 2.97 - 2.76 (m, 2H), 2.46 (t, $J$ = 5.2 Hz, 4H), 2.30 - 2.18 (m, 4H), 1.13 - 1.10 (m, 1H), 0.69 - 0.66 (m, 1H), 0.45 - 0.42 (m, 1H), 0.41 - 0.31 (m, 2H). LCMS (ESI$^+$) m/z: 539.2 [M+H]$^+$, HPLC Method B: $R_T$ =6.92 min, purity:98.0%.

**Example 5: Synthesis of Compound 5**

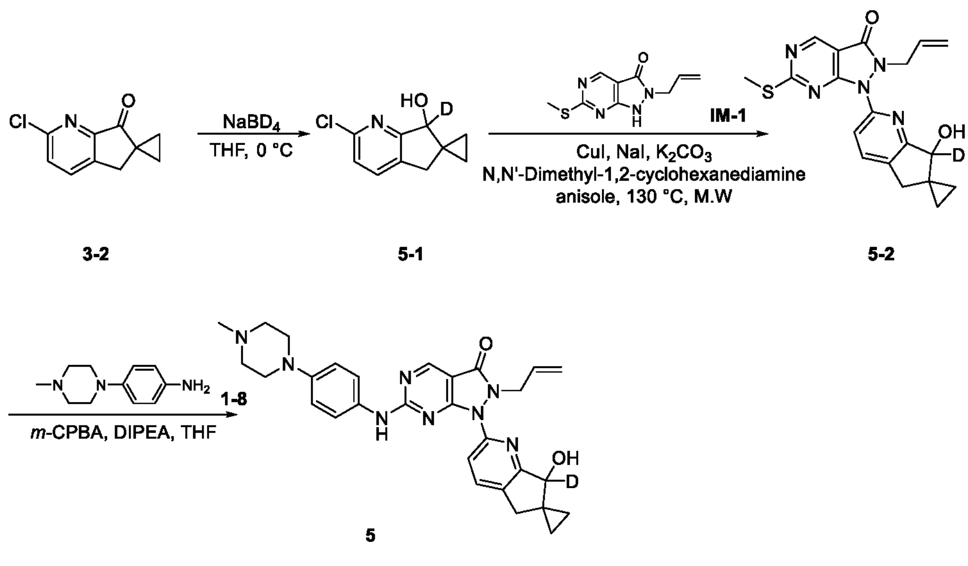


3-2 5-1 5-2

5

**Step 1: Synthesis of Compound 5-1:**

A dry single-necked flask was added with Substrate 3-2 (40.21 mg, 207.64 μmol) and MeOH (1.2 mL). The mixture was stirred to dissolved, and then at 0°C, slowly added with $NaBD_4$ (9.56 mg, 228.40 μmol). At 0°C, the reaction was stirred for 1 hours with LC-MS monitoring. After the reaction was completed, the mixture was added with water and extracted with EA for three times. The organic phase was combined, treated with anhydrous sodium sulfate, and then filtered and dried under evaporation, purified by reverse phase column chromatography to give the product 5-1 (37 mg, 188.15 μmol, 90.62% yield), LCMS (ESI$^+$) m/z: 196 $[M+H]^+$.

**Step 2: Synthesis of Compound 5-2:**

A dry single-necked flask was added with Substrate **5-1** (37 mg, 188.15 μmol) and anisole (2 mL). The mixture was stirred to dissolved, and then added with **IM-1** (50.18 mg, 225.78 μmol), CuI (71.67 mg, 376.30 μmol), $K_2CO_3$ (65.01 mg, 470.38 μmol), N,N'-dimethyl-1,2-cyclohexylene diamine (107.05 mg, 752.60 μmol) and NaI (56.40 mg, 376.30 μmol), and subjected to microwave at 130°C. The reaction was stirred for 5 hours with LC-MS monitoring. After the reaction was completed, the mixture was added with water and extracted with EA for three times. The organic phase was combined, treated with anhydrous sodium sulfate, and then filtered and dried under evaporation, purified by reverse phase column chromatography to give the product **5-2** (13 mg, 34.08 μmol, 18.11% yield), LCMS (ESI$^+$) m/z: 383 $[M+H]^+$.

**Step 3: Synthesis of Compound 5:**

Following the synthesis method of Example **3** Step **4,** the same synthesis method was performed, expect that in Step **4, 3-4** was replaced with **5-2** (13 mg, 33.99 μmol), to give Compound 5 (8.5 mg, 15.28 μmol, 44.96% yield). **$^1$H NMR** (600 MHz, DMSO-$d_6$) δ 10.14 (s, 1H), 8.82 (s, 1H), 7.94 (s, 1H), 7.68 (d, J=7.8 Hz, 1H), 7.59 (s, 2H), 6.91 (d, *J*=8.4 Hz, 2H), 5.69 - 5.67 (m, 1H), 5.35 (s, 1H), 5.02 (d, *J*=10.2 Hz, 1H), 4.90 (d, *J*=16.8 Hz, 1H), 4.63 - 4.49 (m, 2H), 3.10 - 3.08(m, 4H), 3.07 - 3.04 (m, 1H), 2.78 (d, *J*=18.0 Hz, 1H), 2.47 -2.45 (m, 4H), 2.22 (s, 3H), 1.01 - 0.98(m, 1H), 0.69 (t, *J*=7.8 Hz, 2H), 0.53 - 0.49(m, 1H). LCMS (ESI$^+$) m/z: 526.4 $[M+H]^+$, HPLC Method B: $R_T$=6.47 min, purity: 96.2%.

**Example 6: Synthesis of Compound 6**

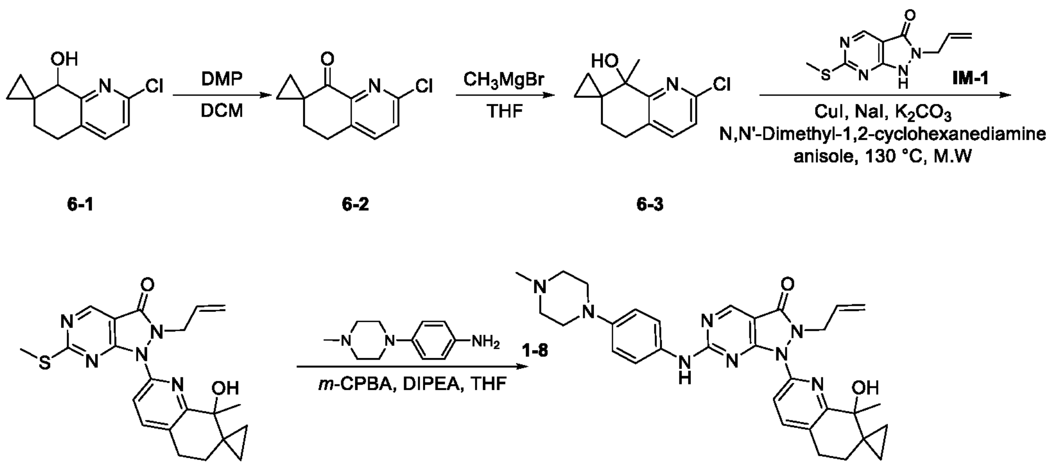


6-1 6-2 6-3

6-4 6

**Step 1: Synthesis of Compound 6-2:**

A dry single-necked flask was added with Substrate **6-1** (157 mg, 748.79 μmol) and DCM (10 mL). The mixture was stirred to dissolved, and then added with DMP (635.18 mg, 1.50 mmol). At 20°C, the reaction was stirred for 16 hours with LC-MS monitoring. After the reaction was completed, the mixture was added with saturated sodium bicarbonate solution and extracted with DCM for three times. The organic phase was combined, treated with anhydrous sodium sulfate, and then filtered and dried under evaporation, purified by reverse phase column chromatography to give the product **6-2** (141 mg, 679.01 μmol, 90.68% yield), LCMS (ESI$^+$) m/z: 208.2 $[M+H]^+$.

**Step 2: Synthesis of Compound 6-3:**

Following the synthesis method of Example **3** Step **2,** the same synthesis method was performed, expect that in Step **2, 3-2** was replaced with **6-2** (141 mg, 679.01 μmol), to give Compound **6-3** (150 mg, crude), LCMS (ESI$^+$) m/z: 224.2 [M+H]$^+$.

**Step 3: Synthesis of Compound 6-4:**

Following the synthesis method of Example **3** Step **3,** the same synthesis method was performed, expect that in Step **3, 3-3** was replaced with **6-3** (95.39 mg, 429.15 μmol), to give Compound **6-4** (75 mg, 183.15 μmol, 51.21% yield), LCMS (ESI$^+$) m/z: 410.4 [M+H]$^+$.

**Step 4: Synthesis of Compound 6:**

Following the synthesis method of Example **3** Step **4,** the same synthesis method was performed, expect that in Step **4, 3-4** was replaced with **6-4** (45 mg, 109.89 μmol), to give Compound **6** (28.5 mg, 51.56 μmol, 45.97% yield). **$^1$H NMR** (600 MHz, DMSO-$d_6$) δ 10.12 (s, 1H), 8.82 (s, 1H), 7.79 (s, 1H), 7.69 (d, *J* = 8.4 Hz, 1H), 7.59 (s, 2H), 6.93 (d, *J* = 8.4 Hz, 2H), 5.70 - 5.64 (m, 1H), 5.04 - 4.95 (m, 1H), 4.86 (d, *J* = 17.4 Hz, 1H), 4.81 (d, *J* = 15.6 Hz, 1H), 4.69 (s, 1H), 4.69 - 4.63 (m, 1H), 3.10 (t, *J* = 4.8 Hz, 4H), 2.90 - 2.86 (m, 1H), 2.81 - 2.79 (m, 1H), 2.47 (s, 4H), 2.23 (s, 3H), 2.01 - 1.96 (m, 1H), 1.48 - 1.39 (m, 4H), 0.86 - 0.85 (m, 1H), 0.64 - 0.62 (m, 1H), 0.34 - 0.31 (m, 1H), 0.22 - 0.21 (m, 1H). LCMS (ESI$^+$) m/z: 553.2 [M+H]$^+$, HPLC Method B: $R_T$=7.91 min, purity:99.7%.

Compound **6** was resolved by SFC to give the following two compounds: **6a** $R_T$= 2.75 min; **6b** $R_T$=4.41 min

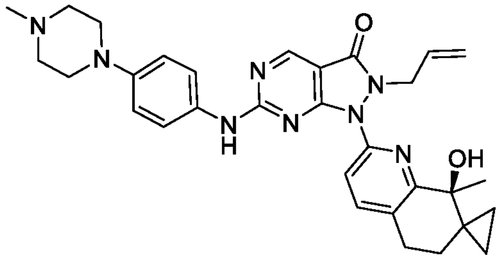

**6a**

,

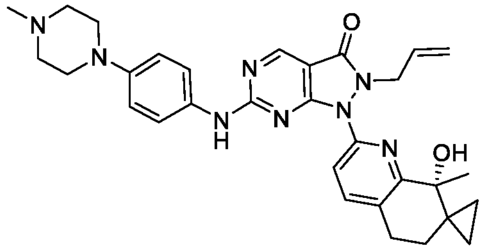

**6b**

**6a** structure characterization: **$^1$H NMR** (400 MHz, DMSO-$d_6$) δ 10.11 (s, 1H), 8.82 (s, 1H), 7.79 (d, *J* = 8.4 Hz, 1H), 7.69 (d, *J* = 8.4 Hz, 1H), 7.58 (d, *J* = 8.4 Hz, 2H), 6.99 - 6.85 (m, 2H), 5.72 - 5.62 (m, 1H), 5.00 - 4.98 (m, 1H), 4.98 - 4.84 (m, 1H), 4.79 (s, 1H), 4.69 (d, *J* = 1.8 Hz, 1H), 4.68 - 4.66 (m, 1H), 3.10 (t, *J* = 4.8 Hz, 4H), 2.89 - 2.78 (m, 2H), 2.46 (t, *J* = 4.8 Hz, 4H), 2.22 (s, 3H), 1.99 - 1.97 (m, 1H), 1.46 - 1.38 (m, 4H), 0.87 - 0.85 (m, 1H), 0.65 - 0.61 (m, 1H), 0.33 - 0.30 (m, 1H), 0.23 - 0.20 (m, 1H). LCMS (ESI$^+$) m/z: 553.2 [M+H]$^+$, HPLC Method B: $R_T$=7.91 min, purity:97.9%.

**6b** structure characterization: **$^1$H NMR** (400 MHz, DMSO-$d_6$) δ 10.11 (s, 1H), 8.82 (s, 1H), 7.79 (d, *J* = 8.4 Hz, 1H), 7.69 (d, *J* = 8.4 Hz, 1H), 7.67 - 7.49 (m, 2H), 6.92 (d, *J* = 8.8 Hz, 2H), 5.72 - 5.62 (m, 1H), 5.03 - 4.94 (m, 1H), 4.88 - 4.84 (m, 1H), 4.79 (s, 1H), 4.69 (s, 1H), 4.67 - 4.65 (m, 1H), 3.10 (t, *J* = 4.8 Hz, 4H), 2.93 - 2.77 (m, 2H), 2.46 (t, *J* = 4.8 Hz, 4H), 2.22 (s, 3H), 1.99 - 1.97 (m, 1H), 1.47 - 1.36 (m, 4H), 0.87 - 0.84 (m, 1H), 0.65 - 0.62 (m, 1H), 0.33 - 0.30 (m, 1H), 0.22 - 0.20 (m, 1H). LCMS (ESI$^+$) m/z: 553.2 [M+H]$^+$, HPLC Method B: $R_T$=7.91 min, purity:99.1%.

**Example 7: Synthesis of Compound 7**

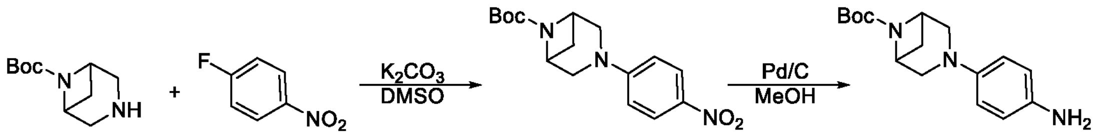


7-1 7-2 7-3 7-4

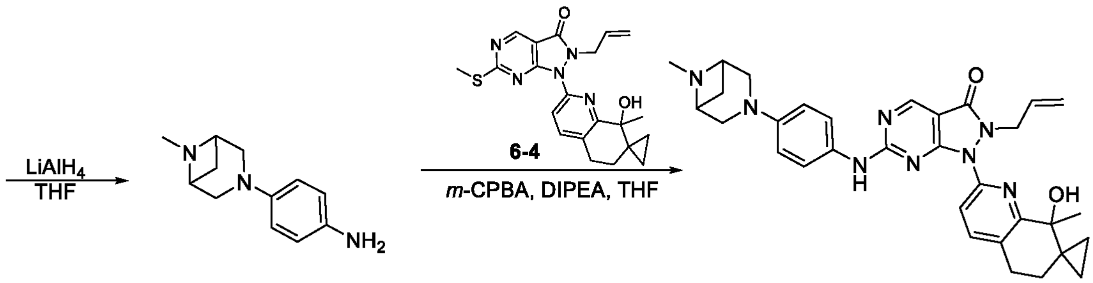


7-5 7

**Step 1: Synthesis of Compound 7-3:**

A dry single-necked flask was added with Substrate **7-1** (400 mg, 2.02 mmol) and DMSO (5 mL). The mixture was stirred to dissolved, and then added with **7-2** (313.14 mg, 2.22 mmol) and potassium carbonate (836.50 mg, 6.05 mmol). At 65°C, the reaction was stirred for 10 hours with LC-MS monitoring. After the reaction was completed, the mixture was added with water and extracted with EA for three times. The organic phase was combined, treated with anhydrous sodium sulfate, and then filtered and dried under evaporation, purified by reverse phase column chromatography to give the product **7-3** (633 mg, 1.98 mmol, 98.24% yield), LCMS (ESI$^+$) m/z: 319 $[M+H]^+$.

**Step 2: Synthesis of Compound 7-4:**

A dry single-necked flask was added with Substrate **7-3** (633 mg, 1.98 mmol) and methanol (5 mL). The mixture was stirred to dissolved, and then added with Pd/C (60 mg, 494.03 μmol). Under $H_2$, the reaction was stirred under room temperature for 12 hours with LC-MS monitoring. After the reaction was completed, the reaction solution was filtrated with celite, and the filtrate was dried under evaporation, to give crude product **7-4** (459 mg, crude), LCMS (ESI$^+$) m/z: 290 $[M+H]^+$.

**Step 3: Synthesis of Compound 7-5:**

A dry single-necked flask was added with Substrate **7-4** (150 mg, 518.36 μmol) and THF (5 mL). The mixture was stirred to dissolved, and then at 0°C, slowly added with $LiAlH_4$ (19.67 mg, 518.36 μmol). The at 65°C, the reaction was stirred for 4 hours with LC-MS monitoring. After the reaction was completed, at 0°C added with water to quench the reaction. After treated with 10% sodium hydroxide solution, the reaction was further added with water, filtrated, and the filtrate was dried under evaporation, purified by reverse phase column chromatography to give the product **7-5** (70 mg, 0.34 mmol, 66.43% yield), LCMS (ESI$^+$) m/z: 204 $[M+H]^+$.

**Step 4: Synthesis of Compound 7:**

Following the synthesis method of Example **3** Step **4,** the same synthesis method was performed, expect that in Step **4, 3-4** was replaced with **6-4** (15 mg, 36.63 μmol), and **1-8** was replaced with **7-5** (8.94 mg, 43.96 μmol), to give Compound 7 (10.4 mg, 16.21 μmol, 44.25% yield). **$^1$H NMR** (600 MHz, DMSO-$d_6$) δ 10.06 (s, 1H), 8.79 (s, 1H), 7.76 (d, *J* = 8.4 Hz, 1H), 7.71 (d, *J* = 8.4 Hz, 1H), 7.66 - 7.47 (m, 2H), 6.72 (d, *J* = 8.4 Hz, 2H), 5.72 - 5.63 (m, 1H), 4.99 (d, *J* = 10.2 Hz, 1H), 4.86 (d, *J* = 17.4 Hz, 1H), 4.80 (s, 1H), 4.68 (s, 1H), 4.64 (d, *J* = 15.6 Hz, 1H), 3.75 - 3.54 (m, 2H), 3.46 (d, *J* = 10.8 Hz, 2H), 2.88 - 2.77 (m, 2H), 2.50 (s, 3H), 2.12 - 1.92 (m, 4H), 1.57 (s, 1H), 1.45 - 1.39 (m, 4H), 0.87 - 0.85 (m, 1H), 0.65 - 0.61 (m, 1H), 0.33 - 0.29 (m, 1H), 0.26 - 0.19 (m, 1H). LCMS (ESI$^+$) m/z: 565.2 $[M+H]^+$, HPLC Method B: $R_T$=8.49 min, purity:88.0%.

Compound **7** was resolved by SFC to give the following two compounds: **7a** $R_T$= 3.564 min; **7b** $R_T$= 6.246 min

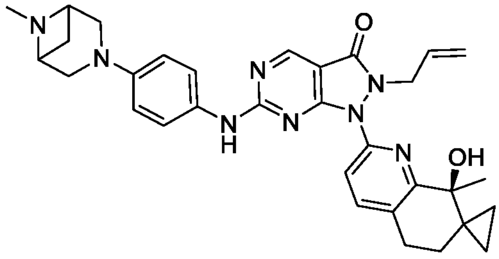

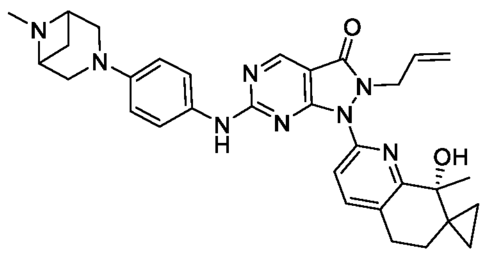

7a 7b

Compound **7a**(8.3 mg, 13.52 μmol); SFC residence time t = 3.564 min. **$^1$H NMR** (400 MHz, DMSO-$d_6$) δ 10.06 (s, 1H), 8.80 (s, 1H), 7.76 (d, *J* =8.4 Hz, 1H), 7.70 (d, *J* =8.4 Hz, 1H), 7.59 (s, 2H), 6.71 (d, *J* =8.8 Hz, 2H), 5.72 - 5.62 (m, 1H), 5.00 - 4.98 (m, 1H), 4.88 - 4.79 (m, 2H), 4.68(s, 1H), 4.67 - 4.61 (m, 1H), 4.80 - 4.77 (m, 1H), 3.58 (d, *J* =5.2 Hz, 2H), 3.43 (d, *J* =10.8 Hz, 2H), 3.27 (d, *J* =10.8 Hz, 2H), 2.92 - 2.76 (m, 2H), 2.46 - 2.41 (m, 1H), 2.02 - 1.94 (m, 4H), 1.53 (d, *J* =8.0 Hz, 1H), 1.46 - 1.42 (m, 1H), 1.40 (s, 3H), 0.87-0.84 (m, 1H), 0.68 - 0.60 (m, 1H), 0.34 - 0.30 (m, 1H), 0.23 - 0.19 (m, 1H). LCMS (ESI) m/z: 565.4 $[M+H]^+$, HPLC Method B: $R_T$=8.36 min, purity> 92.1%.

Compound **7b**(8.3 mg, 13.91 μmol); SFC residence time t = 6.246 min. **$^1$H NMR** (400 MHz, DMSO-$d_6$) δ 10.06 (s, 1H), 8.79 (s, 1H), 7.76 (d, *J* =8.4 Hz, 1H), 7.70 (d, *J* =8.4 Hz, 1H), 7.59 (s, 2H), 6.71 (d, *J* = 8.8 Hz, 2H), 5.72 - 5.62 (m, 1H), 5.00 - 4.98 (m, 1H), 4.88 - 4.79 (m, 2H), 4.68 (s, 1H), 4.67 - 4.61 (m, 1H), 4.80 - 4.77 (m, 1H), 3.58 (d, *J* =5.2 Hz, 2H), 3.43 (d, *J* =10.8 Hz, 2H), 3.27 (d, *J* =10.8 Hz, 2H), 2.92 - 2.76 (m, 2H), 2.45 - 2.40 (m, 1H), 2.02 - 1.95 (m, 4H), 1.53 (d, *J* =8.0 Hz, 1H), 1.46 - 1.42 (m, 1H), 1.40 (s, 3H), 0.87 - 0.84 (m, 1H), 0.68 - 0.60 (m, 1H), 0.34 - 0.30 (m, 1H), 0.23 - 0.19 (m, 1H). LCMS (ESI) m/z: 565.4 $[M+H]^+$, HPLC Method B: $R_T$=8.30 min, purity> 95.2%.

**Example 8: Synthesis of Compound 8**

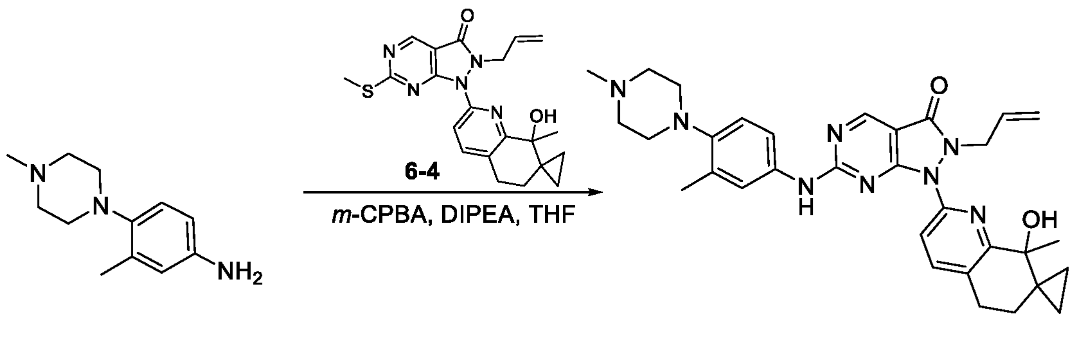


8-1 8

**Step 1: Synthesis of Compound 8:**

Following the synthesis method of Example **3** Step **4,** the same synthesis method was performed, expect that in Step **4, 3-4** was replaced with **6-4** (15 mg, 36.63 μmol), and **1-8** was replaced with **8-1** (9.02 mg, 43.96 μmol), to give Compound **8** (7.4 mg, 12.14 μmol, 33.15% yield). **$^1$H NMR** (400 MHz, DMSO-$d_6$) δ 10.12 (s, 1H), 8.84 (s, 1H), 7.76 (d, *J* = 8.4 Hz, 1H), 7.72 (s, 1H), 7.70 (s, 1H), 7.45 - 7.36 (m, 1H), 6.99 (d, *J* = 8.4 Hz, 1H), 5.72 - 5.63 (m, 1H), 5.00 - 4.98 (m, 1H),, 4.91 - 4.83 (m, 1H), 4.83 - 4.75 (m, 1H), 4.70 (s, 1H), 4.67 - 4.61 (m, 1H), 2.90 - 2.77 (m, 6H), 2.52 - 2.45 (m, 4H), 2.25 (s, 6H), 2.01 - 1.97 (m, 1H), 1.47 - 1.38 (m, 4H), 0.87 - 0.84 (m, 1H), 0.65 - 0.60 (m, 1H), 0.36 - 0.29 (m, 1H), 0.23 - 0.19 (m, 1H). LCMS ($ESI^+$) m/z: 567.2 $[M+H]^+$, HPLC Method B: $R_T$=8.83 min, purity:93.0%.

**Example 9: Synthesis of Compound 9**

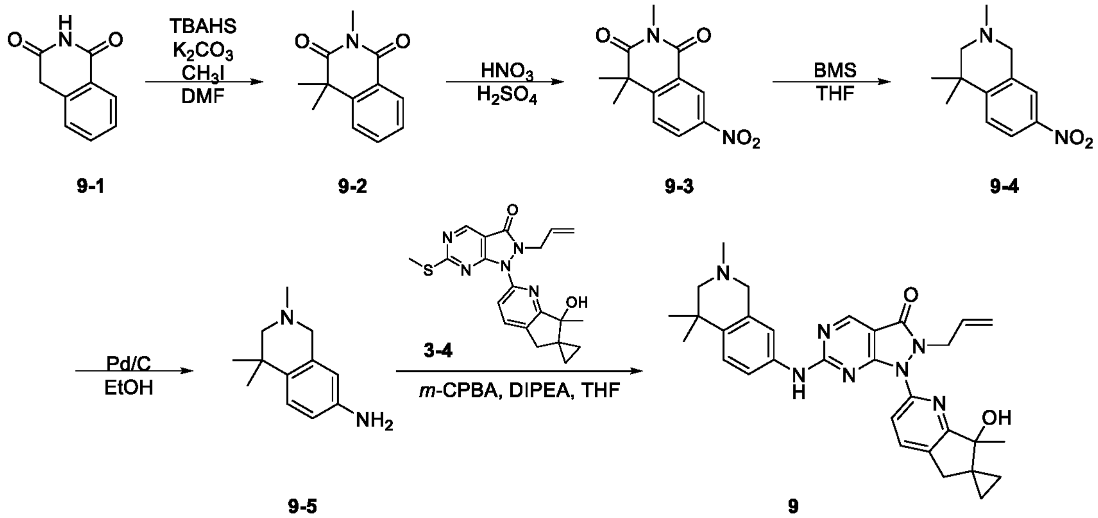


9-1 9-2 9-3 9-4 9-5 9

**Step 1: Synthesis of Compound 9-2:**

A dry single-necked flask was added with Substrate **9-1** (3 g, 18.62 mmol) and DMF (20 mL). The mixture was stirred to dissolved, and then added with iodomethane (8.7 g, 61.43 mmol), TBAHS (630 mg, 18.62 mmol) and potassium carbonate (8.48 g, 61.43 mmol). At 70°C, the reaction was stirred for 15 hours with LC-MS monitoring. After the reaction was completed, the mixture was added with water to quench the reaction, added with extracted with EA for three times. The organic phase was combined, treated with anhydrous sodium sulfate, and then filtered and dried under evaporation, purified by reverse phase column chromatography to give the product **9-2** (3.4 g, 16.73 mmol, 89.87% yield), LCMS (ESI$^+$) m/z: 204 $[M+H]^+$.

**Step 2: Synthesis of Compound 9-3:**

A dry single-necked flask was added with Substrate **9-2** (3.4 g, 16.73 mmol) and $H_2SO_4$ (10 mL). The mixture was stirred to dissolved, and then at 0°C, slowly dropwise added with $HNO_3$ (1.02 g, 16.24 mmol). At 0°C, the reaction was stirred with TLC monitoring. After the reaction was completed, 0°C added with water to quench the reaction, added with extracted with EA for three times. The organic phase was combined, treated with anhydrous sodium sulfate, and then filtered and dried under evaporation, purified by reverse phase column chromatography to give the product **9-3** (3.9 g, 15.71 mmol, 96.76% yield).

**Step 3: Synthesis of Compound 9-4:**

A dry single-necked flask was added with Substrate **9-3** (270 mg, 1.09 mmol) and THF (10 mL). The mixture was stirred to dissolved, and then added with BMS (334.8 mg, 4.35 mmol)). At 70°C, the reaction was stirred for 24 hours with LC-MS monitoring. After the reaction was completed, the mixture was added with saturated sodium sulfite solution to quench the reaction, added with extracted with EA for three times. The organic phase was combined, treated with anhydrous sodium sulfate, and then filtered and dried under evaporation, purified by reverse phase column chromatography to give the product **9-4** (200 mg, 907.99 μmol, 83.48% yield), LCMS (ESI$^+$) m/z: 220 $[M+H]^+$.

**Step 4: Synthesis of Compound 9-5:**

A dry single-necked flask was added with Substrate **9-4** (200 mg, 907.99 μmol) and ethanol (10 mL). The mixture was stirred to dissolved, and then added with Pd/C (110.28 mg, 907.99 μmol). Under $H_2$, the reaction was stirred under room temperature for 4 hours with LC-MS monitoring. After the reaction was completed, the reaction solution was filtrated with celite, and the filtrate was dried under evaporation, to give crude product **9-5** (170 mg, crude), LCMS (ESI$^+$) m/z: 191 $[M+H]^+$.

**Step 5: Synthesis of Compound 9:**

Following the synthesis method of Example **3** Step **4,** the same synthesis method was performed, expect that in Step **4, 1-8** was replaced with **9-5** (8.66 mg, 45.51 μmol), to give Compound **9** (1.7 mg, 2.98 μmol, 7.84% yield). **$^1$H NMR**

(400 MHz, DMSO-$d_6$) δ 10.18 (s, 1H), 8.87 (s, 1H), 7.88 (d, $J$ = 8.0 Hz, 1H), 7.71 (d, $J$ = 8.0 Hz, 1H), 7.54 (s, 1H), 7.42 (d, $J$ = 8.4 Hz, 1H), 7.29 (d, $J$ = 8.4 Hz, 1H), 5.75 - 5.65 (m, 1H), 5.04 (s, 1H), 5.04 - 5.00 (m, 1H), 4.91 - 4.86 (m, 1H), 4.80 - 4.67 (m, 1H), 4.61 - 4.56 (m, 1H), 3.45 - 3.37 (m, 2H), 2.91 (s, 2H), 2.43 - 2.28 (m, 5H), 1.26 - 1.20 (m, 9H), 0.95 - 0.90 (m, 1H), 0.72 - 0.67 (m, 1H), 0.61 - 0.56 (m, 1H), 0.49 - 0.45 (m, 1H). LCMS (ESI$^+$) m/z: 538.3 [M+H]$^+$, HPLC Method B: $R_T$=8.20 min, purity:94.1%.

**Example 10: Synthesis of Compound 10**

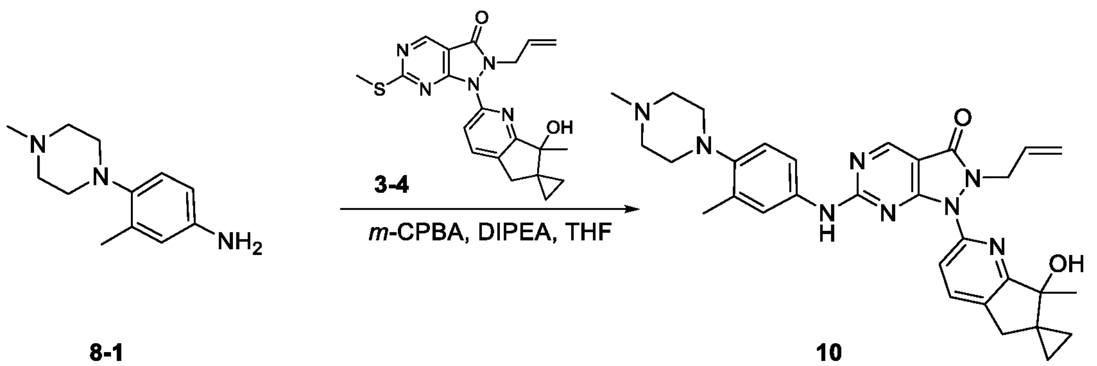


8-1 10

**Step 1: Synthesis of Compound 10:**

Following the synthesis method of Example **3** Step **4,** the same synthesis method was performed, expect that in Step **4, 1-8** was replaced with **8-1** (9.34 mg, 45.51 μmol), to give Compound **10** (7.6 mg, 13.11 μmol, 34.55% yield). **$^1$H NMR** (400 MHz, DMSO-$d_6$) δ 10.14 (s, 1H), 8.85 (s, 1H), 7.89 (d, $J$ = 8.0 Hz, 1H), 7.72 (d, $J$ = 8.0 Hz, 1H), 7.66 (s, 1H), 7.47 - 7.39 (m, 1H), 6.99 (d, $J$ = 8.4 Hz, 1H), 5.75 - 5.65 (m, 1H), 5.03 (s, 1H), 5.03 - 4.97 (m, 1H), 4.90 - 4.86 (m, 1H), 4.74 (d, $J$ = 15.6 Hz, 1H), 4.63- 4.57 (m, 1H), 2.89 (d, $J$ = 2.4 Hz, 2H), 2.83 (t, $J$ = 4.8 Hz, 4H), 2.58 - 2.51 (m, 4H), 2.28 (s, 3H), 2.24 (s, 3H), 1.22 (s, 3H), 0.95 - 0.90 (m, 1H), 0.71 - 0.66 (m, 1H), 0.60 - 0.56 (m, 1H), 0.48 - 0.45 (m, 1H). LCMS (ESI$^+$) m/z: 553.4 [M+H]$^+$, HPLC Method B: $R_T$=7.62 min, purity:95.3%.

**Example 11: Synthesis of Compound 11**

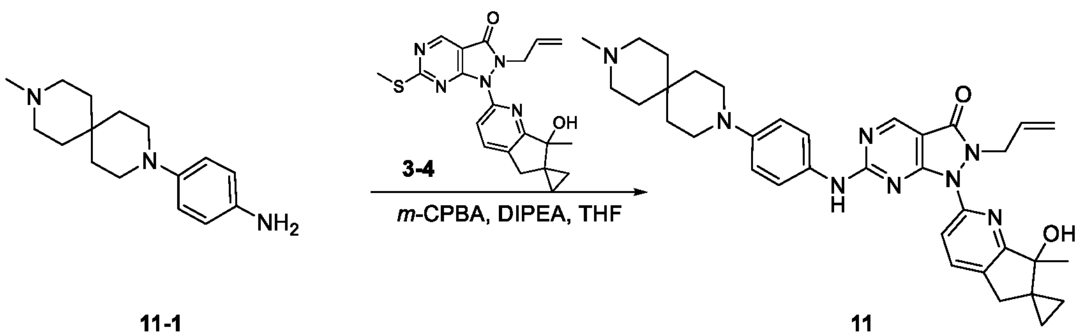


11-1 11

**Step 1: Synthesis of Compound 11:**

Following the synthesis method of Example **3** Step **4,** the same synthesis method was performed, expect that in Step **4, 1-8** was replaced with **11-1** (15.74 mg, 60.69 μmol), to give Compound **11** (5.33 mg, 8.16 μmol, 16.14% yield). **$^1$H NMR** (600 MHz, DMSO-$d_6$) δ 10.12 (s, 1H), 8.82 (s, 1H), 7.91 (s, 1H), 7.69 (d, $J$ = 8.4 Hz, 1H), 7.58 (s, 2H), 6.91 (d, $J$ = 8.4 Hz, 2H), 5.72 - 5.66 (m, 1H), 5.03 - 4.99 (m, 2H), 4.87 (d, $J$ = 17.4 Hz, 1H), 4.76 - 4.73 (m, 1H), 4.61 - 4.59 (m 1H), 3.09 - 3.07 (m, 4H), 2.92 - 2.86 (m, 2H), 2.43 - 2.39 (m, 4H), 2.64 (s, 3H), 1.55 - 1.49 (m, 8H), 1.22 (s, 3H), 0.94 - 0.91 (m, 1H), 0.70 - 0.67 (m, 1H), 0.59 - 0.56 (m, 1H), 0.48 - 0.46 (m, 1H). LCMS (ESI$^+$) m/z: 607.3 [M+H]$^+$, HPLC Method B: $R_T$=8.06 min, purity: 93.4%.

Compound **11** was resolved by SFC to give the following two compounds: **11a** $R_T$= 3.475 min; **11b** $R_T$= 5.377min

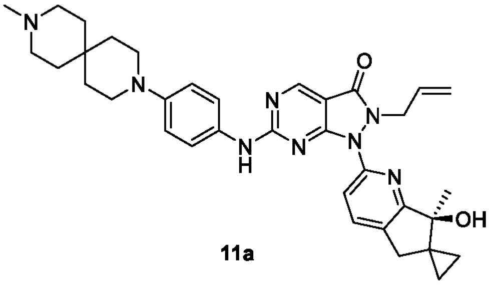

**11a**

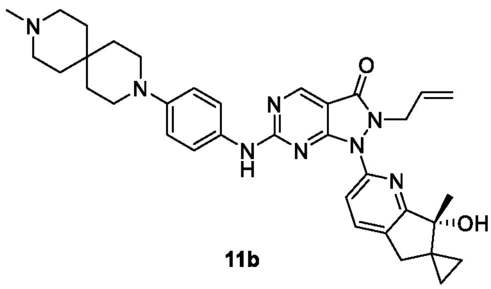

**11b**

**Compound 11a**(12.86 mg, 21.19 μmol); SFC residence time t = 3.475 min. **$^1$H NMR** (400 MHz, DMSO-$d_6$) δ 10.11 (s, 1H), 8.82 (s, 1H), 7.91 (d, *J* = 7.2 Hz, 1H), 7.70 (d, *J* = 8.0 Hz, 1H), 7.56 (s, 2H), 6.91 (d, *J* = 9.2 Hz, 2H), 5.74 - 5.64 (m, 1H), 5.02 - 4.99 (m, 2H), 4.88 (dd, *J* = 17.2, 1.2 Hz, 1H), 4.72 - 4.58 (m, 2H), 3.08 (t, *J* = 5.6 Hz, 4H), 2.94 - 2.85 (m, 2H), 2.29 (s, 4H), 2.16 (s, 3H), 1.53 (t, *J* = 5.6 Hz, 4H), 1.46 (t, *J* = 5.6 Hz, 4H), 1.22 (s, 3H), 0.95 - 0.90 (m, 1H), 0.71 - 0.66 (m, 1H), 0.60 - 0.55 (m, 1H), 0.48 - 0.43 (m, 1H). LCMS (ESI) m/z: 607.3 [M+H]$^+$, HPLC Method B: $R_T$=8.35 min, purity> 94.6%.

Compound **11b**(12.1 mg, 19.94 μmol); SFC residence time t = 5.377 min. **$^1$H NMR** (400 MHz, DMSO-$d_6$) δ 10.11 (s, 1H), 8.82 (s, 1H), 7.91 (d, *J* = 7.2 Hz, 1H), 7.69 (d, *J* = 8.0 Hz, 1H), 7.56 (s, 2H), 6.91 (d, *J* = 9.2 Hz, 2H), 5.74 - 5.64 (m, 1H), 5.02 - 4.99 (m, 2H), 4.88 (dd, *J* = 17.2, 1.2 Hz, 1H), 4.72 - 4.58 (m, 2H), 3.08 (t, *J* = 5.6 Hz, 4H), 2.94 - 2.85 (m, 2H), 2.29 (s, 4H), 2.16 (s, 3H), 1.53 (t, *J* = 5.6 Hz, 4H), 1.46 (t, *J* = 5.6 Hz, 4H), 1.22 (s, 3H), 0.95 - 0.90 (m, 1H), 0.71 - 0.66 (m, 1H), 0.60 - 0.55 (m, 1H), 0.48 - 0.43 (m, 1H). LCMS (ESI) m/z: 607.3 [M+H]$^+$, HPLC Method B: $R_T$= 7.89 min, purity> 97.2%.

**Example 12: Synthesis of Compound 12**

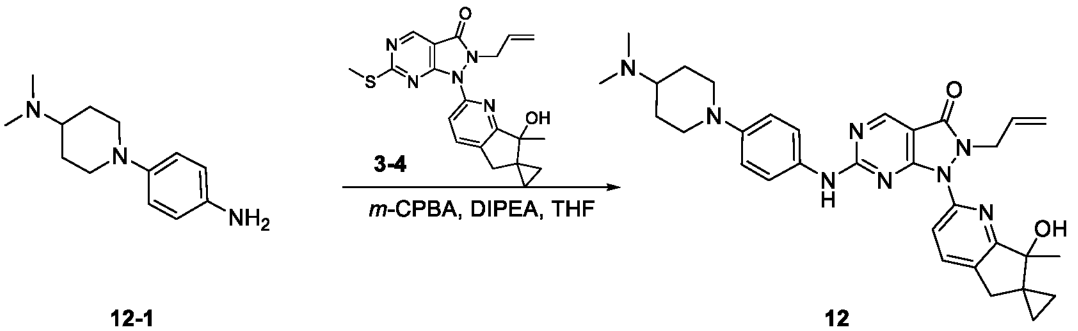


**12-1**

**12**

**Step 1: Synthesis of Compound 12:**

Following the synthesis method of Example **3** Step **4,** the same synthesis method was performed, expect that in Step **4, 1-8** was replaced with **12-1** (16.64 mg, 75.86 μmol), to give Compound **12** (9.38 mg, 15.64 μmol, 30.93% yield). **$^1$H NMR** (600 MHz, DMSO-$d_6$) δ 10.14 (s, 1H), 8.82 (s, 1H), 7.92 - 7.90 (m, 1H), 7.69 (d, *J* = 7.8 Hz, 1H), 7.58 (s, 2H), 6.92 (d, *J* = 9.0 Hz, 2H), 5.72 - 5.66 (m, 1H), 5.03 - 4.99 (m, 2H), 4.87 (d, *J* = 17.4 Hz, 1H), 4.73 (s, 1H), 4.61 - 4.58 (m, 1H), 3.68 (d, *J* = 12.0 Hz, 2H), 2.92 - 2.86 (m, 2H), 2.63 (t, *J* = 12.0 Hz, 2H), 2.38 - 2.32 (m, 7H), 1.91 - 1.87 (m, 2H), 1.55 - 1.49 (m, 2H), 1.22 (s, 3H), 0.94 - 0.91 (m, 1H), 0.70 - 0.67 (m, 1H), 0.59 - 0.56 (m, 1H), 0.48 - 0.46 (m, 1H). LCMS (ESI$^+$) m/z: 567.2 [M+H]$^+$, HPLC Method B: $R_T$=7.29 min, purity: 93.3%.

**Example 13: Synthesis of Compound 13**

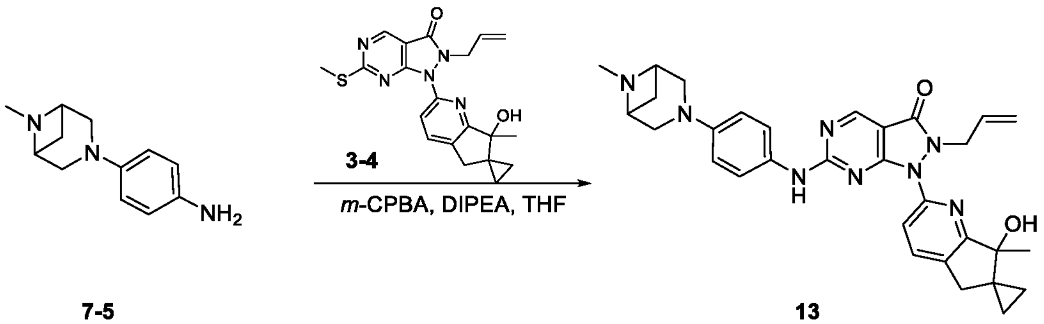


7-5 13

**Step 1: Synthesis of Compound 13:**

Following the synthesis method of Example **3** Step **4,** the same synthesis method was performed, expect that in Step **4, 1-8** was replaced with **7-5** (19.76 mg, 97.21 μmol), to give Compound **13** (14 mg, 22.88 μmol, 47.08% yield). **$^1$H NMR** (600 MHz, Chloroform-*d*) δ 8.81 (s, 1H), 7.69 - 7.67 (m, 2H), 7.49 (s, 2H), 6.72 (d, *J* = 9.0 Hz, 2H), 5.74 - 5.68 (m, 1H), 5.02 (d, *J* = 9.6 Hz, 1H), 4.94 (d, *J* = 17.4 Hz, 1H), 4.84 - 4.80 (m, 2H), 4.03 (s, 2H), 3.64 - 3.58 (m, 4H), 2.95 (d, *J* = 6.6 Hz, 2H), 2.32 (s, 3H), 1.77 (s, 2H), 1.41 (s, 3H), 1.12 - 1.08 (m, 1H), 0.91 - 0.87 (m, 1H), 0.70 - 0.67 (m, 1H), 0.60 - 0.56 (m, 1H). LCMS (ESI$^+$) m/z: 551.2 [M+H]$^+$, HPLC Method B: $R_T$= 7.16 min, purity: 90.0%.

**Example 14: Synthesis of Compound 14**

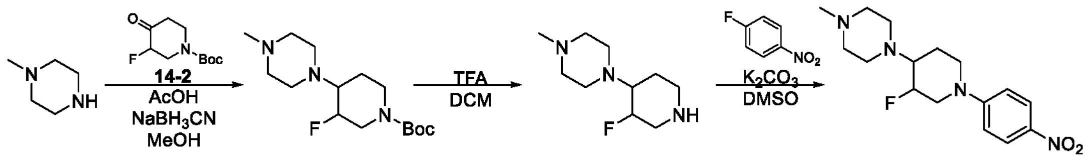


14-1 14-3 14-4 14-5

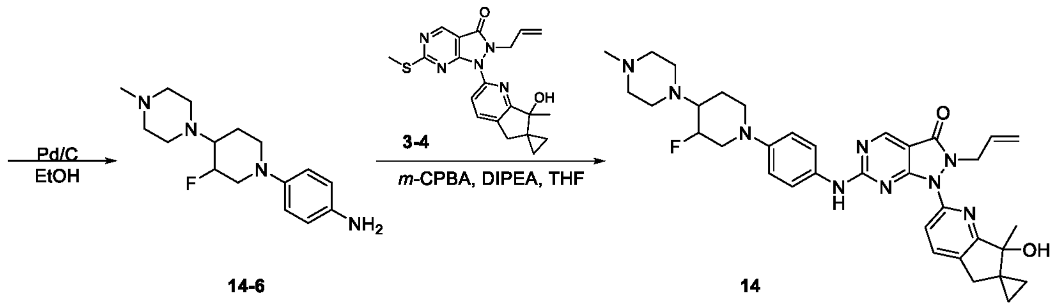


14-6 14

**Step 1: Synthesis of Compound 14-3:**

A dry single-necked flask was added with Substrate **14-1** (217.24 mg, 1 mmol). The mixture was dissolved with methanol (3 mL), added with **14-2** (200.32 mg, 2 mmol), acetic acid (3.00 mg, 50.00 μmol). The reaction was stirred under room temperature for 0.5 hours, added with $NaBH_3CN$ (94.26 mg, 1.50 mmol), and then heated to 50°C to react 4 hours with LC-MS monitoring. After the reaction was completed, the solvent was evaporated, added with water and extracted with EA for three times. The organic phase was combined, treated with anhydrous sodium sulfate, and then filtered and dried under evaporation, purified by reverse phase column chromatography to give the product **14-3** (200 mg, 663.57 μmol, 66.36% yield), LCMS (ESI$^+$) m/z: 302 [M+H]$^+$.

**Step 2: Synthesis of Compound 14-4:**

A dry single-necked flask was added with Substrate **14-3** (200 mg, 663.57 μmol). The mixture was dissolved with DCM (1 mL), added with TFA (756.61 mg, 6.64 mmol). The reaction was stirred under room temperature for 0.5 hours with LC-MS monitoring. After the reaction was completed, the solvent was evaporated to give crude product **14-4** (100 mg, 496.81 μmol, 74.87% yield), LCMS (ESI+) m/z: 202 [M+H]+.

**Step 3: Synthesis of Compound 14-5:**

A dry single-necked flask was added with Substrate **14-4** (140 mg, 695.54 μmol) and DMSO (3 mL). The mixture was stirred to dissolved, and then added with p-fluoro-nitrobenzene (98.14 mg, 695.54 μmol) and potassium carbonate (288.38 mg, 2.09 mmol). At 80°C, the reaction was stirred for 4 hours with LC-MS monitoring. After the reaction was completed, the mixture was added with water and extracted with EA for three times. The organic phase was combined, treated with anhydrous sodium sulfate, and then filtered and dried under evaporation, purified by reverse phase column chromatography to give the product **14-5** (95 mg, 294.69 μmol, 42.37% yield), LCMS ($ESI^+$) m/z: 322 $[M+H]^+$.

**Step 4: Synthesis of Compound 14-6:**

A dry single-necked flask was added with Substrate **14-5** (95 mg, 294.69 μmol) and methanol (5 mL). The mixture was stirred to dissolved, and then added with Pd/C (10.74 mg, 88.41 μmol). Under $H_2$, the reaction was stirred under room temperature for 12 hours with LC-MS monitoring. After the reaction was completed, the reaction solution was filtrated with celite, and the filtrate was dried under evaporation, to give crude product **14-6** (73 mg, crude), LCMS ($ESI^+$) m/z: 293 $[M+H]^+$.

Step 5: **Synthesis of Compound 14:**

Following the synthesis method of Example **3** Step **4,** the same synthesis method was performed, expect that in Step **4, 1-8** was replaced with **14-6** (15 mg, 51.30 μmol), to give Compound **14** (4.1 mg, 5.72 μmol, 22.31% yield). **$^1$H NMR** (400 MHz, DMSO-$d_6$) δ 10.13 (s, 1H), 8.82 (s, 1H), 7.91 (s, 1H), 7.70 (d, $J$ = 8.0 Hz, 1H), 7.57 (s, 2H), 6.92 (d, $J$ = 8.8 Hz, 2H), 5.75 - 7.64 (m, 1H),, 5.13 - 4.97 (m, 3H), 4.88 (d, $J$ = 17.2 Hz, 1H), 4.73 (s, 1H), 4.60 (d, $J$ = 15.2 Hz, 1H), 3.88 (t, $J$ = 12.4 Hz, 1H), 3.75 (d, $J$ = 12.4 Hz, 1H), 2.90 (d, $J$ = 2.4 Hz, 2H), 2.78 - 2.69 (m, 1H), 2.60 (s, 4H), 2.42 - 2.22 (m, 6H), 2.15 (s, 3H), 1.94 - 1.81 (m, 6H), 1.75 (d, $J$ = 12.4 Hz, 1H), 1.22 (s, 3H), 0.95 - 0.84 (m, 6H), 0.73 - 0.66 (m, 6H), 0.61 - 0.53 (m, 1H), 0.48 - 0.43 (m, 1H). LCMS ($ESI^+$) m/z: 640.3 $[M+H]^+$, HPLC Method B: $R_T$=6.63 min, purity: 89.4%.

**Example 15: Synthesis of Compound 15**

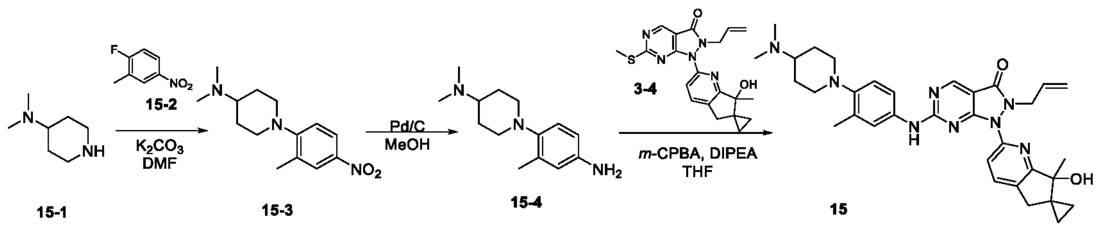


**Step 1: Synthesis of Compound 15-3:**

A dry single-necked flask was added with Substrate **15-1** (181.83 mg, 1.42 mmol) and DMF (10 mL). The mixture was stirred to dissolved, and then added with **15-2** (200 mg, 1.29 mmol) and potassium carbonate (534.53 mg, 3.87 mmol). At 90°C, the reaction was stirred for 3 hours with LC-MS monitoring. After the reaction was completed, the mixture was added with water and extracted with EA for three times. The organic phase was combined, treated with anhydrous sodium sulfate, and then filtered and dried under evaporation, to give crude product **15-3** (100 mg, crude), LCMS ($ESI^+$) m/z: 263 $[M+H]^+$.

**Step 2: Synthesis of Compound 15-4:**

A dry single-necked flask was added with Substrate **15-3** (100 mg, 379.75 μmol) and methanol (5 mL). The mixture was stirred to dissolved, and then added with Pd/C (230.60 mg, 1.90 mmol). Under $H_2$, the reaction was stirred under room temperature for 0.5 hours with LC-MS monitoring. After the reaction was completed, the reaction solution was filtrated with celite, and the filtrate was dried under evaporation, to give crude product **15-4** (109 mg, crude), LCMS ($ESI^+$) m/z: 234 $[M+H]^+$.

**Step 3: Synthesis of Compound 15:**

Following the synthesis method of Example **3** Step **4,** the same synthesis method was performed, expect that in Step **4, 1-8** was replaced with **15-4** (8.85 mg, 37.93 μmol), to give Compound **15** (1.75 mg, 2.59 μmol, 10.23% yield). **$^1$H NMR** (600 MHz, DMSO-$d_6$) δ 10.12 (s, 1H), 8.85 (s, 1H), 7.88 (d, *J* = 8.4 Hz, 1H), 7.71 (d, *J* = 8.0 Hz, 1H), 7.65 (s, 1H), 7.43 - 7.40 (m, 1H), 6.97 (d, *J* = 8.8 Hz, 1H), 5.74 - 5.66 (m, 1H), 5.03 (s, 1H), 5.03 - 5.00 (m, 1H), 4.90 - 4.85 (m, 1H), 4.77 - 4.71 (m, 1H), 4.62 - 4.57 (m, 1H), 3.03 (d, *J* =12.0 Hz, 2H), 2.89 - 2.87 (m, 2H), 2.59 - 2.53 (m, 2H), 2.24 - 2.18 (m, 10H), 1.83 (d, *J* =11.6 Hz, 2H),1.58 - 1.49 (m, 2H), 1.22 (s, 3H), 0.95 - 0.89 (m, 1H), 0.71 - 0.66 (m, 1H), 0.61 - 0.56 (m, 1H), 0.48 - 0.44(m, 1H). LCMS (ESI$^+$) m/z: 581.3 [M+H]$^+$, HPLC Method B: $R_T$=8.41 min, purity: 88.68%.

**Example 16: Synthesis of Compound 16**

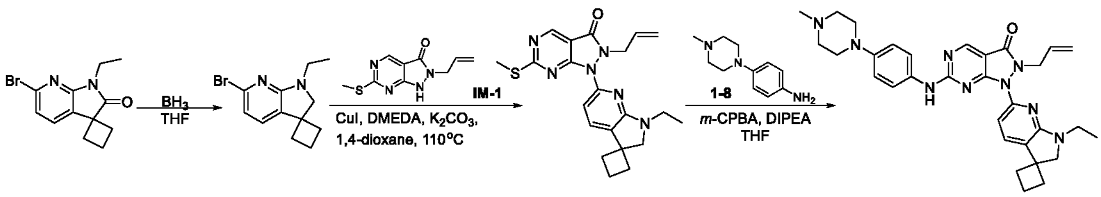


**2-5** **16-1** **16-2** **16**

**Step 1: Synthesis of Compound 16-1:**

A dry single-necked flask was added with Substrate **2-5** (60 mg, 0.21 mmol) and THF (1 mL). The mixture was stirred to dissolved, and then added with a complex of borane and tetrahydrofuran (1 mL). Under room temperature, the reaction was stirred for 5 hours with LC-MS monitoring. After the reaction was completed, the mixture was added with water to quench, and extracted with EA for three times. The organic phase was combined, treated with anhydrous sodium sulfate, and then filtered and dried under evaporation, purified by reverse phase column chromatography to give the product **16-1** (40 mg, 0.15 mmol, 70.18% yield), LCMS (ESI$^+$) m/z: 267 [M+H]$^+$.

**Step 2: Synthesis of Compound 16-2:**

Following the synthesis method of Example **2** Step **5,** the same synthesis method was performed, expect that in Step **5, 2-5** was replaced with **16-1** (40 mg, 0.15 mmol), to give Compound **16-2** (30 mg, 73.43 μmol, 48.83% yield), LCMS (ESI$^+$) m/z: 409 [M+H]$^+$.

**Step 3: Synthesis of Compound 16:**

Following the synthesis method of Example **3** Step **4,** the same synthesis method was performed, expect that in Step **4, 3-4** was replaced with **16-2** (30 mg, 73.43 μmol), to give Compound **16** (6.31 mg, 11.44 μmol, 15.58 % yield). **$^1$H NMR** (600 MHz, DMSO-$d_6$) δ 10.05 (s, 1H), 8.78 (s, 1H), 7.71 (s, 1H), 7.70 - 7.52 (m, 2H), 7.98 - 7.82 (m, 3H), 5.72 - 7.68 (m, 1H), 5.05 (d, *J* = 10.2 Hz, 2H), 4.93 (d, *J* = 16.8 Hz, 1H), 4.51 (s, 2H), 3.62 (s, 2H), 3.31 (d, *J* = 14.4 Hz, 1H), 3.08 (s, 4H), 2.46 (s, 4H), 2.45 (s, 2H), 2.28 (s, 3H), 2.20 (t, *J* = 14.4 Hz, 3H), 2.03 - 1.99 (m, 2H), 1.09 (t, *J* = 7.2 Hz, 3H). LCMS (ESI$^+$) m/z: 552.4 [M+H]$^+$, HPLC Method B: $R_T$=7.03 min, purity: 95.7%.

**Example 17: Synthesis of Compound 17**

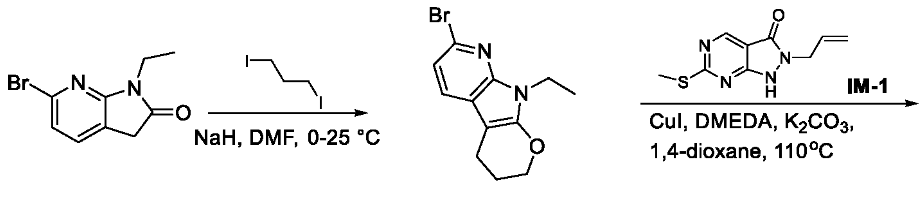


2-4 17-1

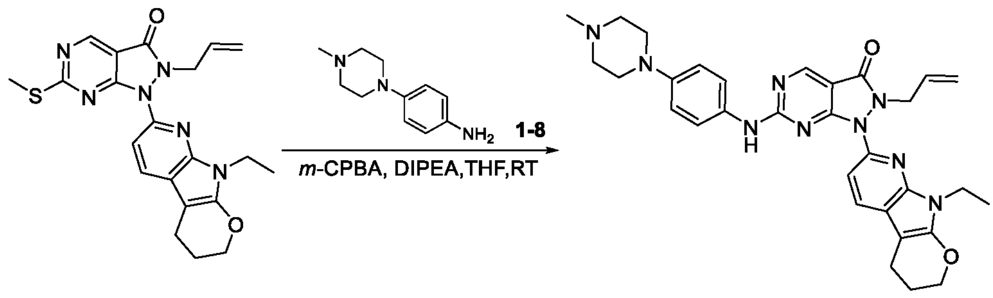


17-2 17

**Step 1: Synthesis of Compound 17-1:**

A dry single-necked flask was added with Substrate **2-4** (200 mg, 829.6 μmol) and DMF (10 mL). The mixture was stirred to dissolved, and then added with NaH (119.5 mg, 4.98 mmol). At 0°C, the reaction was stirred for 0.5 hours, and then added with 1,3-diiodopropane (736.4 mg, 2.48 mmol). Under room temperature, the reaction was stirred for 0.5 hours with LC-MS monitoring. After the reaction was completed, the mixture was added with water to quench the reaction, and extracted with EA for three times. The organic phase was combined, treated with anhydrous sodium sulfate, and then filtered and dried under evaporation, purified by reverse phase column chromatography to give the product **17-1** (70 mg, 250 μmol, 29.87% yield), LCMS ($ESI^+$) m/z: 281 $[M+H]^+$.

**Step 2: Synthesis of Compound 17-2:**

Following the synthesis method of Example **2** Step **5,** the same synthesis method was performed, expect that in Step **5, 2-5** was replaced with **17-1** (70 mg, 250 μmol), to give Compound **17-2** (25 mg, 59.17 μmol, 23.69% yield), LCMS ($ESI^+$) m/z: 423 $[M+H]^+$.

**Step 3: Synthesis of Compound 17:**

Following the synthesis method of Example **3** Step **4,** the same synthesis method was performed, expect that in Step **4, 3-4** was replaced with **17-2** (25 mg, 59.17 μmol), to give Compound 17 (5 mg, 8.84 μmol, 14.94% yield). **$^1$H NMR** (600 MHz, DMSO-$d_6$) δ 10.25 (s, 1H), 8.85 (s, 1H), 8.22 (s, 1H), 7.73 (s, 1H), 7.62 (s, 2H), 6.92 (d, *J* = 8.6 Hz, 2H), 5.75 - 5.65 (m, 1H), 5.08 - 5.03 (m, 1H), 4.95 - 4.89 (m, 1H), 4.59 (d, *J* = 5.9 Hz, 2H), 4.16 (t, *J* = 6.0 Hz, 2H), 3.82 (q, *J* = 7.0 Hz, 2H), 3.10 (t, *J* = 4.9 Hz, 4H), 2.82 (t, *J* = 6.1 Hz, 2H), 2.46 (t, *J* = 4.9 Hz, 4H), 2.22 (s, 3H), 2.20 - 2.15 (m, 2H), 1.15 (t, *J* = 7.1 Hz, 3H). LCMS ($ESI^+$) m/z: 598.2 $[M+MeOH+H]^+$, HPLC Method B: $R_T$=7.23 min, purity: 80.0%.

**Example 18: Synthesis of Compound 18**

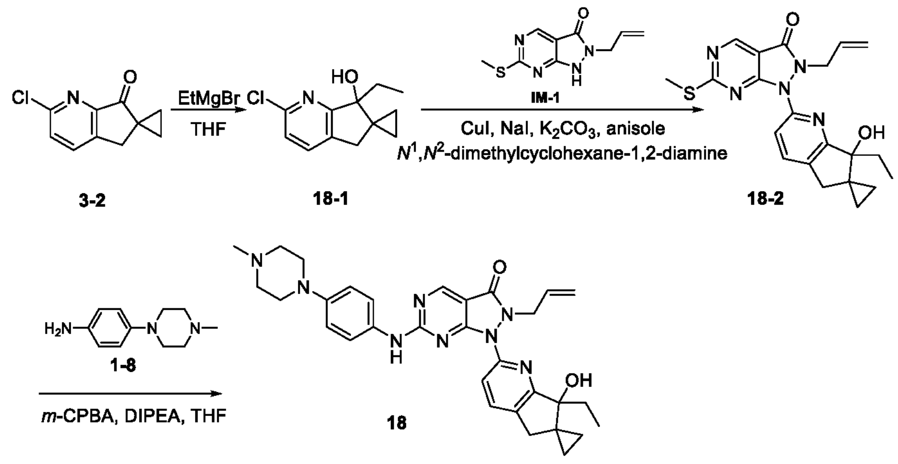


**Step 1: Synthesis of Compound 18-1:**

A dry three-necked flask was added with Substrate **3-2**(100 mg, 516.45 μmol), added with anhydrous tetrahydrofuran (2 mL) to dissolve, under the protection of nitrogen atmosphere cooled to 0°C, and then slowly dropwise added with a solution of ethylmagnesium bromide in tetrahydrofuran (2.07 mmol, 1.04 mL, 2M), and the temperature was kept to react for 1 hour with LC-MS monitoring. The reaction solution was added with saturated ammonium chloride aqueous solution, extracted with ethyl acetate for three times. The organic phase was dried with anhydrous sodium sulfate, filtrated, concentrated under reduced pressure. The residue was purified by column chromatography to give Compound **18-1** (112.00 mg, 500.67 μmol). LCMS (ESI) m/z: 224.2 $[M+H]^+$.

**Step 2: Synthesis of Compound 18-2:**

A dry microwave tube was added with Substrate **18-1**(112.00 mg, 500.67 μmol), Substrate **IM-1**(111.00 mg, 499.40 μmol), copper (I) iodide (190.22 mg, 998.80 μmol), sodium iodide (149.71 mg, 998.80 μmol), potassium carbonate (172.29 mg, 1.25 mmol) and trans-(1R,2R)-N,N'-dimethyl-1,2-cyclohexylene diamine (284.48 mg, 2.00 mmol), and then added with anisole (3 mL). Under nitrogen atmosphere, the reaction was heated by microwave to 130 °C for 3 hours with LC-MS monitoring. The reaction solution was cooled to room temperature, filtrated, washed with ethyl acetate twice, concentrated under reduced pressure. The residue was purified by column chromatography to give Compound **18-2**(90 mg, 220.31 μmol). LCMS (ESI) m/z: 410.2 $[M+H]^+$.

**Step 3: Synthesis of Compound 18:**

A dry single-necked flask was added with Substrate **18-2**(16 mg, 39.07 μmol), dissolved in tetrahydrofuran (2 mL), and then added with m-chloroperoxybenzoic acid (12.14 mg, 70.33 μmol). Under room temperature, the reaction was performed for 1 hour. Then the reaction solution was added with N,N-diisopropyl ethyl amine (50.40 mg, 390.72 μmol) and Substrate 4-(4-methylpiperazino)aniline (8.97 mg, 46.89 μmol), reacted at 45°C overnight with LC-MS monitoring. After the reaction was completed, the reaction solution was cooled to room temperature, concentrated under reduced pressure. The residue was purified by preparative high-performance liquid chromatography to give Compound **18** (5.41 mg, 9.79 μmol). **$^1$H NMR** (600 MHz, DMSO-$d_6$) δ 10.16 (s, 1H), 8.83 (s, 1H), 7.90 (s, 1H), 7.72 - 7.70 (d, *J* = 8.4 Hz, 1H), 7.60 (s, 2H), 6.95 - 6.93 (d, *J* = 8.4 Hz, 2H), 5.72 - 5.64 (m, 1H), 5.02 - 5.01 (m, 1H), 4.95 (s, 1H), 4.89 - 4.86 (m, 1H), 4.84 - 4.71 (m, 1H), 4.60 - 4.52 (m, 1H), 3.26 - 3.05 (m, 4H), 3.01 (m, 1H), 2.74 (m, 1H), 2.48 - 2.31 (m, 7H), 1.78 - 1.73 (m, 1H), 1.60 - 1.54 (m, 1H), 0.96 - 0.95 (m, 1H), 0.71 - 0.69 (m, 5H), 0.41 - 0.39 (m, 1H). LCMS (ESI) m/z: 553.3 $[M+H]^+$, HPLC Method B: $R_T$=7.26 min, purity: > 97.4%.

**Example 19: Synthesis of Compound 19**

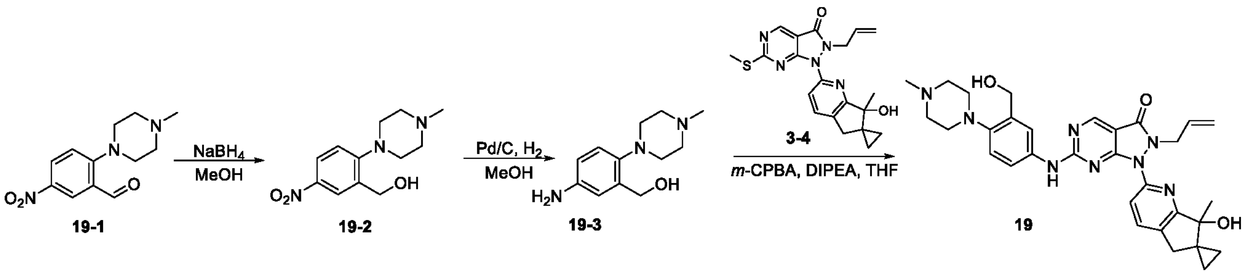


19-1 19-2 19-3 19

**Step 1: Synthesis of Compound 19-2:**

A dry single-necked flask was added with Substrate **19-1**(142.09 mg, 570.04 μmol), and then added with methanol (2 mL) to dissolve, cooled to 0°C, added with sodium borohydride (86.26 mg, 2.28 mmol), and the temperature was kept constant to react for 1 hour with LC-MS monitoring for complete reaction. The reaction solution was concentrated under reduced pressure. The residue was added with water, extracted with ethyl acetate for three times. The organic phase was dried with anhydrous sodium sulfate, filtrated, concentrated under reduced pressure to give Compound **19-2** (130 mg, 517.35 μmol). LCMS (ESI) m/z: 252.3 $[M+H]^+$.

**Step 2: Synthesis of Compound 19-3:**

A dry single-necked flask was added with Substrate **19-2**(125.64 mg, 0.5 mmol), dissolved in methanol (2 mL), and then added with Pd-C (20 mg). Under hydrogen atmosphere, the reaction was performed under room temperature for 2 hours with LC-MS monitoring for complete reaction. The reaction solution was filtrated with celite, washed with methanol twice. The filtrate was concentrated under reduced pressure to give Compound **19-3**(100 mg, 451.88 μmol). LCMS (ESI) m/z: 222.3 $[M+H]^+$.

**Step 3: Synthesis of Compound 19:**

A dry single-necked flask was added with Substrate **3-4**(10 mg, 25.29 μmol), dissolved in tetrahydrofuran (1 mL), and then added with m-chloroperoxybenzoic acid (9.60 mg, 55.63 μmol). Under room temperature, the reaction was performed for 1 hour. Then the reaction solution was added with N,N-diisopropyl ethyl amine (32.68 mg, 252.86 μmol) and Substrate **19-3**(33.57 mg, 151.72 μmol). The reaction was performed at 45°C overnight with LC-MS monitoring. After the reaction was completed, the reaction solution was cooled to room temperature, concentrated under reduced pressure. The residue was purified by preparative high-performance liquid chromatography to give Compound **19** (4 mg, 6.57 μmol). **$^1$H NMR** (600 MHz, DMSO-$d_6$) δ 10.20 (s, 1H), 8.85 (s, 1H), 8.01 (s, 1H), 7.88 (d, *J* = 7.8 Hz, 1H), 7.79 (d, *J* = 7.8 Hz, 1H), 7.45 - 7.48 (m, 1H), 7.03 (d, *J* = 8.4 Hz, 1H), 5.74 - 5.67 (m, 1H), 5.12 (s, 1H), 5.02 - 4.99 (m, 2H), 4.87 (d, *J* = 16.8 Hz, 1H), 4.75 (s, 1H), 4.63 - 4.60 (m, 1H), 4.57 (d, *J* = 4.2 Hz, 2H), 2.88 (d, *J* = 5.4 Hz, 2H), 2.82 (t, *J* = 4.8 Hz, 4H), 2.46 (s, 4H), 2.23 (s, 3H), 1.21 (s, 3H), 0.93 - 0.90 (m, 1H), 0.70 - 0.66 (m, 1H), 0.60 - 0.57 (m, 1H), 0.47 - 0.44 (m, 1H). LCMS (ESI) m/z: 569.3 $[M+H]^+$, HPLC Method B: $R_T$=6.37 min, purity: > 93.4%.

**Example 20: Synthesis of Compound 20**

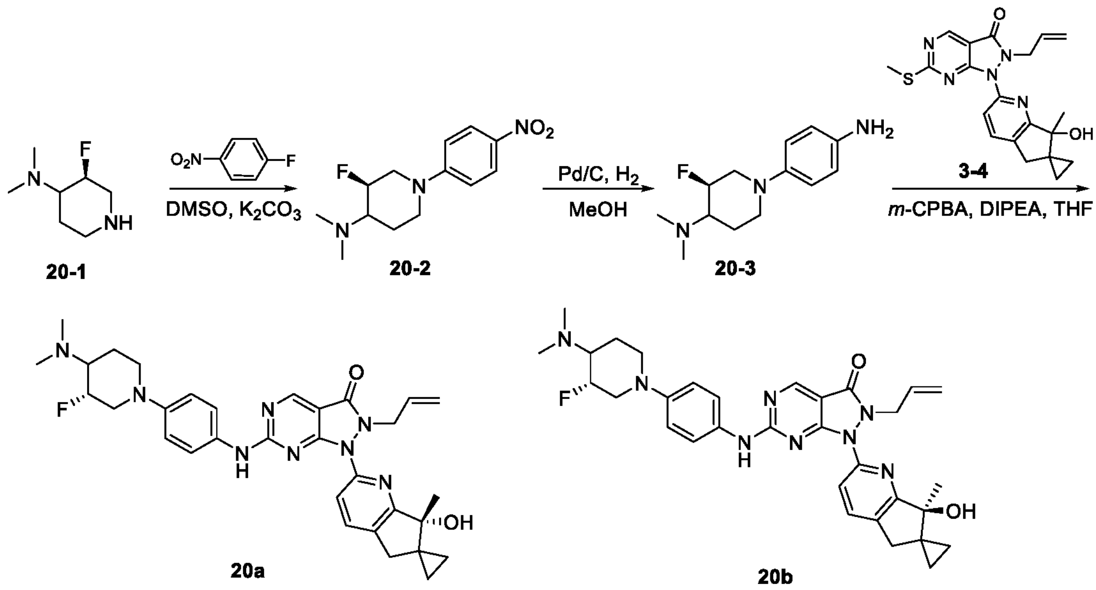


20-1 20-2 20-3

20a 20b

**Step 1: Synthesis of Compound 20-2:**

A dry single-necked flask was added with Substrate **20-1**(100 mg, 683.97 μmol), dissolved in dimethyl sulfoxide (2 mL), and then added with p-fluoro-nitrobenzene (96.51 mg, 683.97 μmol) and potassium carbonate (94.53 mg, 683.97 μmol). At 80°C, the reaction was performed for 4 hours with LC-MS monitoring for complete reaction. The reaction solution was cooled to room temperature, added with water, and then extracted with ethyl acetate for four times. The organic phase was dried with anhydrous sodium sulfate, filtrated, concentrated under reduced pressure. The residue was purified by column chromatography to give Compound **20-2**(120 mg, 448.94 μmol). LCMS (ESI) m/z: 267.1 $[M+H]^+$.

**Step 2: Synthesis of Compound 20-3:**

A dry single-necked flask was added with Substrate **20-2**(110 mg, 411.52 μmol), dissolved in methanol (2 mL), and then added with Pd-C (15 mg). Under hydrogen atmosphere, the reaction was performed under room temperature for 2 hours with LC-MS monitoring for complete reaction. The reaction solution was filtrated with celite, washed with methanol twice. The filtrate was concentrated under reduced pressure to give Compound **20-3**(90 mg, 379.24 μmol). LCMS (ESI) m/z: 238.2 $[M+H]^+$.

**Step 3: Synthesis of Compound 20a & 20b:**

A dry single-necked flask was added with Substrate **3-4**(10 mg, 25.29 μmol), dissolved in tetrahydrofuran (0.5 mL), and then added with m-chloroperoxybenzoic acid (6.55 mg, 37.93 μmol). Under room temperature, the reaction was performed for 1 hour. Then the reaction solution was added with N,N-diisopropyl ethyl amine (32.68 mg, 252.86 μmol) and Substrate **20-3**(12.00 mg, 50.57 μmol). The reaction was performed at 45°C overnight with LC-MS monitoring. After the reaction was completed, the reaction solution was cooled to room temperature, concentrated under reduced pressure. The residue was purified by preparative high-performance liquid chromatography to give Compound **20a**(1.0 mg, 1.47 μmol)(**20a** and **20b** were diastereomers, and separated directly to give the product. The structure is written randomly, and the absolute configuration is undetermined. residence time shall prevail, the same below.). **$^1$H NMR** (600 MHz, DMSO-$d_6$) δ 10.13 (s, 1H), 8.82 (s, 1H), 7.92 (s, 1H), 7.70 (d, *J* = 8.4 Hz, 1H), 7.58 (s, 2H), 6.92 (d, *J* = 8.4 Hz, 2H), 5.73 - 5.66 (m, 1H), 5.09 - 4.96 (m, 3H), 4.88 (d, *J* = 17.4 Hz, 1H), 4.74 (d, *J* = 12.6 Hz, 1H), 4.60 (s, 1H), 3.88 (t, *J* = 12.6 Hz, 1H), 3.74 (d, *J* = 12.0 Hz, 1H), 2.95 - 2.86 (m, 2H), 2.85 - 2.76 (m, 1H), 2.68 (t, *J* = 11.4 Hz, 1H), 2.40 - 2.31 (m, 1H), 2.28 (s, 6H), 1.90 - 1.83 (m, 1H), 1.76 (d, *J* = 12.6 Hz, 1H), 1.22 (s, 3H), 0.94 - 0.90 (m, 1H), 0.70 - 0.64 (m, 1H), 0.60 - 0.55 (m, 1H), 0.49 - 0.42 (m, 1H). LCMS (ESI) m/z: 567.2 $[M+H]^+$, HPLC Method B: $R_T$=8.69 min, purity> 88.7%.

Compound 20b(1.0 mg, 1.50 μmol). **$^1$H** NMR (600 MHz, DMSO-$d_6$) δ 10.15 (s, 1H), 8.83 (s, 1H), 7.93 (s, 1H), 7.69 (d, *J* = 8.4 Hz, 1H), 7.60 (s, 2H), 6.96 (d, *J* = 8.4 Hz, 2H), 5.72 - 5.66 (m, 1H), 5.04 - 4.85 (m, 3H), 4.80 - 4.74 (m, 1H), 4.73 - 4.64 (m, 1H), 4.60 (s, 1H), 3.87 (s, 1H), 3.57 (d, *J* = 12.0 Hz, 1H), 2.90 (d, *J* = 5.4 Hz, 2H), 2.71 - 2.59 (m, 3H), 2.30 (s, 6H), 1.82 (s, 1H), 1.60 - 1.54 (m, 1H), 1.22 (s, 3H), 0.94 - 0.90 (m, 1H), 0.70 - 0.67 (m, 1H), 0.62 - 0.55 (m, 1H), 0.48 - 0.45 (m, 1H).LCMS (ESI) m/z: 567.2 $[M+H]^+$, HPLC Method B: $R_T$=8.70 min, purity> 90.2%.

**Example 21: Synthesis of Compound 21**

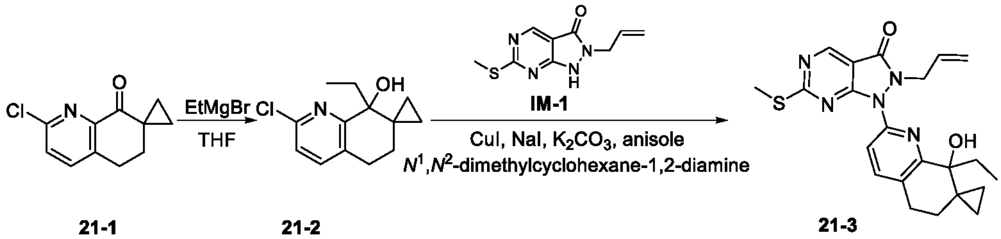


21-1 21-2 21-3

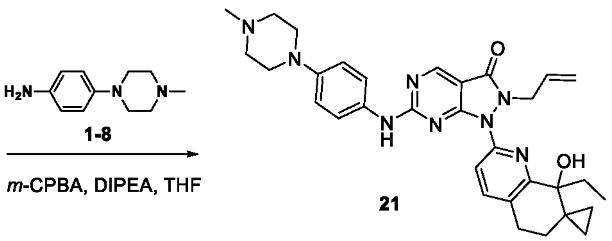


21

**Step 1: Synthesis of Compound 21-2:**

A dry three-necked flask was added with Substrate **21-1**(80 mg, 385.25 μmol), added with anhydrous tetrahydrofuran (4.5 mL) to dissolve, under the protection of nitrogen atmosphere cooled to 0°C, and then slowly dropwise added with a solution of ethylmagnesium bromide in tetrahydrofuran (1.54 mmol, 0.77 mL, 2M), and the temperature was kept to react for 2 hours with LC-MS monitoring. The reaction solution was added with saturated ammonium chloride aqueous solution, extracted with ethyl acetate for three times. The organic phase was dried with anhydrous sodium sulfate, filtrated, concentrated under reduced pressure. The residue was purified by column chromatography to give Compound 21-2(68 mg, 286.05 μmol). LCMS (ESI) m/z: 238.1 $[M+H]^+$.

**Step 2: Synthesis of Compound 21-3:**

A dry microwave tube was added with Substrate **21-2**(68 mg, 286.05 μmol), Substrate **IM-1**(73.86 mg, 332.29 μmol), copper (I) iodide (108.95 mg, 572.09 μmol), sodium iodide (85.75 mg, 572.09 μmol), potassium carbonate (98.83 mg, 715.11 μmol) and trans-(1R,2R)-N,N'-dimethyl-1,2-cyclohexylene diamine (162.75 mg, 1.14 mmol), and then added with anisole (3 mL). Under nitrogen atmosphere, the reaction was heated by microwave to 130 °C for 4 hours with LC-MS monitoring. The reaction solution was cooled to room temperature, filtrated, washed with ethyl acetate twice, concentrated under reduced pressure. The residue was purified by column chromatography to give Compound **21-3**(57 mg, 134.58 μmol). LCMS (ESI) m/z: 424.2 $[M+H]^+$.

**Step 3: Synthesis of Compound 21:**

A dry single-necked flask was added with Substrate **21-3**(20 mg, 47.44 μmol), dissolved in tetrahydrofuran (2 mL), and then added with m-chloroperoxybenzoic acid (16.37 mg, 94.89 μmol). Under room temperature, the reaction was performed for 1 hour. Then the reaction solution was added with N,N-diisopropyl ethyl amine (61.75 mg, 477.78 μmol) and Substrate 4-(4-methylpiperazino)aniline (40 mg, 209.13 μmol). The reaction was performed at 45°C overnight with LC-MS monitoring. After the reaction was completed, the reaction solution was cooled to room temperature, concentrated under reduced pressure. The residue was purified by preparative high-performance liquid chromatography to give Compound **21** (12 mg, 20.54 μmol). **$^1$H NMR** (400 MHz, Chloroform-*d*) δ 8.81 (s, 1H), 7.64 - 7.58 (m, 2H), 7.47 (d, *J* = 8.8 Hz, 2H), 6.93 (d, *J* = 8.8 Hz, 2H), 5.76 - 5.66 (m, 1H), 5.08 - 5.05 (m, 1H), 5.00 - 4.94 (m, 1H), 4.75 - 4.61 (m, 2H), 3.56 (s, 1H), 3.28 (t, *J* = 4.8 Hz, 4H), 3.04 - 2.95 (m, 1H), 2.91 - 2.85 (m, 1H), 2.73 (t, *J* = 4.8 Hz, 4H), 2.50 - 2.43 (m, 4H), 2.02 - 1.93(m, 1H), 1.80 - 1.71 (m, 1H), 1.19 - 1.14 (m, 1H), 1.02 (t, *J* = 7.6 Hz, 3H), 0.75 - 0.71 (m, 1H), 0.49 - 0.44 (m, 1H), 0.07 - 0.01 (m, 1H). LCMS (ESI) m/z: 567.3 $[M+H]^+$, HPLC Method B: $R_T$=8.54 min, purity> 97.0%.

**Example 22: Synthesis of Compound 22**

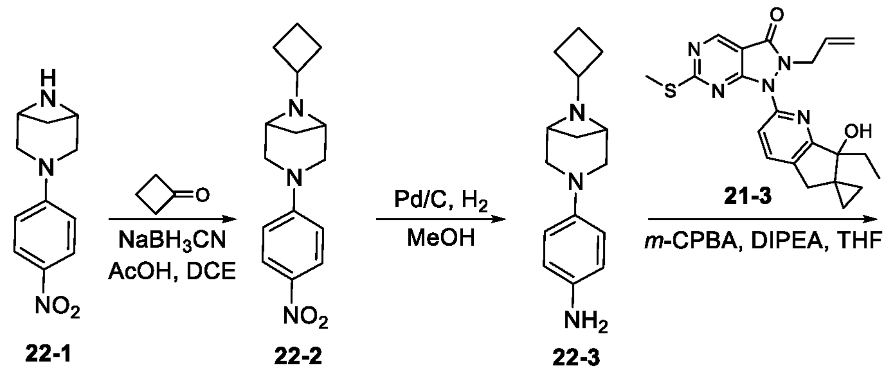


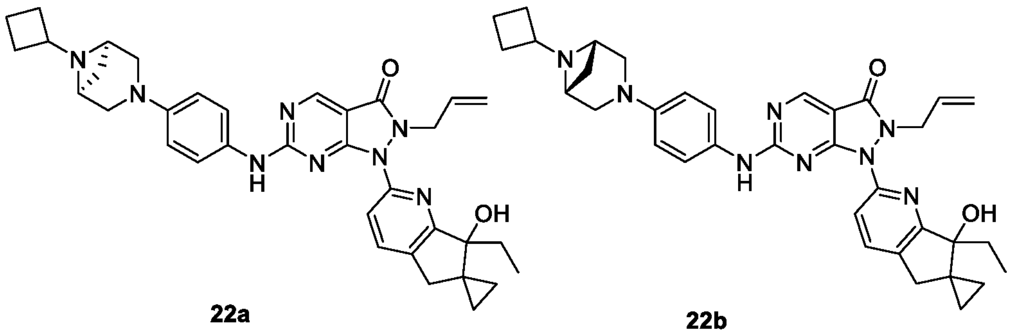

22a

22b

**Step 1: Synthesis of Compound 22-2:**

A dry single-necked flask was added with Substrate **22-1**(90 mg, 410.51 μmol), dissolved in 1,2-dichloroethane (5 mL), added with acetic acid (49.30 mg, 821.02 μmol) and cyclobutanone (143.86 mg, 2.05 mmol), stirred under room temperature for half an hour, and then added with sodium cyanoborohydride (435.02 mg, 2.05 mmol), and heated to 50°C to react for 2 hours with LC-MS monitoring for complete reaction. The reaction solution was concentrated under reduced pressure, added with saturated sodium bicarbonate aqueous solution to adjust to pH=9, and then the aqueous phase was extracted with dichloromethane for four times. The organic phase was dried with anhydrous sodium sulfate, filtrated, concentrated under reduced pressure to give Compound **22-2**(93 mg, 340.25 μmol). LCMS (ESI) m/z: 274.1 $[M+H]^+$.

**Step 2: Synthesis of Compound 22-3:**

A dry single-necked flask was added with Substrate **22-2**(93 mg, 340.25 μmol), dissolved in methanol (3 mL), and then added with Pd-C (10 mg). Under hydrogen atmosphere, the reaction was performed under room temperature for 2 hours with LC-MS monitoring for complete reaction. The reaction solution was filtrated with celite, washed with methanol twice. The filtrate was concentrated under reduced pressure to give Compound **22-3**(80 mg, 328.74 μmol). LCMS (ESI) m/z: 244.2 $[M+H]^+$.

**Step 3: Synthesis of Compound 22a & 22b:**

A dry single-necked flask was added with Substrate **22-3**(18 mg, 43.96 μmol), dissolved in tetrahydrofuran (2 mL), and then added with m-chloroperoxybenzoic acid (15.17 mg, 87.91 μmol). Under room temperature, the reaction was performed for 1 hour. Then the reaction solution was added with N,N-diisopropyl ethyl amine (28.40 mg, 219.78 μmol) and Substrate **21-3**(16.04 mg, 65.93 μmol). The reaction was performed at 45°C overnight with LC-MS monitoring. After the reaction was completed, the reaction solution was cooled to room temperature, concentrated under reduced pressure. The residue was purified by preparative high-performance liquid chromatography to give Compound **22a** (2.40 mg, 3.62 μmol). **$^1$H NMR** (400 MHz, DMSO-$d_6$) δ 10.08 (s, 1H), 8.80 (s, 1H), 7.88 (d, *J* = 8.4 Hz, 1H), 7.72 (d, *J* = 8.0 Hz, 1H), 7.64 - 7.55 (m, 2H), 6.79 - 6.68 (m, 1H), 5.74 - 5.64 (m, 1H), 5.03 - 5.01 (m, 1H), 4.95 (s, 1H), 4.88 (d, *J* =17.2 Hz, 1H), 4.81 - 4.71

(m, 1H), 4.59 - 4.53 (m, 1H), 3.89 - 3.76 (m, 2H), 3.48 - 3.42 (m, 4H), 3.03 - 2.99 (m, 1H), 2.75 - 2.71 (m, 1H), 2.48 (s, 1H), 2.19-2.12 (m, 2H), 1.79 - 1.73 (m, 1H), 1.61 - 1.52 (m, 2H), 0.99 - 0.94 (m, 1H), 0.85 - 0.79 (m, 1H), 0.73 - 0.68 (m, 5H), 0.41 - 0.35 (m, 3H), 0.08 - 0.05 (m, 2H). LCMS (ESI) m/z: 605.3 $[M+H]^+$, HPLC Method B: $R_T$=8.26 min, purity> 91.2%.

Compound **22b** (6.40 mg, 10.03μmol). **$^1$H NMR** (400 MHz, DMSO-$d_6$) δ 10.08 (s, 1H), 8.80 (s, 1H), 7.88 (d, *J* = 8.4 Hz, 1H), 7.72 (d, *J* = 8.0 Hz, 1H), 7.60 (s, 2H), 6.69 (d, *J* = 7.2 Hz, 1H), 5.74 - 5.64 (m, 1H), 5.03 - 5.01 (m, 1H), 4.95 (s, 1H), 4.86 (d, *J* =17.2 Hz, 1H), 4.70 - 4.77 (m, 1H), 4.59 - 4.53 (m, 1H), 3.81 - 3.71 (m, 2H), 3.19 - 3.08 (m, 4H), 3.01 (d, *J* =16.4 Hz, 2H), 2.73 (d, *J* =16.4 Hz, 1H), 2.42 - 2.37 (m, 1H), 2.03 - 1.91 (m, 2H), 1.79 - 1.73 (m, 5H), 1.61 - 1.52 (m, 2H), 0.99 - 0.94 (m, 1H), 0.72 - 0.68 (m, 5H), 0.39 - 0.35 (m, 2H). LCMS (ESI) m/z: 605.3 $[M+H]^+$, HPLC Method B: $R_T$=8.37 min, purity> 94.8%.

**Example 23: Synthesis of Compound 23**

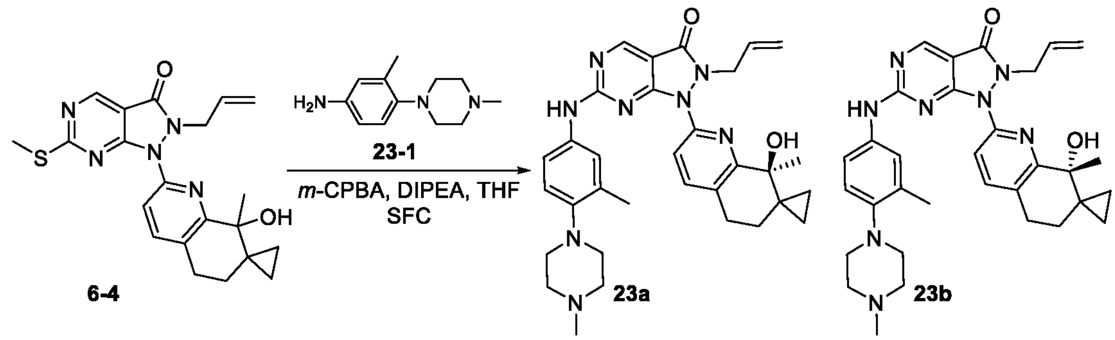


6-4 23a 23b

**Step 1: Synthesis of Compound 23a & 23b:**

A dry single-necked flask was added with Substrate **6-4**(30 mg, 73.26 μmol), dissolved in tetrahydrofuran (1 mL), and then added with m-chloroperoxybenzoic acid (22.76 mg, 131.87 μmol). Under room temperature, the reaction was performed for 1 hour. Then the reaction solution was added with N,N-diisopropyl ethyl amine (94.68 mg, 732.59 μmol) and Substrate **23-1**(30.08 mg, 146.52 μmol). The reaction was performed at 45°C overnight with LC-MS monitoring. After the reaction was completed, the reaction solution was cooled to room temperature, concentrated under reduced pressure. The residue was purified by preparative high-performance liquid chromatography to give the product, and then chirally resolved by supercritical fluid chromatography to give Compound **23a** (12.00 mg, 18.78 μmol); SFC residence time t = 2.302 min. **$^1$H NMR** (400 MHz, DMSO-$d_6$) δ 10.11 (s, 1H), 8.83 (s, 1H), 7.79 - 7.57 (m, 3H), 7.41 - 7.38 (m, 1H), 6.98 (d, *J* = 8.8 Hz, 1H), 5.72 - 5.60 (m, 1H), 5.01 - 4.96 (m, 1H), 4.88 - 4.77 (m, 2H), 4.69 (s, 1H), 4.67 - 4.58 (m, 1H), 2.92 - 2.74 (m, 6H), 2.47 (s, 4H), 2.24 (s, 3H), 2.23 (s, 3H), 2.03 - 1.92 (m, 1H), 1.39 (s, 4H), 0.86 - 0.82 (m, 1H), 0.64 - 0.62 (m, 1H), 0.30 - 0.29 (m, 1H), 0.22 - 0.20 (m, 1H). LCMS (ESI) m/z: 567.2 $[M+H]^+$, HPLC Method B: $R_T$=8.69 min, purity> 86.0%.

Compound **23b**(11.00 mg, 17.51 μmol); SFC residence time t = 3.705 min. **$^1$H NMR** (400 MHz, DMSO-$d_6$) δ 10.12 (s, 1H), 8.83 (s, 1H), 7.79 - 7.57 (m, 3H), 7.41 - 7.38 (m, 1H), 6.98 (d, *J* = 8.8 Hz, 1H), 5.72 - 5.60 (m, 1H), 5.01 - 4.86 (m, 1H), 4.89 - 4.74 (m, 2H), 4.72 - 4.57 (m, 2H), 2.94 - 2.77 (m, 6H), 2.46 (s, 4H), 2.24 (s, 3H), 2.22 (s, 3H), 2.03 - 1.92 (m, 1H), 1.39 (s, 4H), 0.86 - 0.82 (m, 1H), 0.64 - 0.62 (m, 1H), 0.31 - 0.29 (m, 1H), 0.22 - 0.20 (m, 1H). LCMS (ESI) m/z: 567.4 $[M+H]^+$, HPLC Method B: $R_T$=8.70 min, purity > 87.8%.

**Example 24: Synthesis of Compound 24**

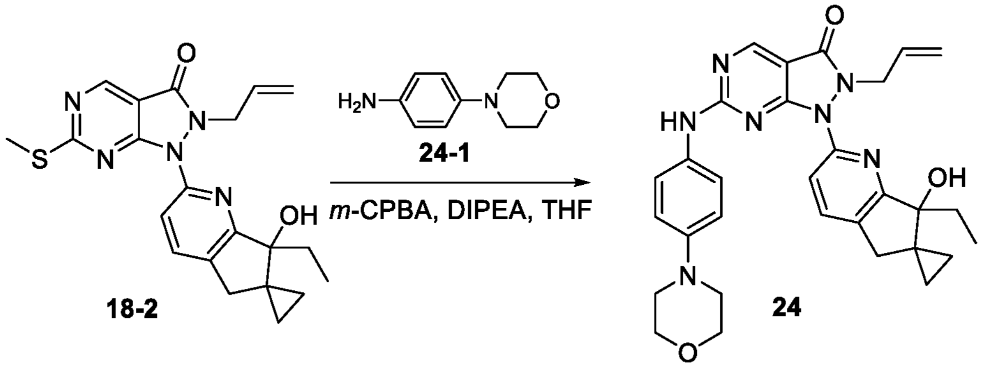


18-2 24

A dry single-necked flask was added with Substrate **18-2**(10 mg, 24.42 μmol), dissolved in tetrahydrofuran (1 mL), and then added with m-chloroperoxybenzoic acid (8.43 mg, 48.84 μmol). Under room temperature, the reaction was performed for 1 hour. Then the reaction solution was added with N,N-diisopropyl ethyl amine (31.56 mg, 244.20 μmol) and Substrate **24-1**(21.76 mg, 122.10 μmol). At 50°C, the reaction was performed overnight with LC-MS monitoring. After the reaction was completed, the reaction solution was cooled to room temperature, concentrated under reduced pressure. The residue was purified by preparative high-performance liquid chromatography to give Compound **24** (4 mg, 6.92 μmol). **$^1$H NMR** (400 MHz, DMSO-$d_6$) δ 10.15 (s, 1H), 8.83 (s, 1H), 7.91 (d, *J* = 12.4 Hz, 1H), 7.71 (d, *J* = 12.4 Hz, 1H), 7.61 (s, 2H), 6.93 (d, *J* = 13.2 Hz, 2H), 5.74 - 5.64 (m, 1H), 5.04 - 5.00 (m, 1H), 4.95 (s, 1H), 4.90 - 4.85 (m, 1H), 4.77 (s, 1H), 4.59 - 4.53 (m, 1H), 3.76 - 3.73 (m, 4H), 3.08 - 3.06 (m, 4H), 3.01 (d, *J* = 25.2 Hz, 1H), 2.74 (d, *J* = 24.4 Hz, 1H), 1.81 - 1.72 (m, 1H), 1.61 - 1.52 (m, 1H), 0.99 - 0.94 (m, 1H), 0.73 - 0.68 (m, 5H), 0.40 - 0.35 (m, 1H). LCMS (ESI) m/z: 540.2 [M+H]$^+$, HPLC Method B: $R_T$=7.49 min, purity> 93.2%.

**Example 25: Synthesis of Compound 25**

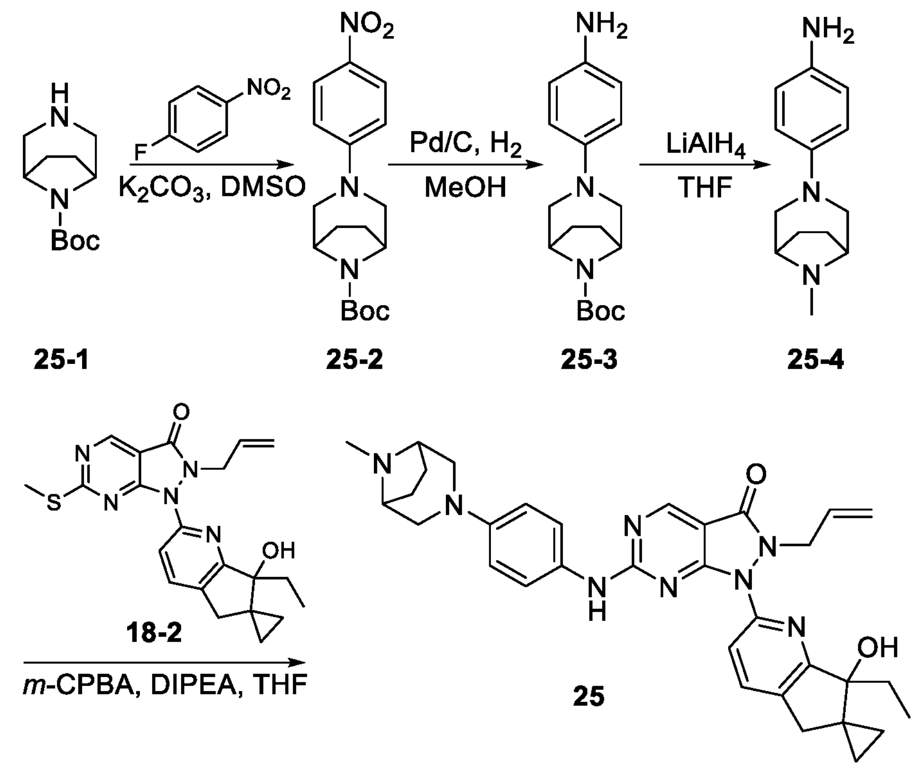


25-1 25-2 25-3 25-4

18-2

25

**Step** 1: **Synthesis of Compound 25-2:**

A dry single-necked flask was added with Substrate **25-1**(332 mg, 1.56 mmol), dissolved in dimethyl sulfoxide (5

mL), and then added with p-fluoro-nitrobenzene (242.73 mg, 1.72 mmol) and potassium carbonate (648.42 mg, 4.69 mmol). At 80°C, the reaction was performed for 12 hours with LC-MS monitoring for complete reaction. The reaction solution was cooled to room temperature, added with water, and then extracted with ethyl acetate for four times. The organic phase was dried with anhydrous sodium sulfate, filtrated, concentrated under reduced pressure. The residue was purified by column chromatography to give Compound **25-2**(512 mg, 1.54 mmol). LCMS (ESI) m/z: 278.1 $[M+H]^+$.

**Step 2: Synthesis of Compound 25-3:**

A dry single-necked flask was added with Substrate **25-2**(640 mg, 1.92 mmol), dissolved in methanol (10 mL), and then added with Pd-C (60 mg). Under hydrogen atmosphere, the reaction was performed under room temperature for 12 hours with LC-MS monitoring for complete reaction. The reaction solution was filtrated with celite, washed with methanol twice. The filtrate was concentrated under reduced pressure to give Compound **25-3**(570 mg, 1.88 mmol). LCMS (ESI) m/z: 248.2 $[M+H]^+$.

**Step 3: Synthesis of Compound 25-4:**

A dry three-necked flask was added with Substrate **25-3**(200 mg, 659.20 μmol), added with anhydrous tetrahydrofuran (5 mL) to dissolve, under the protection of nitrogen atmosphere cooled to 0°C, and then slowly dropwise added with a solution of lithium aluminum hydride in tetrahydrofuran (3.3 mL, 3.30 mmol, 1M). The reaction was refluxed at 65 °C for 4 hours with LC-MS monitoring. The reaction solution was added with water, 10% sodium hydroxide aqueous solution, stirred for half an hour, dried with anhydrous sodium sulfate, filtrated with celite, concentrated under reduced pressure. The residue was purified by column chromatography to give Compound **25-4**(89 mg, 409.55 μmol). LCMS (ESI) m/z: 218.2 $[M+H]^+$.

**Step** 4: **Synthesis of Compound 25:**

A dry single-necked flask was added with Substrate **18-2**(10 mg, 24.42 μmol), dissolved in tetrahydrofuran (1 mL), and then added with m-chloroperoxybenzoic acid (7.59 mg, 43.96 μmol). Under room temperature, the reaction was performed for 1 hour. Then the reaction solution was added with N,N-diisopropyl ethyl amine (31.56 mg, 244.20 μmol) and Substrate **25-4**(10.60 mg, 48.78 μmol). At 50°C, the reaction was performed overnight with LC-MS monitoring. After the reaction was completed, the reaction solution was cooled to room temperature, concentrated under reduced pressure. The residue was purified by preparative high-performance liquid chromatography to give Compound **25** (2 mg, 2.98μmol). **$^1$H NMR** (400 MHz, DMSO-$d_6$) δ 10.09 (s, 1H), 8.81 (s, 1H), 7.90 (d, *J* = 7.6 Hz, 1H), 7.71 (d, *J* = 8.0 Hz, 1H), 7.55 (s, 2H), 6.78 (d, *J* = 8.8 Hz, 2H), 5.73 - 5.62 (m, 1H), 5.02 (dd, *J* = 10.0, 1.6 Hz, 1H), 4.95 (s, 1H), 4.87 (d, *J* = 17.2 Hz, 1H), 4.77 (s, 1H), 4.61 - 4.49 (m, 1H), 3.20 (s, 2H), 3.01 (d, *J* = 16.4 Hz, 1H), 2.76 (dd, *J* = 18.0, 13.6 Hz, 3H), 2.22 (s, 3H), 1.98 - 1.90 (m, 2H), 1.80 - 1.72 (m, 1H), 1.66 - 1.53 (m, 3H), 0.99 - 0.93 (m, 1H), 0.70 (t, *J* = 7.2 Hz, 5H), 0.42 - 0.33 (m, 1H). LCMS (ESI) m/z: 579.4 $[M+H]^+$, HPLC Method B: $R_T$= 7.93 min, purity> 86.1%.

**Example 26: Synthesis of Compound 26**

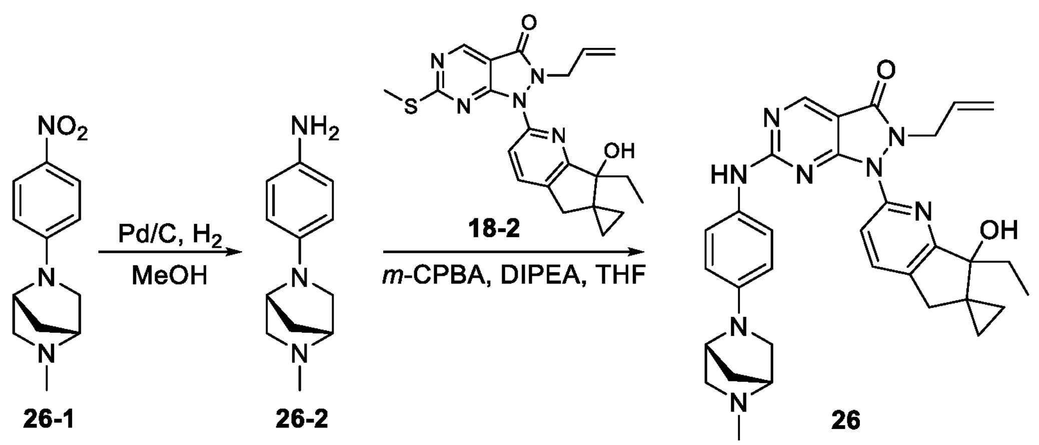


26-1 26-2 26

**Step 1: Synthesis of Compound 26-2:**

A dry single-necked flask was added with Substrate **26-1**(200 mg, 857.39 μmol), dissolved in methanol (5 mL), and then added with Pd-C (20 mg). Under hydrogen atmosphere, the reaction was performed under room temperature for 12 hours with LC-MS monitoring for complete reaction. The reaction solution was filtrated with celite, washed with methanol twice. The filtrate was concentrated under reduced pressure to give Compound **26-2**(170 mg, 836.28 μmol). LCMS (ESI) m/z: 204.1 $[M+H]^+$.

**Step 2: Synthesis of Compound 26:**

A dry single-necked flask was added with Substrate **18-2**(10 mg, 24.42 μmol), dissolved in tetrahydrofuran (2 mL), and then added with m-chloroperoxybenzoic acid (9.27 mg, 53.72 μmol). Under room temperature, the reaction was performed for 1 hour. Then the reaction solution was added with N,N-diisopropyl ethyl amine (15.78 mg, 122.10 μmol) and Substrate **26-2**(10.92 mg, 53.72 μmol). At 50°C, the reaction was performed overnight with LC-MS monitoring. After the reaction was completed, the reaction solution was cooled to room temperature, concentrated under reduced pressure. The residue was purified by preparative high-performance liquid chromatography to give Compound **26** (2.03 mg, 3.59 μmol). **$^1$H NMR** (400 MHz, DMSO-$d_6$) δ 10.02 (s, 1H), 8.79 (s, 1H), 7.88 (d, *J* = 8.0 Hz, 1H), 7.70 (d, *J* = 8.0 Hz, 1H), 7.51 - 7.48 (m, 2H), 6.55 (d, *J* = 8.8 Hz, 2H), 5.73 - 5.64 (m, 1H), 5.03 - 5.00 (m, 1H), 4.95 (s, 1H), 4.88 (d, *J* =17.2 Hz, 1H), 4.80 - 4.77 (m 1H), 4.58 - 4.52 (m, 1H), 4.26 (s, 1H), 3.41 (s, 1H), 3.33 - 3.30 (m, 1H), 3.12 (d, *J* = 9.2 Hz, 1H), 3.01 (d, *J* = 16.4 Hz, 1H), 2.78 - 2.72 (m, 2H), 2.47 - 2.44 (m, 1H), 2.23 (s, 3H), 1.84 (d, *J* = 9.2 Hz, 1H), 1.79 - 1.74 (m, 1H), 1.61 - 1.52 (m, 1H), 0.98 - 0.93 (m, 1H), 0.72 - 0.68 (m, 5H), 0. 39 - 0.34 (m, 1H). LCMS (ESI) m/z: 565.3 $[M+H]^+$, HPLC Method B: $R_T$ = 7.65 min, purity> 95.9%.

**Example 27: Synthesis of Compound 27**

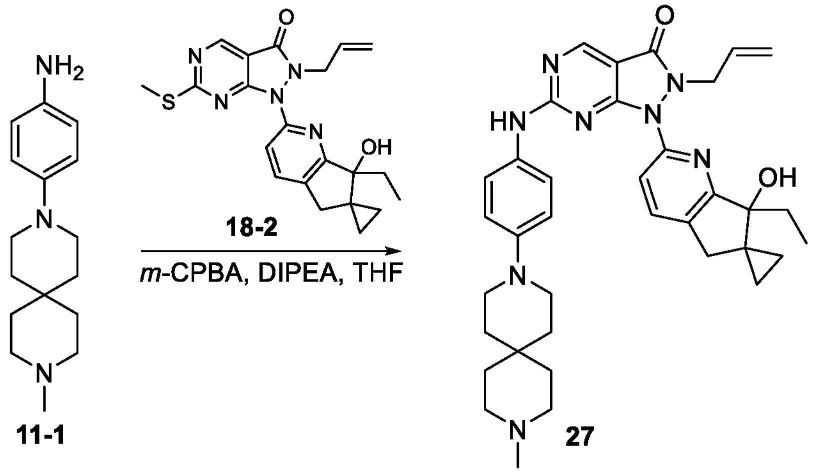


11-1 27

A dry single-necked flask was added with Substrate **18-2**(30 mg, 73.26 μmo), dissolved in tetrahydrofuran (2 mL), and then added with m-chloroperoxybenzoic acid (25.28 mg, 146.52 μmol). Under room temperature, the reaction was performed for 1 hour. Then the reaction solution was added with N,N-diisopropyl ethyl amine (94.68 mg, 732.59 μmol) and Substrate **11-1**(38.01 mg, 146.52 μmol). At 50°C, the reaction was performed overnight with LC-MS monitoring. After the reaction was completed, the reaction solution was cooled to room temperature, concentrated under reduced pressure. The residue was purified by preparative high-performance liquid chromatography to give Compound **27** (15.00 mg, 22.64 μmol). **$^1$H NMR** (400 MHz, DMSO-$d_6$) δ 10.11 (s, 1H), 8.82 (s, 1H), 7.91 (d, *J* = 8.0 Hz, 1H), 7.71 (d, *J* = 8.0 Hz, 1H), 7.57 (s, 2H), 6.91 (d, *J* = 9.2 Hz, 2H), 5.74 - 5.63 (m, 1H), 5.03 - 5.00 (m, 1H), 4.95 (s, 1H), 4.87 (dd, *J* = 17.3, 1.7 Hz, 1H), 4.77 (s, 1H), 4.55 (dd, *J* = 16.0, 6.7 Hz, 1H), 3.08 (t, *J* = 5.7 Hz, 4H), 3.01 (d, *J* = 16.6 Hz, 1H), 2.74 (d, *J* = 16.6 Hz, 1H), 2.28 (t, *J* = 5.4 Hz, 4H), 2.15 (s, 3H), 1.79 - 1.74 (m, 7.3 Hz, 1H), 1.62 - 1.50 (m, 5H), 1.46 (t, *J* = 5.6 Hz, 4H), 0.97 - 0.93 (m, 1H), 0.73 - 0.68 (m, 5H), 0.40 - 0.36 (m, 1H). LCMS (ESI) m/z: 621.2 $[M+H]^+$, HPLC Method B: $R_T$= 8.72 min, purity> 93.7%.

**Example 28: Synthesis of Compound 28**

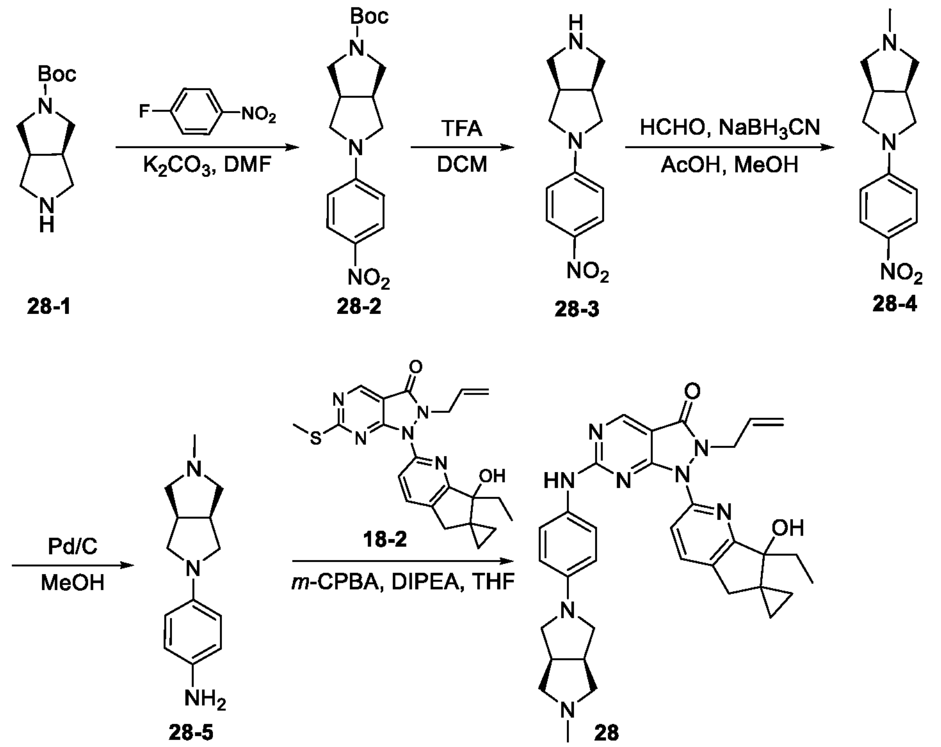

28-1 28-2 28-3 28-4
28-5 28

**Step 1: Synthesis of Compound 28-2:**

A dry single-necked flask was added with Substrate **28-1** (500 mg, 2.36 mmol), dissolved in N,N-dimethyl formamide (7 mL), and then added with p-fluoro-nitrobenzene (302.12 mg, 2.14 mmol) and potassium carbonate (887.73 mg, 6.42 mmol), 90°C, the reaction was performed for 12 hours with LC-MS monitoring for complete reaction. The reaction solution was cooled to room temperature, added with water, and then extracted with ethyl acetate for three times. The organic phase was dried with anhydrous sodium sulfate, filtrated, concentrated under reduced pressure. The residue was purified by column chromatography to give Compound **28-2**(670 mg, 2.01 mmol). LCMS (ESI) m/z: 278.1 $[M+H-56]^+$.

**Step 2: Synthesis of Compound 28-3:**

A dry single-necked flask was added with Substrate **28-2**(670 mg, 2.01 mmol), dissolved in dichloromethane (5 mL), added with trifluoroacetic acid (2.5 mL). Under room temperature, the reaction was performed for 2 hours with LC-MS monitoring for complete reaction. The reaction solution was concentrated under reduced pressure to give crude Compound **28-3**(670 mg, 2.87 mmol). LCMS (ESI) m/z: 234.2 $[M+H]^+$.

**Step 3: Synthesis of Compound 28-4:**

A dry single-necked flask was added with Substrate **28-3**(670 mg, 2.87 mmol), dissolved in methanol (8 mL), added with acetic acid (344.96 mg, 5.74 mmol) and formalin solution (862.42 mg, 28.72 mmol, 37 %), stirred under room temperature for half an hour, and then added with sodium cyanoborohydride (360.99 mg, 5.74 mmol). At 50°C, the reaction was performed for 3 hours with LC-MS monitoring for complete reaction. The reaction solution was concentrated under reduced pressure, added with saturated sodium bicarbonate aqueous solution to adjust to pH=9, and then the aqueous phase was extracted with ethyl acetate for three times. The organic phase was dried with anhydrous sodium sulfate, filtrated, concentrated under reduced pressure to give Compound **28-4**(520 mg, 2.10 mmol). LCMS (ESI) m/z: 248.2 $[M+H]^+$.

**Step 4: Synthesis of Compound 28-5:**

A dry single-necked flask was added with Substrate **28-4**(520 mg, 2.10 mmol), dissolved in methanol (7 mL), and then added with Pd-C (50 mg). Under hydrogen atmosphere, the reaction was performed under room temperature for 3 hours with LC-MS monitoring for complete reaction. The reaction solution was filtrated with celite, washed with methanol twice. The filtrate was concentrated under reduced pressure to give Compound **28-5**(400 mg, 1.84 mmol). LCMS (ESI)

m/z: 218.1 [M+H]+.

**Step 5: Synthesis of Compound 28:**

A dry single-necked flask was added with Substrate **18-2**(15 mg, 36.63 μmol), dissolved in tetrahydrofuran (2 mL), and then added with m-chloroperoxybenzoic acid (13.91 mg, 80.59 μmol). Under room temperature, the reaction was performed for 1 hour. Then the reaction solution was added with N,N-diisopropyl ethyl amine (47.34 mg, 366.30 μmol) and Substrate **28-5**(17.51 mg, 80.59 μmol). At 50°C, the reaction was performed overnight with LC-MS monitoring. After the reaction was completed, the reaction solution was cooled to room temperature, concentrated under reduced pressure. The residue was purified by preparative high-performance liquid chromatography to give Compound **28** (8.09 mg, 11.21 μmol). **$^1$H NMR** (400 MHz, DMSO-*d*$_6$) δ 10.02 (s, 1H), 8.80 (s, 1H), 7.88 (d, *J* = 7.6 Hz, 1H), 7.71 (d, *J* = 8.4 Hz, 1H), 7.54 (s, 2H), 6.63 (d, *J* = 8.8 Hz, 2H), 5.74 - 5.64 (m, 1H), 5.03 - 5.00 (m, 1H), 4.95 (s, 1H), 4.89 - 4.85 (m, 1H), 4.80 - 4.77 (m, 1H), 4.58 - 4.53 (m, 1H), 3.31 (s, 2H), 3.06 - 2.99 (m, 3H), 2.89 - 2.84 (m, 2H), 2.74 (d, *J* = 17.2 Hz, 1H), 2.58 - 2.55 (m, 2H), 2.40 - 2.37 (m, 2H), 2.22 (s, 3H), 1.79-1.74 (m, 1H), 1.59 - 1.54 (m, 1H), 0.98 - 0.94 (m, 1H), 0. 72 - 0.68 (m, 5H), 0. 39 - 0.31 (m, 1H). LCMS (ESI) m/z: 579.4 [M+H]+, HPLC Method B: $R_T$= 8.17, purity> 80.2%.

**Example 29: Synthesis of Compound 29**

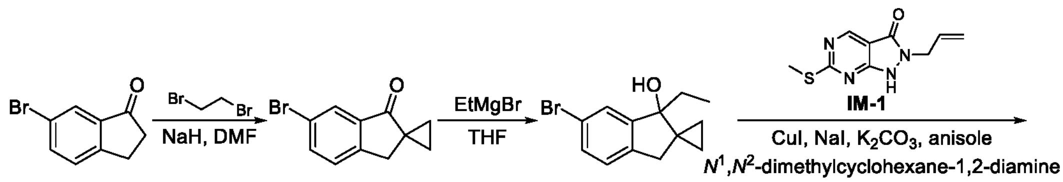


29-1 29-2 29-3

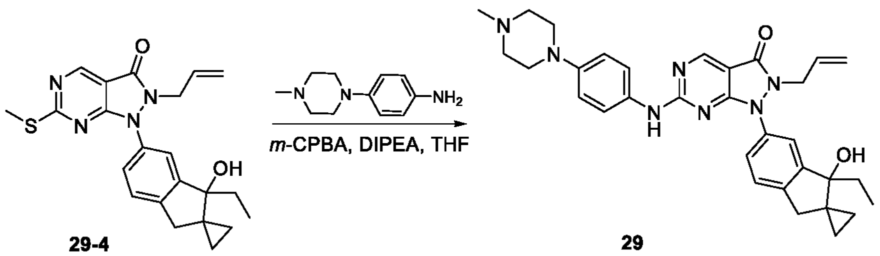


29-4 29

**Step 1: Synthesis of Compound 29-2:**

A dry three-necked flask was added with sodium hydride (696.50 mg, 17.41 mmol, 60%), added with N,N-dimethyl formamide (50 mL) to dissolve, under the protection of nitrogen atmosphere cooled to 0°C, and then slowly dropwise added with a solution of Substrate **29-1**(1.05 g, 4.98 mmol) and 1, 2-dibromoethane (3.27 g, 17.41 mmol) in N,N-dimethyl formamide (20 mL), and the temperature was kept to react for 2 hours with LC-MS monitoring. The reaction solution was added with saturated ammonium chloride aqueous solution, and then extracted with dichloromethane for four times. The organic phase was washed with water for three times, washed with saturated brine solution once. The organic phase was dried with anhydrous sodium sulfate, filtrated, concentrated under reduced pressure. The residue was purified by column chromatography to give Compound **29-2**(820 mg, 3.46 mol). LCMS (ESI) m/z: 238.2 [M+H]+.

**Step 2: Synthesis of Compound 29-3:**

A dry three-necked flask was added with Substrate **29-2**(800 mg, 3.46 mol), added with tetrahydrofuran (10 mL) to dissolve, under the protection of nitrogen atmosphere cooled to 0°C, and then slowly dropwise added with a solution of ethylmagnesium bromide in tetrahydrofuran (2 M, 6.92 mL), and the temperature was kept to react for 1 hour with LC-MS monitoring. The reaction solution was added with saturated ammonium chloride aqueous solution, extracted with ethyl acetate for three times. The organic phase was dried with anhydrous sodium sulfate, filtrated, concentrated under reduced pressure. The residue was purified by column chromatography to give Compound **29-3**(665 mg, 2.49 mmol). LCMS (ESI) m/z: 268.1 [M+H]+.

**Step 3: Synthesis of Compound 29-4:**

A dry microwave tube was added with Substrate **29-3**(30 mg, 112.29 μmol), Substrate **IM-1**(26.21 mg, 117.91 μmol), copper (I) iodide (42.77 mg, 224.58 μmol), sodium iodide (33.66 mg, 224.58 μmol), potassium carbonate (38.80 mg, 280.73 μmol) and trans-(1R,2R)-N,N'-dimethyl-1,2-cyclohexylene diamine (63.89 mg, 449.17 μmol), and then added with anisole (1 mL). Under nitrogen atmosphere, the reaction was heated by microwave to 130 °C for 3.5 hours with LC-MS monitoring. The reaction solution was cooled to room temperature, filtrated, concentrated under reduced pressure. The residue was purified by column chromatography to give Compound **29-4**(8 mg, 19.58 μmol). LCMS (ESI) m/z: 409.1 $[M+H]^+$.

**Step 4: Synthesis of Compound 29:**

A dry single-necked flask was added with Substrate **29-4**(8 mg, 19.58 μmol), dissolved in tetrahydrofuran (1 mL), and then added with m-chloroperoxybenzoic acid (6.08 mg, 35.25 μmol). Under room temperature, the reaction was performed for 1 hour. Then the reaction solution was added with N,N-diisopropyl ethyl amine (25.31 mg, 195.83 μmol) and Substrate 4-(4-methylpiperazino)aniline (7.49 mg, 39.17 μmol), reacted at 45°C overnight with LC-MS monitoring. After the reaction was completed, the reaction solution was cooled to room temperature, concentrated under reduced pressure. The residue was purified by preparative high-performance liquid chromatography to give Compound **29**(2.9 mg, 4.38 μmol). **$^1$H NMR** (400 MHz, DMSO-$d_6$) δ 10.05 (s, 1H), 8.80 (s, 1H), 7.55 (s, 2H), 7.41 - 7.29 (m, 2H), 7.24 (s, 1H), 6.85 (d, *J* = 9.2 Hz, 2H), 5.74 - 5.63 (m, 1H), 5.13 - 5.07 (m, 1H), 4.94 (d, *J* = 17.2 Hz, 1H), 4.86 (s, 1H), 4.23 (d, *J* = 12.0 Hz, 1H), 3.05 (t, *J* = 5.2 Hz, 4H), 2.45 (d, *J* = 4.8 Hz, 4H), 2.22 (s, 3H), 1.67 (dd, *J* = 13.9, 7.2 Hz, 1H), 1.59 - 1.54 (m, 1H), 1.23 (s, 3H), 0.93 (d, *J* = 6.4 Hz, 1H), 0.72 (t, *J* = 7.2 Hz, 3H), 0.67 (d, *J* = 7.2 Hz, 2H), 0.33 (t, *J* = 5.2 Hz, 1H). LCMS (ESI) m/z: 552.3 $[M+H]^+$, HPLC Method B: $R_T$= 7.57, purity> 83.3%.

**Example 30: Synthesis of Compound 30**

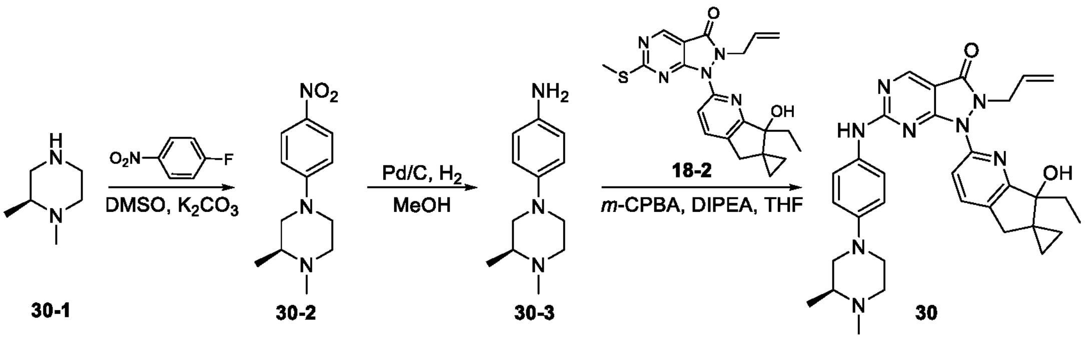


30-1 30-2 30-3 30

**Step 1: Synthesis of Compound 30-2:**

A dry single-necked flask was added with Substrate **30-1**(230 mg, 2.01 mmol), dissolved in dimethyl sulfoxide (5 mL), and then added with p-fluoro-nitrobenzene (298.42 mg, 2.11 mmol) and potassium carbonate (1.39 g, 10.07 mmol). At 100°C, the reaction was performed for 4 hours with LC-MS monitoring for complete reaction. The reaction solution was cooled to room temperature, added with water, and then extracted with ethyl acetate for four times. The organic phase was dried with anhydrous sodium sulfate, filtrated, concentrated under reduced pressure. The residue was purified by column chromatography to give Compound **30-2**(450 mg, 1.91 mmol). LCMS (ESI) m/z: 236.2 $[M+H]^+$.

**Step 2: Synthesis of Compound 30-3:**

A dry single-necked flask was added with Substrate **30-2**(450 mg, 1.91 mmol), dissolved in methanol (3 mL), and then added with Pd-C (40 mg). Under hydrogen atmosphere, the reaction was performed under room temperature for 2 hours with LC-MS monitoring for complete reaction. The reaction solution was filtrated with celite, washed with methanol twice. The filtrate was concentrated under reduced pressure to give Compound **30-3**(380 mg, 1.85 mmol). LCMS (ESI) m/z: 206.1 $[M+H]^+$.

**Step 3: Synthesis of Compound 30:**

A dry single-necked flask was added with Substrate **18-2**(20 mg, 48.84 μmol), dissolved in tetrahydrofuran (2 mL), and then added with m-chloroperoxybenzoic acid (15.17 mg, 87.91 μmol). Under room temperature, the reaction was performed for 1 hour. Then the reaction solution was added with N,N-diisopropyl ethyl amine (63.12 mg, 488.40 μmol) and Substrate **30-3**(20.05 mg, 97.68 μmol). At 50°C, the reaction was performed overnight with LC-MS monitoring. After the reaction was completed, the reaction solution was cooled to room temperature, concentrated under reduced pressure. The residue was purified by preparative high-performance liquid chromatography to give Compound **30**(4.9 mg, 8.32 μmol). **$^1$H NMR** (400 MHz, DMSO-$d_6$) δ 10.14 (s, 1H), 8.83 (s, 1H), 7.92 (s, 1H), 7.71 (d, *J* = 8.0 Hz, 1H), 7.58 (s, 2H), 6.92 (d, *J* = 8.8 Hz, 2H), 5.73 - 5.63 (m, 1H), 5.02 (dd, *J* = 10.2, 1.5 Hz, 1H), 4.97 (s, 1H), 4.87 (d, *J* = 17.2 Hz, 1H), 4.78 (s, 1H), 4.56 (d, *J* = 15.6 Hz, 1H), 3.48 (s, 2H), 3.02 (d, *J* = 16.4 Hz, 1H), 2.84 - 2.69 (m, 4H), 2.37 - 2.30 (m, 2H), 2.21 (s, 3H), 1.77 (dd, *J* = 13.6, 7.2 Hz, 1H), 1.56 (dd, *J* = 13.6, 7.2 Hz, 1H), 1.05 (d, *J* = 6.0 Hz, 3H), 0.98 - 0.91 (m, 1H), 0.70 (t, *J* = 7.2 Hz, 5H), 0.42 - 0.31 (m, 1H). LCMS (ESI) m/z: 567.3 [M+H]$^+$, HPLC Method B: $R_T$= 7.57, purity> 96.1%.

**Example 31: Synthesis of Compound 31**

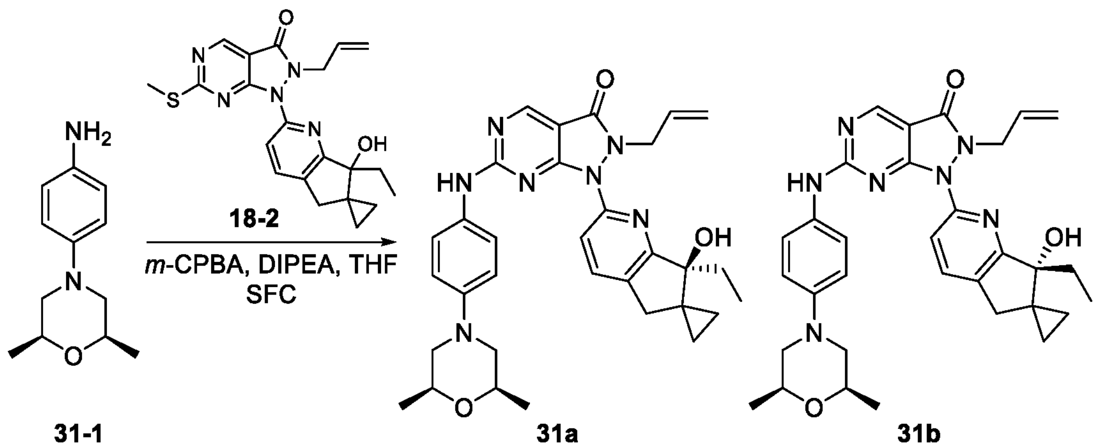


31-1 31a 31b

**Step 1: Synthesis of Compound 31a & 31b:**

A dry single-necked flask was added with Substrate **18-2**(20 mg, 48.84 μmol), dissolved in tetrahydrofuran (2 mL), and then added with m-chloroperoxybenzoic acid (15.18 mg, 87.91 μmol). Under room temperature, the reaction was performed for 1 hour. Then the reaction solution was added with N,N-diisopropyl ethyl amine (63.00 mg, 488.40 μmol) and Substrate **31-1**(12.09 mg, 58.61 μmol). At 50°C, the reaction was performed overnight with LC-MS monitoring. After the reaction was completed, the reaction solution was cooled to room temperature, concentrated under reduced pressure. The residue was purified by preparative high-performance liquid chromatography to give the product, and then chirally resolved by supercritical fluid chromatography to give Compound **31a**(5.1 mg, 8.92 μmol); SFC residence time t = 2.264 min. **$^1$H NMR** (400 MHz, DMSO-$d_6$) δ 10.16 (s, 1H), 8.83 (s, 1H), 7.91 (s, 1H), 7.72 - 7.70 (m, 1H), 7.60 (s, 2H), 6.94 - 6.92 (d, *J* = 8.8 Hz, 2H), 5.74 - 5.64 (m, 1H), 5.03 - 5.00 (m, 1H), 4.97 (s, 1H), 4.89 - 4.85 (m, 1H), 4.82 - 4.72 (m, 1H), 4.58 - 4.53 (m, 1H), 3.74 - 3.66 (m, 2H), 3.55 - 3.50 (m, 2H), 3.05 - 2.98 (m, 1H), 2.76 - 2.72 (m, 1H), 2.24 - 2.18 (m, 2H), 1.79 - 1.74 (m, 1H), 1.59 - 1.53 (m, 1H), 1.19 - 1.26 (m, 6H), 0.98 - 0.93 (m, 1H), 0.72 - 0.68 (m, 5H), 0.41 - 0.32 (m, 1H). LCMS (ESI) m/z: 568.4 [M+H]$^+$, HPLC Method B: $R_T$=8.29 min, purity> 99.3%.

Compound 31b(5.1 mg, 17.60 μmol); SFC residence time t = 3.730 min. **$^1$H NMR** (400 MHz, DMSO-$d_6$) δ 10.16 (s, 1H), 8.83 (s, 1H), 8.00 - 7.88 (m, 1H), 7.72 - 7.70 (m, 1H), 7.66 - 7.55 (m, 2H), 6.94 - 6.91 (d, *J* = 8.8 Hz, 2H), 5.74 - 5.64 (m, 1H), 5.03 - 5.00 (m, 1H), 4.97 (s, 1H), 4.89 - 4.85 (m, 1H), 4.85 - 4.72 (m, 1H), 4.63 - 4.50 (m, 1H), 3.74 - 3.66 (m, 2H), 3.55 - 3.50 (m, 2H), 3.06 - 2.98 (m, 1H), 2.79 - 2.71 (m, 1H), 2.26 - 2.17 (m, 2H), 1.82 - 1.72 (m, 1H), 1.61 - 1.51 (m, 1H), 1.19 - 1.12 (m, 6H), 0.98 - 0.92 (m, 1H), 0.74 - 0.66 (m, 5H), 0.43 - 0.32 (m, 1H). LCMS (ESI) m/z: 568.3 [M+H]$^+$, HPLC Method B: $R_T$= 8.30 min, purity> 95.7%.

**Example 32: Synthesis of Compound 32**

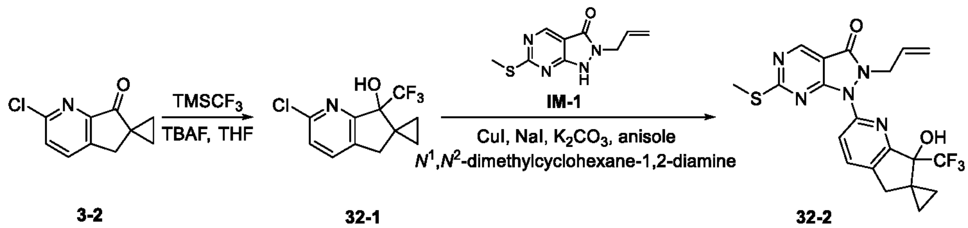


3-2 32-1 32-2

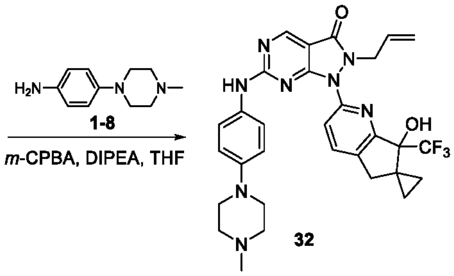


32

**Step 1: Synthesis of Compound 32-1:**

A dry three-necked flask was added with Substrate **3-2**(200 mg, 1.03 mol), added with tetrahydrofuran (5 mL) to dissolve, under the protection of nitrogen atmosphere, cooled to 0°C, and then slowly dropwise added with (trifluoromethyl)trimethylsilane (732.28 mg, 5.15 mol) and tetrabutylammonium fluoride (1.03 mL, 1M), and the temperature was kept to react for 1 hour with LC-MS monitoring. The reaction solution was concentrated under reduced pressure. The residue was purified by column chromatography to give Compound **32-1**(220 mg, 834.47 μmol). LCMS (ESI) m/z: 264.1 $[M+H]^+$.

**Step 2: Synthesis of Compound 32-2:**

A dry microwave tube was added with Substrate **32-1**(220 mg, 834.47 μmol), Substrate **IM-1**(194.75 mg, 876.19 μmol), copper (I) iodide (318.05 mg, 1.67 mmol), sodium iodide (250.32 mg, 1.67 mmol), potassium carbonate (288.86 mg, 2.09 mmol) and trans-(1R,2R)-N,N'-dimethyl-1,2-cyclohexylene diamine (475.08 mg, 3.34 mmol), and then added with anisole (4 mL). Under nitrogen atmosphere, the reaction was heated by microwave to 130°C for 3.5 hours with LC-MS monitoring. The reaction solution was cooled to room temperature, filtrated, concentrated under reduced pressure. The residue was purified by column chromatography to give Compound **32-2**(150 mg, 333.74 μmol). LCMS (ESI) m/z: 450.2 $[M+H]^+$.

**Step 3: Synthesis of Compound 32:**

A dry single-necked flask was added with Substrate **32-2**(20 mg, 44.50 μmol), dissolved in tetrahydrofuran (2 mL), and then added with m-chloroperoxybenzoic acid (13.82 mg, 80.10 μmol). Under room temperature, the reaction was performed for 1 hour. Then the reaction solution was added with N,N-diisopropyl ethyl amine (57.40 mg, 444.99 μmol) and Substrate 4-(4-methylpiperazino)aniline (**1-8**, 10.21 mg, 53.40 μmol). At 50°C, the reaction was performed overnight with LC-MS monitoring. After the reaction was completed, the reaction solution was cooled to room temperature, concentrated under reduced pressure. The residue was purified by preparative high-performance liquid chromatography to give Compound: **32**(6.2 mg, 9.65 μmol). **$^1$H NMR** (400 MHz, DMSO-$d_6$) δ 10.21 (s, 1H), 8.84 (s, 1H), 8.14 - 8.00 (m, 1H), 7.94 - 7.87 (m, 1H), 7.67 - 7.50 (m, 2H), 6.96 - 6.90 (m, 2H), 6.80 - 6.60 (m, 1H), 5.74 - 5.60 (m ,1H), 5.03 - 4.94 (m, 1H), 4.90 - 4.72 (m, 2H), 4.63 - 4.50 (m, 1H), 3.14 - 3.04 (m, 4H), 2.78 - 2.69 (m, 1H), 2.48 - 2.41 (m, 4H), 2.22 (s, 3H), 1.94 - 1.76 (m, 1H), 1.09 - 0.99 (m, 2H), 0.94 - 0.86 (m, 1H), 0.52 - 0.44 (m, 1H). LCMS (ESI) m/z: 593.3 $[M+H]^+$, HPLC Method B: $R_T$= 7.79 min, purity> 92.3%.

**Example 33: Synthesis of Compound 33**

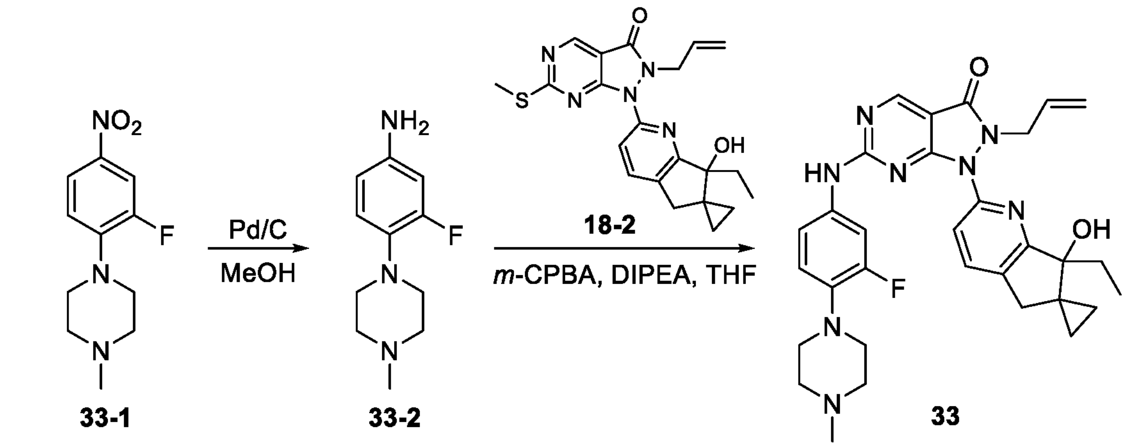


33-1 33-2 33

**Step 1: Synthesis of Compound 33-2:**

A dry single-necked flask was added with Substrate **33-1**(160 mg, 668.77 μmol), dissolved in methanol (5 mL), and then added with Pd-C (20 mg). Under hydrogen atmosphere, the reaction was performed under room temperature for 2 hours with LC-MS monitoring for complete reaction. The reaction solution was filtrated with celite, washed with methanol twice. The filtrate was concentrated under reduced pressure to give Compound **33-2**(68 mg, 324.95 μmol). LCMS (ESI) m/z: 210.2 $[M+H]^+$.

**Step 2: Synthesis of Compound 33:**

A dry single-necked flask was added with Substrate **18-2**(20 mg, 36.63 μmol), dissolved in tetrahydrofuran (2 mL), and then added with m-chloroperoxybenzoic acid (16.55 mg, 65.93 μmol). Under room temperature, the reaction was performed for 1 hour. Then the reaction solution was added with N,N-diisopropyl ethyl amine (45.56 mg, 352.52 μmol) and Substrate **33-2**(30 mg, 143.36 μmol). At 50°C, the reaction was performed overnight with LC-MS monitoring. After the reaction was completed, the reaction solution was cooled to room temperature, concentrated under reduced pressure. The residue was purified by preparative high-performance liquid chromatography to give Compound **33**(6.37 mg, 11.16 μmol). **$^1$H NMR** (400 MHz, Chloroform-*d*) δ 8.78 (s, 1H), 7.81 - 7.70 (m, 3H), 6.99 - 6.85 (m, 2H), 5.75 - 5.65 (m, 1H), 5.03 (d, *J* = 10.4 Hz, 1H), 4.95 - 4.85 (m, 2H), 4.70 - 4.64 (m, 1H), 3.18 - 3.10 (m, 5H), 2.80 - 2.76 (m, 5H), 2.47 (s, 3H), 1.99 - 1.90 (m, 1H), 1.79 - 1.70 (m, 1H), 1.19 - 1.14 (m, 1H), 0.94 - 0.89 (m, 1H), 0.85 - 0.79 (m, 4H), 0.51 - 0.46 (m, 1H). LCMS (ESI) m/z: 571.3 $[M+H]^+$, HPLC Method B: $R_T$= 7.71 min, purity> 87.6%.

**Example 34: Synthesis of Compound 34**

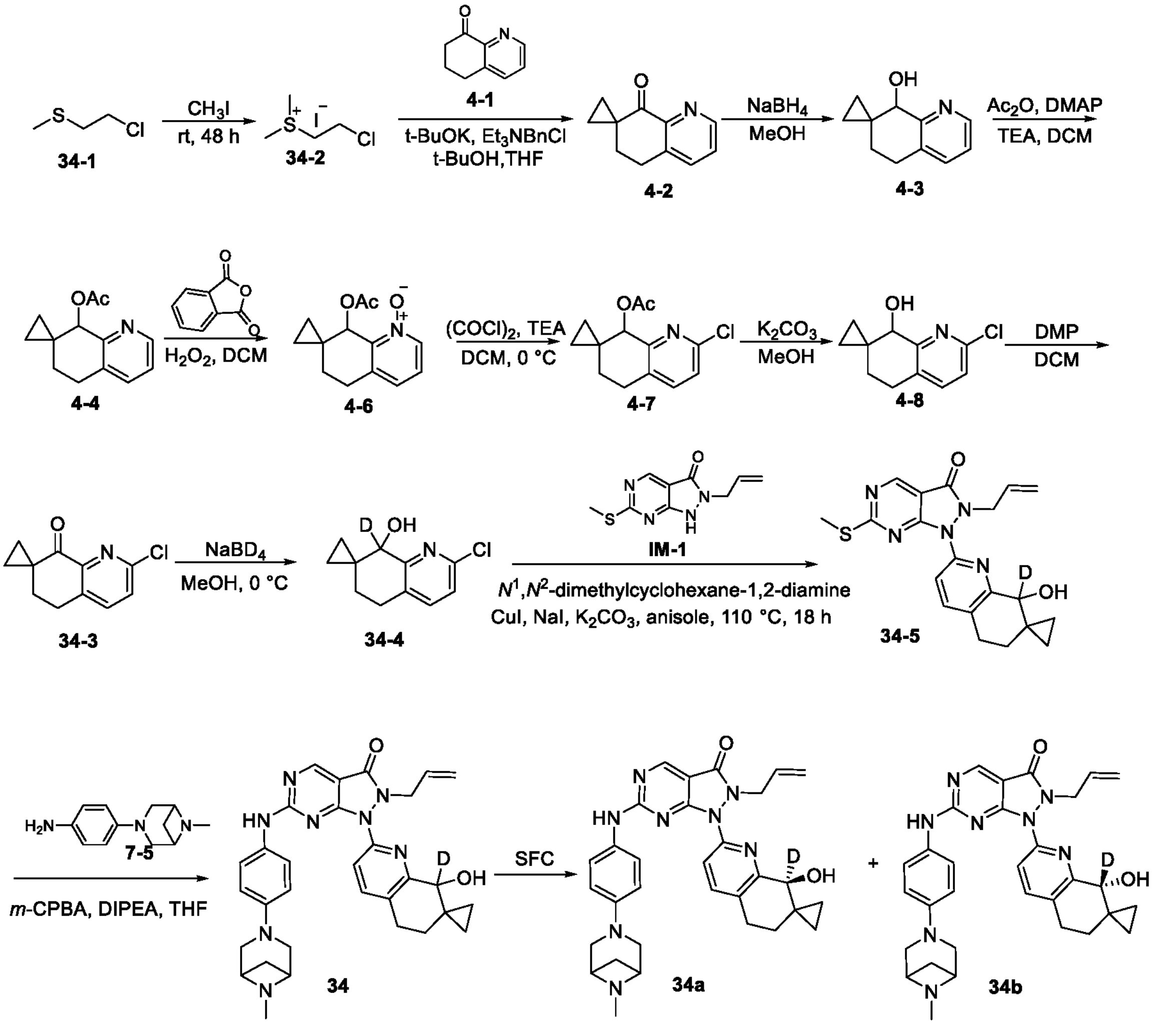


**Step 1: Synthesis of Compound 34-2**:

A dry single-necked flask was added with Substrate **34-1**(100.00 g, 904.08 mmol), and then added with iodomethane (256.65 g, 1.81 mol). The reaction was performed under room temperature for 48 hours, to give yellow solid. The reaction solution was added with methyl-tert-butyl ether, slurried, filtrated, dried in vaccuo to give Compound **34-2**(189.00 g, 748.40 mmol).

**Step 2: Synthesis of Compound 4-2:**

A dry three-necked flask was added with Substrate **4-1**(30.00 g, 203.84 mmol), and then added with tetrahydrofuran (750 mL) and t-butanol (1500 mL) to dissolve, under the protection of nitrogen atmosphere, cooled to -40°C. The reaction solution was dropwise added with a solution of potassium t-butoxide (73.19 g, 652.29 mmol) in tetrahydrofuran (650 mL) solution, and the temperature was kept constant to react for 40 minutes, and then added with triethylbenzylammonium chloride (4.64 g, 20.38 mmol) and Substrate **34-2**(77.22 g, 305.72 mmol). Under room temperature, the reaction was performed for 18 hours with TLC monitoring for complete reaction. The reaction solution was slowly added with saturated ammonium chloride solution, concentrated under reduced pressure to remove the organic phase. The aqueous phase was extracted with dichloromethane for four times. The organic phase was dried with anhydrous sodium sulfate, filtrated, concentrated under reduced pressure. The residue was purified by column chromatography (petroleum ether: ethyl acetate=1:1 elution) to give Compound **4-2**(11.00 g, 63.51 mmol). LCMS ($ESI^+$) m/z: 174.1 $[M+H]^+$.

Compound **4-3 ~ 4-8** synthesis can be referred to **Example 4,** synthesis method

**Step 3: Synthesis of Compound 34-3:**

A dry single-necked flask was added with Substrate **4-8**(27.50 g, 131.12 mmol), added with dichloromethane (300 mL) to dissolve, and cooled with ice bath, added with Dess-Martin oxidant (83.42 g, 196.68 mmol). Under room temperature, the reaction was performed for 2 hours with TLC monitoring for complete reaction. The reaction solution was added with saturated sodium bicarbonate aqueous solution to adjust pH to 8, and then extracted with dichloromethane for three times. The organic phase was dried with anhydrous sodium sulfate, filtrated, concentrated under reduced pressure. The residue was purified by column chromatography (petroleum ether: ethyl acetate=2:1 elution) to give Compound **34-3** (18.55 g, 89.33 mmol). LCMS ($ESI^+$) m/z: 208.1 $[M+H]^+$.

**Step 4: Synthesis of Compound 34-4:**

A dry single-necked flask was added with Substrate **34-3**(18.55 g, 89.33 mmol), and then added with methanol (270 mL) to dissolve, cooled to 0°C, slowly added with sodium borodeuteride (4.48 g, 107.20 mmol), and the temperature was kept constant to react for 1 hour with TLC monitoring for complete reaction. The reaction solution was concentrated under reduced pressure. The residue was added with water, extracted with dichloromethane for three times. The organic phase was dried with anhydrous sodium sulfate, filtrated, concentrated under reduced pressure to give Compound **34-4** (18.00g, 85.44 mmol). LCMS ($ESI^+$) m/z: 211.0 $[M+H]^+$. **$^1$H NMR** (400 MHz, DMSO-$d_6$) δ 7.63 (d, *J* = 8.0 Hz, 1H), 7.32 (d, *J* = 8.0 Hz, 1H), 5.35 (s, 1H), 2.86 - 2.67 (m, 2H), 2.22 - 2.15 (m, 1H), 1.12 - 1.06 (m, 1H), 0.73 - 0.62 (m, 1H), 0.44 - 0.40 (m, 1H), 0.38 - 0.30 (m, 2H).

**Step 5: Synthesis of Compound 34-5:**

A dry sealed tube was added with Substrate **34-4**(5 g, 23.73 mmol), Substrate **IM-1**(5.55 g, 24.92 mmol), copper (I) iodide (4.5 g, 23.73 mmol), sodium iodide (7.14 g, 47.46 mmol), potassium carbonate (8.2 g, 59.33 mmol) and trans-(1R,2R)-N,N'-dimethyl-1,2-cyclohexylene diamine (6.76 g, 47.46 mmol), and then added with anisole (80 mL). Under nitrogen atmosphere, the reaction was performed at 110°C for 18 hours with TLC monitoring for complete reaction. The reaction solution was cooled to room temperature, filtrated, washed with ethyl acetate twice. The filrate was washed with ammonia solution twice, washed with saturated brine solution twice. The organic phase was dried with anhydrous sodium sulfate, filtrated, concentrated under reduced pressure. The residue was purified by column chromatography (petroleum ether: ethyl acetate=1:1 elution) to give Compound **34-5**(4.60 g, 11.60 mmol). LCMS ($ESI^+$) m/z: 397.1 $[M+H]^+$.

**Step 6: Synthesis of Compound 34 & 34a & 34b:**

A dry single-necked flask was added with Substrate **34-5**(57 mg, 143.77 μmol), dissolved in tetrahydrofuran (5 mL), and then added with m-chloroperoxybenzoic acid (44.66 mg, 258.78 μmol). Under room temperature, the reaction was performed for 1 hour. Then the reaction solution was added with N,N-diisopropyl ethyl amine (46 mg, 177.34 μmol) and Substrate **7-5** (43.84 mg, 215.66 μmol). At 50°C, the reaction was performed overnight with LC-MS monitoring. After the reaction was completed, the reaction solution was cooled to room temperature, concentrated under reduced pressure. The residue was purified by preparative high-performance liquid chromatography to give Compound **34**(4.2 mg, 6.40 μmol). **$^1$H NMR** (400 MHz, DMSO-$d_6$) δ 10.06 (s, 1H), 8.78 (s, 1H), 7.81 (d, *J* = 8.4 Hz, 1H), 7.72 (d, *J* = 8.4 Hz, 1H), 7.66 - 7.45 (m, 2H), 6.71 (d, *J* = 8.8 Hz, 2H), 5.73 - 5.64 (m, 1H), 5.28 (s, 1H), 5.00 (dd, *J* = 10.0, 1.6 Hz, 1H), 4.89 (dd, *J* = 17.2, 1.6 Hz, 1H), 4.74 - 4.56 (m, 2H), 3.59 (d, *J* = 6.0 Hz, 2H), 3.44 (d, *J* = 11.2 Hz, 2H), 3.29 - 3.25 (m, 2H), 2.88 - 2.80 (m, 2H), 2.47 - 2.39 (m, 1H), 2.29 - 2.22 (m, 1H), 2.00 (s, 3H), 1.54 (d, *J* = 8.4 Hz, 1H), 1.16 - 1.08 (m, 1H), 0.71 - 0.64 (m, 1H), 0.45 - 0.40 (m, 1H), 0.40 - 0.31 (m, 2H). LCMS (ESI) m/z: 552.3 $[M+H]^+$, HPLC Method B: $R_T$= 7.26 min, purity> 84.0%.

**34** was synthesized by a similar method, and then chirally resolved by supercritical fluid chromatography to give Compound **34a**(10.1 mg, 18.31 μmol); SFC residence time t = 4.312 min. **$^1$H NMR** (600 MHz, DMSO-$d_6$) δ 10.07 (s, 1H), 8.78 (s, 1H), 7.81 (d, *J* = 8.4 Hz, 1H), 7.72 (d, *J* = 8.4 Hz, 1H), 7.61 (s, 2H), 6.71 (d, *J* = 8.4 Hz, 2H), 5.74 - 5.64 (m, 1H), 5.29 (s, 1H), 5.00 (d, *J* = 10.2 Hz, 1H), 4.89 (d, *J* = 17.4 Hz, 1H), 4.70 (s, 1H), 4.64 - 4.54 (m, 1H), 3.59 (d, *J* = 6.0 Hz, 2H), 3.44 (d, *J* = 11.4 Hz, 2H), 3.28 (d, *J* = 10.8 Hz, 2H), 2.92 - 2.81 (m, 2H), 2.43 (d, *J* = 7.2 Hz, 1H), 2.41 - 2.29 (m, 1H), 2.00 (s, 3H), 1.54 (d, *J* = 8.4 Hz, 1H), 1.15 - 1.07 (m, 1H), 0.72 - 0.63 (m, 1H), 0.45 - 0.41 (m, 1H), 0.40 - 0.31 (m, 2H). LCMS (ESI) m/z: 552.3 $[M+H]^+$, HPLC Method B: $R_T$= 7.29 min, purity> 94.5%.

Compound **34b**(10.5 mg, 19.03 μmol); SFC residence time t = 7.619 min. **$^1$H NMR** (600 MHz, DMSO-$d_6$) δ 10.07 (s, 1H), 8.79 (s, 1H), 7.81 (d, *J* = 8.4 Hz, 1H), 7.72 (d, *J* = 8.4 Hz, 1H), 7.61 (s, 2H), 6.71 (d, *J* = 9.0 Hz, 2H), 5.73 - 5.63 (m, 1H), 5.40 - 5.18 (m, 1H), 5.00 (d, *J* = 10.2 Hz, 1H), 4.89 (d, *J* = 17.4 Hz, 1H), 4.76 - 4.54 (m, 2H), 3.59 (d, *J* = 6.0Hz, 2H), 3.44 (d, *J* = 11.4 Hz, 2H), 3.28 (d, *J* = 10.8 Hz, 2H), 2.91 - 2.81 (m, 2H), 2.44 (d, *J* = 7.2 Hz, 1H), 2.41 - 2.30 (m, 1H), 2.00 (s, 3H), 1.54 (d, *J* = 8.4 Hz, 1H), 1.14 - 1.08 (m, 1H), 0.71 - 0.63 (m, 1H), 0.45 - 0.41 (m, 1H), 0.40 - 0.31 (m, 2H). LCMS (ESI) m/z: 552.3 $[M+H]^+$, HPLC Method B: $R_T$= 7.38 min, purity> 84.4%.

**Example 35: Synthesis of Compound 35**

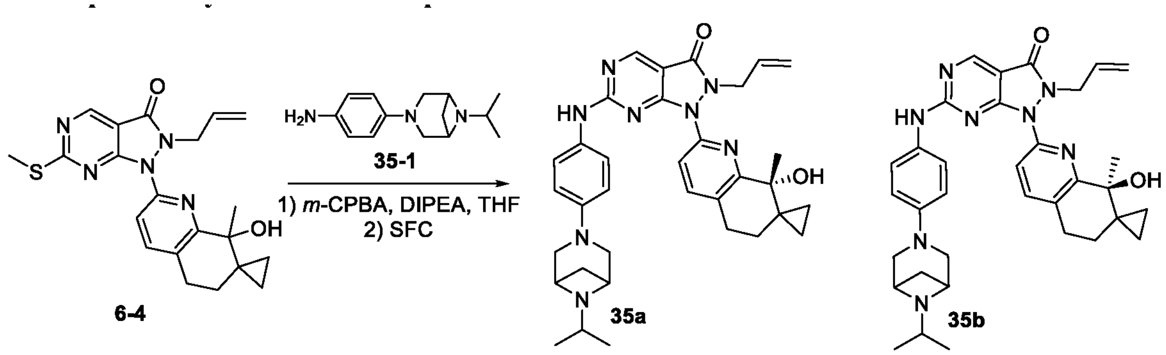


6-4 35a 35b

**Step 1: Synthesis of Compound 35a & 35b**

A dry single-necked flask was added with Substrate **6-4**(40 mg, 97.68 μmol), dissolved in tetrahydrofuran (4 mL), and then added with m-chloroperoxybenzoic acid (30.34 mg, 175.82 μmol). Under room temperature, the reaction was performed for 1 hour. Then the reaction solution was added with N,N-diisopropyl ethyl amine (126.24 mg, 976.79 μmol) and Substrate **35-1**(25.22 mg, 131.87 μmol). The reaction was performed at 45°C overnight with LC-MS monitoring. After the reaction was completed, the reaction solution was cooled to room temperature, concentrated under reduced pressure. The residue was purified by preparative high-performance liquid chromatography to give the product, and then chirally resolved by supercritical fluid chromatography to give Compound **35a**(10.0 mg, 14.69 μmol); SFC residence time t = 3.112 min. **$^1$H NMR** (400 MHz, DMSO-$d_6$) δ 10.05 (s, 1H), 8.79 (s, 1H), 7.79 - 7.67 (m, 2H), 7.59 (s, 2H), 6.69 (d, *J* = 8.8 Hz, 2H), 5.74 - 5.62 (m, 1H), 5.02 - 4.98 (m, 1H), 4.90 - 4.73 (m, 2H), 4.68 (s, 2H), 3.71 (d, *J* = 6.0 Hz, 2H), 3.42 (d, *J* = 11.2 Hz, 2H), 3.24 (d, *J* = 11.2 Hz, 2H), 2.93 - 2.75 (m, 2H), 2.49 - 2.44 (m, 1H), 2.35 (q, *J* = 6.4 Hz, 1H), 2.04 - 1.93 (m, 1H), 1.50 (d, *J* = 8.0 Hz, 1H), 1.40 (s, 4H), 0.88 (d, *J* = 6.0 Hz, 7H), 0.66 - 0.58 (m, 1H), 0.36 - 0.27 (m, 1H), 0.25 - 0.17 (m, 1H). LCMS (ESI) m/z: 593.2 $[M+H]^+$, HPLC Method B: $R_T$= 8.55 min, purity> 85.5%.

Compound **35b**(10.0 mg, 14.21 μmol); SFC residence time t = 4.854 min. **$^1$H NMR** (400 MHz, DMSO-$d_6$) δ 10.05 (s, 1H), 8.79 (s, 1H), 7.87 - 7.67 (m, 2H), 7.59 (s, 2H), 6.69 (d, *J* = 8.8 Hz, 2H), 5.74 - 5.64 (m, 1H), 4.99 (dd, *J* = 10.4, 1.6 Hz, 1H), 4.91 - 4.75 (m, 2H), 4.72 - 4.57 (m, 2H), 3.71 (d, *J* = 6.0 Hz, 2H), 3.42 (d, *J* = 11.2 Hz, 2H), 3.24 (d, *J* = 11.2 Hz, 2H), 2.93 - 2.77 (m, 2H), 2.49 - 2.44 (m, 1H), 2.35 (q, *J* = 6.4 Hz, 1H), 2.05 - 1.91 (m, 1H), 1.50 (d, *J* = 8.0 Hz, 1H), 1.40 (s, 4H), 0.88 (d, *J* = 6.0 Hz, 7H), 0.65 - 0.58 (m, 1H), 0.34 - 0.27 (m, 1H), 0.23 - 0.17 (m, 1H). LCMS (ESI) m/z: 593.2 $[M+H]^+$, HPLC Method B: $R_T$= 8.59 min, purity> 84.0%.

**Example 36: Synthesis of Compound 36**

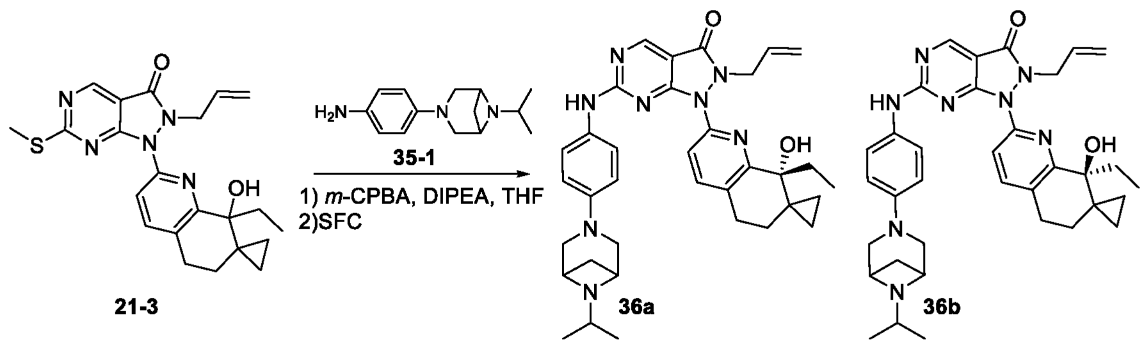


21-3 36a 36b

**Step 1: Synthesis of Compound 36a & 36b**

A dry single-necked flask was added with Substrate **21-3**(45 mg, 106.25 μmol), dissolved in tetrahydrofuran (2 mL), and then added with m-chloroperoxybenzoic acid (33.00 mg, 191.25 μmol). Under room temperature, the reaction

was performed for 1 hour. Then the reaction solution was added with N,N-diisopropyl ethyl amine (137.32 mg, 1.06 mmol) and Substrate **35-1**(49.16 mg, 212.50 μmol). The reaction was performed at 45°C overnight with LC-MS monitoring. After the reaction was completed, the reaction solution was cooled to room temperature, concentrated under reduced pressure. The residue was purified by preparative high-performance liquid chromatography to give the product, and then chirally resolved by supercritical fluid chromatography to give Compound **36a**(14.24 mg, 23.47 μmol); SFC residence time t = 2.868 min. **$^1$H NMR** (400 MHz, DMSO-$d_6$) δ 10.05 (s, 1H), 8.78 (s, 1H), 7.77 (d, *J* = 8.8 Hz, 1H), 7.68 (d, *J* = 8.4 Hz, 1H), 7.58 (s, 2H), 6.69 (d, *J* = 8.8 Hz, 2H), 5.74 - 7.63 (m, 1H), 5.03 - 4.96 (m, 1H), 4.85 (d, *J* = 17.2 Hz, 1H), 4.75 (d, *J* = 16.0 Hz, 1H), 4.61 (d, *J* = 15.6 Hz, 1H), 4.40 (s, 1H), 3.71 (d, *J* = 6.0 Hz, 2H), 3.42 (d, *J* = 11.2 Hz, 2H), 3.24 (d, *J* = 11.2 Hz, 2H), 2.95 - 2.80 (m, 1H), 2.83 (dd, *J* = 17.2, 6.0 Hz, 1H), 2.47 (d, *J* = 6.0 Hz, 1H), 2.38 - 2.28 (m, 2H), 1.95 - 1.88 (m, 1H), 1.79 - 1.71 (m, 1H), 1.50 (d, *J* = 8.0 Hz, 1H), 1.17 - 1.11 (m, 1H), 0.93 (t, *J* = 7.2 Hz, 3H), 0.88 (d, *J* = 6.0 Hz, 7H), 0.61 (d, *J* = *5.6* Hz, 1H), 0.43 - 0.38 (m, 1H), 0.00 (s, 1H). LCMS (ESI) m/z: 607.2 [M+H]$^+$, HPLC Method B: $R_T$ = 9.29 min, purity > 89.7%.

Compound **36b**(13.16 mg, 21.69 μmol); SFC residence time t = 3.896 min. **$^1$H NMR** (400 MHz, DMSO-$d_6$) δ 10.05 (s, 1H), 8.78 (s, 1H), 7.77 (d, *J* = 8.4 Hz, 1H), 7.68 (d, *J* = 8.2 Hz, 1H), 7.59 (s, 2H), 6.69 (d, *J* = 8.6 Hz, 2H), 5.72 - 5.60 (m, 1H), 5.00 (dd, *J* = 10.2, 1.6 Hz, 1H), 4.85 (d, *J* = 17.1 Hz, 1H), 4.75 (d, *J* = 15.3 Hz, 1H), 4.62 (s, 1H), 4.40 (s, 1H), 3.71 (d, *J* = 5.9 Hz, 2H), 3.42 (d, *J* = 11.0 Hz, 2H), 3.24 (d, *J* = 11.0 Hz, 2H), 2.95 - 2.80 (m, 1H), 2.83 (dd, *J* = 17.0, 5.9 Hz, 1H), 2.49 - 2.44 (m, 1H), 2.38 - 2.28 (m, 2H), 1.95 - 1.88 (m, 1H), 1.79 - 1.71 (m, 1H), 1.50 (d, *J* = 8.0 Hz, 1H), 1.16 - 1.11 (m, 1H), 0.93 (t, *J* = 7.4 Hz, 3H), 0.88 (d, *J* = 6.0 Hz, 6H), 0.85 (d, *J* = 5.8 Hz, 1H), 0.65 - 0.58 (m, 1H), 0.43 - 0.38 (m, 1H), -0.00 (s, 1H). LCMS (ESI) m/z: 607.2 [M+H]$^+$, HPLC Method B: $R_T$=9.31 min, purity> 87.9%.

**Example 37: Synthesis of Compound 37**

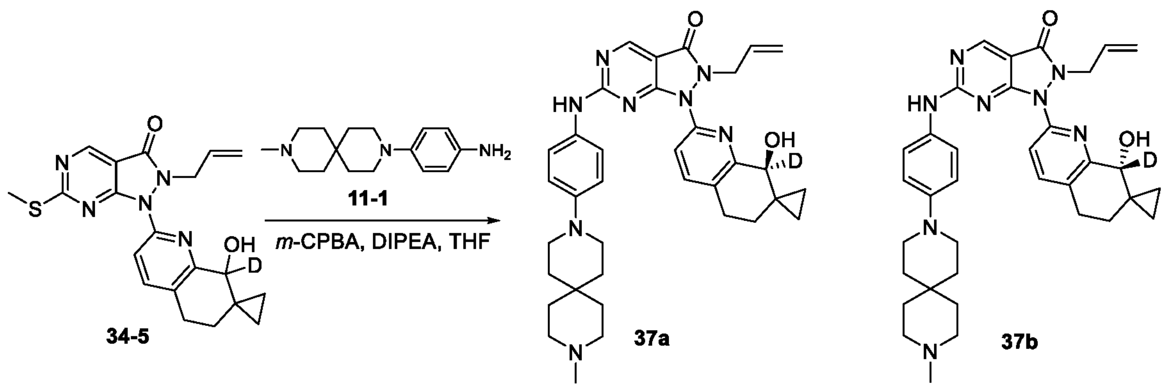


34-5 37a 37b

**Step 20: Synthesis of Compound 37a & 37b:**

A dry single-necked flask was added with Substrate **34-5**(4.10 g, 10.35 mmol), dissolved in tetrahydrofuran (55 mL), and then added with m-chloroperoxybenzoic acid (3.79 g, 18.63 mmol). Under room temperature, the reaction was performed for 1 hour. Then the reaction solution was added with N,N-diisopropyl ethyl amine (5.33 g, 41.29 mmol) and Substrate **11-1**(3.22 g, 12.41 mmol). The reaction was performed at 45°C overnight with LC-MS monitoring. After the reaction was completed, the reaction solution was cooled to room temperature, concentrated under reduced pressure. The residue was purified by preparative high-performance liquid chromatography (acetonitrile/0.01% ammonium bicarbonate aqueous solution elution, 60mL/minutes) to give the compound, and the compound is further chirally resolved by supercritical fluid chromatography to give Compound **37a**(1.30 g, 2.14 mmol ), SFC residence time t = 5.043 min. LCMS (ESI$^+$) m/z: 608.2 [M+H]$^+$, **$^1$H NMR** (600 MHz, DMSO-$d_6$) δ 10.12 (s, 1H), 8.80 (s, 1H), 7.83 (s, 1H), 7.70 (d, *J* = 8.4 Hz, 1H), 7.64 - 7.42 (m, 2H), 6.91 (d, *J* = 9.0 Hz, 2H), 5.70 - 5.64 (m, 1H), 5.30 (s, 1H), 5.00 (d, *J* = 10.2 Hz, 1H), 4.89 (d, *J* = 17.4 Hz, 1H), 4.69 - 4.62 (m, 2H), 3.11 - 3.07 (m, 4H), 2.86 - 2.81 (m, 2H), 2.27 (q, *J* = 12.6, 9.0 Hz, 5H), 2.15 (s, 3H), 1.53 (t, *J* = 5.4 Hz, 4H), 1.46 (t, *J* = 5.4 Hz, 4H), 1.13 - 1.10 (m, 1H), 0.69 - 0.65 (m, 1H), 0.49 - 0.42 (m, 1H), 0.39 - 0.32 (m, 2H), HPLC Method B: RT=8.83 min, purity > 94.1 %.

Compound **37b**(1.50 g, 2.47 mmol), SFC residence time t =9.666 min. LCMS (ESI$^+$) m/z: 608.2 [M+H]$^+$, **$^1$H NMR** (600 MHz, DMSO-$d_6$) δ 10.11 (s, 1H), 8.80 (s, 1H), 7.83 (s, 1H), 7.70 (d, *J* = 8.4 Hz, 1H), 7.66 - 7.47 (m, 2H), 6.91 (d, *J* = 8.4 Hz, 2H), 5.70 - 5.64(m, 1H), 5.29 (s, 1H), 5.00 (d, *J* = 10.2 Hz, 1H), 4.89 (d, *J* = 17.4 Hz, 1H), 4.69 (s, 1H), 4.62 (d, *J* = 15.0 Hz, 1H), 3.09 (t, *J* = 5.4 Hz, 4H), 2.88 - 2.79 (m, 2H), 2.29 (d, *J* = 6.0 Hz, 4H), 2.24 (d, *J* = 17.4 Hz, 1H), 2.16 (s, 3H), 1.54 (t, *J* = 5.4 Hz, 4H), 1.47 (t, *J* = 5.4Hz, 4H), 1.13 - 1.09 (m, 1H), 0.69 - 0.66 (m, 1H), 0.46 - 0.42 (m, 1H), 0.39 - 0.33 (m, 2H), HPLC Method B: RT=8.09 min, purity > 96.5 %.

**Example 38: Synthesis of Compound 38**

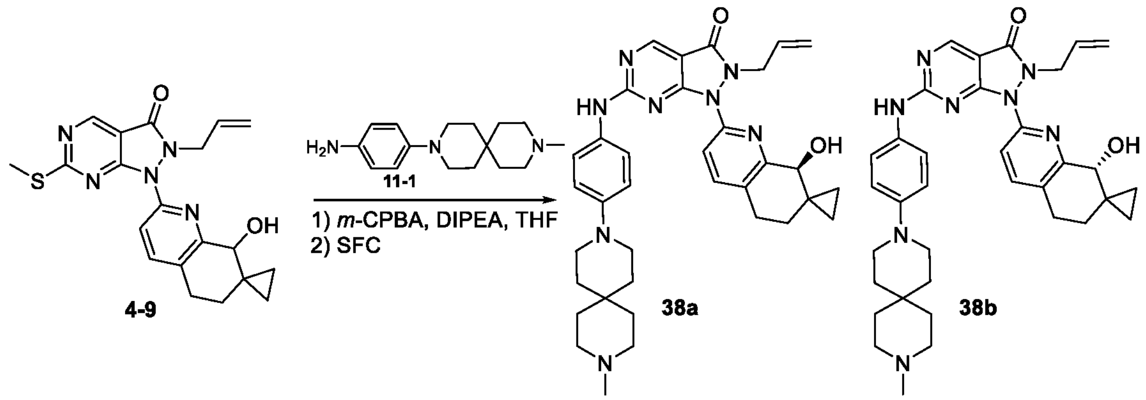


**Step 1: Synthesis of Compound 38a & 38b:**

A dry single-necked flask was added with Substrate **4-9**(80 mg, 202.29 μmol), dissolved in tetrahydrofuran (10 mL), and then added with m-chloroperoxybenzoic acid (61.60 mg, 303.43 μmol). Under room temperature, the reaction was performed for 1 hour. Then the reaction solution was added with N,N-diisopropyl ethyl amine (260.58 mg, 2.02 mmol) and Substrate **11-1**(78.71 mg, 303.43 μmol). The reaction was performed at 45°C overnight with LC-MS monitoring. After the reaction was completed, the reaction solution was cooled to room temperature, concentrated under reduced pressure. The residue was purified by preparative high-performance liquid chromatography to give the product, and then chirally resolved by supercritical fluid chromatography to give Compound **38a** (32.43 mg, 53.45 μmol); SFC residence time t = 4.513 min. **$^1$H NMR** (600 MHz, DMSO-$d_6$) δ 10.11 (s, 1H), 8.80 (s, 1H), 7.83 (d, *J* = 7.8 Hz, 1H), 7.70 (d, *J* = 8.4 Hz, 1H), 7.58 (s, 2H), 6.91 (d, *J* = 8.4 Hz, 2H), 5.67 - 5.65 (m, 1H), 5.31 (d, *J* = 5.4 Hz, 1H), 5.01 - 4.99 (m, 1H), 4.93 - 4.85 (m, 1H), 4.69 (s, 1H), 4.64 - 4.64 (m, 1H), 3.75 (d, *J* = 5.4 Hz, 1H), 3.09 - 3.07 (m, 4H), 2.87 - 2.80 (m, 2H), 2.30 - 2.26 (m, 4H), 2.24 - 2.22 (m, 1H), 2.16 (s, 3H), 1.54 - 1.52 (m, 4H), 1.47 - 1.45 (m, 4H), 1.14 - 1.10 (m, 1H), 0.69 - 0.67 (m, 1H), 0.46 - 0.41 (m, 1H), 0.41 - 0.30 (m, 2H). LCMS (ESI) m/z: 607.4 $[M+H]^+$, HPLC Method B: $R_T$= 8.39 min, purity > 94.4%.

Compound **38b** (32.44 mg, 53.46 μmol); SFC residence time t = 8.668 min. **$^1$H NMR** (600 MHz, DMSO-$d_6$) δ 10.11 (s, 1H), 8.80 (s, 1H), 7.83 (d, *J* = 7.8 Hz, 1H), 7.70 (d, *J* = 8.4 Hz, 1H), 7.58 (s, 2H), 6.91 (d, *J* = 8.4 Hz, 2H), 5.67 - 5.55 (m, 1H), 5.31 (d, *J* = 5.4 Hz, 1H), 5.00 (d, *J* = 10.2 Hz, 1H), 4.89 (d, *J* = 17.4 Hz, 1H), 4.69 (s, 1H), 4.65 - 4.52 (m, 1H), 3.75 (d, *J* = 5.4 Hz, 1H), 3.09 - 3.07 (m, 4H), 2.87 - 2.80 (m, 2H), 2.84 - 2.77 (m, 1H), 2.35 - 2.26 (m, 4H), 2.26 - 2.22 (m, 1H), 2.17 (s, 3H), 1.54 - 1.52 (m, 4H), 1.47 - 1.45 (m, 4H), 1.14 - 1.10 (m, 1H), 0.68 - 0.66 (m, 1H), 0.46 - 0.42 (m, 1H), 0.37 - 0.35 (m, 2H). LCMS (ESI) m/z: 607.4 $[M+H]^+$, HPLC Method B: $R_T$= 8.40 min, purity > 95.7%.

**Example 39: Synthesis of Compound 39**

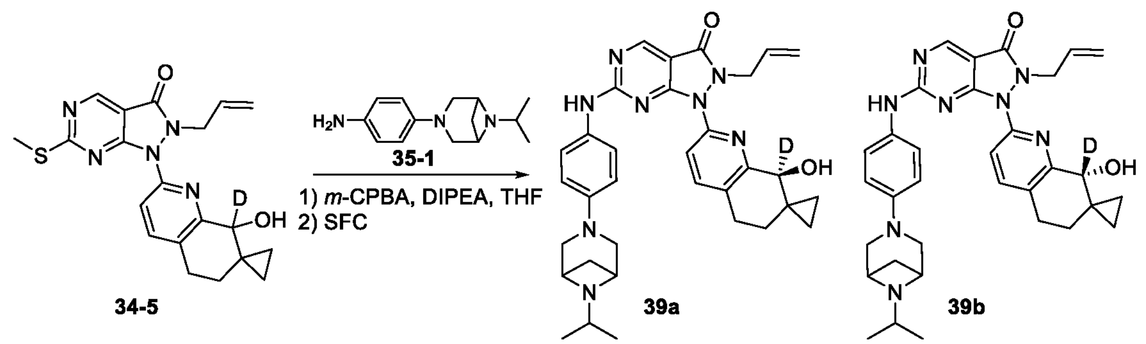


**Step 1: Synthesis of Compound 39a & 39b**

A dry single-necked flask was added with Substrate **34-5**(40 mg, 100.89 μmol), dissolved in tetrahydrofuran (5

mL), and then added with m-chloroperoxybenzoic acid (36.87 mg, 181.60 μmol). Under room temperature, the reaction was performed for 1 hour. Then the reaction solution was added with N,N-diisopropyl ethyl amine (130.39 mg, 1.01 mmol) and Substrate **35-1**(46.68 mg, 201.77 μmol). The reaction was performed at 45°C overnight with LC-MS monitoring. After the reaction was completed, the reaction solution was cooled to room temperature, concentrated under reduced pressure. The residue was purified by preparative high-performance liquid chromatography to give the product, and then chirally resolved by supercritical fluid chromatography to give Compound **39a**(10.1 mg, 16.50 μmol); SFC residence time t = 3.831 min. **$^1$H NMR** (600 MHz, DMSO-$d_6$) δ 10.06 (s, 1H), 8.78 (s, 1H), 7.81 (d, *J* = 8.4 Hz, 1H), 7.71 (d, *J* = 8.4 Hz, 1H), 7.65 - 7.41 (m, 2H), 6.69 (d, *J* = 8.4 Hz, 2H), 5.70 - 5.60 (m, 1H), 5.29 (s, 1H), 5.00 (d, *J* = 10.2 Hz, 1H), 4.89 (d, *J* = 17.4 Hz, 1H), 4.77 - 4.64 (m, 1H), 4.62 (d, *J* = 15.0 Hz, 1H), 3.71 (d, *J* = 6.0 Hz, 2H), 3.42 (d, *J* = 11.4 Hz, 2H), 3.25 (d, *J* = 11.4 Hz, 2H), 2.85 - 2.81 (m, 2H), 2.49 - 2.46 (m, 1H), 2.35 (q, *J* = 6.8 Hz, 1H), 2.26 - 2.23 (m, 1H), 1.50 (d, *J* = 7.8 Hz, 1H), 1.12 - 1.10 (m, 1H), 0.88 (d, *J* = 6.0 Hz, 6H), 0.69 - 0.66 (m, 1H), 0.45 - 0.42 (m, 1H), 0.39 - 0.34 (m, 2H). LCMS (ESI) m/z: 580.2 $[M+H]^+$, HPLC Method B: $R_T$= 7.43 min, purity> 94.7%.

Compound **39b**(10.4 mg, 17.49 μmol); SFC residence time t = 6.371 min. **$^1$H NMR** (600 MHz, DMSO-$d_6$) δ 10.06 (s, 1H), 8.78 (s, 1H), 7.81 (d, *J* = 8.4 Hz, 1H), 7.71 (d, *J* = 8.4 Hz, 1H), 7.67 - 7.38 (m, 2H), 6.69 (d, *J* = 8.4 Hz, 2H), 5.72 - 5.60 (m, 1H), 5.29 (s, 1H), 5.04 - 4.96 (m, 1H), 4.89 (d, *J* = 17.4 Hz, 1H), 4.77 - 4.64 (m, 1H), 4.62 (d, *J* = 15.0 Hz, 1H), 3.71 (d, *J* = 6.0 Hz, 2H), 3.42 (d, *J* = 10.8 Hz, 2H), 3.25 (d, *J* = 10.8 Hz, 2H), 2.89 - 2.79 (m, 2H), 2.48 - 2.44 (m, 1H), 2.35 (d, *J* = 11.4 Hz, 1H), 2.28 - 2.22 (m, 1H), 1.50 (d, *J* = 7.8 Hz, 1H), 1.13 - 1.10 (m, 1H), 0.88 (d, *J* = 6.0 Hz, 6H), 0.71 - 0.65 (m, 1H), 0.45 - 0.42 (m, 1H), 0.39 - 0.34 (m, 2H). LCMS (ESI) m/z: 580.2 $[M+H]^+$, HPLC Method B: $R_T$=7.44 min, purity> 97.5%.

**Example 40: Synthesis of Compound 40:**

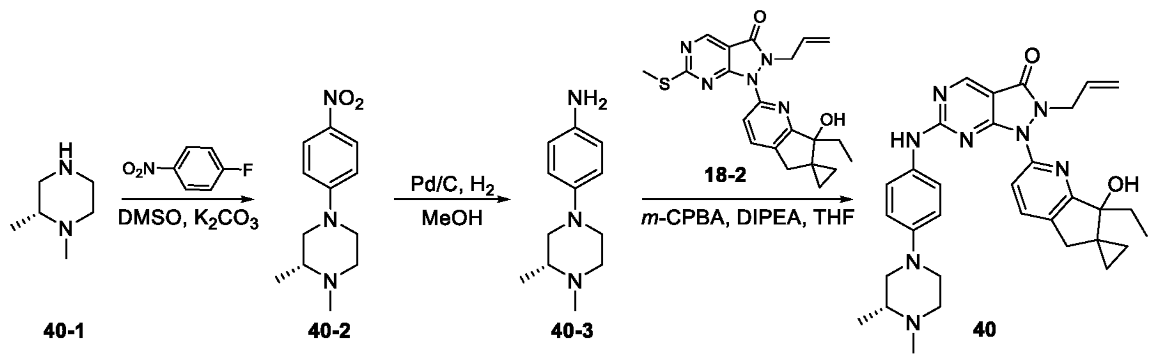


40-1 40-2 40-3 40

**Step 1: Synthesis of Compound 40-2:**

A dry single-necked flask was added with Substrate **40-1**(180 mg, 1.58 mmol), dissolved in dimethyl sulfoxide (3 mL), and then added with p-fluoro-nitrobenzene (233.54 mg, 1.66 mmol) and potassium carbonate (1.09 g, 7.88 mmol). At 100°C, the reaction was performed for 12 hours with LC-MS monitoring for complete reaction. The reaction solution was cooled to room temperature, added with water, and then extracted with ethyl acetate for four times. The organic phase was dried with anhydrous sodium sulfate, filtrated, concentrated under reduced pressure. The residue was purified by column chromatography to give Compound **40-2**(300 mg, 1.28 mmol). LCMS (ESI) m/z: 236.2 $[M+H]^+$.

**Step 2: Synthesis of Compound 40-3:**

A dry single-necked flask was added with Substrate **40-2**(300 mg, 1.28 mmol), dissolved in methanol (5 mL), and then added with Pd-C (30 mg). Under hydrogen atmosphere, the reaction was performed under room temperature for 2 hours with LC-MS monitoring for complete reaction. The reaction solution was filtrated with celite, washed with methanol twice. The filtrate was concentrated under reduced pressure to give Compound **40-3**(230 mg, 1.12 mmol). LCMS (ESI) m/z: 206.1 $[M+H]^+$.

**Step 3: Synthesis of Compound 40:**

A dry single-necked flask was added with Substrate **18-2**(20 mg, 48.84 μmol), dissolved in tetrahydrofuran (1 mL), and then added with m-chloroperoxybenzoic acid (15.17 mg, 87.91 μmol). Under room temperature, the reaction was performed for 1 hour. Then the reaction solution was added with N,N-diisopropyl ethyl amine (63.12 mg, 488.40 μmol) and

Substrate **40-3**(20.05 mg, 97.68 μmol). At 50°C, the reaction was performed overnight with LC-MS monitoring. After the reaction was completed, the reaction solution was cooled to room temperature, concentrated under reduced pressure. The residue was purified by preparative high-performance liquid chromatography to give Compound **40**(4 mg, 7.06 μmol). **$^1$H NMR** (400 MHz, DMSO-$d_6$) δ 10.12 (s, 1H), 8.82 (s, 1H), 7.91 (d, *J* = 8.0 Hz, 1H), 7.71 (d, *J* = 8.4 Hz, 1H), 7.58 (s, 2H), 6.92 (d, *J* = 8.8 Hz, 2H), 5.75 - 5.64 (m, 1H), 5.03 - 4.98 (m, 1H), 4.95 (s, 1H), 4.88 (dd, *J* = 17.2, 1.6 Hz, 1H), 4.78 (d, *J* = 16.4 Hz, 1H), 4.56 (dd, *J* = 16.0, 6.4 Hz, 1H), 3.48 (t, *J* = 10.8 Hz, 2H), 3.02 (d, *J* = 16.4 Hz, 1H), 2.82 (d, *J* = 11.2 Hz, 1H), 2.75 (d, *J* = 13.6 Hz, 1H), 2.72 - 2.66 (m, 1H), 2.38 - 2.30 (m, 1H), 2.27 (d, *J* = 11.2 Hz, 1H), 2.22 (s, 3H), 2.13 (d, *J* = 8.8 Hz, 1H), 1.80 - 1.75 (m, 1H), 1.62 - 1.53 (m, 1H), 1.06 (d, *J* = 6.0 Hz, 3H), 0.99 - 0.92 (m, 1H), 0.76 - 0.67 (m, 5H), 0.42 - 0.33 (m, 1H). LCMS (ESI) m/z: 567.3 $[M+H]^+$, HPLC Method B: $R_T$= 7.59, purity> 88.5%.

**Example 41: Synthesis of Compound 41**

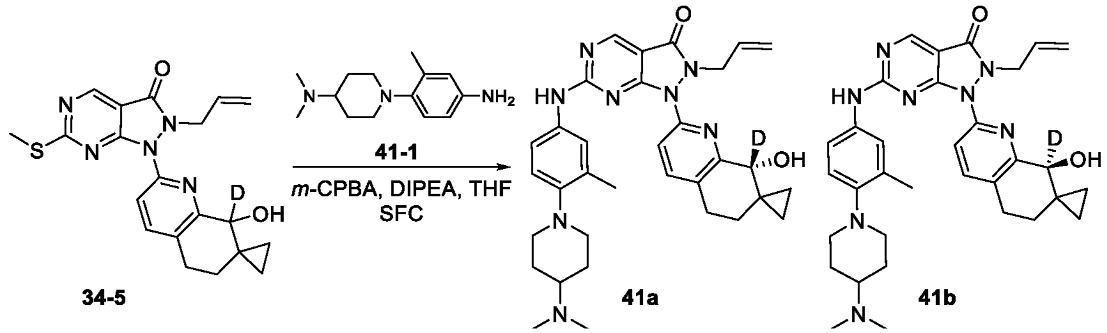


34-5 41a 41b

**Step 1: Synthesis of Compound 41a & 41b**

A dry single-necked flask was added with Substrate **34-5**(40 mg, 100.89 μmol), dissolved in tetrahydrofuran (3 mL), and then added with m-chloroperoxybenzoic acid (36.87 mg, 181.60 μmol). Under room temperature, the reaction was performed for 1 hour. Then the reaction solution was added with N,N-diisopropyl ethyl amine (130.39 mg, 1.01 mmol) and Substrate **41-1**(47.08 mg, 201.77 μmol). The reaction was performed at 45°C overnight with LC-MS monitoring. After the reaction was completed, the reaction solution was cooled to room temperature, concentrated under reduced pressure. The residue was purified by preparative high-performance liquid chromatography to give the product, and then chirally resolved by supercritical fluid chromatography to give Compound **41a**(5.1 mg, 8.45 μmol); SFC residence time t = 3.476 min. **$^1$H NMR** (400 MHz, DMSO-$d_6$) δ 10.12 (s, 1H), 8.83 (s, 1H), 7.84 - 7.78 (m, 1H), 7.76 - 7.70 (m, 1H), 7.70 - 7.60 (m, 1H), 7.43 - 7.37 (m, 1H), 7.00 - 6.94 (m, 1H), 5.74 - 5.60 (m ,1H), 5.29 (s, 1H), 5.03 - 4.96 (m, 1H), 4.93 - 4.83 (m, 1H), 4.74 - 4.58 (m, 2H), 3.10 - 3.01 (m, 2H), 2.92 - 2.76 (m, 2H), 2.62 - 2.53 (m, 2H), 2.30 - 2.20 (m, 1H), 1.90 - 1.80 (m, 2H), 1.60 - 1.48 (m, 2H), 1.15 - 1.14 (m, 1H), 0.71 - 0.62 (s, 1H), 0.47 - 0.31 (m, 3H). LCMS (ESI) m/z: 582.3 $[M+H]^+$, HPLC Method B: $R_T$= 8.13 min, purity> 96.4%.

Compound **41b**(5.2 mg, 8.47 μmol); SFC residence time t = 8.440 min. **$^1$H NMR** (400 MHz, DMSO-$d_6$) δ 10.12 (s, 1H), 8.83 (s, 1H), 7.88 - 7.76 (m, 1H), 7.76 - 7.69 (m, 1H), 7.70 - 7.55 (m, 1H), 7.44 - 7.31 (m, 1H), 7.02 - 6.94 (m, 1H), 5.74 - 5.60 (m ,1H), 5.29 (s, 1H), 5.03 - 4.96 (m, 1H), 4.93 - 4.83 (m, 1H), 4.77 - 4.56 (m, 2H), 3.10 - 3.01 (m, 2H), 2.92 - 2.76 (m, 2H), 2.62 - 2.53 (m, 2H), 2.30 - 2.20 (m, 1H), 1.90 - 1.79 (m, 2H), 1.60 - 1.48 (m, 2H), 1.17 - 1.06 (m, 1H), 0.71 - 0.62 (s, 1H), 0.47 - 0.31 (m, 3H). LCMS (ESI) m/z: 582.4 $[M+H]^+$, HPLC Method B: $R_T$= 8.56 min, purity> 94.8%.

**Example 42: Synthesis of Compound 42**

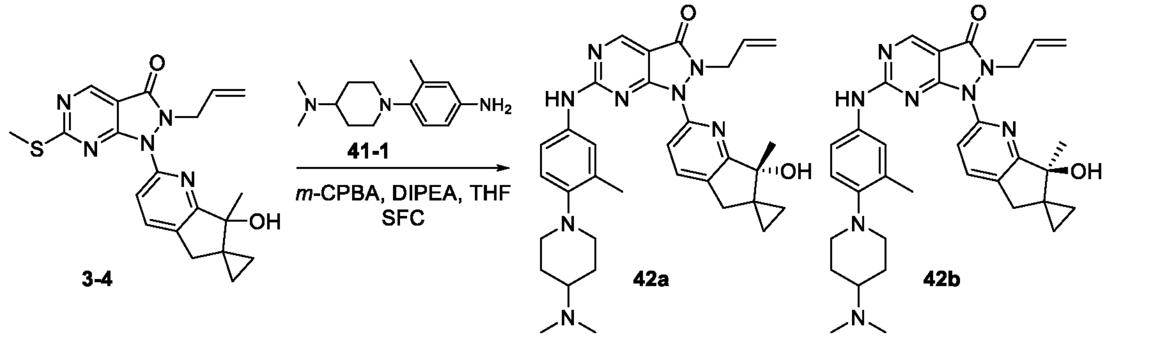


3-4 42a 42b

**Step 1: Synthesis of Compound 42a & 42b:**

A dry single-necked flask was added with Substrate **3-4**(30 mg, 75.86 μmol), dissolved in tetrahydrofuran (1.5 mL), and then added with m-chloroperoxybenzoic acid (26.18 mg, 151.72 μmol). Under room temperature, the reaction was performed for 1 hour. Then the reaction solution was added with N,N-diisopropyl ethyl amine (98.04 mg, 758.58 μmol) and Substrate **41-1**(17.70 mg, 75.86 μmol). At 50°C, the reaction was performed overnight with LC-MS monitoring. After the reaction was completed, the reaction solution was cooled to room temperature, concentrated under reduced pressure. The residue was purified by preparative high-performance liquid chromatography to give the product, and then chirally resolved by supercritical fluid chromatography to give Compound **42a**(4.0 mg, 6.39 μmol); SFC residence time t = 2.339 min. **$^1$H NMR** (400 MHz, DMSO-$d_6$) δ 10.12 (s, 1H), 8.85 (s, 1H), 7.88 (d, *J*= 8.0 Hz, 1H), 7.72 (d, *J* = 8.0 Hz, 1H), 7.65 (s, 1H), 7.43 - 7.40 (m, 1H), 6.97 (d, *J* = 8.4 Hz, 1H), 5.74 - 5.65 (m, 1H), 5.03 - 4.99 (m, 2H), 4.90 - 4.85 (m, 1H), 4.74 (d, *J* = 16.4 Hz, 1H), 4.62 - 4.57 (m, 1H), 3.04 (d, *J* = 11.6 Hz, 2H), 2.89 (s, 2H), 2.60 - 2.49 (m, 2H), 2.23 (s, 9H), 1.84 (d, *J* = 12.0 Hz, 2H), 1.58-1.50 (m, 2H), 1.22 (s, 4H), 0.95 - 0.90 (m, 1H), 0.70 - 0.66 (m, 1H), 0.61 - 0.56 (m, 1H), 0.48 - 0.43 (m, 1H). LCMS (ESI) m/z: 581.3[M+H]$^+$, HPLC Method B: $R_T$=8.01 min, purity> 92.7%.

Compound **42b**(4.0 mg, 6.40 μmol); SFC residence time t = 5.345 min. **$^1$H NMR** (400 MHz, DMSO-$d_6$) δ 10.12 (s, 1H), 8.84 (s, 1H), 7.88 (d, *J* = 8.0 Hz, 1H), 7.72 (d, *J* = 8.0 Hz, 1H), 7.65 (s, 1H), 7.43 - 7.40 (m, 1H), 6.97 (d, *J* = 8.8 Hz, 1H), 5.74 - 5.65 (m, 1H), 5.03 - 4.99 (m, 2H), 4.74 (d, *J* = 16.0 Hz, 1H), 4.62 - 4.57 (m, 1H), 3.04 (d, *J* = 11.6 Hz, 2H), 2.89 (d, *J* = 2.0 Hz, 2H), 2.60 - 2.54 (m, 2H), 2.23 (d, *J* = 4.0 Hz, 9H), 1.84 (d, *J* = 12.4 Hz, 2H), 1.57 - 1.49 (m, 2H), 1.26 - 1.22 (m, 4H), 0.95 - 0.90 (s, 1H), 0.72 - 0.66 (m, 1H), 0.60 - 0.56 (m, 1H), 0.48 - 0.43 (m, 1H). LCMS (ESI) m/z: 581.3 [M+H]$^+$, HPLC Method B: $R_T$= 8.11 min, purity> 92.9%.

**Example 43: Synthesis of Compound 43**

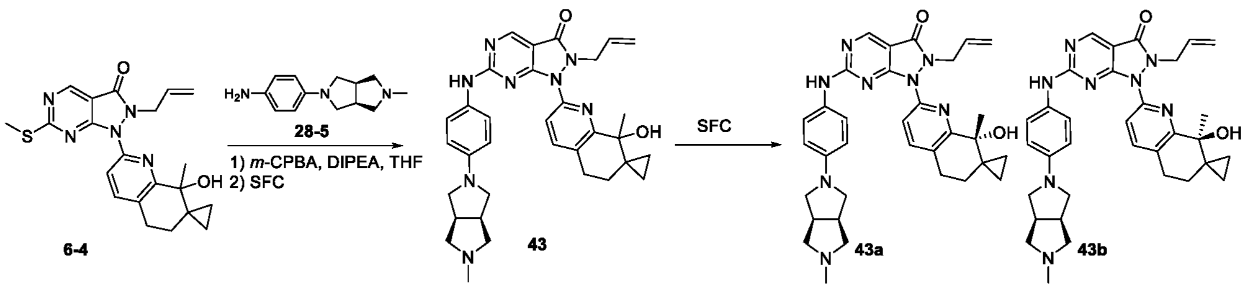


6-4 43 43a 43b

**Step 1: Synthesis of Compound 43 & 43a & 43b:**

A dry single-necked flask was added with Substrate **6-4**(105 mg, 256.41 μmol), dissolved in tetrahydrofuran (5 mL), and then added with m-chloroperoxybenzoic acid (79.65 mg, 461.54 μmol). Under room temperature, the reaction was performed for 1 hour. Then the reaction solution was added with N,N-diisopropyl ethyl amine (330.24 mg, 2.56 mmol) and Substrate **28-5**(111.44 mg, 512.82 μmol). The reaction was performed at 45°C overnight with LC-MS monitoring. After the reaction was completed, the reaction solution was cooled to room temperature, concentrated under reduced pressure. The residue was purified by preparative high-performance liquid chromatography to give Compound **43**(20.09 mg, 28.74 μmol). **$^1$H NMR** (400 MHz, DMSO-$d_6$) δ 10.05 (s, 1H), 8.79 (s, 1H), 7.77 - 7.68 (m, 2H), 7.53 (s, 2H), 6.63 (d, *J* =8.8 Hz, 2H), 5.72 - 5.62 (m, 1H), 4.99 (dd, *J* =10.2, 1.6 Hz, 2H), 4.88 - 4.79 (m, 2H), 4.69 - 4.61 (m, 2H), 3.31 (s, 2H), 3.06 - 3.03 (m, 2H),

2.93 - 2.76 (m, 4H), 2.59 - 2.55(m, 2H), 2.40 - 2.37 (m, 2H), 2.22(s, 3H), 2.02 - 1.95 (m, 1H), 1.45 - 1.40 (m, 4H), 1.87 - 0.84(m, 1H), 0.65 - 0.61 (m, 1H), 0.34 - 0.30(m, 1H), 0.23 - 0.19 (m, 1H). LCMS (ESI) m/z: 579.3 [M+H]$^+$, HPLC Method B: $R_T$= 8.97min, purity> 82.8%.

Compound **43** was synthesized by a similar method, and then chirally resolved by supercritical fluid chromatography to give Compound **43a**(2.3 mg, 3.50 μmol); SFC residence time t = 3.946 min. **$^1$H NMR** (600 MHz, DMSO-$d_6$) δ 10.05 (s, 1H), 8.79 (s, 1H), 7.76 (s, 1H), 7.69 (d, *J* = 8.4 Hz, 1H), 7.54 (s, 2H), 6.64 (d, *J* = 8.4 Hz, 2H), 5.73 - 5.64 (m, 1H), 4.99 (dd, *J* = 10.2, 1.8 Hz, 1H), 4.86 (d, *J* = 17.4 Hz, 1H), 4.80 (s, 1H), 4.68 (s, 1H), 4.64 (d, *J* = 13.8 Hz, 1H), 3.06 - 3.01 (m, 2H), 2.90 - 2.84 (m, 3H), 2.82 - 2.78 (m, 1H), 2.59 - 2.55 (m, 2H), 2.38 (dd, *J* = 9.0, 3.0 Hz, 2H), 2.22 (s, 3H), 2.02 - 1.95 (m, 1H), 1.45 - 1.42 (m, 1H), 1.40 (s, 3H), 0.88 - 0.84 (m, 1H), 0.65 - 0.61 (m, 1H), 0.34 - 0.30 (m, 1H), 0.23 - 0.19 (m, 1H). LCMS (ESI) m/z: 579.4 [M+H]$^+$, HPLC Method B: $R_T$= 8.76min, purity> 88.0%.

Compound **43b**(9.3 mg, 13.90 μmol); SFC residence time t = 6.047 min. **$^1$H NMR** (600 MHz, DMSO-$d_6$) δ 10.05 (s, 1H), 8.79 (s, 1H), 7.76 (d, *J* = 8.4 Hz, 1H), 7.69 (d, *J* = 8.4 Hz, 1H), 7.54 (s, 2H), 6.63 (d, *J* = 8.4 Hz, 2H), 5.72 - 5.62 (m, 1H), 4.99 (dd, *J*= 10.2, 1.8 Hz, 1H), 4.86 (d, *J* = 17.4 Hz, 1H), 4.80 (s, 1H), 4.68 (s, 1H), 4.67 - 4.59 (m, 1H), 3.08 - 3.02 (m, 2H), 2.90 - 2.84 (m, 3H), 2.82 - 2.78 (m, 1H), 2.57 (t, *J* = 7.8 Hz, 2H), 2.42 - 2.37 (m, 2H), 2.22 (s, 3H), 2.02 - 1.95 (m, 1H), 1.46 - 1.42 (m, 1H), 1.40 (s, 3H), 0.87 - 0.84 (m, 1H), 0.66 - 0.61 (m, 1H), 0.35 - 0.30 (m, 1H), 0.24 - 0.19 (m, 1H). LCMS (ESI) m/z: 579.4 [M+H]$^+$, HPLC Method B: $R_T$= 8.81 min, purity> 82.1%.

**Example 44: Synthesis of Compound 44**

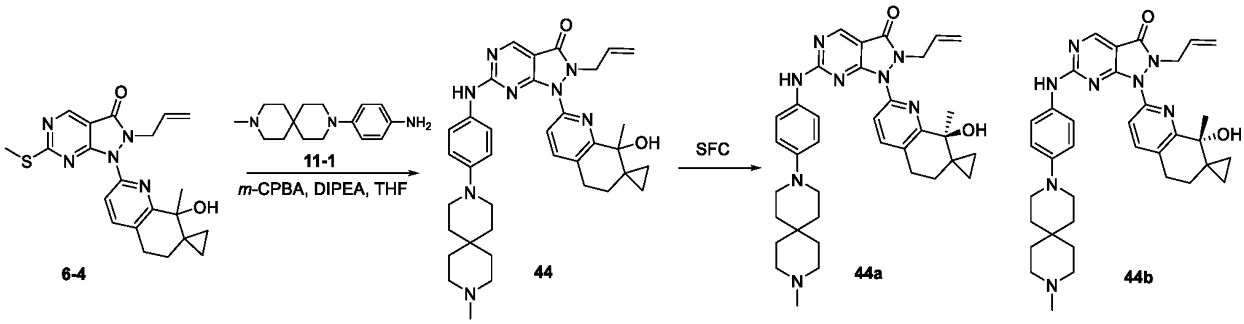


6-4 44 44a 44b

**Step 1: Synthesis of Compound 44 & 44a & 44b:**

A dry single-necked flask was added with Substrate **6-4**(90 mg, 219.78 μmol), dissolved in tetrahydrofuran (4 mL), and then added with m-chloroperoxybenzoic acid (68.27 mg, 395.06 μmol). Under room temperature, the reaction was performed for 1 hour. Then the reaction solution was added with N,N-diisopropyl ethyl amine (282.51 mg, 2.19 mmol) and Substrate **11-1**(114.02 mg, 439.56). The reaction was performed at 45°C overnight with LC-MS monitoring. After the reaction was completed, the reaction solution was cooled to room temperature, concentrated under reduced pressure. The residue was purified by preparative high-performance liquid chromatography to give Compound **44**(21.5 mg, 34.63 μmol). **$^1$H NMR** (400 MHz, DMSO-$d_6$) δ 10.10 (s, 1H), 8.81 (s, 1H), 7.78 (d, *J* = 8.4 Hz, 1H), 7.69 (d, *J* = 8.4 Hz, 1H), 7.56 (s, 2H), 6.92 (d, *J* = 8.8 Hz, 2H), 5.73 - 5.62 (m, 1H), 5.03 - 4.98 (m, 1H), 4.92 - 4.75 (m, 2H), 4.69 - 4.58 (m, 2H), 3.09 (t, *J* = 5.6 Hz, 4H), 2.92 - 2.76 (m, 2H), 2.38 - 2.28 (m, 4H), 2.19 (s, 3H), 2.04 - 1.96 (m, 1H), 1.54 (t, *J* = 5.6 Hz, 4H), 1.47 (t, *J* = 5.6 Hz, 4H), 1.45 - 1.42 (m, 1H), 1.40 (s, 3H), 0.88 - 0.82 (m, 1H), 0.65 - 0.59 (m, 1H), 0.36 - 0.29 (m, 1H), 0.25 - 0.18 (m, 1H). LCMS (ESI) m/z: 621.2 [M+H]$^+$, HPLC Method B: $R_T$= 9.63min, purity> 96.8%.

Compound **44** was synthesized by a similar method, and then chirally resolved by supercritical fluid chromatography to give Compound **44a**(1.6 mg, 2.23 μmol); SFC residence time t = 3.819 min. **$^1$H NMR** (600 MHz, DMSO-$d_6$) δ 10.10 (s, 1H), 8.80 (s, 1H), 7.82 - 7.73 (m, 1H), 7.71 - 7.65 (m, 1H), 7.63 - 7.48 (m, 2H), 6.96 - 6.86 (m, 2H), 5.72 - 5.62 (m ,1H), 5.03 - 4.95 (m, 1H), 4.91 - 4.74 (m, 2H), 4.73 - 4.58 (m, 2H), 3.29 (s, 1H), 3.10 - 3.06 (m, 4H), 2.93 - 2.84 (m, 1H), 2.83 - 2.76 (m, 1H), 2.36 - 2.25 (m, 3H), 2.24 - 2.13 (m, 3H), 2.03 - 1.95 (m, 1H), 1.56 - 1.51 (m, 4H), 1.50 - 1.45 (m, 4H), 1.40 (s, 3H), 1.25 - 1.21 (m, 1H), 0.88 - 0.82 (m, 1H), 0.66 - 0.58 (m, 1H), 0.35 - 0.29 (m, 1H), 0.24 - 0.18 (m, 1H). LCMS (ESI) m/z: 621.4 [M+H]$^+$, HPLC Method B: $R_T$= 10.05 min, purity> 86.7%.

Compound 4**4b**(1.5 mg, 2.21 μmol); SFC residence time t = 6.629 min. **$^1$H NMR** (600 MHz, DMSO-$d_6$) δ 10.10 (s, 1H), 8.80 (s, 1H), 7.82 - 7.73 (m, 1H), 7.71 - 7.65 (m, 1H), 7.61 - 7.49 (m, 2H), 6.96 - 6.86 (m, 2H), 5.71 - 5.61 (m ,1H), 5.03 - 4.95 (m, 1H), 4.89 - 4.83 (m, 1H), 4.83 - 4.74 (m, 1H), 4.73 - 4.67 (m,1H), 4.67 - 4.59 (m, 1H), 3.10 - 3.06 (m, 4H), 2.92 - 2.85 (m, 1H), 2.83 - 2.76 (m, 1H), 2.32 - 2.27 (m, 3H), 2.20 - 2.13 (m, 3H), 2.02 - 1.95 (m, 1H), 1.56 - 1.51 (m, 4H), 1.50 - 1.45 (m, 4H), 1.40 (s, 3H), 1.26 - 1.17 (m, 2H), 0.88 - 0.82 (m, 1H), 0.66 - 0.58 (m, 1H), 0.35 - 0.29 (m, 1H), 0.24 - 0.18 (m, 1H). LCMS

(ESI) m/z: 621.4 $[M+H]^+$, HPLC Method B: $R_T$= 10.03 min, purity> 91.4%.

**Example 45: Synthesis of Compound 45**

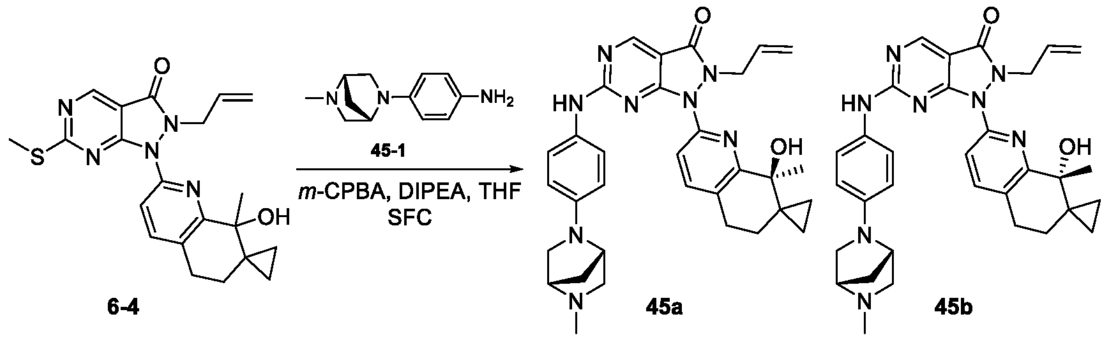


**6-4** **45a** **45b**

**Step 1: Synthesis of Compound 45a & 45b:**

A dry single-necked flask was added with Substrate **6-4**(40 mg, 97.68 μmol), dissolved in tetrahydrofuran (4 mL), and then added with m-chloroperoxybenzoic acid (30.34 mg, 175.82 μmol). Under room temperature, the reaction was performed for 1 hour. Then the reaction solution was added with N,N-diisopropyl ethyl amine (126.01 mg, 976.79 μmol) and Substrate **45-1**(39.71 mg, 195.36 μmol). The reaction was performed at 45°C overnight with LC-MS monitoring. After the reaction was completed, the reaction solution was cooled to room temperature, concentrated under reduced pressure. The residue was purified by preparative high-performance liquid chromatography to give the product, and then chirally resolved by supercritical fluid chromatography to give Compound **45a**(8.52 mg, 14.66 μmol); SFC residence time t = 3.602 min. **$^1$H NMR** (600 MHz, DMSO-$d_6$) δ 10.00 (s, 1H), 8.78 (s, 1H), 7.79 - 7.62 (m, 2H), 7.57 - 7.31 (m, 2H), 6.56 (d, *J* = 7.8 Hz, 2H), 5.72 - 5.60 (m ,1H), 5.04 - 4.94 (m, 1H), 4.91 - 4.72 (m, 2H), 4.72 - 4.56 (m, 2H), 4.27 (s, 1H), 3.42 - 3.40 (m, 2H), 3.16 - 3.10 (m, 1H), 2.92 - 2.82 (m, 1H), 2.82 - 2.74 (m, 2H), 2.48 - 2.44 (m, 1H), 2.24 (s, 3H), 2.02 - 1.92 (m, 1H), 1.90- 1.81 (m, 1H), 1.79 - 1. 71 (m, 1H), 1.47 - 1.37 (m, 4H), 0.90 - 0.80 (m, 1H), 0.66 - 0.58 (m, 1H), 0.36 - 0.26 (m, 1H), 0.24 - 0.15 (m, 1H). LCMS(ESI) m/z: 565.4$[M+H]^+$, HPLC Method B Rt = 8.36 min, purity > 97.13%.

Compound **45b**(7.8 mg, 13.39 μmol); SFC residence time t = 7.195 min. **$^1$H NMR** (600 MHz, DMSO-$d_6$) δ 10.00 (s, 1H), 8.78 (s, 1H), 7.79 - 7.62 (m, 2H), 7.57 - 7.15 (m, 2H), 6.57 - 6.41 (m, 2H), 5.72 - 5.60 (m ,1H), 5.04 - 4.94 (m, 1H), 4.91 - 4.72 (m, 2H), 4.72 - 4.56 (m, 2H), 4.27 (s, 1H), 3.42 - 3.40 (m, 2H), 3.16 - 3.10 (m, 1H), 2.92 - 2.82 (m, 1H), 2.82 - 2.74 (m, 2H), 2.48 - 2.44 (m, 1H), 2.25 (s, 3H), 2.02 - 1.92 (m, 1H), 1.90 - 1.81 (m, 1H), 1.79 - 1. 71 (m, 1H), 1.47 - 1.37 (m, 4H), 0.90 - 0.80 (m, 1H), 0.66 - 0.58 (m, 1H), 0.36 - 0.26 (m, 1H), 0.24 - 0.15 (m, 1H). LCMS(ESI) m/z: 565.3$[M+H]^+$, HPLC Method B Rt = 8.317 min, purity > 96.94%.

**Example 46: Synthesis of Compound 46**

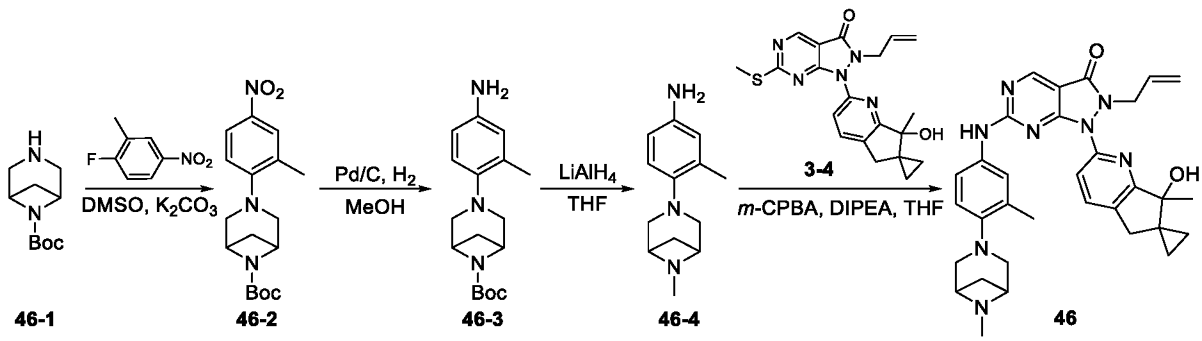


**46-1** **46-2** **46-3** **46-4** **46**

**Step 1: Synthesis of Compound 46-2:**

A dry single-necked flask was added with Substrate **46-1**(400 mg, 2.02 mmol), dissolved in dimethyl sulfoxide (5

mL), and then added with 2-fluoro-5-nitrotoluene (310 mg, 2.00 mmol) and potassium carbonate (390.35 mg, 2.82 mmol), 120°C, the reaction was performed for 6 hours with LC-MS monitoring for complete reaction. The reaction solution was cooled to room temperature, added with water, and then extracted with ethyl acetate for four times. The organic phase was dried with anhydrous sodium sulfate, filtrated, concentrated under reduced pressure. The residue was purified by column chromatography to give Compound **46-2**(290 mg, 869.87 μmol). LCMS (ESI) m/z: 278.2 $[M+H-56]^+$.

**Step 2: Synthesis of Compound 46-3:**

A dry single-necked flask was added with Substrate **46-2**(290 mg, 869.87 μmol), dissolved in methanol (5 mL), and then added with Pd-C (30 mg). Under hydrogen atmosphere, the reaction was performed under room temperature for 2 hours with LC-MS monitoring for complete reaction. The reaction solution was filtrated with celite, washed with methanol twice. The filtrate was concentrated under reduced pressure to give Compound **46-3**(240 mg, 791.03 mmol). LCMS (ESI) m/z: 248.0 $[M+H-56]^+$.

**Step 3: Synthesis of Compound 46-4:**

A dry three-necked flask was added with Substrate **46-3**(120 mg, 395.52 μmol), added with anhydrous tetrahydrofuran (3 mL) to dissolve, under the protection of nitrogen atmosphere cooled to 0°C, and then slowly dropwise added with a solution of lithium aluminum hydride in tetrahydrofuran (1.98 mL, 1.98 mmol, 1M). The reaction was heated under reflux at 65°C for 4 hours with LC-MS monitoring. The reaction solution was added with water, 10% sodium hydroxide aqueous solution, stirred for half an hour, dried with anhydrous sodium sulfate, filtrated with celite, concentrated under reduced pressure. The residue was purified by column chromatography to give Compound **46-4**(38 mg, 174.87 μmol). LCMS (ESI) m/z: 218.2 $[M+H]^+$.

**Step 4: Synthesis of Compound 46:**

A dry single-necked flask was added with Substrate **3-4**(15 mg, 37.93 μmol), dissolved in tetrahydrofuran (2 mL), and then added with m-chloroperoxybenzoic acid (7.59 mg, 68.27 μmol). Under room temperature, the reaction was performed for 1 hour. Then the reaction solution was added with N,N-diisopropyl ethyl amine (48.93 mg, 379.30 μmol) and Substrate **46-4**(16.49 mg, 75.86 μmol). At 50°C, the reaction was performed overnight with LC-MS monitoring. After the reaction was completed, the reaction solution was cooled to room temperature, concentrated under reduced pressure. The residue was purified by preparative high-performance liquid chromatography to give Compound **46** (2.0 mg, 3.10 μmol). **$^1$H NMR** (600 MHz, DMSO-$d_6$) δ 10.15 (s, 1H), 8.85 (s, 1H), 7.88 (d, *J* = 7.8 Hz, 1H), 7.74 - 7.70 (m, 2H), 7.48 - 7.42 (m, 1H), 7.29 (d, *J* = 8.4 Hz, 1H), 5.73 - 5.67 (m, 1H), 5.03 - 5.00 (m, 2H), 4.88 (d, *J* = 16.8 Hz, 1H), 4.73 - 4.58 (m, 2H), 3.52 (d, *J* = 10.8 Hz, 4H), 3.17 (d, *J* = 4.8 Hz, 1H), 3.04 - 3.03 (m, 2H), 2.92 - 2.86 (m, 2H), 2.27 (s, 3H), 2.20 (s, 3H), 1.99 - 1.96(m, 1H), 1.22 (s, 3H), 0.94 - 0.90 (m, 1H), 0.70 - 0.67 (m, 1H), 0.59 - 0.56 (m, 1H), 0.47 - 0.44 (m, 1H). LCMS (ESI) m/z: 565.3 $[M+H]^+$, HPLC Method B: $R_T$ = 7.66 min, purity> 87.6%.

**Example 47: Synthesis of Compound 47**

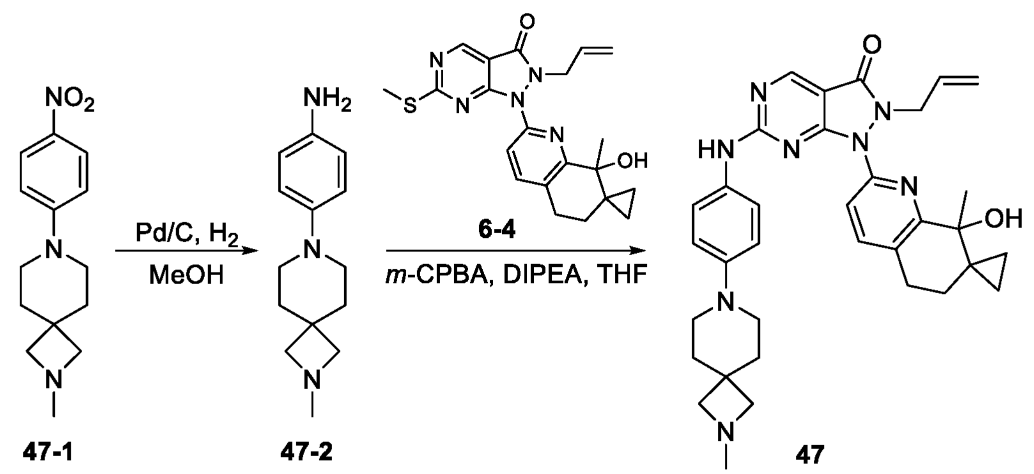


**47-1** **47-2** **47**

**Step 1: Synthesis of Compound 47-2:**

A dry single-necked flask was added with Substrate **47-1**(300 mg, 1.15 mmol), dissolved in methanol (5 mL), and then added with Pd-C (30 mg). Under hydrogen atmosphere, the reaction was performed under room temperature for 2 hours with LC-MS monitoring for complete reaction. The reaction solution was filtrated with celite, washed with methanol twice. The filtrate was concentrated under reduced pressure to give Compound **47-2**(230 mg, 994.23 μmol). LCMS (ESI) m/z: 232.2 $[M+H]^+$.

**Step 2: Synthesis of Compound 47:**

A dry single-necked flask was added with Substrate **6-4**(15 mg, 36.63 μmol), dissolved in tetrahydrofuran (1 mL), and then added with m-chloroperoxybenzoic acid (11.38 mg, 65.93 μmol). Under room temperature, the reaction was performed for 1 hour. Then the reaction solution was added with N,N-diisopropyl ethyl amine (47.34 mg, 366.30 μmol) and Substrate **47-2**(16.95 mg, 73.26 μmol). The reaction was performed at 45°C overnight with LC-MS monitoring. After the reaction was completed, the reaction solution was cooled to room temperature, concentrated under reduced pressure. The residue was purified by preparative high-performance liquid chromatography to give Compound **47**(4.0 mg, 6.75 μmol). **$^1$H NMR** (400 MHz, DMSO-*d6*) δ 8.97 - 8.66 (m, 1H), 7.79 - 7.45 (m, 3H), 7.35 (d, *J* = 8.4 Hz, 1H), 7.13 (d, *J* = 8.4 Hz, 1H), 6.68 - 6.47 (m, 2H), 5.68 - 5.59 (m, 2H), 5.00 (d, *J* = 10.4 Hz, 1H), 4.86 (d, *J* = 14.8 Hz, 1H), 4.76 - 4.57 (m, 2H), 4.16 (s, 3H), 3.85 (d, *J* = 10.0 Hz, 1H), 3.78 - 3.66 (m, 1H), 3.59 (s, 3H), 3.23 (s, 3H), 2.85 - 2.71 (m, 2H), 2.16 (s, 2H), 1.99 - 1.89 (m, 3H), 1.40 (s, 4H), 0.85 - 0.83 (m, 1H), 0.64 - 0.62 (m, 1H), 0.34 - 0.31 (m, 1H), 0.26 - 0.18 (m, 1H). LCMS(ESI) m/z: 593.4 $[M+H]^+$, HPLC Method B $R_T$ = 7.58 min, purity >98.6%. LCMS (ESI) m/z: 593.4 $[M+H]^+$, HPLC Method B: $R_T$ = 7.55 min, purity> 98.8%.

**Example 48: Synthesis of Compound 48**

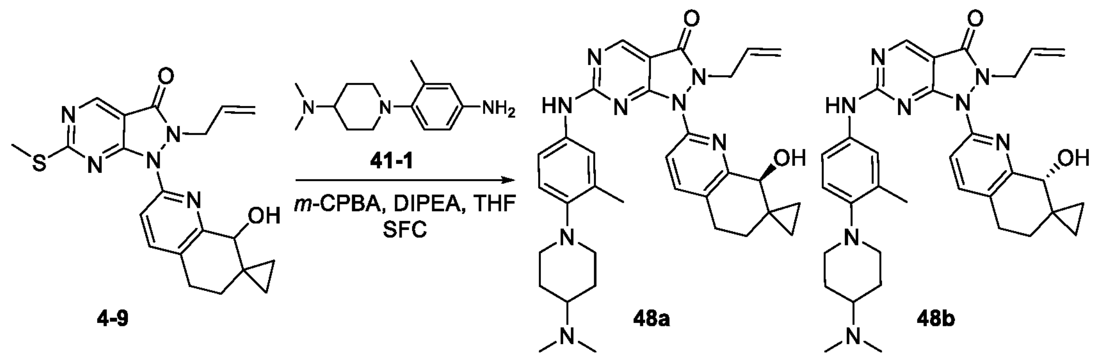


4-9 48a 48b

**Step 1: Synthesis of Compound 48a & 48b**

A dry single-necked flask was added with Substrate **4-9**(20 mg, 50.57 μmol), dissolved in tetrahydrofuran (2 mL), and then added with m-chloroperoxybenzoic acid (17.45 mg, 101.14 μmol). Under room temperature, the reaction was performed for 1 hour. Then the reaction solution was added with N,N-diisopropyl ethyl amine (65.36 mg, 505.72 μmol) and Substrate **41-1**(11.80 mg, 50.57 μmol). The reaction was performed at 45°C overnight with LC-MS monitoring. After the reaction was completed, the reaction solution was cooled to room temperature, concentrated under reduced pressure. The residue was purified by preparative high-performance liquid chromatography to give the product, and then chirally resolved by supercritical fluid chromatography to give Compound **48a**(4.93 mg, 7.62 μmol). **$^1$H NMR** (400 MHz, DMSO-$d_6$) δ 10.13 (s, 1H), 8.83 (s, 1H), 7.80 (d, *J* = 8.4 Hz, 1H), 7.72 (d, *J* = 8.4 Hz, 1H), 7.67 - 7.66 (m, 1H), 7.42 - 7.39 (m, 1H), 6.97 (d, *J* = 8.8 Hz, 1H), 5.72 - 5.62 (m, 1H), 5.31 (d, *J* = *5.6* Hz, 1H), 5.01 (dd, *J* = 1.2, 10.4 Hz, 1H), 4.88 (dd, *J* = 10.4, 1.2 Hz, 1H), 4.72 - 4.64 (m, 2H), 3.74 (d, *J* = 5.6 Hz, 1H), 3.04 (d, *J* = 12.0 Hz, 2H), 2.87 - 2.84(m, 1H), 2.57 (t, *J* = 10.8 Hz, 2H), 2.28 - 2.18 (m, 11H), 1.83 (d, *J* = 11.2 Hz, 2H), 1.57 - 1.52 (m, 2H), 1.15 - 1.09 (m, 1H), 0.69 - 0.66 (m, 1H), 0.45 - 0.42 (m, 1H), 0.39 - 0.34 (m, 2H). LCMS (ESI) m/z: 581.2 $[M+H]^+$, HPLC Method B: $R_T$= 8.46 min, purity> 89.8%.

Compound **48b**(4.43 mg, 6.42 μmol). **$^1$H NMR** (400 MHz, DMSO-$d_6$) δ 10.12 (s, 1H), 8.83 (s, 1H), 7.80 (d, *J* = 8.4 Hz, 1H), 7.72 (d, *J* = 8.4 Hz, 1H), 7.67 - 7.66 (m, 1H), 7.42 - 7.39 (m, 1H), 6.97 (d, *J* = 8.8 Hz, 1H), 5.72 - 5.62 (m, 1H), 5.31 (d, *J* = 5.6 Hz, 1H), 5.01 (dd, *J* = 1.2, 10.4 Hz, 1H), 4.88 (dd, *J* = 10.4, 1.2 Hz, 1H), 4.72 - 4.60 (m, 2H), 3.74 (d, *J* = 5.6 Hz, 1H), 3.04 (d, *J* = 12.0 Hz, 2H), 2.87 - 2.84 (m, 1H), 2.57 (t, *J* = 10.8 Hz, 2H), 2.28 - 2.18 (m, 11H), 1.83 (d, *J*= 11.2 Hz, 2H), 1.57 -

1.52 (m, 2H), 1.15 - 1.09 (m, 1H), 0.69 - 0.66 (m, 1H), 0.45 - 0.42 (m, 1H), 0.39 - 0.34 (m, 2H). LCMS (ESI) m/z: 581.2 $[M+H]^+$, HPLC Method B: $R_T$= 8.50 min, purity> 84.1%.

**Example 49: Synthesis of Compound 49**

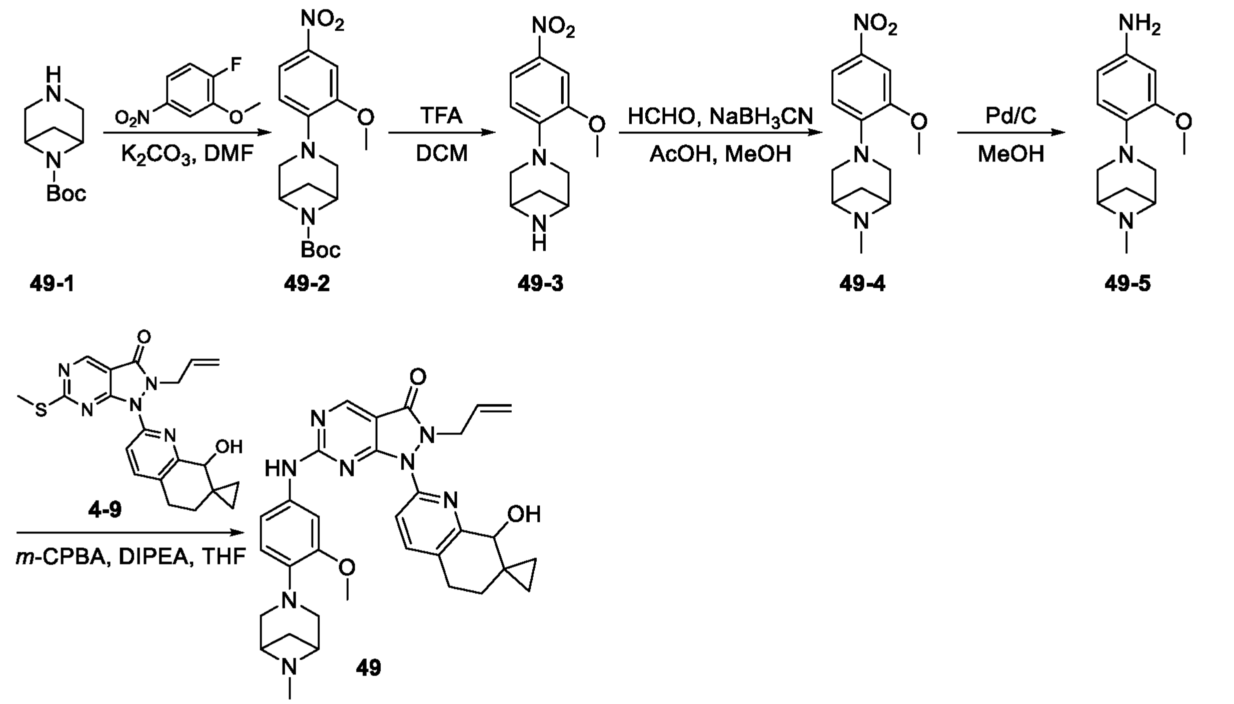


49-1 49-2 49-3 49-4 49-5

49

**Step 1: Synthesis of Compound 49-2:**

A dry single-necked flask was added with Substrate **49-1** (416 mg, 2.10 mmol), dissolved in dimethyl sulfoxide (5 mL), and then added with 2-fluoro5- nitroanisole (342 mg, 2.00 mmol) and potassium carbonate (386.68 mg, 2.80 mmol). At 110°C, the reaction was performed for 4 hours with LC-MS monitoring for complete reaction. The reaction solution was cooled to room temperature, added with water, and then extracted with ethyl acetate for three times. The organic phase was dried with anhydrous sodium sulfate, filtrated, concentrated under reduced pressure. The residue was purified by column chromatography to give Compound **49-2**(470 mg, 1.35 mmol). LCMS (ESI) m/z: 294.3 $[M+H-56]^+$.

**Step 2: Synthesis of Compound 49-3:**

A dry single-necked flask was added with Substrate **49-2**(200 mg, 572.44 μmol), dissolved in dichloromethane (3 mL), added with trifluoroacetic acid (3 mL). Under room temperature, the reaction was performed for 1 hour with LC-MS monitoring for complete reaction. The reaction solution was concentrated under reduced pressure to give crude Compound **49-3**(140 mg, 561.65 umol). LCMS (ESI) m/z: 250.1 $[M+H]^+$.

**Step 3: Synthesis of Compound 49-4:**

A dry single-necked flask was added with Substrate **49-3**(140 mg, 561.65 μmol), dissolved in methanol (5 mL), added with acetic acid (0.5 mL) and formalin solution (191.30 mg, 5.62 mmol, 37 %), stirred under room temperature for half an hour, and then added with sodium cyanoborohydride (105.88 mg, 1.68 mmol). At 50°C, the reaction was performed for 6 hours with LC-MS monitoring for complete reaction. The reaction solution was concentrated under reduced pressure, added with saturated sodium bicarbonate aqueous solution to adjust to pH=9, and then the aqueous phase was extracted with ethyl acetate for three times. The organic phase was dried with anhydrous sodium sulfate, filtrated, concentrated under reduced pressure to give Compound **49-4**(32 mg, 121.54 μmol). LCMS (ESI) m/z: 264.2 $[M+H]^+$.

**Step 4: Synthesis of Compound 49-5:**

A dry single-necked flask was added with Substrate **49-4**(32 mg, 121.54 μmol), dissolved in methanol (3 mL), and then added with Pd-C (5 mg). Under hydrogen atmosphere, the reaction was performed under room temperature for 2 hours with LC-MS monitoring for complete reaction. The reaction solution was filtrated with celite, washed with methanol twice. The filtrate was concentrated under reduced pressure to give Compound **49-5**(25.0 mg, 107.15 umol). LCMS (ESI) m/z: 234.1 $[M+H]^+$.

**Step 5: Synthesis of Compound 49:**

A dry single-necked flask was added with Substrate **4-9**(20 mg, 50.57 μmol), dissolved in tetrahydrofuran (2 mL), and then added with m-chloroperoxybenzoic acid (15.54 mg, 90.03 μmol). Under room temperature, the reaction was performed for 1 hour. Then the reaction solution was added with N,N-diisopropyl ethyl amine (138.49 mg, 1.07 mmol) and Substrate **49-5**(25.0 mg, 107.15 μmol), 25°C, the reaction was performed overnight with LC-MS monitoring. After the reaction was completed, the reaction solution was cooled to room temperature, concentrated under reduced pressure. The residue was purified by preparative high-performance liquid chromatography to give Compound **49** (3.0 mg, 4.71 μmol).**$^1$H** NMR (600 MHz, Chloroform-d) δ 8.83 (s, 1H), 7.67 - 7.63 (m, 2H), 7.17 (s, 1H), 7.10 - 7.09 (m, 1H), 7.00 - 6.98 (m, 1H), 5.76 - 5.69 (m, 1H), 5.06 (d, *J= 10.2* Hz, 1H), 4.98 (dd, *J* = 17.4, 1.2 Hz, 1H), 4.69 - 4.63 (m, 2H), 4.28 (s, 1H), 3.80 (d, *J* = 4.2 Hz, 2H), 3.77 (s, 3H), 3.75 - 3.72 (m, 2H), 3.59 (d, *J* = 9.6 Hz, 2H), 2.91 - 2.89 (m, 2H), 2.76 (s, 1H), 2.35 (s, 3H), 2.04 (s, 1H), 2.00 - 1.95 (m, 2H), 1.63 - 1.59 (m, 1H), 0.84 - 0.83 (m, 1H), 0.68 - 0.66 (m, 1H), 0.48 - 0.44 (m, 2H). LCMS (ESI) m/z: 581.3 $[M+H]^+$, HPLC Method B: $R_T$= 7.19 min, purity> 91.1%.

**Example 50: Synthesis of Compound 50**

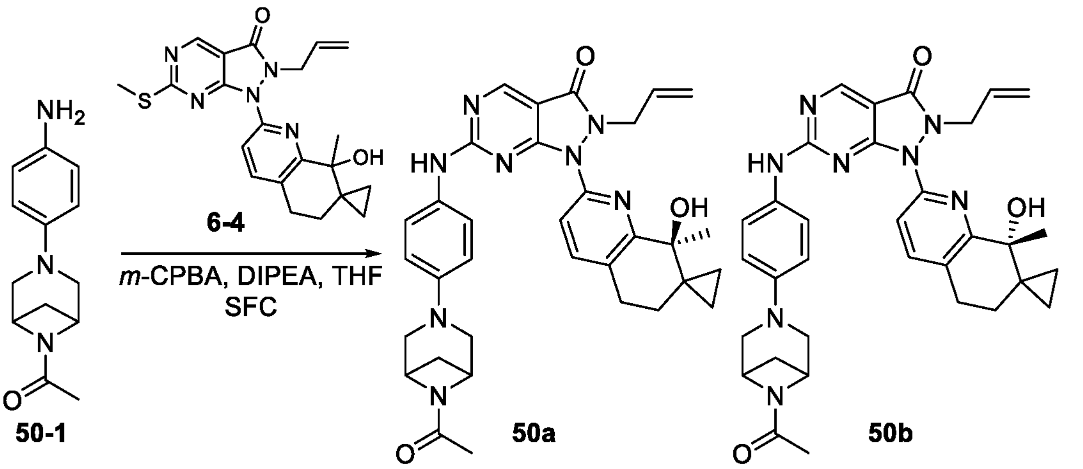


6-4

50-1

50a

50b

**Step 1: Synthesis of Compound 50a & 50b**

A dry single-necked flask was added with Substrate **6-4**(30 mg, 73.26 μmol), dissolved in tetrahydrofuran (2 mL), and then added with m-chloroperoxybenzoic acid (22.76 mg, 131.87 μmol). Under room temperature, the reaction was performed for 1 hour. Then the reaction solution was added with N,N-diisopropyl ethyl amine (81.02 mg, 626.91 μmol) and Substrate **50-1**(29 mg, 125.38 μmol), 25°C, the reaction was performed overnight with LC-MS monitoring. After the reaction was completed, the reaction solution was cooled to room temperature, concentrated under reduced pressure. The residue was purified by preparative high-performance liquid chromatography to give the product, and then chirally resolved by supercritical fluid chromatography to give Compound **50a(10.1** mg, 16.68 μmol); SFC residence time t = 6.088 min. **$^1$H NMR** (600 MHz, DMSO-$d_6$) δ 10.06 (s, 1H), 8.79 (s, 1H), 7.76 (s, 1H), 7.70 (d, *J* = 7.8 Hz, 1H), 7.58 (s, 2H), 6.68 (d, *J* = 9.0 Hz, 2H), 5.70 - 5.63 (m, 1H), 4.99 (d, *J* = 10.2 Hz, 1H), 4.87 - 4.80 (m, 2H), 4.68 (s, 1H), 4.65 - 4.63 (m, 2H), 4.40 (s, 1H), 3.70 (d, *J* =9.6 Hz, 1H), 3.60 (d, *J* =9.6 Hz, 1H), 3.49 (d, *J* = 10.8 Hz, 1H), 3.31 (s, 1H), 2.91-2.86 (m, 1H), 2.81 - 2.78 (m, 1H), 2.64 - 2.61 (m, 1H), 2.01 - 1.96 (m, 1H), 1.82 (s, 3H), 1.58 (d, *J* = 8.4 Hz, 1H), 1.45 - 1.40 (m, 4H), 0.86 - 0.84 (m, 1H), 0.64 - 0.61 (m, 1H), 0.33 - 0.30 (m, 1H), 0.22 - 0.19 (m, 1H). LCMS (ESI) m/z: 593.3 $[M+H]^+$, HPLC Method B: $R_T$= 7.16 min, purity> 97.9%.

Compound **54b**(9.5 mg, 15.44 μmol); SFC residence time t = 9.232 min. **$^1$H NMR** (600 MHz, DMSO-$d_6$) δ 10.06

(s, 1H), 8.79 (s, 1H), 7.76 (s, 1H), 7.70 (d, *J* = 8.4 Hz, 1H), 7.58 (s, 2H), 6.68 (d, *J* = 8.4 Hz, 2H), 5.70 - 5.63 (m, 1H), 5.00 - 4.98 (d, *J*= 10.2 Hz, 1H), 4.87 - 4.80 (m, 2H), 4.68 (s, 1H), 4.65 - 4.63 (m, 2H), 4.40 (s, 1H), 3.70 (d, *J* = 10.2 Hz, 1H), 3.60 (d, *J* = 10.2 Hz, 1H), 3.49 (d, *J* = 10.8 Hz, 1H), 3.30 (s, 1H), 2.91 - 2.86 (m, 1H), 2.81 - 2.78 (m, 1H), 2.64 - 2.61 (m, 1H), 2.01 - 1.96(m, 1H), 1.82 (s, 3H), 1.58 (d, *J*= 8.4 Hz, 1H), 1.45 - 1.40 (m, 4H), 0.86 - 0.84 (m, 1H), 0.64 - 0.61 (m, 1H), 0.33 - 0.30 (m, 1H), 0.22 - 0.19 (m, 1H). LCMS (ESI) m/z: 581.3 $[M+H]^+$, HPLC Method B: $R_T$= 7.17 min, purity> 96.3%.

**Example 51: Synthesis of Compound 51**

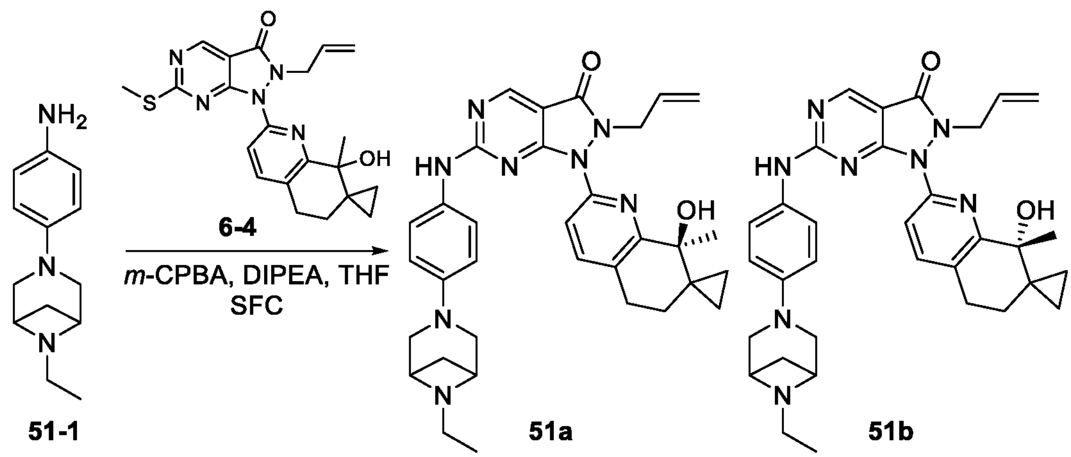


51-1 51a 51b

**Step 1: Synthesis of Compound 51a & 51b**

A dry single-necked flask was added with Substrate **6-4**(30 mg, 73.26 μmol), dissolved in tetrahydrofuran (2 mL), and then added with m-chloroperoxybenzoic acid (22.76 mg, 131.87 μmol). Under room temperature, the reaction was performed for 1 hour. Then the reaction solution was added with N,N-diisopropyl ethyl amine (89.21 mg, 690.26 μmol) and Substrate **51-1**(30 mg, 138.05 μmol), 25°C, the reaction was performed overnight with LC-MS monitoring. After the reaction was completed, the reaction solution was cooled to room temperature, concentrated under reduced pressure. The residue was purified by preparative high-performance liquid chromatography to give the product, and then chirally resolved by supercritical fluid chromatography to give Compound **51a**(9.1 mg, 14.28 μmol); SFC residence time t = 3.568 min. **$^1$H NMR** (400 MHz, Chloroform-d) δ 8.81 (s, 1H), 7.66 (d, J=12.0 Hz, 1H), 7.57 (d, J=12.0 Hz, 1H), 7.46 (s, 2H), 6.72 (d, *J* = 13.8 Hz, 2H), 5.78 - 5.68 (m, 1H), 5.07 (dd, *J* = 15.6, 1.2 Hz, 1H), 4.99 (dd, *J* = 25.2, 1.8 Hz, 1H), 4.78 - 4.61 (m, 2H), 3.97 (s, 2H), 3.59 (d, *J* = 16.8 Hz, 2H), 3.43 (d, *J* = 16.8 Hz, 2H), 3.02 - 2.93 (m, 1H), 2.86 - 2.80 (m, 2H), 2.54 - 2.52 (m, 2H), 2.36 - 2.28 (m, 1H), 1.54 (s, 3H), 1.30 - 1.24(m, 2H), 1.10 (t, *J* = 10.8 Hz, 3H), 1.03 - 0.98 (m, 1H), 0.88 - 0.83 (m, 1H), 0.44 - 0.39 (m, 1H), 0.25 - 0.20 (m, 1H). LCMS (ESI) m/z: 579.3 $[M+H]^+$, HPLC Method B: $R_T$= 8.55 min, purity> 90.8%.

Compound **51b**(7.9 mg, 13.65 μmol); SFC residence time t = 5.059 min. **$^1$H NMR** (400 MHz, Chloroform-d) δ 8.81 (s, 1H), 7.65 (d, J=12.0 Hz, 1H), 7.57 (d, J=12.0 Hz, 1H), 7.49 (s, 2H), 6.72 (d, *J* = 13.8 Hz, 2H), 5.77 - 5.68 (m, 1H), 5.07 (dd, *J* = 15.6, 1.2 Hz, 1H), 4.98 (dd, *J* = 25.2, 1.8 Hz, 1H), 4.78 - 4.61 (m, 2H), 4.06 (s, 2H), 3.62 (d, *J* = 16.8 Hz, 2H), 3.48 (d, *J* = 16.8 Hz, 2H), 3.34 (s, 1H), 3.02 - 2.93 (m, 1H), 2.86 - 2.80 (m, 2H), 2.60 - 2.59 (m, 2H), 2.36 - 2.28 (m, 1H), 1.54 (s, 3H), 1.30 - 1.24(m, 2H), 1.15 (t, *J* = 10.8 Hz, 3H), 1.03 - 0.98 (m, 1H), 0.88 - 0.83 (m, 1H), 0.44 - 0.39 (m, 1H), 0.25 - 0.20 (m, 1H). LCMS (ESI) m/z: 579.3 $[M+H]^+$, HPLC Method B: $R_T$= 8.40 min, purity> 95.4%.

**Example 52: Synthesis of Compound 52**

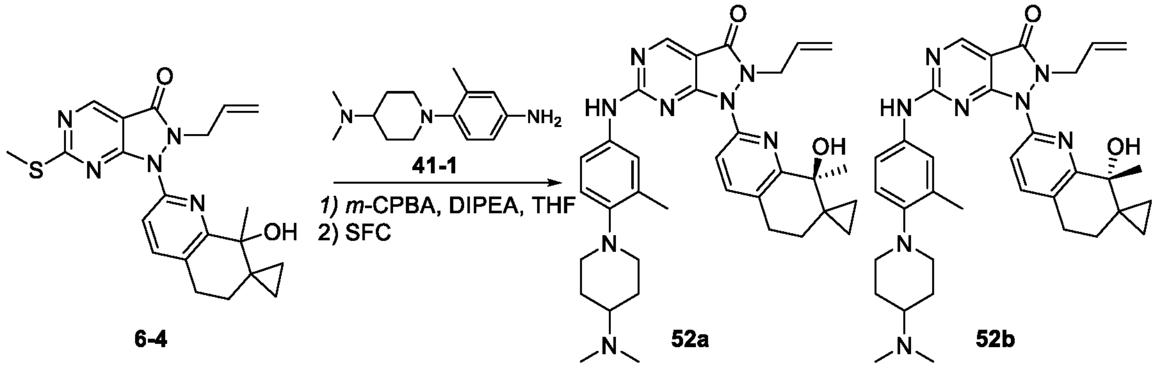


6-4 52a 52b

**Step 1: Synthesis of Compound 52a & 52b**

A dry single-necked flask was added with Substrate **6-4**(30 mg, 73.26 μmol), dissolved in tetrahydrofuran (2 mL), and then added with m-chloroperoxybenzoic acid (31.61 mg, 183.15 μmol). Under room temperature, the reaction was performed for 1 hour. Then the reaction solution was added with N,N-diisopropyl ethyl amine (94.68 mg, 732.59 μmol) and Substrate **41-1**(34.19 mg, 146.52 μmol).The reaction was performed at 45°C overnight with LC-MS monitoring. After the reaction was completed, the reaction solution was cooled to room temperature, concentrated under reduced pressure. The residue was purified by preparative high-performance liquid chromatography to give the product, and then chirally resolved by supercritical fluid chromatography to give Compound **52a**(6.2 mg, 9.00 μmol); SFC residence time t = 3.064 min. **$^1$H NMR** (400 MHz, DMSO-$d_6$) δ 10.12 (s, 1H), 8.83 (s, 1H), 7.75 (d, *J* = 8.4 Hz, 1H), 7.72 - 7.65 (m, 2H), 7.41 - 7.38 (m, 1H), 6.97 (d, *J* = 8.8 Hz, 1H), 5.72 - 5.62 (m, 1H), 4.99 (dd, *J* = 10.4, 1.2 Hz, 1H), 4.88 - 4.83 (m, 2H), 4.70 (s, 1H), 4.66 - 4.61 (m, 1H), 3.04 (d, *J* = 12.0 Hz, 1H), 2.93 - 2.77 (m, 2H), 2.59 - 2.54 (m, 2H), 2.25 - 2.15 (m, 11H), 2.02 - 1.95 (m, 1H), 1.83 (d, *J* = 11.6 Hz, 2H), 1.58 - 1.48 (m, 2H), 1.46 - 1.40 (m, 4H), 0.87 - 0.84 (m, 1H), 0.65 - 0.60 (m, 1H), 0.35 - 0.30 (m, 1H), 0.23 - 0.19 (m, 1H). LCMS (ESI) m/z: 595.4 [M+H]$^+$, HPLC Method B: $R_T$= 10.14 min, purity> 86.3%.

Compound **52b**(6.56 mg, 9.89 μmol); SFC residence time t = 5.193 min. **$^1$H NMR** (400 MHz, DMSO-$d_6$) δ 10.12 (s, 1H), 8.83 (s, 1H), 7.75 (d, *J* = 8.4 Hz, 1H), 7.72 - 7.65 (m, 2H), 7.41 - 7.38 (m, 1H), 6.97 (d, *J* = 8.8 Hz, 1H), 5.72 - 5.62 (m, 1H), 4.99 (dd, *J*=10.4, 1.2 Hz, 1H), 4.88 - 4.78 (m, 2H), 4.70 (s, 1H), 4.67 - 4.61 (m, 1H), 3.04 (d, *J* = 12.0 Hz, 1H), 2.93 - 2.77 (m, 2H), 2.59 - 2.54(m, 2H), 2.25 - 2.15 (m, 11H), 2.02 - 1.95 (m, 1H), 1.83 (d, J=11.6 Hz, 2H), 1.58 - 1.48 (m, 2H), 1.46 - 1.40 (m, 4H), 0.87 - 0.84 (m, 1H), 0.65 - 0.60 (m, 1H), 0.35 - 0.30 (m, 1H), 0.23 - 0.19 (m, 1H). LCMS (ESI) m/z: 595.4 [M+H]$^+$, HPLC Method B: $R_T$= 10.32 min, purity> 89.7%.

**Example 53: Synthesis of Compound 53**

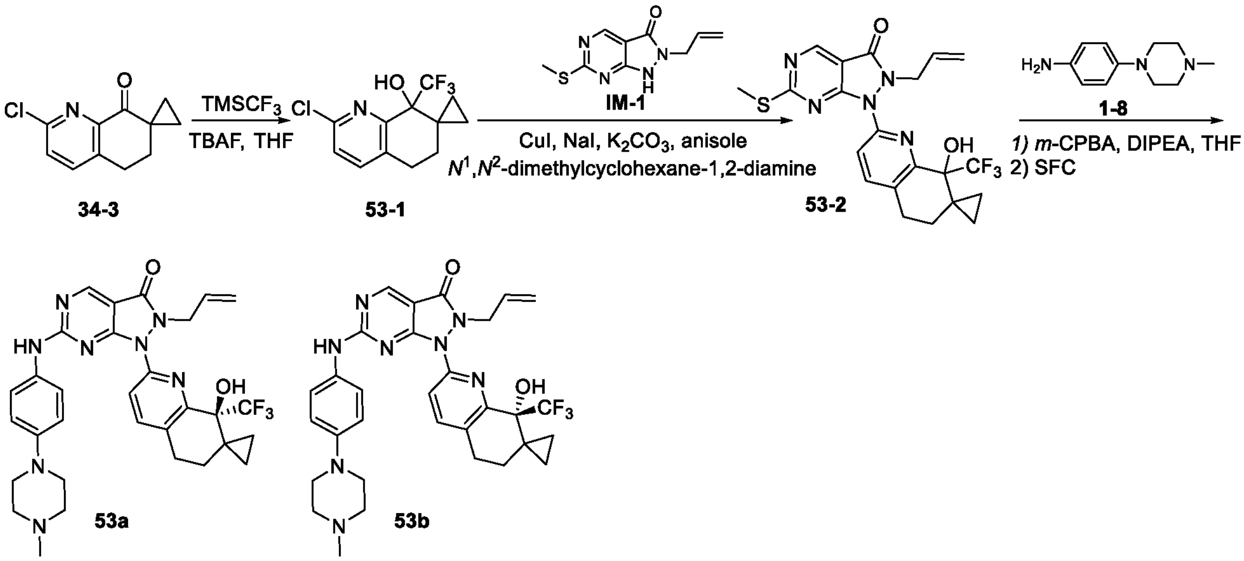


34-3 53-1 53-2 53a 53b

**Step 1: Synthesis of Compound 53-1:**

A dry three-necked flask was added with Substrate **34-3**(180 mg, 866.82 μmol), added with tetrahydrofuran (5 mL) to dissolve, under the protection of nitrogen atmosphere cooled to 0°C, and then slowly dropwise added with (trifluoromethyl)trimethylsilane (616.28 mg, 4.33 mmol) and tetrabutylammonium fluoride (1 M, 866.82 μL), and the temperature was kept to react for 1 hour with LC-MS monitoring. The reaction solution was concentrated under reduced pressure. The residue was purified by column chromatography to give Compound **53-1**(209 mg, 752.69 μmol). LCMS (ESI) m/z: 278.1 $[M+H]^+$.

**Step 2: Synthesis of Compound 53-2:**

A dry microwave tube was added with Substrate **53-1**(209 mg, 752.69 μmol), Substrate **IM-1**(175.66 mg, 790.33 μmol), copper (I) iodide (286.70 mg, 1.51 mmol), sodium iodide (225.64 mg, 1.51 mmol), potassium carbonate (260.07 mg, 1.88 mmol) and trans-(1R,2R)-N,N'-dimethyl-1,2-cyclohexylene diamine (428.25 mg, 3.01 mmol), and then added with anisole (15 mL). Under nitrogen atmosphere, the reaction was heated by microwave to 130 °C for 3 hours with LC-MS monitoring. The reaction solution was cooled to room temperature, filtrated, concentrated under reduced pressure. The residue was purified by column chromatography to give Compound 53-2(210 mg, 453.10 μmol).LCMS (ESI) m/z: 464.3 $[M+H]^+$.

**Step 3**: **Synthesis of Compound 53a & 53b:**

A dry single-necked flask was added with Substrate **53-2**(40 mg, 86.30 μmol), dissolved in tetrahydrofuran (4 mL), and then added with m-chloroperoxybenzoic acid (31.54 mg, 155.35 μmol). Under room temperature, the reaction was performed for 1 hour. Then the reaction solution was added with N,N-diisopropyl ethyl amine (111.54 mg, 863.05 μmol) and Substrate 4-(4-methylpiperazino)aniline (33.02 mg, 172.61 μmol).The reaction was performed at 45°C overnight with LC-MS monitoring. After the reaction was completed, the reaction solution was cooled to room temperature, concentrated under reduced pressure. The residue was purified by preparative high-performance liquid chromatography to give the product, and then chirally resolved by supercritical fluid chromatography to give Compound **53a**(12.6 mg, 18.88 μmol); SFC residence time t = 2.141 min. **$^1$H NMR** (600 MHz, Methanol-*d*$_4$) δ 10.17 (s, 1H), 8.83 (s, 1H), 8.06 - 7.92 (m, 1H), 7.89 (d, *J* = 8.4 Hz, 1H), 7.58 (s, 2H), 6.94 (d, *J* = 8.4 Hz, 2H), 6.03 (s, 1H), 5.73 - 5.62 (m, 1H), 4.97 (d, *J*= 10.2 Hz, 1H), 4.88 - 4.73 (m, 2H), 4.59 (dd, *J*= 16.2, 7.2 Hz, 1H), 3.11 (t, *J* = 4.8 Hz, 4H), 3.05 - 2.99 (m, 1H), 2.93 (dd, *J* = 17.2, 6.0 Hz, 1H), 2.47 (t, *J* = 4.8 Hz, 4H), 2.23 (s, 3H), 1.25 - 1.17 (m, 2H), 1.05 - 1.09 (m, 1H), 0.74 - 0.72 (m, 1H), 0.60 - 0.52 (m, 1H), 0.22 - 0.20 (m, 1H). LCMS (ESI) m/z: 607.5 $[M+H]^+$, HPLC Method B: $R_T$= 8.74 min, purity> 90.9%.

Compound **53b**(13.7 mg, 20.60 μmol); SFC residence time t = 3.522 min. **$^1$H NMR** (600 MHz, Methanol-*d*$_4$) δ 10.17 (s, 1H), 8.83 (s, 1H), 8.07 - 7.92 (m, 1H), 7.89 (d, *J* = 8.4 Hz, 1H), 7.58 (s, 2H), 6.94 (d, *J* = 8.4 Hz, 2H), 6.03 (s, 1H), 5.73 - 5.62 (m, 1H), 5.02 - 4.93 (m, 1H), 4.86 - 4.73 (m, 2H), 4.59 (dd, *J* = 16.2, 7.2 Hz, 1H), 3.11 (t, *J* = 4.8 Hz, 4H), 3.05 - 2.99 (m, 1H), 2.93 (dd, *J* = 17.2, 6.0 Hz, 1H), 2.46 (t, *J* = 4.8 Hz, 4H), 2.23 (s, 3H), 1.25 - 1.16 (m, 2H), 1.05 - 1.09 (m, 1H), 0.74 - 0.72 (m, 1H), 0.60 - 0.52 (m, 1H), 0.22 - 0.20 (m, 1H). LCMS (ESI) m/z: 607.1 $[M+H]^+$, HPLC Method B: $R_T$= 8.77 min, purity> 91.2%.

**Example 54: Synthesis of Compound 54**

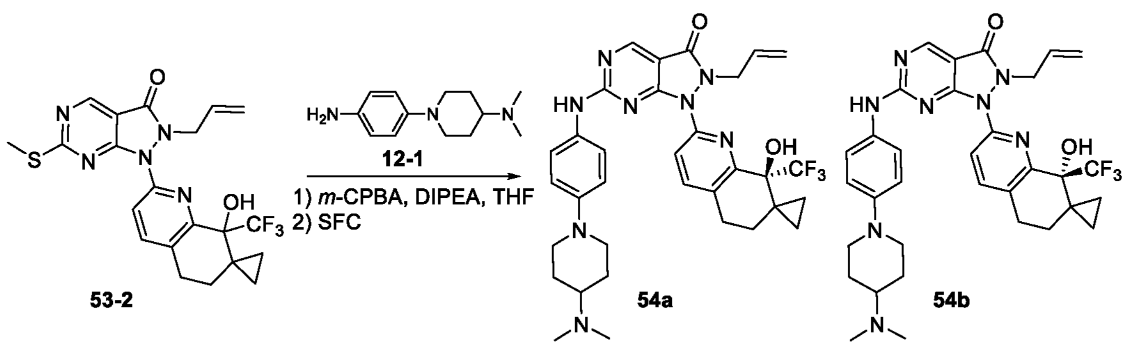


53-2 54a 54b

**Step 1: Synthesis of Compound 54a & 54b:**

A dry single-necked flask was added with Substrate **53-2**(40 mg, 86.30 μmol), dissolved in tetrahydrofuran (4 mL), and then added with m-chloroperoxybenzoic acid (31.54 mg, 155.35 μmol). Under room temperature, the reaction was performed for 1 hour. Then the reaction solution was added with N,N-diisopropyl ethyl amine (111.54 mg, 863.05 μmol) and Substrate **12-1**(37.86 mg, 172.61 μmol).The reaction was performed at 45°C overnight with LC-MS monitoring. After the reaction was completed, the reaction solution was cooled to room temperature, concentrated under reduced pressure. The residue was purified by preparative high-performance liquid chromatography to give the product, and then chirally resolved by supercritical fluid chromatography to give Compound **54a(8.2** mg, 12.61 μmol); SFC residence time t = 2.764 min. **$^1$H NMR** (600 MHz, Methanol-$d_4$) δ 10.17 (s, 1H), 8.83 (s, 1H), 8.06 - 7.92 (m, 1H), 7.89 (d, *J* = 8.4 Hz, 1H), 7.58 (s, 2H), 6.94 (d, *J* = 8.4 Hz, 2H), 6.03 (s, 1H), 5.73 - 5.62 (m, 1H), 4.97 (d, *J* = 10.2 Hz, 1H), 4.88 - 4.73 (m, 2H), 4.59 (dd, *J* = 16.2, 7.2 Hz, 1H), 3.11 (t, *J* = 4.8 Hz, 4H), 3.06 - 2.98 (m, 1H), 2.93 (dd, *J*= 17.2, 6.0 Hz, 1H), 2.47 (t, *J* = 4.8 Hz, 4H), 2.23 (s, 3H), 1.25 - 1.17 (m, 2H), 1.03 - 0.97 (m, 1H), 0.70 - 0.75 (m, 1H), 0.60 - 0.52 (m, 1H), 0.23 - 0.18 (m, 1H). LCMS (ESI) m/z: 635.4 $[M+H]^+$, HPLC Method B: $R_T$= 9.66 min, purity> 97.6%.

Compound **54b**(10.0 mg, 14.98 μmol); SFC residence time t = 4.157 min. **$^1$H NMR** (600 MHz, Methanol-$d_4$) δ 10.17 (s, 1H), 8.83 (s, 1H), 8.07 - 7.92 (m, 1H), 7.89 (d, *J* = 8.4 Hz, 1H), 7.58 (s, 2H), 6.94 (d, *J* = 8.4 Hz, 2H), 6.03 (s, 1H), 5.73 - 5.62 (m, 1H), 5.02 - 4.93 (m, 1H), 4.86 - 4.73 (m, 2H), 4.59 (dd, *J* = 16.2, 7.2 Hz, 1H), 3.11 (t, *J* = 4.8 Hz, 4H), 3.06 - 2.98 (m, 1H), 2.93 (dd, *J* = 17.2, 6.0 Hz, 1H), 2.46 (t, *J* = 4.8 Hz, 4H), 2.23 (s, 3H), 1.25 - 1.16 (m, 2H), 1.03 - 0.97 (m, 1H), 0.70 - 0.75 (m, 1H), 0.60 - 0.52 (m, 1H), 0.23 - 0.18 (m, 1H). LCMS (ESI) m/z: 635.4 $[M+H]^+$, HPLC Method B: $R_T$= 9.75 min, purity> 95.1%.

**Example 55: Synthesis of Compound 55**

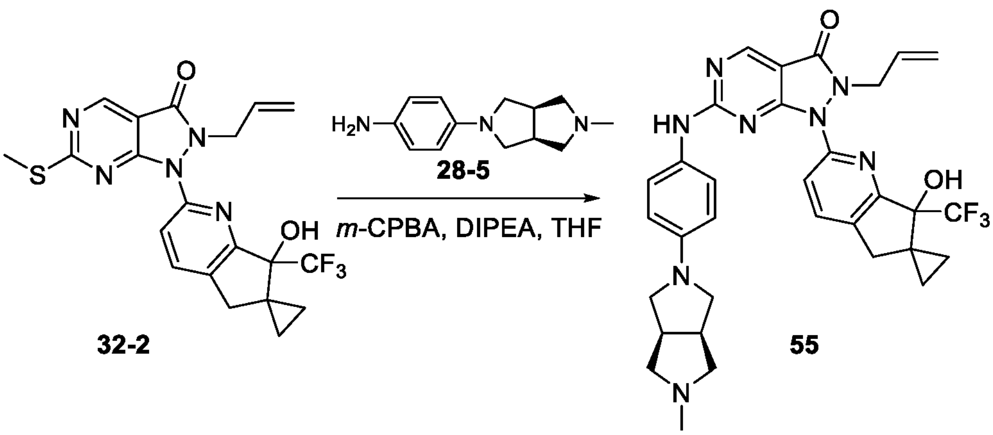


32-2

55

**Step** 1: **Synthesis of Compound 55:**

A dry single-necked flask was added with Substrate **32-2**(40 mg, 89.00 μmol), dissolved in tetrahydrofuran (2 mL), and then added with m-chloroperoxybenzoic acid (38.40 mg, 222.49 μmol). Under room temperature, the reaction was performed for 1 hour. Then the reaction solution was added with N,N-diisopropyl ethyl amine (115.02 mg, 889.98 μmol) and Substrate **28-5**(38.68 mg, 178.00 μmol).At 50°C, the reaction was performed overnight with LC-MS monitoring. After the reaction was completed, the reaction solution was cooled to room temperature, concentrated under reduced pressure. The residue was purified by preparative high-performance liquid chromatography to give Compound **55**(12.38 mg, 17.33 μmol).**$^1$H NMR** (400 MHz, DMSO-$d_6$) δ 10.12 (s, 1H), 8.82 (s, 1H), 8.04 - 8.02 (m, 1H), 7.91 (d, *J* = 8.0 Hz, 1H), 7.53 (s, 2H), 6.69 (s, 1H), 6.54 (d, *J* = 8.4 Hz, 2H), 5.72 - 5.62 (m, 1H), 4.98 (d, *J* = 10.0 Hz, 1H), 4.82 (d, *J* = 16.8 Hz, 2H), 4.57 (dd, *J* = 7.2, 16.0 Hz, 1H), 3.30 (s, 2H), 3.22 - 3.18 (m, 1H), 3.05 (d, *J* = 8.8 Hz, 2H), 2.87 - 2.86 (m, 2H), 2.75 - 2.71 (m, 1H), 2.56 (t, *J* = 8.8 Hz, 2H), 2.40 - 2.22 (m, 2H), 2.22 (s, 3H), 1.04 - 0.99 (m, 2H), 0.91 - 0.88 (m, 1H), 0.49 - 0.47 (m, 1H). LCMS (ESI) m/z: 619.3 $[M+H]^+$, HPLC Method B: $R_T$= 8.79 min, purity> 86.6%.

**Example 56: Synthesis of Compound 56**

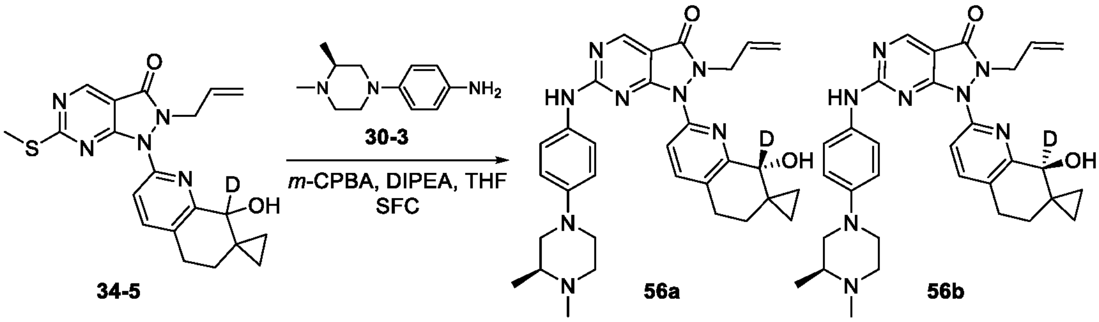


34-5 56a 56b

**Step 1: Synthesis of Compound 56a & 56b:**

A dry single-necked flask was added with Substrate **34-5**(35 mg, 88.28 μmol), dissolved in tetrahydrofuran (1 mL), and then added with m-chloroperoxybenzoic acid (27.42 mg, 158.90 μmol). Under room temperature, the reaction was performed for 1 hour. Then the reaction solution was added with N,N-diisopropyl ethyl amine (114.09 mg, 882.76 μmol,) and Substrate **30-3**(36.25 mg, 176.55 μmol).The reaction was performed at 45°C overnight with LC-MS monitoring. After the reaction was completed, the reaction solution was cooled to room temperature, concentrated under reduced pressure. The residue was purified by preparative high-performance liquid chromatography to give the product, and then chirally resolved by supercritical fluid chromatography to give Compound **56a**(11.0 mg, 19.87 μmol); SFC residence time t = 3.027 min. **$^1$H NMR** (400 MHz, DMSO-$d_6$) δ 10.11 (s, 1H), 8.81 (s, 1H), 7.83 (d, *J* = 8.4 Hz, 1H), 7.69 (d, *J* = 8.4 Hz, 1H), 7.58 (s, 2H), 6.92 (d, *J* = 9.2 Hz, 2H), 5.74 - 5.62 (m, 1H), 5.29 (s, 1H), 5.02 - 4.97 (m, 1H), 4.93 - 4.85 (m, 1H), 4.68 (s, 1H), 4.61 (dd, *J* = 15.9, 5.7 Hz, 1H), 3.52 - 3.43 (m, 2H), 2.89 - 2.78 (m, 3H), 2.72 - 2.65 (m, 1H), 2.37 - 2.31 (m, 1H), 2.30 - 2.22 (m, 2H), 2.21 (s, 3H), 2.17 - 2.10 (m, *J* = 9.7, 6.3, 2.9 Hz, 1H), 1.14 - 1.08 (m, 1H), 1.06 (d, *J* = 6.2 Hz, 3H), 0.71 - 0.63 (m, 1H), 0.46 - 0.41 (m, 1H), 0.40 - 0.32 (m, 2H). LCMS (ESI) m/z: 554.2 [M+H]$^+$, HPLC Method B: $R_T$= 7.15 min, purity> 82.1%.

Compound **56b**(10.8 mg, 19.51 μmol); SFC residence time t = 5.117 min. **$^1$H NMR** (400 MHz, DMSO-$d_6$) δ 10.11 (s, 1H), 8.81 (s, 1H), 7.83 (d, *J* = 8.4 Hz, 1H), 7.69 (d, *J* = 8.4 Hz, 1H), 7.58 (s, 2H), 7.01 - 6.79 (m, 2H), 5.73 - 5.62 (m, 1H), 5.29 (s, 1H), 5.02 - 4.97 (m, 1H), 4.94 - 4.85 (m, 1H), 4.68 (s, 1H), 4.61 (dd, *J* = 15.7, 5.7 Hz, 1H), 3.49 (d, *J* = 11.0 Hz, 2H), 2.88 - 2.78 (m, 3H), 2.72 - 2.64 (m, 1H), 2.34 (dd, *J* = 11.6, 9.9 Hz, 1H), 2.30 - 2.23 (m, 2H), 2.21 (s, 3H), 2.17 - 2.10 (m, *J* = 9.7, 6.3, 2.9 Hz, 1H), 1.15 - 1.08 (m, 1H), 1.06 (d, *J* = 6.2 Hz, 3H), 0.71 - 0.62 (m, 1H), 0.47 - 0.41 (m, 1H), 0.40 - 0.33 (m, 2H). LCMS (ESI) m/z: 554.2 [M+H]$^+$, HPLC Method B: $R_T$= 7.15 min, purity> 87.6%.

**Example 57: Synthesis of Compound 57**

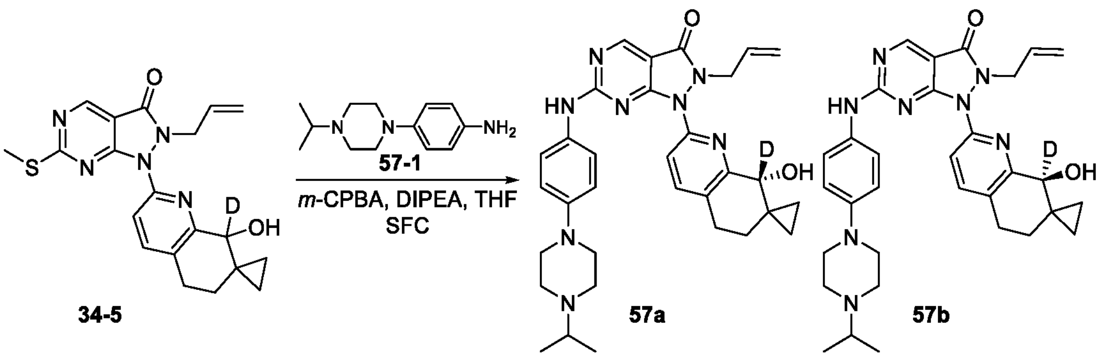


34-5 57a 57b

**Step 1: Synthesis of Compound 57a & 57b:**

A dry single-necked flask was added with Substrate **34-5**(35 mg, 88.28 μmol), dissolved in tetrahydrofuran (1 mL), and then added with m-chloroperoxybenzoic acid (15.23 mg, 88.28 μmol). Under room temperature, the reaction was performed for 1 hour. Then the reaction solution was added with N,N-diisopropyl ethyl amine (114.09 mg, 882.76 μmol,) and Substrate **57-1**(38.72 mg, 176.55 μmol).The reaction was performed at 45°C overnight with LC-MS monitoring. After the reaction was completed, the reaction solution was cooled to room temperature, concentrated under reduced pressure. The residue was purified by preparative high-performance liquid chromatography to give the product, and then chirally

resolved by supercritical fluid chromatography to give Compound **57a**(12.5 mg, 22.02 μmol); SFC residence time t = 3.149 min. **$^1$H NMR** (400 MHz, DMSO-$d_6$) δ 10.11 (s, 1H), 8.81 (s, 1H), 7.83 (d, *J* = 8.4 Hz, 1H), 7.70 (d, *J* = 8.4 Hz, 1H), 7.64 - 7.49 (m, 2H), 6.91 (d, *J* = 9.2 Hz, 2H), 5.74 - 5.63 (m, 1H), 5.29 (s, 1H), 5.00 (dd, *J*= 10.2, 1.6 Hz, 1H), 4.89 (dd, *J* = 17.2, 1.6 Hz, 1H), 4.64 (dd, *J* = 17.2, 1.2 Hz, 2H), 3.09 (t, *J* = 4.8 Hz, 4H), 2.90 - 2.81 (m, 2H), 2.71 - 2.64 (m, 1H), 2.58 (t, *J* = 5.2 Hz, 4H), 2.29 - 2.21 (m, 1H), 1.14 - 1.08 (m, 1H), 1.02 (s, 3H), 1.00 (s, 3H), 0.70 - 0.64 (m, 1H), 0.47 - 0.41 (m, 1H), 0.39 - 0.30 (m, 2H). LCMS (ESI) m/z: 568.2 [M+H]$^+$, HPLC Method B: $R_T$= 7.64 min, purity> 86.5%.

Compound **57b**(12.4 mg, 21.84 μmol); SFC residence time t = 4.956 min. **$^1$H NMR** (400 MHz, DMSO-$d_6$) δ 10.11 (s, 1H), 8.81 (s, 1H), 7.83 (d, *J* = 8.4 Hz, 1H), 7.70 (d, *J* = 8.4 Hz, 1H), 7.58 (s, 2H), 6.91 (d, *J* = 8.8 Hz, 2H), 5.74 - 5.64 (m, 1H), 5.29 (s, 1H), 5.00 (dd, *J* = 10.2, 1.6 Hz, 1H), 4.89 (dd, *J* = 17.2, 1.6 Hz, 1H), 4.64 (dd, *J* = 17.2, 1.6 Hz, 2H), 3.09 (t, *J* = 4.8 Hz, 4H), 2.89 - 2.79 (m, 2H), 2.71 - 2.64 (m, 1H), 2.58 (t, *J* = 5.0 Hz, 4H), 2.29 - 2.21 (m, 1H), 1.14 - 1.07 (m, 1H), 1.02 (s, 3H), 1.00 (s, 3H), 0.70 - 0.64 (m, 1H), 0.47 - 0.41 (m, 1H), 0.39 - 0.31 (m, 2H). LCMS (ESI) m/z: 568.2 [M+H]$^+$, HPLC Method B: $R_T$= 7.63 min, purity> 88.6%.

**Example 58: Synthesis of Compound 58**

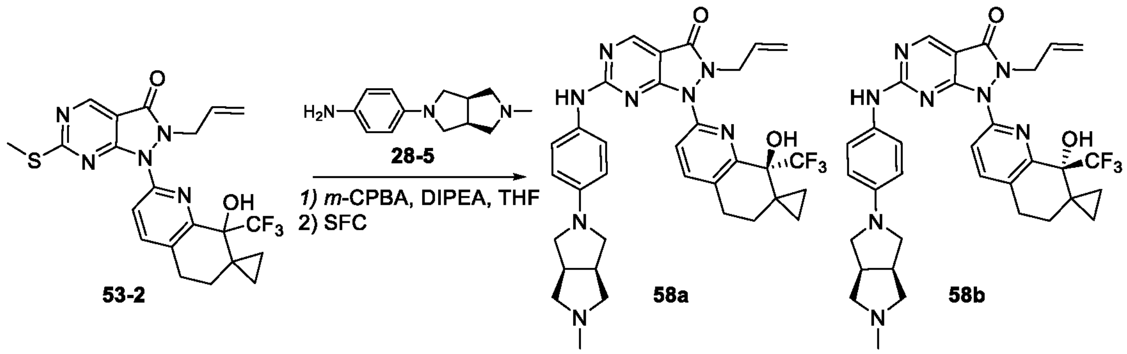


53-2 58a 58b

**Step 1: Synthesis of Compound 58a & 58b:**

A dry single-necked flask was added with Substrate **53-2**(40 mg, 86.30 μmol), dissolved in tetrahydrofuran (2 mL), and then added with m-chloroperoxybenzoic acid (37.23 mg, 215.76 μmol). Under room temperature, the reaction was performed for 1 hour. Then the reaction solution was added with N,N-diisopropyl ethyl amine (111.54 mg, 863.05 μmol) and Substrate **28-5**(18.75 mg, 86.30 μmol).The reaction was performed at 45°C overnight with LC-MS monitoring. After the reaction was completed, the reaction solution was cooled to room temperature, concentrated under reduced pressure. The residue was purified by preparative high-performance liquid chromatography to give the product, and then chirally resolved by supercritical fluid chromatography to give Compound **58a**(7.25 mg, 9.60 μmol); SFC residence time t = 3.125 min. **$^1$H NMR** (400 MHz, DMSO-$d_6$) δ 10.09 (s, 1H), 8.81 (s, 1H), 7.94 - 7.88 (m, 2H), 7.52 (s, 2H), 6.64 (d, *J* = 8.4 Hz, 1H), 6.02 (s, 1H), 5.69 - 5.59 (m, 1H), 4.96 (d, *J* = 9.6 Hz, 1H), 4.80 - 4.76 (m, 2H), 4.61 - 4.56 (m, 1H), 3.30 (s, 2H), 3.06 (d, *J* = 8.8 Hz, 2H), 2.99 - 2.88 (m, 3H), 2.57 (t, *J* = 8.4 Hz, 2H), 2.46 (s, 1H), 2.39 (d, *J= 7.6* Hz, 2H), 2.22 (s, 3H), 1.23 (s, 1H), 1.02 - 0.97 (m, 1H), 0.74 - 0.71 (m, 1H), 0.59 - 0.54 (m, 1H), 0.23 - 0.19 (m, 1H). LCMS (ESI) m/z: 633.3 [M+H]$^+$, HPLC Method B: $R_T$= 10.11 min, purity> 83.8%.

Compound **58b**(6.7 mg, 8.65 μmol); SFC residence time t = 4.825 min. **$^1$H NMR** (400 MHz, DMSO-$d_6$) δ 10.09 (s, 1H), 8.81 (s, 1H), 7.94 - 7.88 (m, 2H), 7.53 - 7.37 (m, 2H), 6.64 (d, *J* = 8.4 Hz, 1H), 6.02 (s, 1H), 5.69 - 5.59 (m, 1H), 4.96 (d, *J* = 9.6 Hz, 1H), 4.80 - 4.76 (m, 2H), 4.61 - 4.56 (m, 1H), 3.30 (s, 2H), 3.06 (d, *J* = 8.8 Hz, 2H), 2.99 - 2.88 (m, 3H), 2.59 (t, *J* = 8.4 Hz, 2H), 2.47 - 2.41 (m, 3H), 2.24 (s, 3H), 1.26 - 1.23 (m, 2H), 1.03 - 0.97 (m, 1H), 0.74 - 0.71 (m, 1H), 0.59 - 0.54 (m, 1H), 0.23 - 0.19 (m, 1H). LCMS (ESI) m/z: 633.2 [M+H]$^+$, HPLC Method B: $R_T$= 10.14min, purity> 81.7%.

**Example 59: Synthesis of Compound 59**

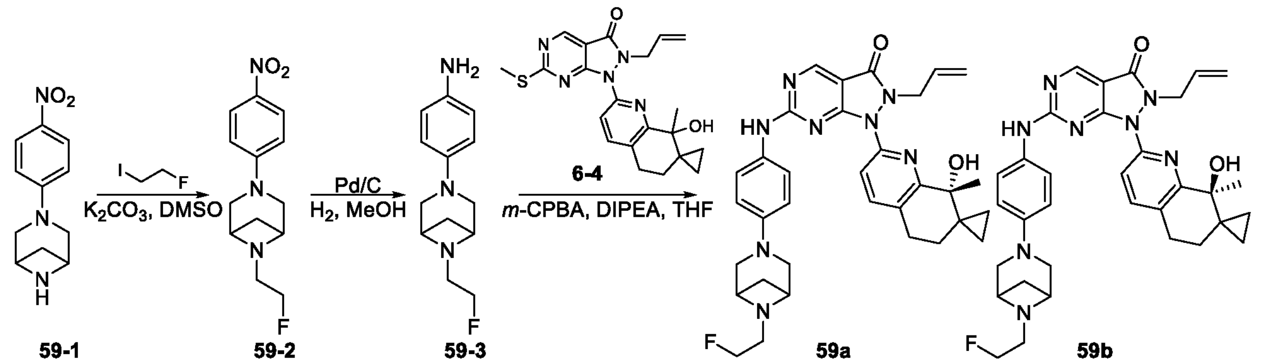


59-1 59-2 59-3 59a 59b

**Step 1: Synthesis of Compound 59-2:**

A dry single-necked flask was added with Substrate **59-1**(66 mg, 301.04 μmol), dissolved in acetone (2 mL), 1-fluoro-2-iodoethane (57 mg, 327.67 μmol) and potassium carbonate (58 mg, 419.68 μmol).At 50°C, the reaction was heated for 6 hours with LC-MS monitoring for complete reaction. The reaction solution was concentrated under reduced pressure, added with water, extracted with ethyl acetate for four times. The organic phase was dried with anhydrous sodium sulfate, filtrated, concentrated under reduced pressure. The residue was purified by column chromatography to give Compound **59-2(55** mg, 207.33 μmol).LCMS (ESI) m/z: 266.2 $[M+H]^+$.

**Step 2: Synthesis of Compound 59-3:**

A dry single-necked flask was added with Substrate **59-2**(55 mg, 207.33 μmol), dissolved in methanol (5mL), and then added with Pd-C (10 mg). Under hydrogen atmosphere, the reaction was performed under room temperature for 2 hours with LC-MS monitoring for complete reaction. The reaction solution was filtrated with celite, washed with methanol twice. The filtrate was concentrated under reduced pressure to give Compound **59-3**(30 mg, 127.50 μmol).LCMS (ESI) m/z: 236.2 $[M+H]^+$.

**Step 3: Synthesis of Compound 59a & 59b:**

A dry single-necked flask was added with Substrate **6-4**(30 mg, 73.26 μmol), dissolved in tetrahydrofuran (2 mL), and then added with m-chloroperoxybenzoic acid (22.76 mg, 131.87 μmol). Under room temperature, the reaction was performed for 1 hour. Then the reaction solution was added with N,N-diisopropyl ethyl amine (54.67 mg, 423.03 μmol) and Substrate **59-3**(30 mg, 127.50 μmol).The reaction was performed at 45°C overnight with LC-MS monitoring. After the reaction was completed, the reaction solution was cooled to room temperature, concentrated under reduced pressure. The residue was purified by preparative high-performance liquid chromatography to give the product, and then chirally resolved by supercritical fluid chromatography to give Compound **59a** (10.8 mg, 16.54 μmol); SFC residence time t = 3.435 min. **$^1$H NMR** (400 MHz, Chloroform-d) δ 8.81 (s, 1H), 7.68 - 7.57 (m, 2H), 7.49 - 7.47 (m, 2H), 6.73 (d, *J* = 8.8 Hz, 2H), 5.77 - 5.68 (m, 1H), 5.08 - 5.06 (m, 1H), 5.01 - 4.96 (m, 1H), 4.79 - 4.57 (m, 4H), 4.03 - 4.02 (m, 2H), 3.68 (d, *J* = 11.2 Hz, 2H), 3.45 (d, *J* = 11.2 Hz, 2H), 3.34 (s, 1H), 3.02 - 2.94 (m, 1H), 2.89 - 2.76 (m, 4H), 2.36 - 2.28 (m, 1H), 1.73 (d, *J* = 8.8 Hz, 1H), 1.55 (s, 3H), 1.29 - 1.24 (m, 2H), 1.03 - 0.99 (m, 1H), 0.88 - 0.84 (m, 1H), 0.44 - 0.39 (m, 1H), 0.25 - 0.20 (m, 1H). LCMS (ESI) m/z: 597.2 $[M+H]^+$, HPLC Method B: $R_T$= 7.95 min, purity> 91.4%.

Compound **59b** (13.6 mg, 22.22μmol); SFC residence time t = 4.794 min. **$^1$H NMR** (400 MHz, Chloroform-d) δ 8.81 (s, 1H), 7.67 (d, *J* = 8.4Hz, 1H), 7.58 (d, *J* = 8.0 Hz, 1H), 7.48 - 7.47 (m, 2H), 6.73 (d, *J* = 8.8 Hz, 2H), 5.77 - 5.68 (m, 1H), 5.08 - 5.05 (m, 1H), 5.01 - 4.96 (m, 1H), 4.79 - 4.54 (m, 4H), 3.98 (d, *J* = 4.0 Hz, 2H), 3.67 (d, *J* = 11.2 Hz, 2H), 3.43 (d, *J* = 11.2 Hz, 2H), 3.34 (s, 1H), 3.02 - 2.95 (m, 1H), 2.85 - 2.73 (m, 4H), 2.36 - 2.28 (m, 1H), 1.70 (d, *J*= 8.8 Hz, 1H), 1.54 (s, 3H), 1.29 - 1.24 (m, 2H), 1.03 - 0.99 (m, 1H), 0.88 - 0.83 (m, 1H), 0.44 - 0.39 (m, 1H), 0.25 - 0.22 (m, 1H). LCMS (ESI) m/z: 597.2 $[M+H]^+$, HPLC Method B: $R_T$= 7.96 min, purity> 97.5%.

**Example 60: Synthesis of Compound 60**

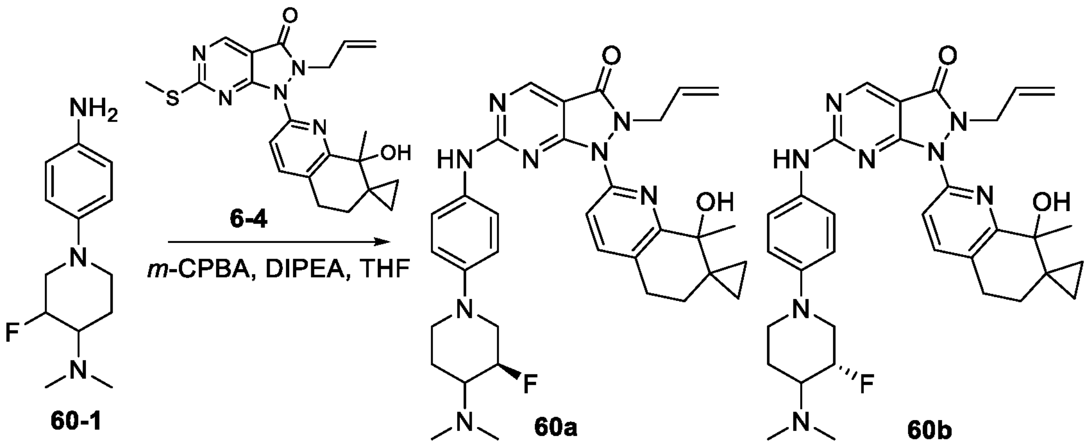


60-1 60a 60b

**Step 1: Synthesis of Compound 60a & 60b:**

A dry single-necked flask was added with Substrate **6-4**(30 mg, 73.26 μmol), dissolved in tetrahydrofuran (2 mL), and then added with m-chloroperoxybenzoic acid (22.76 mg, 131.87 μmol). Under room temperature, the reaction was performed for 1 hour. Then the reaction solution was added with N,N-diisopropyl ethyl amine (54.67 mg, 423.03 μmol) and Substrate 60-1(35 mg, 147.48 μmol).At 50°C, the reaction was performed overnight with LC-MS monitoring. After the reaction was completed, the reaction solution was cooled to room temperature, concentrated under reduced pressure. The residue was purified by preparative high-performance liquid chromatography to give Compound **60a** (8.16 mg, 11.67 μmol).**$^1$H NMR** (400 MHz, Chloroform-*d*) δ 8.81 (s, 1H), 7.66-7.58 (m, 2H), 7.46 (d, *J* = 8.8 Hz, 2H), 6.93 (d, *J* = 8.8 Hz, 2H), 5.77 - 5.67 (m, 1H), 5.15 - 4.96 (m, 3H), 4.80 - 4.63 (m, 2H), 4.00 - 3.94 (m, 1H), 3.76 (d, *J* = 12.0 Hz, 1H), 3.35 (s, 1H), 3.03 - 2.76 (m, 4H), 2.48 (s, 6H), 2.40 - 2.28 (m, 2H), 2.22 - 2.12 (m, 1H), 1.95 - 1.92 (m, 2H), 1.54 (s, 3H), 1.30 - 1.25 (m, 1H), 1.02 - 1.00 (m, 1H), 0.88 - 0.83 (m, 1H), 0.44 - 0.39 (m, 1H), 0.26 - 0.21 (m, 1H). LCMS (ESI) m/z: 599.2 $[M+H]^+$, HPLC Method B: $R_T$= 7.91 min, purity> 85.6%.

Compound **60b** (2 mg, 2.84 μmol).**$^1$H NMR** (400 MHz, Chloroform-*d*) δ 8.82 (s, 1H), 7.65 - 7.58 (m, 2H), 7.48 (d, *J* = 8.8 Hz, 2H), 6.92 (d, *J* = 8.8 Hz, 2H), 5.77 - 5.67 (m, 1H), 5.08 - 5.06 (m, 1H), 5.00 - 4.96 (m, 1H), 4.90 - 4.61 (m, 3H), 3.96 - 3.90 (m, 1H), 3.63 - 3.60 (m, 1H), 3.30 (s, 1H), 3.03 - 2.95 (m, 1H), 2.88 - 2.70 (m, 4H), 2.52 (s, 6H), 2.32 - 2.29 (m, 1H), 2.06 (s, 1H), 1.80 - 1.70 (m, 1H), 1.55 (s, 3H), 1.31 - 1.25 (m, 2H), 1.03 - 0.98 (m, 1H), 0.88 - 0.83 (m, 1H), 0.44 - 0.40 (m, 1H), 0.26 - 0.21 (m, 1H). LCMS (ESI) m/z: 599.2 $[M+H]^+$, HPLC Method B: $R_T$= 8.26 min, purity> 85.1%.

**Example 61: Synthesis of Compound 61**

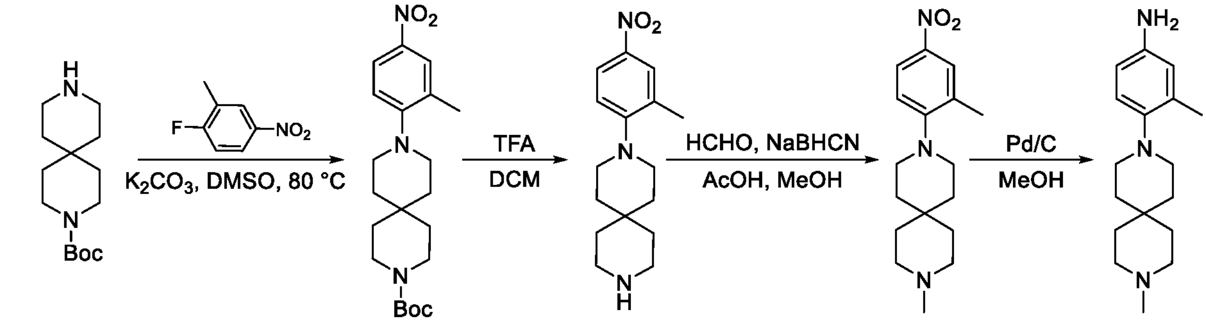


61-1 61-2 61-3 61-4 61-5

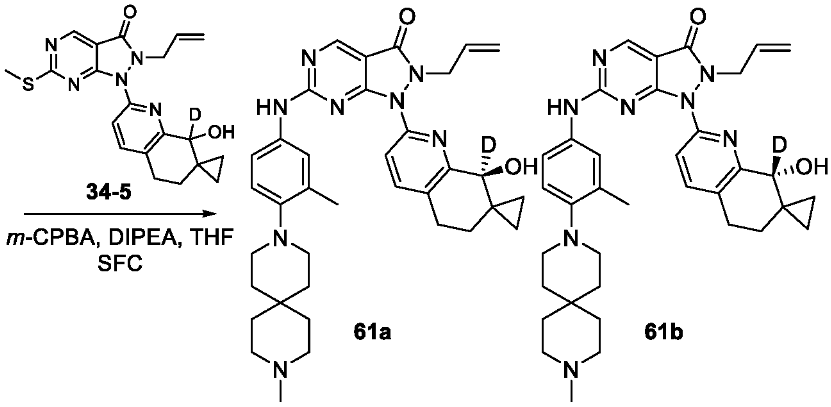


34-5 61a 61b

**Step 1: Synthesis of Compound 61-2:**

A dry single-necked flask was added with Substrate **61-1** (0.5 g, 1.97 mmol), dissolved in dimethyl sulfoxide (10 mL), and then added with p-2-fluoro-5- nitrotoluene(304.93 mg, 1.97 mmol) and potassium carbonate (543.33 mg, 3.93 mmol). At 80°C, the reaction was performed for 16 hours with LC-MS monitoring for complete reaction. The reaction solution was cooled to room temperature, added with water, and then extracted with ethyl acetate for four times. The organic phase was dried with anhydrous sodium sulfate, filtrated, concentrated under reduced pressure. The residue was purified by column chromatography to give Compound **61-2**(740 mg, 1.90 mmol). LCMS (ESI) m/z: 390.2 $[M+H]^+$.

**Step 2: Synthesis of Compound 61-3:**

A dry single-necked flask was added with Substrate **61-2**(740 mg, 1.90 mmol), dissolved in dichloromethane (15 mL), added with trifluoroacetic acid (5 mL). Under room temperature, the reaction was performed for 2 hours with LC-MS monitoring for complete reaction. The reaction solution was concentrated under reduced pressure. The residue was dissolved in water, and added with saturated sodium bicarbonate aqueous solution to adjust to pH=9, and then the aqueous phase was extracted with dichloromethane for four times. The organic phase was dried with anhydrous sodium sulfate, filtrated, concentrated under reduced pressure to give Compound **61-3**(550 mg, 1.90 mmol). LCMS (ESI) m/z: 290.2 $[M+H]^+$.

**Step 3: Synthesis of Compound 61-4:**

A dry single-necked flask was added with Substrate **61-3**(550 mg, 1.90 mmol), dissolved in methanol (15 mL), added with acetic acid (3 mL) and formalin solution (1.91 g, 20.73 mmol, 37 %), stirred under room temperature for 1 hour, and then added with sodium cyanoborohydride (260.60 mg, 4.15 mmol). Under room temperature, the reaction was performed for 18 hours with LC-MS monitoring for complete reaction. The reaction solution was concentrated under reduced pressure, added with saturated sodium bicarbonate aqueous solution to adjust to pH=9, and then the aqueous phase was extracted with dichloromethane for four times. The organic phase was dried with anhydrous sodium sulfate, filtrated, concentrated under reduced pressure to give Compound 61-4(400 mg, 1.32 mmol). LCMS (ESI) m/z: 304.2 $[M+H]^+$.

**Step 4: Synthesis of Compound 61-5:**

A dry single-necked flask was added with Substrate **61-4**(400 mg, 1.32 mmol), dissolved in methanol (10 mL), and then added with Pd-C (50 mg). Under hydrogen atmosphere, the reaction was performed under room temperature for 16 hours with LC-MS monitoring for complete reaction. The reaction solution was filtrated with celite, washed with methanol twice. The filtrate was concentrated under reduced pressure to give Compound **61-5**(340 mg, 1.24 mmol). LCMS (ESI) m/z: 274.4 $[M+H]^+$.

**Step 5: Synthesis of Compound 61a & 61b:**

A dry single-necked flask was added with Substrate **34-5**(33.35 mg, 84.12 μmol), dissolved in tetrahydrofuran (3 mL), and then added with m-chloroperoxybenzoic acid (30.74 mg, 151.42 μmol). Under room temperature, the reaction was performed for 1 hour. Then the reaction solution was added with N,N-diisopropyl ethyl amine (108.72 mg, 841.21 μmol) and Substrate **61-5**(46 mg, 168.24 μmol).The reaction was performed at 45°C overnight with LC-MS monitoring. After the reaction was completed, the reaction solution was cooled to room temperature, concentrated under reduced pressure. The residue was purified by preparative high-performance liquid chromatography to give the product, and then chirally resolved by supercritical fluid chromatography to give Compound **61a** (8.1 mg, 11.75 μmol); SFC residence time t = 3.216 min. $^1$**H NMR** (600 MHz, DMSO-$d_6$) δ 10.13 (s, 1H), 8.83 (s, 1H), 7.81 (d, *J* = 8.4 Hz, 1H), 7.72 (d, *J* = 8.4 Hz, 1H), 7.65 (s, 1H), 7.43 - 7.42 (m, 1H), 7.01 (d, *J* = 8.4 Hz, 1H), 5.70 - 5.64 (m, 1H), 5.29 (s, 1H), 5.00 (d, *J* = 10.2 Hz, 1H), 4.89 (d, *J* = 17.4 Hz, 1H), 4.77 - 4.57 (m, 2H), 2.90 - 2.82 (m, 2H), 2.75 (t, *J* = 5.4 Hz, 4H), 2.32 (s, 4H), 2.26 - 2.22 (m, 4H), 2.18 (s, 3H), 1.55 (t, *J*= 5.4 Hz, 4H), 1.51 (t, *J*= 5.4 Hz, 4H), 1.16 - 1.09 (m, 1H), 0.69 - 0.66 (m, 1H), 0.44 - 0.42(m, 1H), 0.39 - 0.34 (m, 2H). LCMS (ESI) m/z: 622.4 $[M+H]^+$, HPLC Method B: $R_T$= 9.98 min, purity > 90.2%.

Compound **61b** (7.9 mg, 11.60 μmol); SFC residence time t = 5.557 min. $^1$**H NMR** (600 MHz, DMSO-$d_6$) δ 10.14 (s, 1H), 8.83 (s, 1H), 7.81 (d, *J* = 8.4 Hz, 1H), 7.72 (d, *J* = 8.4 Hz, 1H), 7.65 (s, 1H), 7.43 - 7.42 (m, 1H), 7.01 (d, *J* = 8.4 Hz, 1H), 5.70 - 5.64 (m, 1H), 5.30 (s, 1H), 5.00 (d, *J* = 10.2 Hz, 1H), 4.89 (d, *J* = 17.4 Hz, 1H), 4.73 - 4.58 (m, 2H), 2.90 - 2.81 (m, 2H), 2.75 (t, *J* = 5.4 Hz, 4H), 2.34 (s, 4H), 2.27 - 2.22 (m, 4H), 2.19 (s, 3H), 1.55 (t, *J* = 5.4 Hz, 4H), 1.51 (t, *J*= 5.4 Hz, 4H), 1.16 - 1.09 (m, 1H), 0.69 - 0.66 (m, 1H), 0.44 - 0.42(m, 1H), 0.39 - 0.34 (m, 2H). LCMS (ESI) m/z: 622.4 $[M+H]^+$, HPLC Method B: $R_T$= 10.05 min, purity > 91.3%.

**Example 62: Synthesis of Compound 62a and 62b**

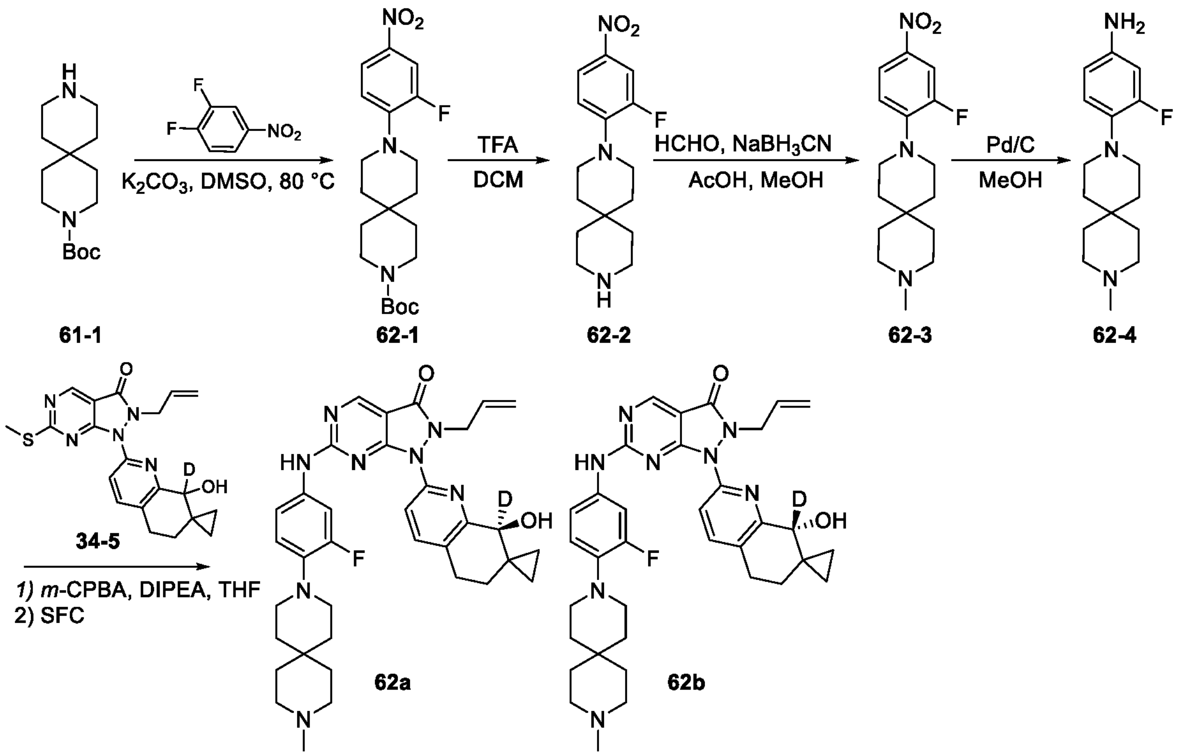


**61-1** **62-1** **62-2** **62-3** **62-4**

**34-5** **62a** **62b**

**Step 1: Synthesis of Compound 62-1:**

A dry single-necked flask was added with Substrate **61-1** (0.5 g, 1.97 mmol), dissolved in dimethyl sulfoxide (10 mL), and then added with 3,4-difluoro-nitrobenzene (312.72 mg, 1.97 mmol) and potassium carbonate (326.00 mg, 2.36 mmol). At 80°C, the reaction was performed for 16 hours with LC-MS monitoring for complete reaction. The reaction solution was cooled to room temperature, added with water, and then extracted with ethyl acetate for four times. The organic phase was dried with anhydrous sodium sulfate, filtrated, concentrated under reduced pressure. The residue was purified by column chromatography to give Compound **62-1**(730 mg, 1.86 mmol). LCMS (ESI) m/z: 394.2 $[M+H]^+$.

**Step 2: Synthesis of Compound 62-2:**

A dry single-necked flask was added with Substrate **62-1**(730 mg, 1.86 mmol), dissolved in dichloromethane (10 mL), added with trifluoroacetic acid (5 mL). Under room temperature, the reaction was performed for 2 hours with LC-MS monitoring for complete reaction. The reaction solution was concentrated under reduced pressure. The residue was dissolved in water, and added with saturated sodium bicarbonate aqueous solution to adjust to pH=9, and then the aqueous phase was extracted with dichloromethane for four times. The organic phase was dried with anhydrous sodium sulfate, filtrated, concentrated under reduced pressure to give Compound **62-2**(540 mg, 1.84 mmol). LCMS (ESI) m/z: 294.1$[M+H]^+$.

**Step 3: Synthesis of Compound 62-3:**

A dry single-necked flask was added with Substrate **62-2**(540 mg, 1.84 mmol), dissolved in methanol (15 mL), added with acetic acid (3 mL) and formalin solution (1.73 g, 18.75 mmol, 37 %), stirred under room temperature for 1 hour, and then added with sodium cyanoborohydride (235.66 mg, 3.75 mmol). Under room temperature, the reaction was performed for 18 hours with LC-MS monitoring for complete reaction. The reaction solution was concentrated under reduced pressure, added with saturated sodium bicarbonate aqueous solution to adjust to pH=9, and then the aqueous phase was extracted with dichloromethane for four times. The organic phase was dried with anhydrous sodium sulfate, filtrated, concentrated under reduced pressure to give Compound **62-3**(500 mg, 1.63 mmol). LCMS (ESI) m/z: 308.1 $[M+H]^+$.

**Step 4: Synthesis of Compound 62-4:**

A dry single-necked flask was added with Substrate **62-3**(500 mg, 1.63 mmol), dissolved in methanol (10 mL), and then added with Pd-C (60 mg). Under hydrogen atmosphere, the reaction was performed under room temperature for 16 hours with LC-MS monitoring for complete reaction. The reaction solution was filtrated with celite, washed with methanol twice. The filtrate was concentrated under reduced pressure to give Compound **62-4**(450 mg, 1.62 mmol). LCMS (ESI) m/z: 278.4 $[M+H]^+$.

**Step 5: Synthesis of Compound 62a & 62b:**

A dry single-necked flask was added with Substrate **34-5**(33.34 mg, 84.09 μmol), dissolved in tetrahydrofuran (3 mL), and then added with m-chloroperoxybenzoic acid (30.73 mg, 151.36 μmol). Under room temperature, the reaction was performed for 1 hour. Then the reaction solution was added with N,N-diisopropyl ethyl amine (108.68 mg, 840.91 μmol) and Substrate **62-4**(46.65 mg, 168.18 μmol).The reaction was performed at 45°C overnight with LC-MS monitoring. After the reaction was completed, the reaction solution was cooled to room temperature, concentrated under reduced pressure. The residue was purified by preparative high-performance liquid chromatography to give the product, and then chirally resolved by supercritical fluid chromatography to give Compound **62a** (6.0 mg, 8.32 μmol); SFC residence time t = 3.230 min. **$^1$H NMR** (600 MHz, DMSO-$d_6$) δ 10.31 (s, 1H), 8.87 (s, 1H), 7.81 (d, *J* = 8.4 Hz, 1H), 7.75 - 7.66 (m, 2H), 7.39 (d, *J* = 8.4 Hz, 1H), 7..04 - 7.01 (m, 1H), 5.72 - 5.63 (m, 1H), 5.30 (s, 1H), 5.00 (dd, *J*= 10.2, 1.8 Hz, 1H), 4.89 (dd, *J* = 17.4, 1.8 Hz, 1H), 4.74 - 4.57 (m, 2H), 2.92 (t, *J* = 5.4 Hz, 4H), 2.88 - 2.80 (m, 2H), 2.35 - 2.23 (m, 5H), 2.18 (s, 3H), 1.56 (t, *J* = 5.4 Hz, 4H), 1.49 (t, *J* = 5.4 Hz, 4H), 1.13 - 1.10 (m, 1H), 0.68 - 0.66 (m, 1H), 0.45 - 0.43 (m, 1H), 0.39 - 0.33 (m, 2H). LCMS (ESI) m/z: 626.2 $[M+H]^+$, HPLC Method B: $R_T$= 9.55 min, purity > 86.8%.

Compound **62b** (6.2 mg, 8.76 μmol); SFC residence time t = 5.103 min. **$^1$H NMR** (600 MHz, DMSO-$d_6$) δ 10.31 (s, 1H), 8.87 (s, 1H), 7.81 (d, *J* = 8.4 Hz, 1H), 7.75 - 7.66 (m, 2H), 7.39 (d, *J* = 8.4 Hz, 1H), 7.04 - 7.01(m, 1H), 5.72 - 5.63 (m, 1H), 5.30 (s, 1H), 5.00 (dd, *J* = 10.2, 1.8 Hz, 1H), 4.89 (dd, *J* = 17.4, 1.8 Hz, 1H), 4.74 - 4.58 (m, 2H), 2.92 (t, *J* = 5.4 Hz, 4H), 2.88 - 2.81 (m, 2H), 2.33 - 2.23 (m, 5H), 2.16 (s, 3H), 1.56 (t, *J* = 5.4 Hz, 4H), 1.49 (t, *J* = 5.4 Hz, 4H), 1.13 - 1.10 (m, 1H), 0.68 - 0.66 (m, 1H), 0.45 - 0.43 (m, 1H), 0.40 - 0.33 (m, 2H). LCMS (ESI) m/z: 626.2 $[M+H]^+$, HPLC Method B: $R_T$= 9.50 min, purity > 88.4%.

**Example 63: Synthesis of Compound 63a and 63b**

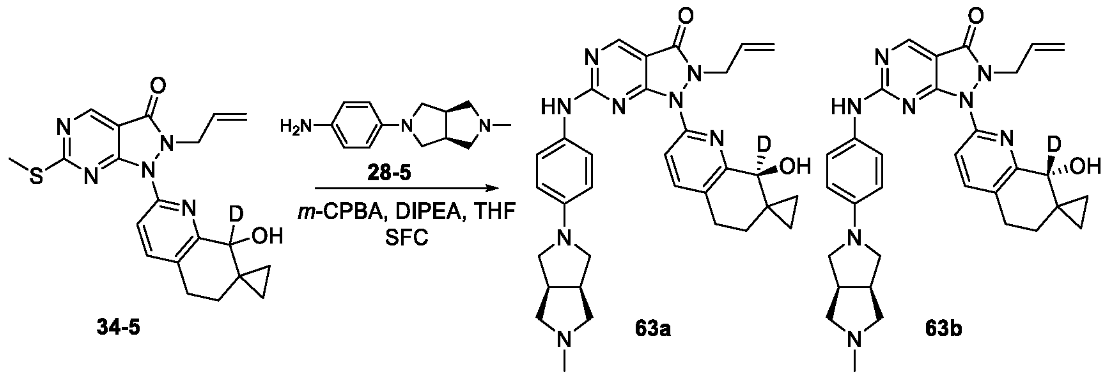


34-5 63a 63b

**Step 1: Synthesis of Compound 63a & 63b:**

A dry single-necked flask was added with Substrate **34-5**(33.33 mg, 83.99 μmol), dissolved in tetrahydrofuran (3 mL), and then added with m-chloroperoxybenzoic acid (30.69 mg, 151.18 μmol). Under room temperature, the reaction was performed for 1 hour. Then the reaction solution was added with N,N-diisopropyl ethyl amine (108.55 mg, 839.88 μmol) and Substrate **28-5**(36.50 mg, 167.98 μmol).The reaction was performed at 45°C overnight with LC-MS monitoring. After the reaction was completed, the reaction solution was cooled to room temperature, concentrated under reduced pressure. The residue was purified by preparative high-performance liquid chromatography to give the product, and then chirally resolved by supercritical fluid chromatography to give Compound **63a** (6.9 mg, 10.11 μmol); SFC residence time t = 3.357 min. **$^1$H NMR** (600 MHz, DMSO-$d_6$) δ 10.06 (s, 1H), 8.79 (s, 1H), 7.81 (d, *J* = 8.4 Hz, 1H), 7.70 (d, *J* = 8.4 Hz, 1H), 7.55 (s, 2H), 6.64 (d, *J* = 8.4 Hz, 2H), 5.72 - 5.63 (m, 1H), 5.28 (s, 1H), 5.04 - 4.96 (m, 1H), 4.89 (d, *J* = 17.4 Hz, 1H), 4.82 - 4.54 (m, 2H), 3.11 - 3.03 (m, 2H), 2.92 - 2.79 (m, 4H), 2.66 - 2.57 (m, 2H), 2.53 - 2.50 (m, 2H), 2.46 - 2.39 (m, 2H), 2.29 - 2.21 (m, 4H), 1.13 - 1.10 (m, 1H), 0.69 - 0.66 (m, 1H), 0.45 - 0.42 (m, 1H), 0.39 - 0.34 (m, 2H). LCMS (ESI) m/z: 566.4 $[M+H]^+$, HPLC Method B: $R_T$= 7.83 min, purity > 82.9%.

Compound **63b** (6.8 mg, 10.53 μmol); SFC residence time t = 5.596 min. **$^1$H NMR** (600 MHz, DMSO-$d_6$) δ 10.06 (s, 1H), 8.79 (s, 1H), 7.81 (d, *J* = 8.4 Hz, 1H), 7.70 (d, *J* = 8.4 Hz, 1H), 7.55 (s, 2H), 6.64 (d, *J* = 8.4 Hz, 2H), 5.72 - 5.63 (m, 1H), 5.29 (s, 1H), 5.00 (d, *J* = 10.2 Hz, 1H), 4.89 (d, *J* = 17.4 Hz, 1H), 4.76 - 4.58 (m, 2H), 3.07 (d, *J* = 8.4 Hz, 2H), 2.93 - 2.81 (m, 4H), 2.62 (d, *J* = 7.2 Hz, 2H), 2.52 - 2.59 (m, 2H), 2.47 - 2.38 (m, 2H), 2.29 - 2.21 (m, 4H), 1.13 - 1.10 (m, 1H), 0.69 - 0.66 (m, 1H), 0.45 - 0.42 (m, 1H), 0.39 - 0.34 (m, 2H). LCMS (ESI) m/z: 566.4 $[M+H]^+$, HPLC Method B: $R_T$= 7.78 min, purity > 87.6%.

**Example 64: Synthesis of Compound 64a & 64b**

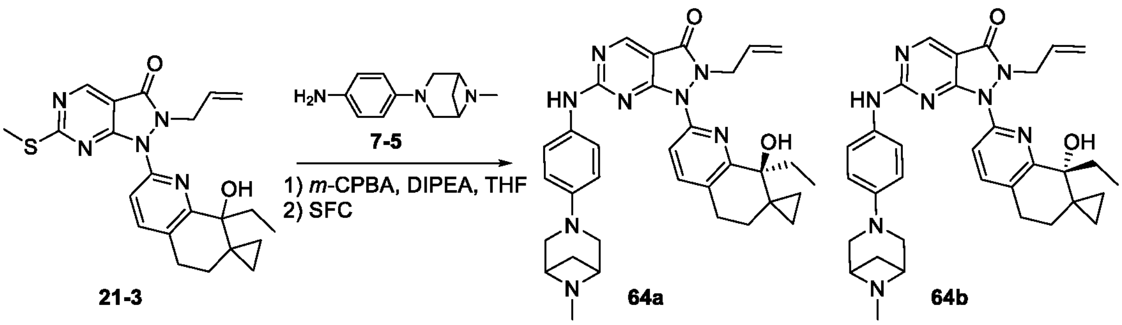


21-3 64a 64b

**Step 1: Synthesis of Compound 64a & 64b**

A dry single-necked flask was added with Substrate **21-3**(40 mg, 94.44 μmol), dissolved in tetrahydrofuran (1 mL), and then added with m-chloroperoxybenzoic acid (29.34 mg, 170.00 μmol). Under room temperature, the reaction

was performed for 1 hour. Then the reaction solution was added with N,N-diisopropyl ethyl amine (122.06 mg, 944.44 μmol) and Substrate **7-5**(38.40 mg, 188.89 μmol).The reaction was performed at 45°C overnight with LC-MS monitoring. After the reaction was completed, the reaction solution was cooled to room temperature, concentrated under reduced pressure. The residue was purified by preparative high-performance liquid chromatography to give the product, and then chirally resolved by supercritical fluid chromatography to give Compound **64a**(10.1 mg, 17.45 μmol); SFC residence time t = 2.944 min. **$^1$H NMR** (600 MHz, DMSO-$d_6$) δ 10.06 (s, 1H), 8.79 (s, 1H), 7.77 (d, $J$ = 8.4 Hz, 1H), 7.69 (d, $J$ = 8.4 Hz, 1H), 7.60 (s, 2H), 6.71 (d, $J$ = 8.4 Hz, 2H), 5.72 - 5.63 (m, 1H), 5.00 (d, $J$ = 10.2 Hz, 1H), 4.85 (d, $J$ = 17.4 Hz, 1H), 4.75 (s, 1H), 4.61 (d, $J$ = 16.2 Hz, 1H), 4.40 (s, 1H), 3.84 (s, 2H), 3.64 - 3.55 (m, 2H), 3.44 (d, $J$ = 10.8 Hz, 2H), 3.27 (d, $J$ = 10.8 Hz, 2H), 2.95 - 2.89 (m, 1H), 2.86 - 2.80 (m, 1H), 2.42 (d, $J$ = 17.4 Hz, 1H), 2.35 - 2.29 (m, 1H), 2.00 (s, 3H), 1.92 (dd, $J$ = 14.4, 7.2 Hz, 1H), 1.75 (dd, $J$ = 14.4, 7.2 Hz, 1H), 1.54 (d, $J$ = 8.4 Hz, 1H), 1.15 - 1.11 (m, 1H), 0.93 (t, $J$ = 7.2 Hz, 3H), 0.87 - 0.85 (m, 1H), 0.64 - 0.57 (m, 1H), 0.42 - 0.36 (m, 1H), 0.00 (d, $J$ = 3.5 Hz, 1H). LCMS (ESI) m/z: 579.4 $[M+H]^+$, HPLC Method B: $R_T$= 9.34 min, purity> 86.7%.

Compound **64b**(8.7 mg, 15.03 μmol); SFC residence time t = 4.409 min. **$^1$H NMR** (600 MHz, DMSO-$d_6$) δ 10.06 (s, 1H), 8.79 (s, 1H), 7.77 (s, 1H), 7.69 (d, $J$ = 8.4 Hz, 1H), 7.60 (s, 2H), 6.71 (d, $J$ = 8.4 Hz, 2H), 5.72 - 5.63 (m, 1H), 5.04 - 4.97 (m, 1H), 4.85 (d, $J$ = 17.4 Hz, 1H), 4.74 (s, 1H), 4.66 - 4.54 (m, 1H), 4.40 (s, 1H), 3.58 (d, $J$ = 6.0 Hz, 2H), 3.43 (d, $J$ = 10.8 Hz, 2H), 3.27 (d, $J$ = 10.8 Hz, 2H), 2.95 - 2.89 (m, 1H), 2.87 - 2.81 (m, 1H), 2.46 - 2.40 (m, 1H), 2.35 - 2.29 (m, 1H), 1.99 (s, 3H), 1.96 - 1.90 (m, 1H), 1.75 (dd, $J$ = 14.4, 7.2 Hz, 1H), 1.53 (d, $J$ = 8.4 Hz, 1H), 1.15 - 1.11 (m, 1H), 0.93 (t, $J$ = 7.2 Hz, 3H), 0.87 - 0.84 (m, 1H), 0.64 - 0.57 (m, 1H), 0.42 - 0.36 (m, 1H), 0.00 (s, 1H). LCMS (ESI) m/z: 579.4 $[M+H]^+$, HPLC Method B: $R_T$ =9.25 min, purity> 91.1%.

**Example 65: Synthesis of Compound 65**

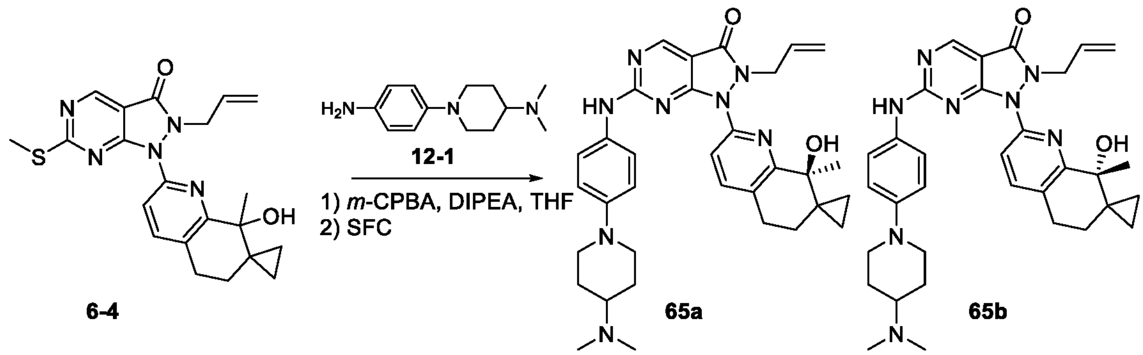


6-4 65a 65b

**Step 1: Synthesis of Compound 65a & 65b:**

A dry single-necked flask was added with Substrate **6-4**(40 mg, 97.68 μmol), dissolved in tetrahydrofuran (1 mL), and then added with m-chloroperoxybenzoic acid (30.34 mg, 175.82 μmol). Under room temperature, the reaction was performed for 1 hour. Then the reaction solution was added with N,N-diisopropyl ethyl amine (126.24 mg, 976.79 μmol) and Substrate **12-1**(42.85 mg, 195.36 μmol).At 50°C, the reaction was performed overnight with LC-MS monitoring. After the reaction was completed, the reaction solution was cooled to room temperature, concentrated under reduced pressure. The residue was purified by preparative high-performance liquid chromatography to give Compound **65a** (14.3 mg, 24.62 μmol); SFC residence time t = 3.425 min. **$^1$H NMR** (600 MHz, DMSO-$d_6$) δ 10.11 (s, 1H), 8.81 (s, 1H), 7.78 (s, 1H), 7.69 (d, $J$ = 8.4 Hz, 1H), 7.56 (s, 2H), 6.93 (d, $J$ = 8.4 Hz, 2H), 5.70 - 5.62 (m, 1H), 4.99 (dd, $J$ = 10.2, 1.8 Hz, 1H), 4.89 - 4.84 (m, 1H), 4.83 - 4.73 (m, 1H), 4.69 (s, 1H), 4.64 (dd, $J$ = 16.2, 6.0 Hz, 1H), 3.66 (d, $J$ = 12.0 Hz, 2H), 2.93 - 2.86 (m, 1H), 2.83 - 2.77 (m, 1H), 2.67 - 2.59 (m, 2H), 2.19 (s, 6H), 2.18 (d, $J$ = 3.6 Hz, 1H), 2.03 - 1.96 (m, 1H), 1.83 (d, $J$ = 12.0 Hz, 2H), 1.52 - 1.45 (m, 2H), 1.45 - 1.42 (m, 1H), 1.40 (s, 3H), 0.91 - 0.84 (m, 1H), 0.68 - 0.60 (m, 1H), 0.35 - 0.30 (m, 1H), 0.23 - 0.18 (m, 1H). LCMS (ESI) m/z: 581.4 $[M+H]^+$, HPLC Method B: $R_T$= 8.79 min, purity> 93.5%.

Compound **65b** (15.6 mg, 26.86 μmol); SFC residence time t = 5.024 min. **$^1$H NMR** (600 MHz, DMSO-$d_6$) δ 10.11 (s, 1H), 8.81 (s, 1H), 7.78 (d, $J$ = 7.8 Hz, 1H), 7.69 (d, $J$ = 8.4 Hz, 1H), 7.64 - 7.41 (m, 2H), 6.93 (d, $J$ = 8.4 Hz, 2H), 5.70 - 5.62 (m, 1H), 4.99 (d, $J$ = 10.2 Hz, 1H), 4.86 (d, $J$ = 17.4 Hz, 1H), 4.81 (d, $J$ = 15.6 Hz, 1H), 4.69 (s, 1H), 4.64 (dd, $J$ = 16.2, 6.0 Hz, 1H), 3.66 (d, $J$ = 12.0 Hz, 2H), 2.93 - 2.86 (m, 1H), 2.83 - 2.77 (m, 1H), 2.67 - 2.59 (m, 2H), 2.20 (s, 6H), 2.19 - 2.13 (m, 1H), 1.96 (m, 1H), 1.86 - 1.81 (m, 2H), 1.48 (dd, $J$ = 11.8, 3.7 Hz, 2H), 1.45 - 1.42 (m, 1H), 1.41 (s, 3H), 0.91 - 0.84 (m, 1H), 0.67 - 0.60 (m, 1H), 0.35 - 0.30 (m, 1H), 0.23 - 0.18 (m, 1H). LCMS (ESI) m/z: 581.4 $[M+H]^+$, HPLC Method B: $R_T$= 8.86min, purity> 93.6%.

**Example 66: Synthesis of Compound 66**

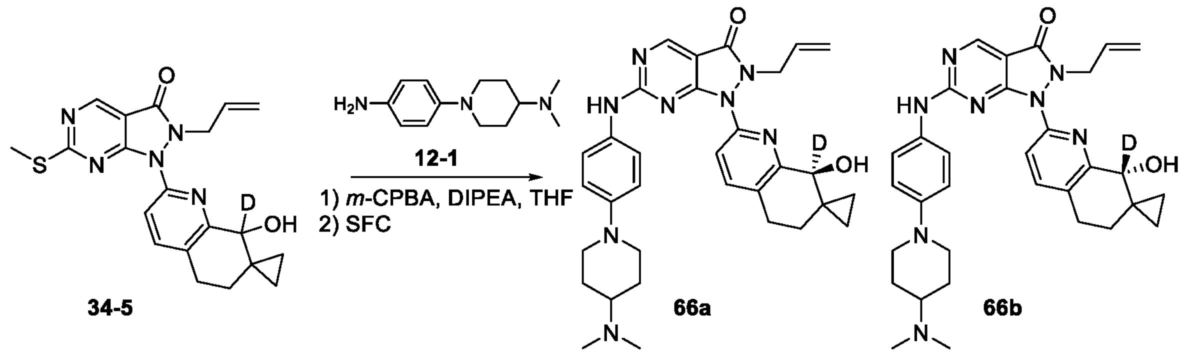


34-5 66a 66b

**Step 1: Synthesis of Compound 66a & 66b:**

A dry single-necked flask was added with Substrate **34-5**(40 mg, 100.89 μmol), dissolved in tetrahydrofuran (1 mL), and then added with m-chloroperoxybenzoic acid (30.72 mg, 151.33 μmol). Under room temperature, the reaction was performed for 1 hour. Then the reaction solution was added with N,N-diisopropyl ethyl amine (65.19 mg, 504.43 μmol) and Substrate **12-1**(33.19 mg, 151.33 μmol). The reaction was performed at 45°C overnight with LC-MS monitoring. After the reaction was completed, the reaction solution was cooled to room temperature, concentrated under reduced pressure. The residue was purified by preparative high-performance liquid chromatography to give the product, and then chirally resolved by supercritical fluid chromatography to give Compound **66a** (12.12 mg, 21.35 μmol); SFC residence time t = 3.579 min. **$^1$H NMR** (600 MHz, DMSO-$d_6$) δ 10.12 (s, 1H), 8.81 (d, *J* = 3.6 Hz, 1H), 7.83 (d, *J* = 8.4 Hz, 1H), 7.70 (d, *J* = 8.4 Hz, 1H), 7.58 (s, 2H), 6.98 - 6.90 (m, 2H), 5.73 - 5.63 (m, 1H), 5.30 (s, 1H), 5.00 (d, *J* = 10.2 Hz, 1H), 4.89 (d, *J* = 17.4 Hz, 1H), 4.69 (s, 1H), 4.65 - 4.56 (m, 1H), 3.66 (d, *J* = 12.0 Hz, 2H), 3.04 (s, 1H), 2.87 - 2.78 (m, 2H), 2.65 - 2.59 (m, 2H), 2.29 - 2.20 (m, 1H), 2.19 (s, 6H), 1.88 - 1.80 (m, 2H), 1.53 - 1.44 (m, 2H), 1.13 - 1.09 (m, 1H), 0.71 - 0.62 (m, 1H), 0.45 - 0.42 (m, 1H), 0.39 - 0.32 (m, 2H). LCMS (ESI) m/z: 568.2 $[M+H]^+$, HPLC Method B: $R_T$= 7.48min, purity > 86.1%.

Compound **66b** (12.56 mg, 22.12 μmol); SFC residence time t = 5.858 min. **$^1$H NMR** (600 MHz, DMSO-$d_6$) δ 10.12 (s, 1H), 8.81 (s, 1H), 7.83 (d, *J* = 8.4 Hz, 1H), 7.70 (d, *J* = 8.4 Hz, 1H), 7.58 (s, 2H), 6.92 (d, *J* = 8.4 Hz, 2H), 5.73 - 5.63 (m, 1H), 5.29 (s, 1H), 5.00 (d, *J* = 10.2 Hz, 1H), 4.89 (d, *J* = 17.4 Hz, 1H), 4.69 (s, 1H), 4.65 - 4.54 (m, 1H), 3.66 (d, *J* = 12.0 Hz, 2H), 3.07 (s, 1H), 2.87 - 2.79 (m, 2H), 2.65 - 2.59 (m, 2H), 2.29 - 2.20 (m, 1H), 2.19 (s, 6H), 1.82 (s, 2H), 1.53 - 1.44 (m, 2H), 1.13 - 1.10 (m, 1H), 0.71 - 0.62 (m, 1H), 0.45 - 0.42 (m, 1H), 0.39 - 0.32 (m, 2H). LCMS (ESI) m/z: 568.2 $[M+H]^+$, HPLC Method B: $R_T$= 7.50 min, purity > 89.5%.

**Example 67: Synthesis of Compound 67**

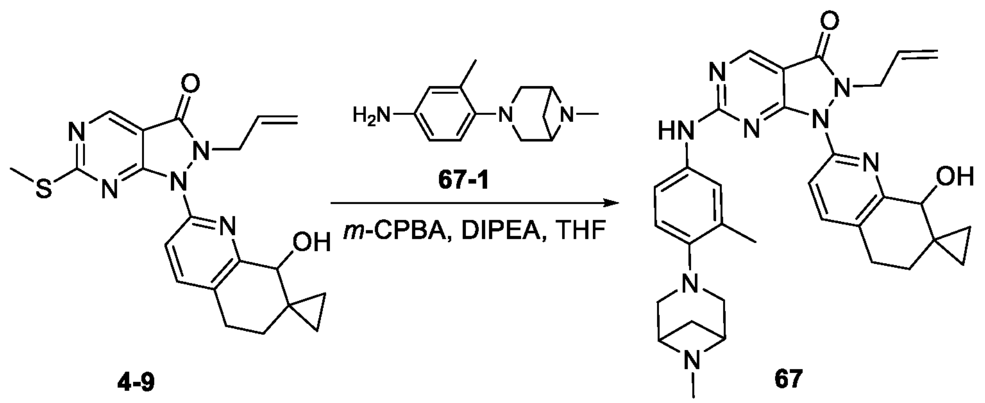


4-9 67

**Step 1: Synthesis of Compound 67:**

A dry single-necked flask was added with Substrate **4-9**(20 mg, 50.57μmol), dissolved in tetrahydrofuran (2 mL), and then added with m-chloroperoxybenzoic acid (15.4 mg, 75.86 μmol). Under room temperature, the reaction was performed for 1 hour. Then the reaction solution was added with N,N-diisopropyl ethyl amine (65.15 mg, 0.51 mmol) and Substrate **67-1**(16.48mg, 75.86 μmol).The reaction was performed at 45°C overnight with LC-MS monitoring. After the reaction was completed, the reaction solution was cooled to room temperature, concentrated under reduced pressure. The residue was purified by preparative high-performance liquid chromatography to give Compound **67** (6.8 mg, 12.02 μmol).**$^1$H NMR** (600 MHz, DMSO-$d_6$) δ 10.18 (s, 1H), 8.85 (s, 1H), 7.80 (d, *J* = 8.4 Hz, 1H), 7.77 - 7.62 (m, 2H), 7.46 (d, *J* = 8.4 Hz, 1H), 7.34 (d, *J* = 9.0 Hz, 1H), 5.71 - 5.65 (m, 1H), 5.33 (d, *J* = 5.4 Hz, 1H), 5.01 (d, *J* = 10.2 Hz, 1H), 4.89 (d, *J* = 17.4 Hz, 1H), 4.71 - 4.62 (m, 2H), 3.75 (d, *J* = 4.8 Hz, 1H), 3.65 - 3.65 (m, 2H), 3.23 - 3.02 (m, 4H), 2.86 - 2.80 (m, 2H), 2.52 (s, 3H), 2.33 - 2.21 (m, 6H), 1.14 - 1.10 (m, 1H), 0.70 - 0.66 (m, 1H), 0.45 - 0.42 (m, 1H), 0.39 - 0.34 (m, 2H). LCMS (ESI) m/z: 565.2 $[M+H]^+$, HPLC Method B: $R_T$= 8.22 min, purity > 87.8%.

**Example 68: Synthesis of Compound 68**

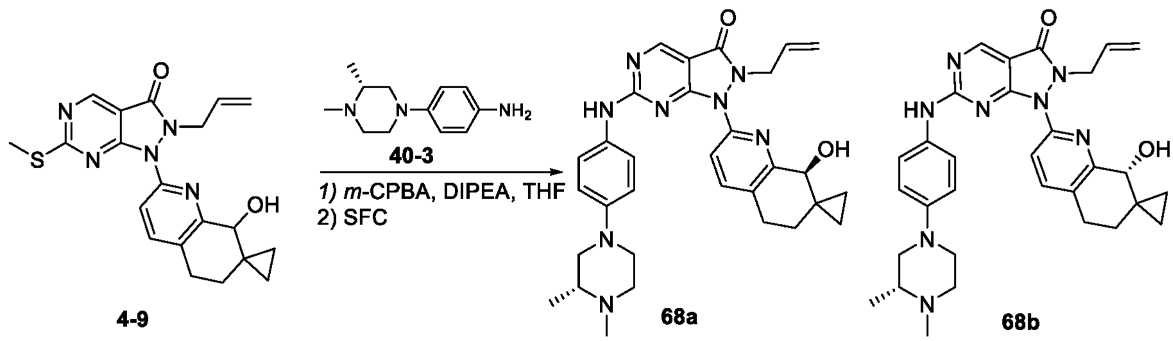


4-9 68a 68b

**Step 1: Synthesis of Compound 68a & 68b:**

A dry single-necked flask was added with Substrate **4-9**(40 mg, 101.14 μmol), dissolved in tetrahydrofuran (1 mL), and then added with m-chloroperoxybenzoic acid (30.80 mg, 151.72 μmol). Under room temperature, the reaction was performed for 1 hour. Then the reaction solution was added with N,N-diisopropyl ethyl amine (65.36 mg, 505.72 μmol) and Substrate **40-3**(31.15 mg, 151.72 μmol).The reaction was performed at 45°C overnight with LC-MS monitoring. After the reaction was completed, the reaction solution was cooled to room temperature, concentrated under reduced pressure. The residue was purified by preparative high-performance liquid chromatography to give the product, and then chirally resolved by supercritical fluid chromatography to give Compound **68a** (10.48 mg, 518.96 μmol); SFC residence time t = 3.121 min. **$^1$H NMR** (600 MHz, DMSO-$d_6$) δ 10.12 (s, 1H), 8.81 (s, 1H), 7.83 (d, *J*=8.4 Hz, 1H), 7.69 (d, *J*=8.4 Hz, 1H), 7.64 - 7.46 (m, 2H), 6.92 (d, *J* = 8.4 Hz, 2H), 5.69 - 5.64 (m, 6.0 Hz, 1H), 5.32 (d, *J* = 5.4 Hz, 1H), 5.03 - 4.98 (m, 1H), 4.91 - 4.85 (m, 1H), 4.69 (s, 1H), 4.63 - 4.60 (m, 5.4 Hz, 1H), 3.75 (d, *J* = 5.4 Hz, 1H), 3.47 (d, *J*= 13.2 Hz, 2H), 2.88 - 2.80 (m, 3H), 2.71 - 2.70 (m, 1H), 2.35 - 2.33 (m, 1H), 2.27 - 2.23 (m, 2H), 2.22 (s, 3H), 2.17 - 2.11 (m, 1H), 1.13 - 1.11 (m, 1H), 1.06 (s, 3H), 0.70 - 0.66 (m, 1H), 0.46 - 0.42 (m, 1H), 0.39 - 0.35 (m, 2H). LCMS (ESI) m/z: 553.3 $[M+H]^+$, HPLC Method B: $R_T$= 7.23min, purity > 96.1%.

Compound **68b** (12.34 mg, 22.33 μmol); SFC residence time t = 5.185 min. **$^1$H NMR** (600 MHz, DMSO-$d_6$) δ 10.12 (s, 1H), 8.81 (s, 1H), 7.84 (d, *J* = 8.4 Hz, 1H), 7.75 - 7.67 (m, 1H), 7.59 (s, 2H), 6.92 (d, *J*= 8.4 Hz, 2H), 5.69 - 5.66 (m, 1H), 5.31 (d, *J* = 5.4 Hz, 1H), 5.05 - 4.96 (m, 1H), 4.94 - 4.87 (m, 1H), 4.69 (s, 1H), 4.62 - 4.60 (m, 1H), 3.75 (d, *J* = 5.4 Hz, 1H), 3.48 - 3.45 (m, 2H), 2.86 - 2.81 (m, 3H), 2.74 - 2.69 (m, 1H), 2.36 - 2.34 (m, 1H), 2.29 - 2.23 (m, 2H), 2.22 (s, 3H), 2.15 (s, 1H), 1.14 - 1.10 (m, 1H), 1.06 (s, 3H), 0.69 - 0.67 (m, 1H), 0.44 - 0.42 (m, 1H), 0.39 - 0.36 (m, 2H). LCMS (ESI) m/z: 553.3 $[M+H]^+$, HPLC Method B: $R_T$= 7.24 min, purity > 94.1%.

**Example 69: Synthesis of Compound 69**

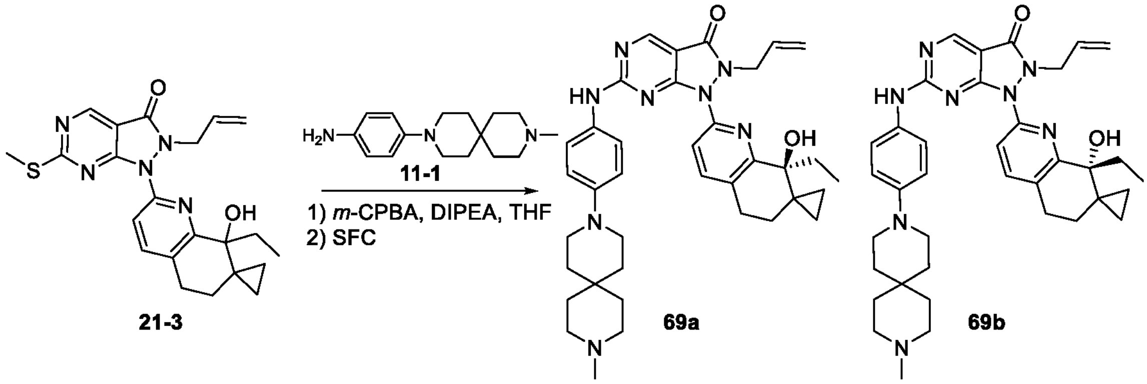


21-3 69a 69b

**Step 1: Synthesis of Compound 69a & 69b:**

A dry single-necked flask was added with Substrate **21-3**(40 mg, 94.44 μmol), dissolved in tetrahydrofuran (1 mL), and then added with m-chloroperoxybenzoic acid (28.76 mg, 141.67 μmol). Under room temperature, the reaction was performed for 1 hour. Then the reaction solution was added with N,N-diisopropyl ethyl amine (61.03 mg, 472.22 μmol) and Substrate B(36.75 mg, 141.67 μmol).The reaction was performed at 45°C overnight with LC-MS monitoring. After the reaction was completed, the reaction solution was cooled to room temperature, concentrated under reduced pressure. The residue was purified by preparative high-performance liquid chromatography to give the product, and then chirally resolved by supercritical fluid chromatography to give Compound **69a** (1.1 mg, 1.73 μmol); SFC residence time t = 3.897 min. **$^1$H NMR** (600 MHz, DMSO-$d_6$) δ 10.11 (s, 1H), 8.81 (d, *J* = 4.2 Hz, 1H), 7.79 (s, 1H), 7.67 (d, *J* = 8.4 Hz, 1H), 7.57 (s, 2H), 6.92 (d, *J* = 9.0 Hz, 2H), 5.74 - 5.62 (m, 1H), 4.99 (dd, *J* = 10.2, 1.8 Hz, 1H), 4.85 (d, *J* = 17.4 Hz, 1H), 4.74 (s, 1H), 4.62 (s, 1H), 4.40 (d, *J* = 4.2 Hz, 1H), 3.09 (s, 4H), 2.98 - 2.89 (m, 1H), 2.84 (dd, *J* = 17.4, 6.0 Hz, 1H), 2.36 - 2.29 (m, 4H), 2.18 (s, 3H), 1.94 - 1.89 (m, 1H), 1.75 (dd, *J* = 14.4, 7.2 Hz, 1H), 1.54 (t, *J* = 5.4 Hz, 4H), 1.47 (t, *J* = 5.4 Hz, 4H), 0.93 (t, *J* = 7.2 Hz, 3H), 0.87 - 0.84 (m, 2H), 0.78 - 0.73 (m, 2H), 0.64 - 0.58 (m, 1H), 0.43 - 0.37 (m, 1H). LCMS (ESI) m/z: 635.4 $[M+H]^+$, HPLC Method B: $R_T$= 7.07 min, purity > 83.2%.

**Compound 69b** (1.0 mg, 1.58 μmol); SFC residence time t = 5.463 min. **$^1$H NMR** (600 MHz, DMSO-$d_6$) δ 10.11 (s, 1H), 8.81 (d, *J = 4.3* Hz, 1H), 7.79 (s, 1H), 7.67 (d, *J* = 8.2 Hz, 1H), 7.57 (s, 2H), 6.92 (d, *J* = 9.0 Hz, 2H), 5.74 - 5.62 (m, 1H), 4.99 (dd, *J* = 10.2, 1.8 Hz, 1H), 4.85 (d, *J* = 17.4 Hz, 1H), 4.74 (s, 1H), 4.61 (s, 1H), 4.40 (d, *J* = 4.8 Hz, 1H), 3.13 - 3.06 (m, 4H), 2.98 - 2.89 (m, 1H), 2.84 (dd, *J* = 16.8, 6.0 Hz, 1H), 2.38 - 2.29 (m, 4H), 2.20 (s, 3H), 1.93 - 1.89 (m, 1H), 1.75 (dd, *J* = 14.4, 7.2 Hz, 1H), 1.54 (t, *J = 5.4* Hz, 4H), 1.48 (t, *J*= 5.4 Hz, 4H), 0.93 (d, *J*= 7.2 Hz, 3H), 0.87 - 0.84 (m, 2H), 0.78 - 0.73 (m, 2H), 0.64 - 0.58 (m, 1H), 0.43 - 0.37 (m, 1H). LCMS (ESI) m/z: 635.4 $[M+H]^+$, HPLC Method B: $R_T$ = 7.08 min, purity > 80.1%.

**Example 70: Synthesis of Compound 70**

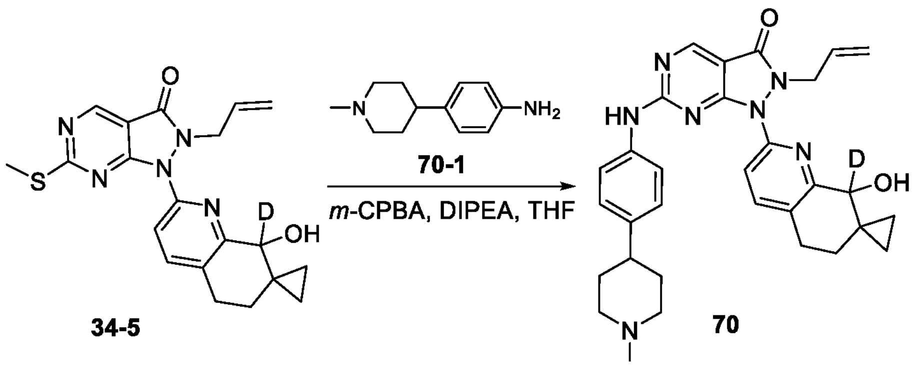


34-5 70

**Step 1: Synthesis of Compound 70:**

A dry single-necked flask was added with Substrate **34-5**(68.41 mg, 172.55 μmol), dissolved in tetrahydrofuran (3 mL), and then added with m-chloroperoxybenzoic acid (59.55 mg, 345.10 μmol). Under room temperature, the reaction was performed for 1 hour. Then the reaction solution was added with N,N-diisopropyl ethyl amine (111.50 mg, 862.75 μmol) and Substrate **70-1**(65.67 mg, 345.10 μmol).The reaction was performed at 45°C overnight with LC-MS monitoring.

After the reaction was completed, the reaction solution was cooled to room temperature, concentrated under reduced pressure. The residue was purified by preparative high-performance liquid chromatography (basic) to give Compound **70** (43.75mg, 90.97 μmol).**$^1$H NMR** (400 MHz, DMSO-$d_6$) δ 10.23 (s, 1H), 8.87 (s, 1H), 7.82 (d, $J$= 8.4 Hz, 1H), 7.75 - 7.63 (m, 3H), 7.27 - 7.12 (m, 2H), 5.74 - 5.61 (m, 1H), 5.30 (s, 1H), 5.02 - 4.99 (m, 1H), 4.91 - 4.87 (m, 1H), 4.64 - 4.51 (m, 2H), 3.22 (s, 3H), 2.93 - 2.78 (m, 2H), 2.68 - 2.51 (m, 5H), 2.31 - 2.20 (m, 1H), 1.88 (d, $J$ = 13.2 Hz, 2H), 1.71 - 1.69 (m, 2H), 1.13 - 1.10 (m, 1H), 0.70 - 0.67 (m, 1H), 0.47 - 0.40 (m, 1H), 0.39 - 0.31 (m, 2H). LCMS (ESI) m/z: 539.2 [M+H]$^+$, HPLC Method B: $R_T$= 6.88 min, purity > 97.1 %.

**Example 71: Synthesis of Compound 71**

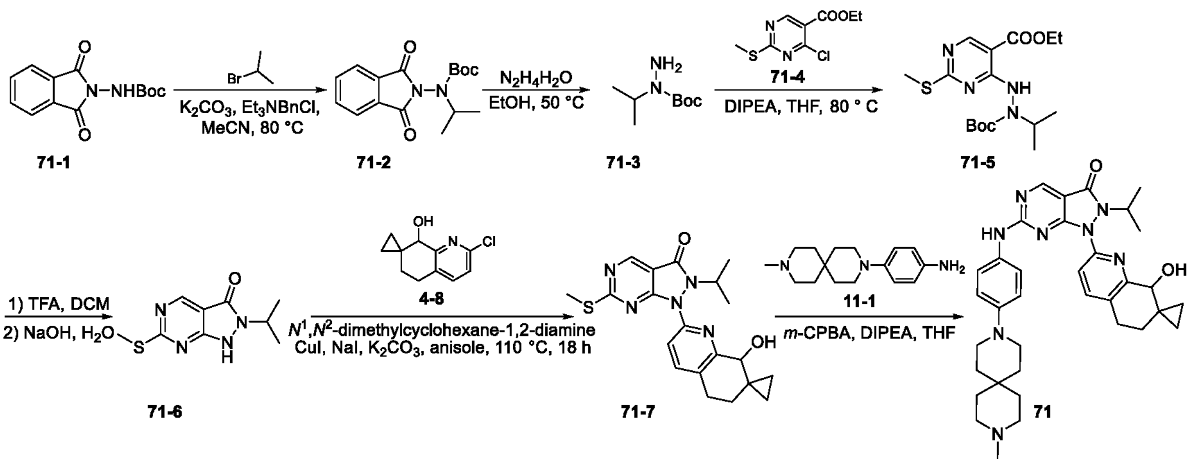


**Step 1: Synthesis of Compound 71-2:**

A single necked flask was added with Substrate **71-1** (2.5 g, 9.54 mmol), potassium carbonate (5.27 g, 38.16 mmol) and benzyltriethylammonium chloride (217.34 mg, 0.95 mmol), and then added with acetonitrile(30 mL) to dissolve, after the solution turned yellow, added with 3-bromopropane (1.58 g, 14.31 mmol). At 80°C, the reaction was stirred for 48 hours with LC-MS monitoring. After the reaction was completed, the reaction solution was cooled to room temperature, filtrated, and then washed with ethyl acetate. The filtrate was added with ethyl acetate and water, and then extracted with ethyl acetate for three times. The organic phase was dried with anhydrous sodium sulfate, filtrated, concentrated under reduced pressure to give crude product. The crude product was further recrystallized in petroleum ether and dichloromethane, filtrated, and dried in vaccuo to give Compound 71-2 (2.00 g, 6.57 mmol), LCMS (ESI) m/z: 205.0 [M+H-100]$^+$.

**Step 2: Synthesis of Compound 71-3:**

A dry single-necked flask was added with Substrate **71-2** (2.00 g, 6.57 mmol), dissolved in ethanol (40 mL), and then added with hydrazine hydrate (10 mL). At 50°C, the reaction was heated for 2 hours with LC-MS monitoring. After the reaction was completed, the reaction solution was cooled to room temperature, filtrated, washed with ethanol for three times. The filtrate was concentrated under reduced pressure. The residue was added with water and ethyl acetate to dissolve, and extracted with ethyl acetate for four times. The organic phase was dried with anhydrous sodium sulfate, filtrated, concentrated under reduced pressure to give Compound **71-3** (1.1 g, 6.31 mmol), LCMS (ESI) m/z: 160.2 [M+H-56+41]$^+$.

**Step 3: Synthesis of Compound 71-5:**

A dry single-necked flask was added with Substrate **71-3** (1.1 g, 6.31 mmol) and Substrate ethyl 4-chloro-2-methylthiopyrimidine-5-carboxylate **(71-4,** 1.40 g, 6.01 mmol), dissolved in tetrahydrofuran (20 mL), and then added with N,N-diisopropyl ethyl amine (1.94 g, 15.03 mmol). At 80°C, the reaction was heated for 16 hours with LC-MS monitoring. After the reaction was completed, the reaction solution was cooled to room temperature, concentrated under reduced pressure to give crude product. Then the crude product was dissolved in ethyl acetate, washed with 1M diluted hydrochloric acid for five times. The organic phase was dried with anhydrous sodium sulfate, filtrated, concentrated under reduced pressure to give Compound **71-5** (2.15 g, 5.81 mmol), LCMS (ESI) m/z: 371.2 [M+H]$^+$.

**Step 4: Synthesis of Compound 71-6:**

A dry single-necked flask was added with Substrate **71-5** (2.15 g, 5.81 mmol), dissolved in dichloromethane (30 mL), and then under ice bath, added with trifluoroacetic acid (10 mL). At 45°C, the reaction was performed for 2 hours with LC-MS monitoring for complete reaction. The reaction solution was cooled with ice bath to 0°C, slowly added with 40 % sodium hydroxide aqueous solution until pH = 11, and then added with methanol (30 mL). The reaction was performed under room temperature for 6 hours under LC-MS monitoring for complete reaction, concentrated under reduced pressure to remove the organic phase, and then added with 3M diluted hydrochloric acid until pH = 1, to give yellow precipitate. The solid was filtrated, and washed with water twice, and the solid was dried in vaccuo to give Compound **71-6**(785.00 mg, 3.50 mmol). LCMS (ESI) m/z: 225.2 $[M+H]^+$.

**Step 5: Synthesis of Compound 71-7:**

A dry sealed tube was added with Substrate **4-8**(183.6 mg, 875.66 μmol), Substrate **71-6**(206.21 mg, 919.44 μmol), copper (I) iodide (166.77 mg, 875.66 μmol), sodium iodide (262.50 mg, 1.75 mmol), potassium carbonate (302.56 mg, 2.19 mmol) and trans-(1R,2R)-*N*, *N*-dimethyl-1,2-cyclohexylene diamine (249.11 mg, 1.75 mmol), and then added with anisole (4 mL). Under nitrogen atmosphere. At 110°C, the reaction was performed for 18 hours with TLC monitoring for complete reaction. The reaction solution was cooled to room temperature, filtrated, washed with ethyl acetate twice. The filrate was washed with ammonia solution twice, washed with saturated brine solution twice. The organic phase was dried with anhydrous sodium sulfate, filtrated, concentrated under reduced pressure. The residue was purified by column chromatography to give Compound B(132 mg, 332.08 μmol).LCMS ($ESI^+$) m/z: 398.1 $[M+H]^+$.

**Step 6: Synthesis of Compound 71:**

A dry single-necked flask was added with Substrate **71-7**(132 mg, 332.08 μmol), dissolved in tetrahydrofuran (5 mL), and then added with m-chloroperoxybenzoic acid (85.96 mg, 498.12 μmol). Under room temperature, the reaction was performed for 1 hour. Then the reaction solution was added with N,N-diisopropyl ethyl amine (214.59 mg, 1.66 mmol) and Substrate **11-1**(172.28 mg, 664.16 μmol).The reaction was performed at 45°C overnight with LC-MS monitoring. After the reaction was completed, the reaction solution was cooled to room temperature, concentrated under reduced pressure. The residue was purified by preparative high-performance liquid chromatography (basic) to give Compound **71**(75.15 mg, 123.45 μmol).**$^1$H NMR** (400 MHz, DMSO-$d_6$) δ 10.05 (s, 1H), 8.74 (s, 1H), 7.83 (d, *J* = 8.4 Hz, 1H), 7.63 - 7.45 (m, 3H), 6.88 (d, *J* = 9.2 Hz, 2H), 5.24 (d, *J* = 5.2 Hz, 1H), 4.23 - 4.11 (m, 1H), 3.72 (d, *J* = 5.2 Hz, 1H), 3.07 (t, *J* = 5.6 Hz, 4H), 2.92 - 2.75 (m, 2H), 2.35 - 2.20 (m, 5H), 2.16 (s, 3H), 1.53 (t, *J* = 5.6 Hz, 4H), 1.46 (t, *J* = 5.6 Hz, 4H), 1.31 (d, *J* = 7.2 Hz, 6H), 1.14 - 1.06 (m, 1H), 0.72 - 0.62 (m, 1H), 0.46 - 0.41 (m, 1H), 0.40 - 0.34 (m, 1H), 0.33 - 0.27 (m, 1H). LCMS (ESI) m/z: 609.3 $[M+H]^+$, HPLC Method B: $R_T$= 7.55 min, purity > 95.5 %.

**Example 72: Synthesis of Compound 72**

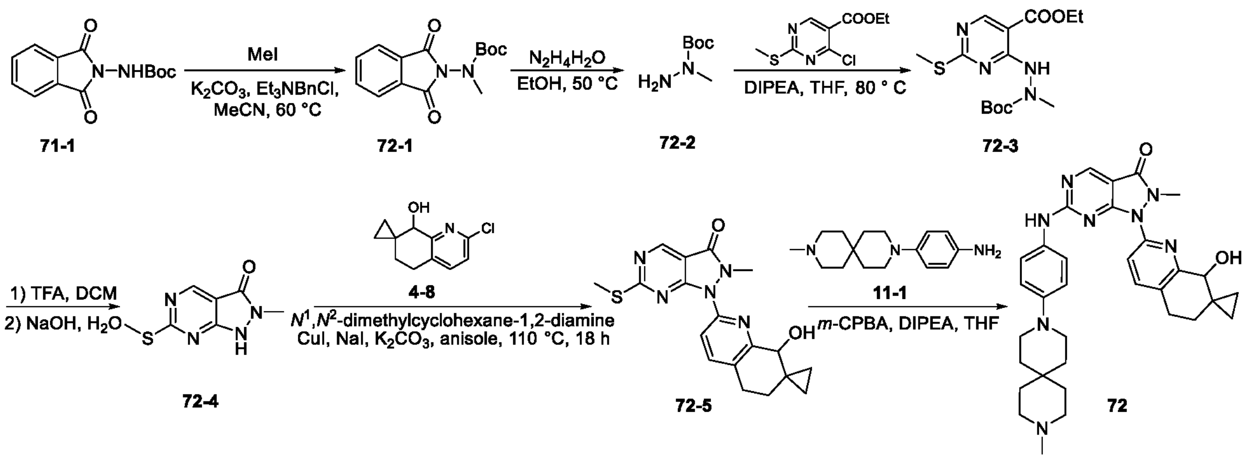


71-1 72-1 72-2 72-3

72-4 72-5 72

**Step 1: Synthesis of Compound 72-1:**

A single necked flask was added with Substrate **71-1**(2.5 g, 9.54 mmol), potassium carbonate (5.27 g, 38.16 mmol) and benzyltriethylammonium chloride (217.34 mg, 0.95 mmol), and then added with acetonitrile(30 mL) to dissolve, after the solution turned yellow, added with iodomethane (2.71 g, 19.08 mmol). At 60°C, the reaction was stirred for 24 hours with LC-MS monitoring. After the reaction was completed, the reaction solution was cooled to room temperature, filtrated, and then washed with ethyl acetate. The filtrate was added with ethyl acetate and water, and then extracted with ethyl acetate for three times. The organic phase was dried with anhydrous sodium sulfate, filtrated, concentrated under reduced pressure to give crude product. The crude product was further recrystallized in petroleum ether and dichloromethane, filtrated, and dried in vaccuo to give Compound **72-1** (1.80 g, 6.51 mmol), LCMS (ESI) m/z: 177.0 $[M+H-100]^+$.

**Step 2: Synthesis of Compound 72-2:**

A dry single-necked flask was added with Substrate **72-1** (1.80 g, 6.51 mmol), dissolved in ethanol (30 mL), and then added with hydrazine hydrate(8 mL). At 50°C, the reaction was heated for 2 hours with LC-MS monitoring. After the reaction was completed, the reaction solution was cooled to room temperature, filtrated, washed with ethanol for three times. The filtrate was concentrated under reduced pressure. The residue was added with water and ethyl acetate to dissolve, and extracted with ethyl acetate for four times. The organic phase was dried with anhydrous sodium sulfate, filtrated, concentrated under reduced pressure to give Compound **72-2** (0.91 g, 6.20 mmol), LCMS (ESI) m/z: 132.2 $[M+H-56+41]^+$.

**Step 3: Synthesis of Compound 72-3:**

A dry single-necked flask was added with Substrate **72-2** (0.91 g, 6.20 mmol) and Substrate ethyl 4-chloro-2-methylthiopyrimidine-5-carboxylate (1.37 g, 5.90 mmol), dissolved in tetrahydrofuran (20 mL), and then added with N,N-diisopropyl ethyl amine (1.90 g, 14.75 mmol). At 80°C, the reaction was heated for 16 hours with LC-MS monitoring. After the reaction was completed, the reaction solution was cooled to room temperature, concentrated under reduced pressure to give crude product. Then the crude product was dissolved in ethyl acetate, washed with 1M diluted hydrochloric acid for five times. The organic phase was dried with anhydrous sodium sulfate, filtrated, concentrated under reduced pressure to give Compound **72-3** (1.96 g, 5.71 mmol), LCMS (ESI) m/z: 343.2 $[M+H]^+$.

**Step 4: Synthesis of Compound 72-4:**

A dry single-necked flask was added with Substrate **72-3** (1.96 g, 5.71 mmol), dissolved in dichloromethane (20 mL), and then under ice bath, added with trifluoroacetic acid (80 mL). At 45°C, the reaction was performed for 2 hours with LC-MS monitoring for complete reaction. The reaction solution was cooled with ice bath to 0°C, slowly added with 40 % sodium hydroxide aqueous solution until pH = 11, and then added with methanol (20 mL). The reaction was performed under room temperature for 6 hours under LC-MS monitoring for complete reaction, concentrated under reduced pressure to remove the organic phase, and then added with 3M diluted hydrochloric acid until pH = 1, to give yellow precipitate. The solid was filtrated, and washed with water twice, and the solid was dried in vaccuo to give Compound **72-4**(647.00 mg, 3.30 mmol). LCMS (ESI) m/z: 197.2 $[M+H]^+$.

**Step 5: Synthesis of Compound 72-5:**

A dry sealed tube was added with Substrate **4-8**(101.76 mg, 485.34 μmol), Substrate **72-4**(100.00 mg, 509.61 μmol), copper (I) iodide (97.05 mg, 509.61 μmol), sodium iodide (153.00 mg, 1.02 mmol), potassium carbonate (175.82 mg, 1.27 mmol) and trans-(1R, 2R)- *N, N'*-dimethyl-1,2-cyclohexylene diamine (144.73 mg, 1.02 mmol), and then added with anisole (2 mL). Under nitrogen atmosphere. At 110°C, the reaction was performed for 18 hours with TLC monitoring for complete reaction. The reaction solution was cooled to room temperature, filtrated, washed with ethyl acetate twice. The filrate was washed with ammonia solution twice, washed with saturated brine solution twice. The organic phase was dried with anhydrous sodium sulfate, filtrated, concentrated under reduced pressure. The residue was purified by column chromatography to give Compound **72-5**(36.00 mg, 97.44 μmol).LCMS ($ESI^+$) m/z: 370.1 $[M+H]^+$.

**Step 6: Synthesis of Compound 72:**

A dry single-necked flask was added with Substrate **72-5**(36.00 mg, 97.44 μmol), dissolved in tetrahydrofuran (1 mL), and then added with m-chloroperoxybenzoic acid (33.63 mg, 194.88 μmol). Under room temperature, the reaction was performed for 1 hour. Then the reaction solution was added with N,N-diisopropyl ethyl amine (125.70 mg, 974.40

μmol) and Substrate **11-1**(50.55 mg, 194.88 mmol). The reaction was performed at 45°C overnight with LC-MS monitoring. After the reaction was completed, the reaction solution was cooled to room temperature, concentrated under reduced pressure. The residue was purified by preparative high-performance liquid chromatography (basic) to give Compound **72**(10.23 mg, 36.44 μmol).**$^1$H NMR** (400 MHz, DMSO-$d_6$) δ 10.07 (s, 1H), 8.79 (s, 1H), 7.86 (d, $J$ = 8.4 Hz, 1H), 7.71 (d, $J$ = 8.4 Hz, 1H), 7.57 (s, 2H), 6.92 (d, $J$ = 9.2 Hz, 2H), 5.30 (d, $J$= 5.2 Hz, 1H), 3.75 (d, $J$ = 5.2 Hz, 1H), 3.38 (s, 3H), 3.09 (t, $J$ = 5.6 Hz, 4H), 2.92 - 2.81 (m, 2H), 2.39 - 2.27 (m, 4H), 2.19 (s, 3H), 1.54 (t, $J$= 5.6 Hz, 4H), 1.48 (t, $J$= 5.6 Hz, 4H), 1.27 - 1.21 (m, 1H), 1.18 - 1.10 (m, 1H), 0.70 - 0.61 (m, 1H), 0.47 - 0.40 (m, 1H), 0.39 - 0.32 (m, 2H). LCMS (ESI) m/z: 581.6 $[M+H]^+$, HPLC Method B: $R_T$= 6.70 min, purity > 92.9 %.

## Biological activity evaluation assay

Unless otherwise specified, some biological evaluation experiments in this part of Example were compared with compound **AZD1775** and **ZnC3** as control. The structural information of **AZD1**775(CAS No.: 955365-80-7) and **ZnC3** (CAS No.: 2376146-48-2) is as follows:

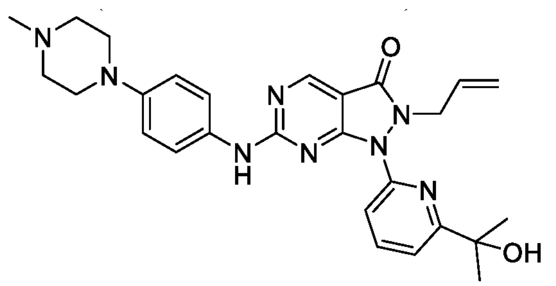

Chemical Formula: $C_{27}H_{32}N_8O_2$
Molecular Weight: 500.61
**AZD1775**

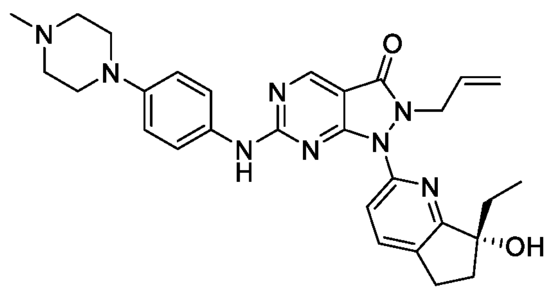

Chemical Formula: $C_{29}H_{34}N_8O_2$
Molecular Weight: 526.65
**ZnC3**

### Test Example 1: The binding of compound to Wee1 protein and Tracer 178 was evaluated by TR-FRET method.

First, solutions of the compounds in different concentration gradients were prepared. The compounds were dissolved in DMSO and the compounds were diluted 4 folds with a total of 10 dose points and 2 parallel replicates for each concentration. DMSO was added as a positive control (maximal signal control) and a negative control (minimal signal control) and a final level of 0.25% DMSO was ensured in each reaction well.

WEE1 (Thermo Fisher, Cat # PR7373A) protein in buffer (50 mM HEPES pH 7.5, 10 mM $MgCl_2$, 1 mM EGTA, 0.01% Brij-35) at 15 nM) in different concentrations of compound, and Tracer 178 (Invitrogen, PV5593) and MAb Anti-GST-Eu crypate (Cisbio, 61GSTKLA) were added to 384-well plates (Corning, cat#3574), centrifuged at 1000 rpm for 1 min and the 384-well plates were incubated in a constant temperature shaker for 60 min at 25°C and 300 rpm. Tracer 178 and MAb Anti-GST-Eu crypate were prepared in buffer (50 mM HEPES pH 7.5, 10 mM $MgCl_2$, 1 mM EGTA, 0.01% Brij-35) with a final reaction concentration of 50 nM for Tracer 178 and a final concentration of 2 nM for MAb Anti-GST-Eu crypate, where the negative control (minimal signal control) used an equal amount of buffer in place of the protein solution.

After incubation, readings were performed using BMG PHERAStar (excitation light at 337 nm and emission light at wavelength values of 620 nm and 665 nm to read the fluorescence signal values). The ratio of the fluorescence signal was calculated: 665/620* 1000 was the final signal value of the enzyme activity, and the TR-FRET signal of the reads obtained from the positive control (maximum signal control) and the negative control (minimum signal control) was normalized to give the inhibition rate for different concentrations of the compound. The IC50 for inhibition of enzyme activity by the compounds was then calculated using GraphPad Prism 6 and fitted with a log (inhibitor) vs. response-Variable slope mode. The fitting equation was: Y=Bottom + (Top-Bottom)/(1+10^((LogIC50-X)*HillSlope)), where Y represents the percentage of residual enzyme activity known and X represents the known concentration of compound after the logarithm.

The Wee1 inhibitory activity of the compounds in the Examples was tested according to the method described above and the results are shown in Table 1, where the $IC_{50}$ of each compound is categorized as follows:

"-" represents $IC_{50}$ measured value of more than 10 μM;
"+" represents $IC_{50}$ measured value of less than or equal to 10 μM and more than 1 μM;
"++" represents $IC_{50}$ measured value of less than or equal to 1 μM and more than 100 nM;

"+++" represents $IC_{50}$ measured value of less than or equal to 100 nM and more than 10 nM;
"++++" represents $IC_{50}$ measured value of less than or equal to 10 nM and more than 5 nM;
"+++++" represents $IC_{50}$ measured value of less than or equal to 5 nM.

Table 1 The inhibitory activity against Wee1 kinase of the compounds of the present invention

| Compound No. | $IC_{50}$/nM | Compound No. | $IC_{50}$/nM | Compound No. | $IC_{50}$/nM |
|---|---|---|---|---|---|
| AZD1775 | +++++ | ZnC3 | +++ | / | / |
| 1 | +++ | 2 | +++ | 3 | ++++ |
| 4 | +++++ | 4a | +++++ | 4b | +++++ |
| 5 | +++++ | 6 | +++++ | 6a | +++++ |
| 7 | +++++ | 7a | ++++ | 17 | +++++ |
| 23a | +++++ | 28 | +++++ | 30 | +++++ |
| 32 | ++++ | 37a | ++++ | 37b | ++++ |
| 39b | ++++ | 41b | +++++ | 44a | +++++ |
| 51a | +++++ | 58b | ++++ | 61b | ++++ |
| 65a | ++++ | 38b | ++++ | / | / |

Conclusion: The compounds of the present invention show good Wee1 kinase inhibitory activity.

**Test Example 2: Evaluation of cell proliferation inhibitory activity**

A. Evaluation of the anti-proliferative effect of compounds on BxPC3, HT-29 and OVCAR-3 cells by the Cell Titer-Glo method

Compound solutions of different concentration gradients were prepared. DMSO was dissolved to a concentration of 10 mM test compound and 10 mM reference compound AZD1775, and the compounds were serially diluted in culture medium for a total of 9 dose points, with 2 parallel replicates set at each concentration. The cell growth group without compound was used as a positive control (maximum signal control) and the medium was used as a negative control (minimum signal control), while ensuring that the final level of DMSO in each reaction well was 0.2%. After removing the medium from the 384-well plate, 25μl of the configured compound at different concentrations was transferred into the well plate and the compound and cells were incubated in the cell incubator at 37°C with 5% $CO_2$ for 3 days.
The 384-well plates were removed from the cell incubator and allowed to equilibrate for 1h to room temperature. 25μl of Cell Titer-Glo assay was added to each well, lysed on a shaker for 2min and then read out (Luminescence) using a BMG PHERAStar after 10min incubation. Calculate the inhibition rate from the luminescence signal.

$$\mathbf{Inhibition\%} = \mathbf{1} - \frac{\mathbf{S}(\text{Compound signal value}) - \mathbf{S}(\text{Negative Control signal value})}{\mathbf{S}(\text{Positive Control signal value}) - \mathbf{S}(\text{Negative Control signal value})}) \times \mathbf{100\%}$$

to calculate the rate of inhibition of the cells by different concentrations of the compounds. The $IC_{50}$ of the compound on cell activity inhibition was calculated by fitting a log(inhibitor) vs. response-Variable slope model to GraphPad Prism 6. The fitted equation was: Y=Bottom + (Top-Bottom)/(1+10^((LogIC50-X)*HillSlope)), where Y represents the rate of inhibition and X represents the concentration of the known compound after Log.
The results of the *in vitro* anti-cell proliferation assays of BxPC3, HT-29 and OVCAR-3 for the example compounds according to the method described above are shown in Table 2, where the $IC_{50}$ of each compound was determined and classified according to the description as follows:

"-" represents $IC_{50}$ measured value of more than 10 μM;
"+" represents $IC_{50}$ measured value of less than or equal to 10 μM and more than 5 μM;
"++" represents $IC_{50}$ measured value of less than or equal to 5 μM and more than 2 μM;
"+++" represents $IC_{50}$ measured value of less than or equal to 2 μM and more than 1 μM;

"++++" represents $IC_{50}$ measured value of less than or equal to 1 μM and more than 0.1 μM;
"+++++" represents $IC_{50}$ measured value of less than or equal to 0.1 μM.

Table 2 Inhibitory activity of compounds of the present invention on the proliferation of BxPC3, HT-29 and OVCAR-3 cells in vitro

| **Anti-prolifer ative activity** | BxPC3 | HT-29 | OVCAR-3 | **Anti-prolifer ative activity** | BxPC3 | HT-29 | OVCAR-3 |
|---|---|---|---|---|---|---|---|
| **AZD1775** | ++++ | ++++ | ++++ | **ZnC3** | ++++ | ++++ | ++++ |
| **1** | + | +++ | Not Tested | **2** | ++ | +++ | Not Tested |
| **3** | +++ | ++++ | +++ | **3a** | +++ | +++ | +++ |
| **3b** | +++ | ++++ | ++++ | **4** | +++ | ++++ | +++ |
| **4a** | ++ | +++ | ++ | **4b** | ++++ | ++++ | ++++ |
| **5** | +++ | +++ | +++ | **6** | +++ | ++++ | ++++ |
| **6a** | +++ | ++++ | ++++ | **6b** | +++ | ++++ | ++++ |
| **7** | ++++ | ++++ | ++++ | **7a** | ++++ | ++++ | ++++ |
| **7b** | ++++ | ++++ | ++++ | **8** | +++ | ++++ | +++ |
| **9** | - | +++ | ++++ | **10** | ++ | +++ | ++++ |
| **11** | ++++ | ++++ | ++++ | **11a** | ++++ | ++++ | ++++ |
| **11b** | ++++ | ++++ | ++++ | **12** | ++ | ++++ | ++++ |
| **13** | ++++ | ++++ | ++++ | **14** | +++ | ++++ | ++++ |
| **15** | ++++ | ++++ | ++++ | **16** | +++ | ++++ | Not Tested |
| **17** | +++ | ++++ | ++++ | **18** | ++ | ++++ | ++ |
| **19** | ++ | ++ | ++ | **20a** | +++ | +++ | +++ |
| **20b** | +++ | +++ | +++ | **21** | +++ | ++++ | ++++ |
| **22a** | +++ | ++++ | ++++ | **22b** | ++++ | ++++ | ++++ |
| **23a** | ++++ | ++++ | ++++ | **23b** | ++++ | ++++ | ++++ |
| **24** | +++ | +++ | +++ | **25** | +++ | +++ | ++++ |
| **26** | ++ | +++ | ++ | **27** | ++++ | ++++ | ++++ |
| **28** | ++++ | ++++ | ++++ | **29** | ++ | +++ | ++++ |
| **30** | +++ | ++++ | +++ | **31a** | + | ++ | + |
| **31b** | ++ | ++ | +++ | **32** | +++ | ++++ | +++ |
| **33** | ++ | +++ | ++ | **34** | +++ | ++++ | +++ |
| **34a** | 2.13 | ++++ | ++ | **34b** | ++++ | ++++ | +++ |
| **35a** | ++++ | ++++ | ++++ | **35b** | ++++ | ++++ | ++++ |
| **36a** | ++++ | ++++ | ++++ | **36b** | ++++ | ++++ | ++++ |
| **37a** | ++++ | ++++ | ++++ | **37b** | ++++ | ++++ | ++++ |
| **38a** | +++ | ++++ | +++ | **38b** | ++++ | ++++ | ++++ |
| **39a** | ++++ | ++++ | ++++ | **39b** | ++++ | ++++ | ++++ |
| **40** | +++ | ++++ | ++++ | **41a** | +++ | ++++ | ++++ |
| **41b** | ++++ | ++++ | ++++ | **42a** | ++++ | ++++ | ++++ |
| **42b** | ++++ | ++++ | ++++ | **43** | ++++ | ++++ | ++++ |
| **43a** | ++++ | ++++ | ++++ | **43b** | ++++ | ++++ | ++++ |

(continued)

| Anti-prolifer ative activity | BxPC3 | HT-29 | OVCAR-3 | Anti-prolifer ative activity | BxPC3 | HT-29 | OVCAR-3 |
|---|---|---|---|---|---|---|---|
| **44** | ++++ | ++++ | ++++ | **44a** | ++++ | ++++ | ++++ |
| **44b** | ++++ | ++++ | ++++ | **45a** | +++ | ++++ | ++++ |
| **45b** | +++ | ++++ | +++ | **46** | +++ | +++ | +++ |
| **47** | - | - | - | **48a** | ++++ | ++++ | ++++ |
| **48b** | ++++ | ++++ | ++++ | **49** | ++ | ++ | ++ |
| **50a** | ++++ | ++++ | ++++ | **50b** | ++++ | ++++ | ++++ |
| **51a** | ++++ | ++++ | ++++ | **51b** | ++++ | ++++ | ++++ |
| **52a** | ++++ | ++++ | ++++ | **52b** | ++++ | ++++ | ++++ |
| **53a** | ++ | +++ | ++ | **53b** | ++++ | ++++ | +++ |
| **54a** | ++ | +++ | ++ | **54b** | ++++ | ++++ | ++++ |
| **55** | ++++ | ++++ | ++++ | **56a** | +++ | +++ | +++ |
| **56b** | ++++ | ++++ | ++++ | **57a** | ++ | +++ | ++ |
| **57b** | ++++ | ++++ | ++++ | **58a** | +++ | ++++ | ++ |
| **58b** | ++++ | ++++ | ++++ | **59a** | ++++ | ++++ | ++++ |
| **59b** | +++ | ++++ | +++ | **60a** | ++++ | ++++ | ++++ |
| **60b** | ++++ | ++++ | ++++ | **61a** | ++++ | ++++ | ++++ |
| **61b** | ++++ | ++++ | ++++ | **62a** | +++ | ++++ | +++ |
| **62b** | ++++ | ++++ | ++++ | **63a** | ++++ | ++++ | ++++ |
| **63b** | +++ | ++++ | +++ | **64a** | ++++ | ++++ | ++++ |
| **64b** | ++++ | ++++ | ++++ | **65a** | ++++ | ++++ | ++++ |
| **65b** | ++++ | ++++ | ++++ | **66a** | ++ | +++ | ++ |
| **66b** | ++++ | ++++ | +++ | **67** | +++ | +++ | +++ |
| **68a** | ++ | +++ | ++ | **68b** | ++++ | ++++ | ++++ |
| **69a** | +++ | ++++ | ++++ | **69b** | +++ | ++++ | +++ |

Conclusion: The compounds of the present invention exhibit good cell proliferation inhibitory activity against tumor cells, and the anti-proliferative activity data of some compounds is equivalent to or even better than the control compound AZD1775.

B. Evaluation of the anti-proliferative effect of compounds by the Cell Titer-Glo method on normal cells HUVEC and HK2.

The test method is the same as the anti-proliferative activity evaluation of tumor cells. The test results of two normal cells are shown in Table 3.

Table 3 Inhibitory activity of compounds of the present invention on the proliferation of HUVEC and HK2 cells

| $IC_{50}/\mu M$ | HUVEC | HK2 | $IC_{50}/\mu M$ | HUVEC | HK2 |
|---|---|---|---|---|---|
| **AZD1775** | >10 | 0.26 | **ZnC3** | >10 | 0.34 |
| **4b** | >10 | 1.29 | **7a** | >10 | 0.67 |
| **11** | >10 | 1.46 | **15** | >10 | 0.76 |
| **28** | >10 | 1.19 | **32** | >10 | 1.14 |
| **37b** | >10 | 1.53 | **39b** | >10 | 0.73 |

(continued)

| $IC_{50}/\mu M$ | HUVEC | HK2 | $IC_{50}/\mu M$ | HUVEC | HK2 |
|---|---|---|---|---|---|
| **41b** | >10 | 0.55 | **44a** | 1.03 | 0.36 |
| **52a** | 3.83 | 0.48 | **55** | >10 | 0.93 |
| **56b** | >10 | 0.99 | **57b** | >10 | 0.92 |
| **58b** | 0.66 | 0.47 | **61b** | 4.46 | 0.49 |
| **65a** | >10 | 0.70 | / | / | / |

Conclusion: the compound of the present invention has a weak inhibitory activity on normal HUVEC cells, but it has a certain inhibitory effect on HK2 cells. Compared with the control compound, most of the present invention compounds show equivalent or lower inhibitory activity, which is more safe.

**Test Example 3: Evaluation of the metabolic stability of liver microsomes (mouse and human) in vitro**

1. Preparation of working solution:

Microsomes were taken out from the -80 °C refrigerator, rapidly melted in a 37 °C water bath and placed on ice until ready to use. The test article was diluted with DMSO to prepare a 10 mM stock solution, and then diluted with acetonitrile to a 0.5 mM secondary stock solution. The microsomes were diluted to 0.75 mg/ml using Buffer C; the secondary stock solution was then added to a final concentration of 1.5 μM of compound as working solution, based on n=2, 5 time points, 350 μL of each compound was prepared and placed on ice prior to use. NADPH was diluted with Buffer C to a working solution of 6 mM for the starter solution. An acetonitrile solution containing an internal standard was prepared as the precipitant, and Verapamil-HCl was chosen as the internal standard at a concentration of 4 ng/ml.

2. Experimental procedure:

A round-bottom well plate was taken, noted as the reaction plate, and the prepared working solution for each compound was dispensed into the well plate according to the number of replicates and time points (0 h samples are also added to the reaction plate), 30 μL/well; the plate was incubated at 37 °C for 10 min. A separate plate with pointed bottom wells, noted as a precipitation plate, was added with 135 μL precipitant per well; 0 h samples were transferred to the plate after 10 min incubation and 15 μL of starter solution was added; the plate was placed on ice before centrifugation.

The diluted starter solution was added in sufficient quantity to the dispensing plate to facilitate the multichannel pipette aspiration operation.

The reaction was carried out on a warm incubation shaker and 15 μL of starter solution/sample is added to the plate using a multichannel pipette. The reaction was mixed with a slight shake to initiate the reaction, which was accurately timed and recorded using a timer;

After the reaction time had elapsed, all the solution in the plate was aspirated using the multichannel pipette and added to the precipitation plate to terminate the reaction at that point in time. After all reactions had been terminated, the plates were shaken for ten minutes on a plate shaker at 600 rpm to precipitate the protein. The plate was centrifuged at 4 °C for 15 minutes at maximum rpm. 80 μL of supernatant was taken, 320 μL of pure water was added and mixed for LC-MS analysis.

3. The test results are shown in Table 4.

Table 4 Liver microsomes stability data of different soecies

| Example Compound No. | Mouse | Human | Rat | Dog | Monkey |
|---|---|---|---|---|---|
| | $T_{1/2}$ (min) | | | | |
| **AZD1775** | 25.99 | 38.12 | 38.08 | 10.76 | 20.09 |
| **4b** | 72.82 | 44.82 | >120 | 41,74 | 22.84 |
| **7a** | 72.56 | 63.40 | 21.4 | 92.48 | 15.75 |

(continued)

| Example Compound No. | Mouse | Human | Rat | Dog | Monkey |
|---|---|---|---|---|---|
| | $T_{1/2}$ (min) | | | | |
| **28** | 51.65 | 29.95 | 30.9 | 38.2 | 17.74 |
| **37b** | 37.8 | >120 | >120 | 69.59 | 37.98 |
| **44** | 62.8 | 77.75 | >120 | 42.81 | 30.09 |
| **52a** | 96.19 | 26.24 | 31.42 | 22.8 | 14.22 |

Conclusion: The compounds of the present invention show good metabolic stability in five different species of liver microsomes.

**Test Example 4:** Solubility evaluation

The compound was placed in a buffer solution and shaken at constant temperature for 24h. The supernatant was prepared into a solution of about 100 μg/ml of the test article, and the solubility was calculated by reversed-phase high performance liquid chromatography with gradient elution and external standard method. Chromatographic conditions: C18 column, mobile phase A: 0.02 M potassium dihydrogen phosphate: acetonitrile=90:10 , mobile phase B: acetonitrile; V :1.0 ml/min, T:35°C, λ :210nm.
The test results are shown in Table 5.

Table 5 Solubility of the compounds at different pH conditions

| Compound No. | Saturated solubility (mg/mL)(37°C, 24h) | | |
|---|---|---|---|
| | **pH 5.0** | **pH 6.5** | **pH 7.4** |
| **37b** | 0.37 | 0.37 | 0.62 |
| **AZD1775** | 0.41 | 0.05 | 0.02 |
| **ZnC3** | 0.35 | 0.59 | 0.68 |

Conclusion: Under three pH conditions, the solubility of the Compound 37b of the present invention is similar to that of the control compound **ZnC3,** and the solubility of the compound of the present invention is obviously better than that of the control compound AZD1775.

**Test Example 5: Membrane Permeability evaluation**

Caco-2 cells were purchased from the American Model Tissue Cell Collection (Rockville, MD). The cell culture medium was modified Eagle's medium (MEM) containing 10% inactivated fetal bovine serum and 1% non-essential amino acids. Cells were inoculated on polycarbonate filter membranes (Cat no. 3396) and incubated at 37°C in a 5% $CO_2$ incubator.
The cells were incubated for 21-28 days after inoculation for transport experiments and the apparent permeability (Papp) of Lucifer Yellow was used to characterize and verify the compactness of the cell monolayer. A stock solution of 10 mM was prepared by dissolving the compound in DMSO and diluted using Hanks Balanced Salt Solution (HBSS, Invitrogen, Cat# 14025-092) containing 25 mM HEPES (pH 7.4) to obtain the working solution. A 10 μM working solution of the compound to be tested was added to the apical side and basolateral side of Caco-2 and incubated at 37°C for 90 min. After the incubation, dilute the samples on the apical side and basolateral side, and the concentrations of compounds on the apical and basolateral sides were detected by LC-MS/MS, and the concentrations of the compounds were quantified by standard curve.
The test results are shown in Table 6.

Table 6 Permeability data of the compounds in Caco2 model

| **Compound No.** | Papp ($10^{-6}$ cm/s) | | Efflux Ration |
|---|---|---|---|
| | A to B | B to A | |
| **Atenolol** | 0.43 | 0.18 | 0.42 |

(continued)

| Compound No. | Papp ($10^{-6}$ cm/s) | | Efflux Ration |
|---|---|---|---|
| | A to B | B to A | |
| **Propranolol** | 14.71 | 13.02 | 0.88 |
| **Quinidine** | 4.05 | 18.72 | 4.62 |
| **AZD1775** | 0.80 | 15.17 | 18.96 |
| **ZnC3** | 0.42 | 15.01 | 35.74 |
| **3b** | 0.73 | 13.07 | 17.90 |
| **4b** | 0.94 | 13.19 | 14.03 |
| **11** | 0.41 | 8.85 | 21.59 |
| **37b** | 0.46 | 9.69 | 21.06 |
| **39b** | 0.38 | 13.62 | 35.84 |
| **65a** | 0.96 | 9.17 | 9.55 |

Conclusion: The membrane permeability of the compounds of the present invention is equivalent to that of the control compound in Caco2 model. Generally speaking, the cell permeability of the compounds of the present invention and the control compound is not good, and they have certain efflux properties.

**Test Example 6: Evaluation of plasma protein binding (PPB)**

1. Experimental procedure

Sample preparation: The compound was dissolved in DMSO to a stock solution of 10 mM, then the compound was diluted with PBS to a secondary stock solution of 0.02 mM, and then the above 0.02 mM was diluted to 1 μM using blank plasma, which was the sample to be incubated.

Dialysis set-up preparation: 400 μL of blank PBS was first added to the white wells of the equilibrated dialysis plate and 200 μL of the configured plasma sample was added to the red wells, and the dialysis plate was sealed with a sealing film.

Recovery plate preparation: Two 96-well deep-well plates, labelled T0 and T5, were prepared and all plasma samples were added at n=2. 300 μL of acetonitrile (Verapamil-HCl, 4 ng/mL) was added directly to the T0 plate, followed by 50 μL of blank PBS mix well for 5 min and left to stand in a 4 °C refrigerator until the end of the incubation.

Experimental Operation: The dialysis device and the T5 plate were incubated together for 5 h in a microplate thermostatic shaker (37 °C, using 300 rpm or minimum speed). At the end of the incubation, 300 μL of acetonitrile (Verapamil-HCl, 4 ng/mL) was added and 50 μL of PBS solution was added. At the end of the dialysis incubation, a new 96-well deep well plate was taken. Add 50 μL of plasma well sample to the corresponding position of the 96-well plate, 300 μL of acetonitrile and 50 μL of blank PBS; take 50 μL of buffer well sample to the corresponding position of the 96-well plate, then add 300 μL of acetonitrile and 50 μL of blank plasma. Add 300 μL of acetonitrile (Verapamil-HCl, 4 ng/mL) to the plasma-containing wells of the T5 plate, and then 50 μL of PBS solution was added. Shake for 5 min to fully precipitate the proteins and centrifuge at 20,000 g for 10 min at 4 °C. Add 200 μL of supernatant to 200 μL of pure water, mix well and perform LC-MS/MS analysis.

2. Data processing and parameter calculation

- $$\text{Plasma protein binding rate} = [(Rpe - Rb)/ Rpe] \times 100\ \%$$
- 

$$Recovery=[(Rpe+ Rb)/ R5h] \times 100\%$$

- 

$$Stability=(R5/R0)\times 100\%$$

Wherein:

- $R_{pe}$= ratio of plasma-side testing sample peak area to internal standard
- $R_b$= ratio of buffer side testing sample peak area to internal standard
- $R_5$= ratio of incubator stability sample peak area to internal standard
- $R_0$= ratio of refrigerator stability sample peak area to internal standard 3. The test results are shown in Table 7.

Table 7 The plasma protein binding of representative compounds (mouse/rat/human)

| Compound | Plasma Protein binding(PPB)/% | | |
|---|---|---|---|
| | Mouse | Rat | Human |
| AZD1775 | 91.3 | 62.6 | 66.38 |
| 3 | 87.75 | Not Tested | Not Tested |
| 4b | 87.71 | 77.45 | Not Tested |
| 6 | 97.2 | Not Tested | Not Tested |
| 7a | 97.21 | 94.82 | Not Tested |
| 11 | 92.17 | Not Tested | Not Tested |
| 15 | 93.51 | Not Tested | Not Tested |
| 21 | 98.86 | Not Tested | Not Tested |
| 28 | 92.29 | 86.08 | Not Tested |
| 32 | 96.87 | Not Tested | Not Tested |
| 37b | 92.46 | 84.10 | 77.58 |
| 41b | 96.26 | Not Tested | Not Tested |
| 44 | 98.46 | 96.98 | Not Tested |
| 61b | 98.46 | 93.61 | Not Tested |

Conclusion: The compounds of the present invention have good plasma protein binding capacity.

**Test Example 7: Evaluation of compound inhibition of cytochrome P450**

Enzymatic experiments were performed to quantify the inhibition of CYP450 enzyme activity of each isoform of CYP450 by small molecule inhibitors through fluorescence generated by the oxidation of the substrate by cytochrome P450. The experiments were performed in 384-well plates (Corning, Cat# 3575) using a reaction buffer of 142.86 mM Potassium Phosphate, pH 7.4. The Solution A components used in the experiments were: 26.13 mM NADP+ (Sigma-aldrich, Cat# N0505) 65.77 mM G6P (J&K, Cat#968161) and 65.42 mM MgCl2 (Sigma-aldrich, Cat#M2670). The Solution B composition used for the experiment was: 40 U/mL G6PDH (Sigma-aldrich, Cat# G6378). The substrate mix was 0.05 X Solution A, 0.01 X Solution B, 50 mM Potassium Phosphate, 0.01 mM BOMCC/ 0.01 mM EOMCC/ 0.001 mM DBOMF. For CYP3A4 and CYP2C9, the reaction system was 50 μL or 20 μL, respectively, including 3 nM CYP3A4 or 120 nM CYP2C9, BOMCC substrate mixed solution and different concentrations of compounds to be tested. For CYP2C19, CYP2D6 and CYP1A2, the reaction system was 20 μL and included 12.5 nM CYP2C19, 80 nM CYP2D6 or 1 nM CYP1A2, EOMCC substrate mix and various concentrations of the compounds to be tested. For CYP2C8, the reaction system is 50 μL and includes 1.5 nM CYP2C8, DBOMF substrate mix and various concentrations of compound to be tested. After preincubation with the enzyme for 10 minutes, the substrate was added and the fluorescence signal was read at different

wavelengths (BOMCC/EOMCC Ex430 nm/Em480 nm, DBOMF Ex490 nm/Em520 nm) using BMG PHERAStar depending on the substrate, with reaction intervals of 30 seconds or more (depending on the actual number of wells) and reaction times of 30 minutes. The data were analyzed and processed using GraphPad Prism 6 software to obtain IC50 values. The test results are shown in Table 8.

Table 8 Inhibitory activity of the compounds on seven CYP subtypes

| $IC_{50}/\mu M$ | CYP3A4 | CYP1A2 | CYP2C9 | CYP2C19 | CYP2D6 | CYP2C8 | CYP 2B6 |
|---|---|---|---|---|---|---|---|
| **AZD1775** | > 10 | > 10 | > 10 | $1<IC_{50}<10$ | > 10 | > 10 | > 10 |
| **3** | > 10 | > 10 | > 10 | > 10 | > 10 | > 10 | Not Tested |
| **7a** | > 10 | > 10 | > 10 | $1<IC_{50}<10$ | > 10 | > 10 | Not Tested |
| **37b** | > 10 | > 10 | > 10 | > 10 | > 10 | > 10 | > 10 |
| **44** | > 10 | > 10 | > 10 | $1<IC_{50}<10$ | > 10 | > 10 | Not Tested |

Conclusion: The compound of the present patent shows no obvious cytochrome P450 enzyme inhibition, and the Compound **37b** of the present patent inhibited all subtypes by more than 10 μM, which was better than the control compound AZD1775.

**Test Example 8: hERG potassium channel inhibition assay**

Experimental procedure:

(I) Experimental materials:

A. CHO (Chinese hamster ovary cells) stably transfected cell line culture

The cell line used for the patch clamp assay was a 10th generation CHO cell overexpressing hERG potassium channel cDNA. CHO hERG cells were cultured in Petri dishes or flasks at 37°C in a 5 % CO2 incubator. Cells were dropped onto circular slides 24-48 hours prior to electrophysiological experiments and cultured in cell culture medium and used for experiments after the cells had been adhered.

Cell culture medium (purchased from Invitrogen) Composition:

- Ham's F12 medium
- 10% (v/v) heat inactivated FBS
- 100 μg/ml Hygromycin B (thaumatin)
- 100 μg/ml Geneticin (Genomycin, G418)

B. Compound preparation

Compound powders are dissolved in the extracellular solution and are subjected to a routine 5 to 10 minute sonication and shaking to ensure complete dissolution of the compound.

The final concentrations of compounds used for electrophysiological assays were 5, 20 μM and the final concentration of DMSO was 0.1%.

(II) Experimental protocol:

A. Experimental procedure for electrophysiological recordings

Cell membrane currents were recorded using a HEKA EPC-10 USB patch-clamp amplifier (HEKA Elektronik, Germany).

1)A coverslip with a large number of uniformly growing individual CHO hERG cells on its surface was taken. Place in a continuous recording cell on an inverted microscope, perfused with extracellular fluid (approximately 1 ml per minute) and recorded continuously, waiting for the current to stabilize.
2) Record HERG channel currents for individual cells using standard whole cell recording mode. The membrane voltage is first clamped at -80 mV and the cell is given a +20 mV stimulus for 5 s to activate the hERG potassium channel, then repolarized to -50 mV for 5 s to generate an outward tail current, which is continuously perfused until the

current is stable, at which point the peak tail current is the control current value.

3) The extracellular solution containing the drug to be tested was then perfused and recorded until the inhibitory effect of the drug on the hERG current reached a steady state, at which point the peak tail current was the post-drug current value.

4) The cells are again perfused with the extracellular solution until the hERG current returns to or approaches the level prior to the addition of the drug, then the perfusion can be continued to test other concentrations or drugs. One or more compound or drug concentrations may be tested on each cell.

5) Cisapride (C4740-10mg, Sigma) is used as a positive control in the experiment to ensure that the cells used respond properly.

(III) Quality control

The following criteria need to be met for the reported experimental data:
Electrophysiological recording parameters

a) Sealing resistance > 500MΩ
b) contact resistance (Ra) < 10MΩ
c) Initial tail current amplitude > 200pA
d) Current rundown (spontaneous reduction) < 2%/min
e) Leakage current < 200pA or 10% of peak hERG current (within 90% of recording time)

The test results are shown in Table 9.

Table 9 hERG inhibitory activity of the compounds

| Compound | Test Concentration/μM | Inhibition/% | Compound | Test Concentration/μM | Inhibition/% |
|---|---|---|---|---|---|
| **AZD1775** | 5 | 35.62 ± 1.43 | **ZnC3** | 5 | 7.38 ± 1.59 |
| | 20 | 55.56 ± 1.83 | | 20 | 18.05 ± 7.58 |
| **4b** | 5 | 23.84 ± 4.39 | **7** | 5 | 88.02 ± 3.87 |
| | 20 | 42.42 ± 5.58 | | 20 | 95.13 ± 1.06 |
| **37b** | 5 | 18.00 ± 4.80 | **39b** | 5 | 47.26 ± 2.27 |
| | 20 | 34.70 ± 4.40 | | 20 | 67.24 ± 1.32 |
| **44** | 5 | 77.68 ± 4.16 | **48b** | 5 | 73.67 ± 0.93 |
| | 20 | 91.56 ± 2.72 | | 20 | 90.92 ± 0.49 |

Conclusion: The hERG inhibitory activity of some compounds of the present patent (such as 4b, 37b) is weaker than that of the control compound AZD1775, and the risk of cardiotoxicity is relatively lower.

Test Example 9: **Pharmacokinetics Evaluation**

**(I) Evaluation of pharmacokinetics on single dose in mice**

The present experiment aimed to study the pharmacokinetics in the plasma of male ICR mice after the administration.

1. Experimental Aim:

The present experiment aimed to obtain the pharmacokinetic profile of the subject compounds in ICR mice (both intravenous and oral)

2. Standard compliance

In this experiment (non-GLP study), the test articles-testing, DMPK animal test, and DMPK analysis were done in Chengdu Hitgen, and all the tests followed the present test protocol, and the relevant SOPs of the related organizations.

3. Test materials, instruments and equipment

3.1 Test materials

3.1.1 Test articles

The following test articles were provided by Chengdu Hitgen and their quality was ensured to meet the requirements.

| | |
|---|---|
| Name/Code | Test compound |
| Nature | Solution |
| Solvent/Dosage | 5%DMSO-10%Solutol-85%HPBCD(20%,W/V) |
| Preservation Condition | Room temperature |
| Concentration of test solution | 0.2 mg/ml (Intravenous), 1 mg/ml (Oral) |
| Volume of test solution | 3 ml, 3 ml |

3.1.2 Test system

Species: SPF grade male ICR mice
Body weight/weekly age: about 30g
Amount: 6
Experimental animal source: Charles River

4. Experimental procedure:

4.1 Solvent Preparation

Intravenous solvents: 5% DMSO - 10% Soluto1-85% HPBCD (20%,WN)
"Gavage solvent/dosage form: 5% DMSO - 10% Soluto1-85% HPBCD (20%,WN) " 4.2 Subjects given (route)
Intravenous: 1 mg/kg in a volume of 5 mL/kg
Gavage: 10 mg/kg in a volume of 10 mL/kg
Ultrasound for 5 min before administration

4.3 Subject Preparation (concentration)

Intravenous: 0.2 mg/mL
Gavage: 1 mg/mL
Fast overnight before administration and feed four hours after administration.

4.4 Sample collection

Blood samples were collected by orbital venous plexus puncture (40-50 L) into anticoagulant tubes containing pre-sprayed EDTA-K2 at 5 min (IV only), 15 min, 30 min, 1 h, 2 h, 4 h, 8 h, and 24 h, respectively, and the supernatant, i.e., the plasma, was centrifuged at 10,000 rpm for 20 min within 1 h. The blood samples were stored in a refrigerator at or below -20 C for LC-MS/MS analysis.

4.5 Biopharmaceutical analytical methods and assays: LC-MS/MS Analytical Detection used

5. Data processing

The samples were detected for drug concentration at each time point by LC-MS/MS. Pharmacokinetic parameters terminal elimination half-life (t1/2), area under the curve (AUC), apparent volume of distribution (Vd), clearance (CL), mean residence time (MRT), and Cmax were calculated using the non-compailinental model of Phoenix WinNonlin 5.2. Bioavailability (F%) was directly from serum concentration results. Mean standard deviation (X±SD) was used for blood concentration and pharmacokinetic parameters, etc. Specific testing and analytical methods were specified in the form of protocol revisions.

6. The test results are shown in Table 10.

Table 10 Pharmacokinetic parameters of compound **AZD1775** and **37b** in ICR mice

| **Parameter** | ***iv* (1 mg/kg)** | | **Parameter** | ***po* (10 mg/kg)** | |
|---|---|---|---|---|---|
| | **AZD1775** | **37b** | | **AZD1775** | **37b** |
| **$C_0$ (ng/mL),** | 250 | 186 | **$C_{max}$ (ng/mL)** | 398 | 307 |
| **$AUC_{last}$ (h*ng/mL)** | **116** | **558** | **$AUC_{last}$ (h*ng/mL)** | **396** | **1607** |
| **$T_{1/2}$ (h)** | 0.5 | 2.99 | **$T_{1/2}$ (h)** | 0.87 | NA |
| **$T_{max}$ (h)** | / | / | **$T_{max}$ (h)** | 0.42 | 2.67 |
| **Cl (L/h/kg)** | 8.05 | 1.58 | **Cl (L/h/kg),** | 26.76 | 4.44 |
| **F/%** | / | / | **F/%** | 32 | 29 |

Conclusion: The pharmacokinetic analysis in mice showed that the compound of the present patent 37b had better pharmacokinetic properties than the control compound AZD1775, with higher drug exposure and slower clearance.

**(II) Pharmacokinetic evaluation on single dose in rats**

Pharmacokinetic properties of the drug in male SD rats were evaluated by the same method with in vivo pharmacokinetic study with mice. The results are shown in Table 11:

Table 11 Pharmacokinetic parameters of compound **AZD1775** and **37b** in SD rats

| **Parameter** | iv (1 mg/kg) | | **Parameter** | po (10 mg/kg) | |
|---|---|---|---|---|---|
| | AZD1775 | 37b | | AZD1775 | 37b |
| **C0 (ng/mL)** | 100.7 | 313.2 | **Cmax (ng/mL)** | 188.5 | 188.6 |
| **AUClast (h*ng/mL)** | **72.3** | **471** | **AUClast (h*ng/mL)** | **881** | **1131** |
| **T1/2 (h)** | 0.91 | 3.97 | **T1/2 (h)** | 2.68 | NA |
| **Tmax (h)** | / | / | **Tmax (h)** | / | 2.67 |
| **Cl (L/h/kg),** | 8.86 | / | **Cl (L/h/kg),** | 10.25 | / |
| **F/%** | / | / | **F/%** | 121.8 | 24.01 |

Results: The maximum blood concentration of the Compound **37b** of the present patent and the control compound **AZD1775** was similar. The Compound **37b** of the present patent had obvious advantages in drug exposure, indicating that the clearance of the Compound **37b** was slower, and the oral exposure level of the Compound **37b** was also better than that of the control compound **AZD1775.**

**(III) Pharmacokinetic evaluation on single dose in Beagle dogs**

Pharmacokinetic properties of the drug in male Beagle dogs were evaluated by the same method with in vivo pharmacokinetic study with mice. The basic information is as follows:

Beagle: weighing 8-11kg, purchased from Jiangsu Marshall Biotechnology Co., Ltd.
Dosage: 2mg/kg (intravenous injection); 10 mg/kg (oral)
Solvent: 5% DMSO+5% Solutol+90% (20% HP-β-CD in Saline)

The test results are shown in Table 12.

Table 12 Pharmacokinetic parameters of compound **AZD1775** and **37b** in Beagle dogs

| **Parameter** | ***iv* (2 mg/kg)** | | **Parameter** | ***po* (10 mg/kg)** | |
|---|---|---|---|---|---|
| | **AZD1775** | **37b** | | **AZD1775** | **37b** |
| **Vss (ng/mL)** | 3.01 | 5.71 | **$C_{max}$ (ng/mL)** | 405 | 171 |
| **$AUC_{last}$ (h*ng/mL)** | 877 | 1602 | **$AUC_{last}$ (h*ng/mL)** | 1624 | 1816 |
| **$T_{1/2}$ (h)** | 1.03 | 5.05 | **$T_{1/2}$ (h)** | 2.34 | 4.69 |
| **$T_{max}$ (h)** | / | / | **$T_{max}$ (h)** | 1.67 | 2.00 |
| **Cl (L/h/kg),** | 2.64 | 1.31 | **F/%** | 37.9 | 22.8 |

Conclusion: In beagle dogs, the pharmacokinetics of the Compound **37b** of the present patent is slightly better than that of the control compound **AZD1775.**

**Test Example 10: Evaluation of drug efficacy in animals (CDX model)**

(I) Pharmacodynamic evaluation of the compound in nude mice of female BALB/c xenograft of human colon cancer HT-29 cells.

Objective: To evaluate the anti-tumor efficacy of compound **37b** and **AZD1775** in nude mice bearing xenogeneic tumor (HT-29).

Experimental method: BALB/c nude mice (provided by Charles River) were selected, 7-8 weeks old and weighing 19-22 g.

The prepared and cultured HT-29 cells were counted, and $5 \times 10^6$ HT-29 cells were mixed in 0.1 mL PBS solution and inoculated subcutaneously on the right wing of mice. When the cells became tumors and the average tumor volume reached 120 $mm^3$, they began to be grouped and administered. The dosage was AZD1775, 60mg/kg, once a day; 37b was given in three dosage groups, 30mg/kg, 60mg/kg and 120mg/kg, once a day. The experimental indicator was to check whether the tumor growth was inhibited. The formula for calculating the tumor volume was: $V = 0.5a \times b^2$, where a and b respectively indicated the long and short diameters of the tumor.

The anti-tumor effects of the compounds were evaluated by TGI(%). The calculation of TGI(%) was: TGI(%)= [1-(the average tumor volume of a treatment group at the end of administration-the average tumor volume of the treatment group at the beginning of administration)/(the average tumor volume of the solvent control group at the end of administration-the average tumor volume of the solvent control group at the beginning of administration)] ×100%.

After 24 days of administration, the antitumor effects of compound 37b and AZD1775 are shown in Table 13:

Table 13 Antitumor Effects of Compound AZD1775 and 37b on HT-29 Model

| Compound | **AZD1775** | **37b** | **37b** | **37b** |
|---|---|---|---|---|
| Dosage | 60mg/kg | 30mg/kg | 60mg/kg | 120mg/kg |
| Dose frequency | qd | qd | qd | qd |
| TGI(%) | 54.5 | 34.7 | 57.7 | 100.0 |

Conclusion: At the dose of 60mg/kg, the compound 37b of the present patent has the same antitumor effect as the control compound AZD1775. At the dose of 120mg/kg, the compound 37b of the present patent has significant tumor growth inhibition.

(II) Pharmacodynamic evaluation of the compound in nude mice of female BALB/c xenograft of human pancreatic cancer BxPC3 cells.

Objective: To evaluate the anti-tumor efficacy of compound **37b** and **AZD1775** in nude mice bearing xenogeneic tumor (BxPC3).

Experimental method: BALB/c nude mice (provided by Charles River) were selected, 7-8 weeks old and weighing 19-22 g.

The prepared and cultured BxPC3 cells were counted, and $1 \times 10^7$ BxPC3 cells were mixed in 0.1 mL PBS solution and inoculated subcutaneously on the right wing of mice. When the cells became tumors and the average tumor volume reached 190 $mm^3$, they began to be grouped and administered. The dosage was AZD1775, 60mg/kg, once a day; 37b was given in three dosage groups, 30mg/kg, 60mg/kg and 120mg/kg, once a day. The experimental indicator was to check whether the tumor growth was inhibited. The formula for calculating the tumor volume was: $V = 0.5a\times b^2$, where a and b respectively indicated the long and short diameters of the tumor.

The anti-tumor effects of the compounds were evaluated by TGI(%). The calculation of TGI(%) was: TGI(%)= [1-(the average tumor volume of a treatment group at the end of administration-the average tumor volume of the treatment group at the beginning of administration)/(the average tumor volume of the solvent control group at the end of administration-the average tumor volume of the solvent control group at the beginning of administration)] ×100%.

After 40 days of administration, the antitumor effects of compound 37b and AZD1775 are shown in Table 14:

Table 13 Antitumor Effects of Compound AZD1775 and 37b on HT-29 Model

| Compound | **AZD1775** | **37b** | **37b** | **37b** |
|---|---|---|---|---|
| Dosage | 60mg/kg | 30mg/kg | 60mg/kg | 120mg/kg |
| Dose frequency | qd | qd | qd | qd |
| TGI(%) | 57.6 | 70.1 | 72.4 | 100.9 |

Conclusion: At the dosage of 30 mg/kg and 30 mg/kg, the compound **37b** of the present invention has better antitumor effect than the control compound AZD1775. At the dose of 120 mg/kg, compound **37b** has significant tumor growth inhibition.

**Test Example 11: Toxicological Evaluation of Compounds**

(I) Single dose toxicity in rats-evaluation of maximum tolerated dose

Objective: To evaluate the tolerance of AZD1775 and 37b in rats after a single dose.
Experimental animals: SD rats (provided by Charles River), 6-8 weeks old, 180-220g.

Test method: the rats were given a single oral dose according to the predetermined dose, and the survival of the rats was followed up (the longest continuous observation was 14 days). See Table 15 for the experimental protocol and results.

Table 15 Experimental Protocol of Maximum Tolerable Dose of AZD 1775 and 37b Rats after Single Dose

| **Group** | **Animal Numbers** | **Sex** | **Dosage (mg/kg)** | Administration Route and Frequence | **Death Numbers** | **Death Rate** |
|---|---|---|---|---|---|---|
| **AZD1775** Low Dosage Group | 3 | Male | 100 | By gavage, Single dose | 0 | 0% |
| | 3 | Female | 100 | By gavage, Single dose | 0 | 0% |
| **AZD1775** Medium Dosage Group | 3 | Male | 200 | By gavage, Single dose | 0 | 0% |
| | 3 | Female | 200 | By gavage, Single dose | 0 | 0% |
| **AZD1775** High Dosage Group | 3 | Male | 300 | By gavage, Single dose | 0 | 0% |
| | 3 | Female | 300 | By gavage, Single dose | 2 | 67% |

(continued)

| Group | Animal Numbers | Sex | Dosage (mg/kg) | Administration Route and Frequence | Death Numbers | Death Rate |
|---|---|---|---|---|---|---|
| **37b** Low Dosage Group | 5 | Male | 200 | By gavage, Single dose | 0 | 0% |
| | 5 | Female | 200 | By gavage, Single dose | 0 | 0% |
| **37b** Secondary | 5 | Male | 300 | By gavage, Single dose | 0 | 0% |
| Low Dosage Group | 5 | Female | 300 | By gavage, Single dose | **0** | **0%** |
| **37b** Medium Dosage Group | 5 | Male | 400 | By gavage, Single dose | **2** | **40%** |
| | 5 | Female | 400 | By gavage, Single dose | **3** | **60%** |
| **37b** High Dosage Group | 5 | Male | 500 | By gavage, Single dose | **2** | **40%** |
| | 3 | Female | 500 | By gavage, Single dose | **2** | **67%** |

Test results: The maximum tolerated dose of the compound 37b of the present invention is higher than that of the control compound AZD1775 in a single dose to rats, and it has obvious safety advantages.

(II) Toxicity evaluation of rats after 14 consecutive days of administration

Objective: to evaluate the tolerance and toxicity of compounds AZD1775, ZnC3 and 37b in rats after continuous administration.

Experimental animals: SD rats (provided by Charles River), 6-8 weeks old, 180-220g.

Test method: The rats were given a predetermined dose orally once a day, and the survival and weight changes of the rats were followed up (the longest continuous administration was 14 days). See Table 16 for the test protocol.

Table 16 Experimental Protocol of 14-day Continuous Administration Toxicity to Rats

| Group | Animal numbers | Dosage (mg/kg) | Administration Route |
|---|---|---|---|
| Control Group | 4 Males+4 Females | Vehicle | By gavage, qd |
| **AZD1775** Low Dosage Group | 7 Males+7 Females (Among them, 4 rats were studied by toxicology and 3 rats were studied by toxicity metabolism kinetics.) | 25 | By gavage, qd |
| **AZD1775** Medium Dosage Group | | 75 | By gavage, qd |
| **37b** Low Dosage Group | | 40 | By gavage, qd |
| **37b** Medium Dosage Group | | 75 | By gavage, qd |
| **37b** High Dosage Group | | 120 | By gavage, qd |
| **ZnC3** | | 75 | By gavage, qd |

Among them, the sampling of toxic metabolic kinetics study is as follows: (1) sampling at the first and last administration; Sampling time points: 15 minutes, 30 minutes, 1 hour, 2 hours, 4 hours, 8 hours and 24 hours.

Test results:

1. The overall survival of rats

After 14 days of continuous administration according to the above administration protocol, it was found that rats in some dose groups died, and the results are shown in Table 17.

Table 17 Survival statistics of rats after continuous administration for 14 days

| **Female** | Control | **AZD1775-** 25 mg/kg | **AZD1775-75** mg/kg | **37b** -40 mg/kg | **37b** -75 mg/kg | **37b** -120 mg/kg | Zn-C3 - 75 mg/kg |
|---|---|---|---|---|---|---|---|
| Survival of Rats | 4/4 | 7/7 | 0/7 | 7/7 | 7/7 | 6/7 | 1/7 |

(continued)

| Male | Control | **AZD1775-** 25 mg/kg | **AZD1775-75** 25 mg/kg | **37b** -40 mg/kg | **37b** -75 mg/kg | **37b** -120 mg/kg | Zn-C3- 75 mg/kg |
|---|---|---|---|---|---|---|---|
| Survival of Rats | 4/4 | 7/7 | 1/7 | 7/7 | 7/7 | 4/7 | 2/7 |

Results: The survival rate of rats in the compound 37b groups was significantly better than that in the control compound AZD1775 group and the control compound ZnC3 group.

2. Toxic metabolic kinetic parameters

The results of toxicity metabolic kinetics test of three compounds in rats are shown in Table 18 (female rats) and Table 19 (male rats).

Table 18 Toxic metabolic kinetic parameters in female rats

| **Female rats dosing group** | first administration blood sampling | | | | day 14 post-administration blood sampling | | | |
|---|---|---|---|---|---|---|---|---|
| | $T_{max}$ | $C_{max}$ | $T_{1/2}$ | $AUC_{last}$ | $T_{max}$ | $C_{max}$ | $T_{1/2}$ | $AUC_{last}$ |
| **AZD1775,25mg/kg** | 1.33±0.58 | 1801±792 | 5.46±1.33 | 9066±2760 | 0.58±0.38 | 852±247 | 4.55±0.56 | 6490±2842 |
| **AZD1775,7mg/kg** | 1.83±1.89 | 1355±639 | / | 15991±9091 | / | / | / | / |
| **37b,40mg/kg** | 2.25±2.47 | 375±275 | 10.83±0.40 | 5572±4537 | 4.00±0.00 | 1466±825 | 6.09±2.27 | 16189±11337 |
| **37b,75mg/kg** | 2,25±2.47 | 839±213 | 9,.56±4.93 | 8937±2772 | 2.00±0.00 | 1617±379 | 5.23±1.02 | 1710±5399 |
| **37b,120mg/kg** | 1.33±0.58 | 2893±1816 | 7.93±2.37 | 19720±5189 | 4.00±0.00 | 2197±694 | 5.94±0.92 | 19090±6565 |
| **ZnC3,75mg/kg** | 16.17±13.57 | 1588±741 | / | 21547±12906 | / | / | / | / |

Table 19 Toxic metabolic kinetic parameters in male rats

| Male rats dosing group | first adnnmistration blood sampling | | | | day 14 post-administration blood sampling | | | |
|---|---|---|---|---|---|---|---|---|
| | $T_{max}$ | $C_{max}$ | $T_{1/2}$ | $AUC_{last}$ | $T_{max}$ | $C_{max}$ | $T_{1/2}$ | $AUO_{last}$ |
| **AZD1775,25 mg/kg** | 2.17±1.76 | 491±104 | 5.11±0.26 | 3187±18 | 3.33±1.15 | 774±297 | / | 7274±1473 |
| **AZD1775,75 mg/kg** | 2.75±2.17 | 875±237 | / | 7487±2221 | | / | / | / |
| **37b,40 mg/kg** | 3.67±3.79 | 487±278 | 9.63±3.13 | 2423±259 | 5.33±2.31 | 1403±812 | 4.38:1:1.27 | 19170±13683 |
| **37b,75 mg/kg** | 6.00±3.46 | 1733±569 | / | 17765±3708 | 3.33±1.15 | 2406±489 | 4.88±0.72 | 24712±4091 |
| **37b,120 mg/kg** | 1.08±0.88 | 1865±161 | 11.07±1.92 | 16922±5243 | / | / | / | / |
| **ZnC3,75 mg/kg** | 0.75±0.43 | 2304±1090 | 12.46±7.61 | 12277±3592 | / | / | / | / |

Conclusion: Based on the analysis of the maximum tolerated dose of single administration in rats and the toxicity of rats after 14 days of administration, the compound 37b of the present invention has obvious advantages over the control compound AZD1775 and the control compound ZnC3 in drug safety.

## Claims

1. A compound represented by Formula I, or a stereoisomer thereof, or a pharmaceutically acceptable salt thereof:

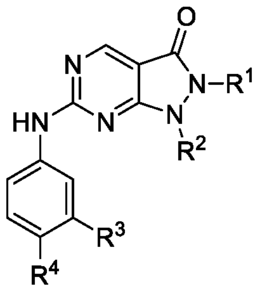

Formula I

wherein,

$R^1$ is selected from a group consisting of $-C_{1\sim6}$ alkyl, $-C_{2\sim6}$ alkenyl, $-C_{2\sim6}$ alkynyl, $-C_{0\sim2}$ alkylene-CN, $-C_{0\sim2}$ alkylene-(3~10-membered cycloalkyl), $-C_{0\sim2}$ alkylene-(3~10-membered heterocycloalkyl);

$R^2$ is selected from a group consisting of

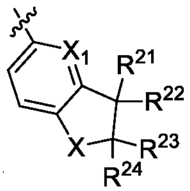 , 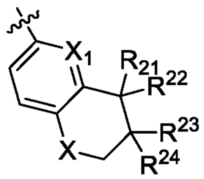 , 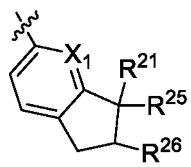 ,

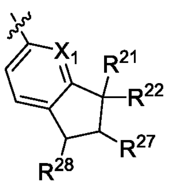 , 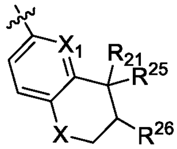 , 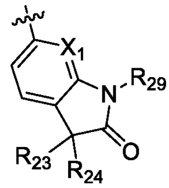 , 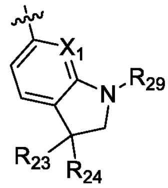 , 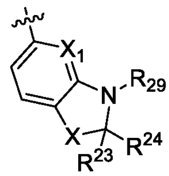 , 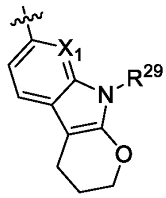 ,

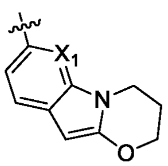 , 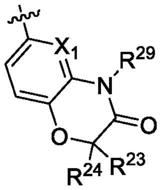 , 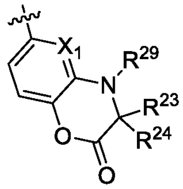 , 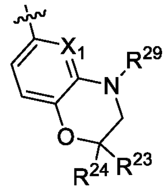 , 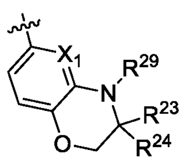 , 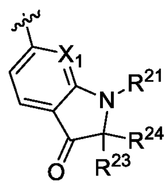 ,

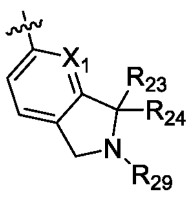 ;

X is selected from a group consisting of O, NH or $CH_2$;
$X_1$ is selected from a group consisting of CH or N;
$R^{21}$, $R^{22}$, $R^{29}$ are independently selected from a group consisting of hydrogen, deuterium, halogen, cyano, nitro, -OH, -$C_{1\sim6}$ alkyl, halogen-substituted $C_{1\sim6}$ alkyl, -$C_{0\sim2}$ alkylene-OH, -O($C_{1\sim6}$ alkyl), -O(halogen-substituted $C_{1\sim6}$ alkyl), -$NH_2$, -$C_{0\sim2}$ alkylene-NH($C_{1\sim6}$ alkyl), -$C_{0\sim2}$ alkylene-N($C_{1\sim6}$ alkyl) ($C_{1\sim6}$ alkyl), -$C_{0\sim2}$ alkylene-(3~10-membered cycloalkyl), -$C_{0\sim2}$ alkylene-(3~10-membered heterocycloalkyl);
$R^{23}$, $R^{24}$ together with the atom adjacent therewith form 3~10-membered carbocyclyl, 3~10-membered heterocyclyl;
$R^{25}$, $R^{26}$ together with the atom adjacent therewith form 3~10-membered carbocyclyl, 3~10-membered heterocyclyl;
$R^{27}$, $R^{28}$ together with the atom adjacent therewith form 3~10-membered carbocyclyl, 3~10-membered heterocyclyl;
$R^3$ is selected from a group consisting of hydrogen, deuterium, halogen, cyano, nitro, -$C_{1\sim6}$ alkyl, halogen-substituted $C_{1\sim6}$ alkyl, -$C_{0\sim2}$ alkylene-OH, -O($C_{1\sim6}$ alkyl), -O(halogen-substituted $C_{1\sim6}$ alkyl), -$NH_2$, -$C_{0\sim2}$ alkylene-NH($C_{1\sim6}$ alkyl), -$C_{0\sim2}$ alkylene-N($C_{1\sim6}$ alkyl) ($C_{1\sim6}$ alkyl);
$R^4$ is selected from a group consisting of 3~12-membered heterocycloalkyl; the heterocycloalkyl is optionally substituted by one, two, three or four independent $R^{41}$;
$R^{41}$ is selected from a group consisting of hydrogen, halogen, cyano, nitro, -OH, -$C_{1\sim6}$ alkyl, halogen-substituted $C_{1\sim6}$ alkyl, -$C_{0\sim2}$ alkylene-OH, -O($C_{1\sim6}$ alkyl), -O(halogen-substituted $C_{1\sim6}$ alkyl), -$NH_2$, -$C_{0\sim2}$ alkylene-NH($C_{1\sim6}$ alkyl), -$C_{0\sim2}$ alkylene-N($C_{1\sim6}$ alkyl) ($C_{1\sim6}$ alkyl), -C(O)$C_{1\sim6}$ alkyl, 3~10-membered carbocyclyl, 3~10-membered heterocyclyl; the carbocyclyl, heterocyclyl are optionally substituted by one, two, three or four independent $R^{31}$;
or, $R^3$, $R^4$ together with the atom adjacent therewith form 3~10-membered carbocyclyl, 3~10-membered heterocyclyl; said carbocyclyl, heterocycloalkyl is optionally substituted by one, two, three or four independent $R^{31}$;
$R^{31}$ is selected from a group consisting of hydrogen, halogen, cyano, nitro, -OH, -$C_{1\sim6}$ alkyl, halogen-substituted $C_{1\sim6}$ alkyl, -$C_{0\sim2}$ alkylene-OH, -O($C_{1\sim6}$ alkyl), -O(halogen-substituted $C_{1\sim6}$ alkyl), -$NH_2$, -$C_{0\sim2}$ alkylene-NH($C_{1\sim6}$ alkyl), -$C_{0\sim2}$ alkylene-N($C_{1\sim6}$ alkyl) ($C_{1\sim6}$ alkyl).

**2.** The compound, or a stereoisomer thereof, or a pharmaceutically acceptable salt thereof according to claim 1, wherein $R^1$ is selected from a group consisting of

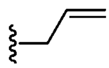
,

methyl, ethyl,

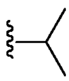
, 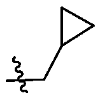
.

**3.** The compound, or a stereoisomer thereof, or a pharmaceutically acceptable salt thereof according to claim 1, wherein: $R^{21}$, $R^{22}$, $R^{29}$ are independently selected from a group consisting of hydrogen, deuterium, cyano, methyl, ethyl, -OH, trifluoromethyl, cyclopropyl, -$CH_2$OH, -$NH_2$.

**4.** The compound, or a stereoisomer thereof, or a pharmaceutically acceptable salt thereof according to claim 1, wherein:

$R^{23}$, $R^{24}$ together with the atom adjacent therewith form cyclopropyl, cyclobutyl, cyclopentyl;
$R^{25}$, $R^{26}$ together with the atom adjacent therewith form cyclopropyl, cyclobutyl, cyclopentyl;
$R^{27}$, $R^{28}$ together with the atom adjacent therewith form cyclopropyl, cyclobutyl, cyclopentyl.

**5.** The compound, or a stereoisomer thereof, or a pharmaceutically acceptable salt thereof according to any one of claims 1, 3 or 4, wherein $R^2$ is selected from a group consisting of

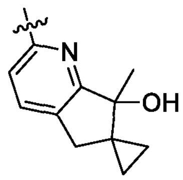 , 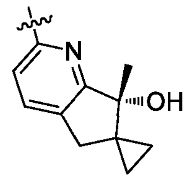 , 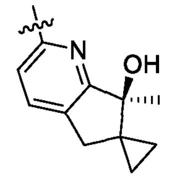 , 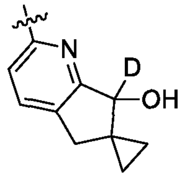 , 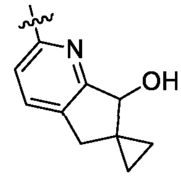 , 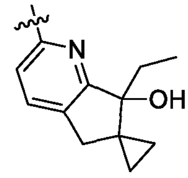 ,

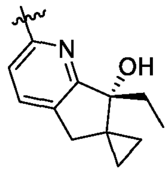 , 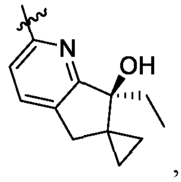 , 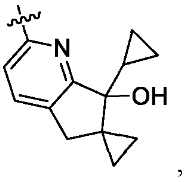 , 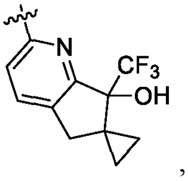 , 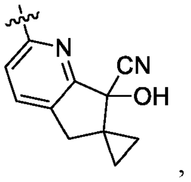 , 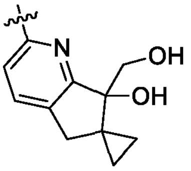 ,

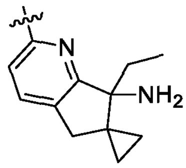 , 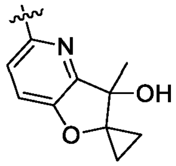 , 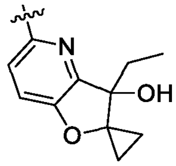 , 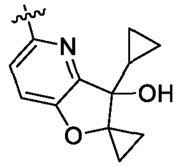 , 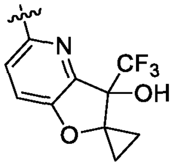 , 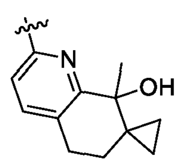 ,

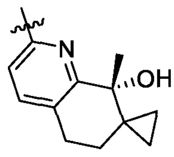 , 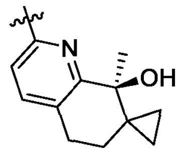 , 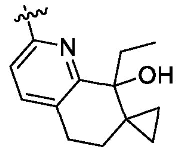 , 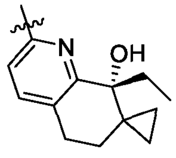 , 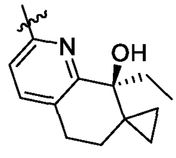 , 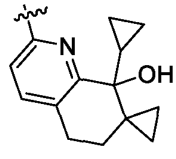 ,

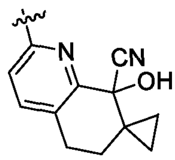 , 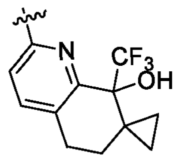 , 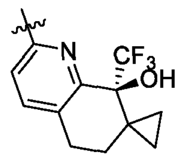 , 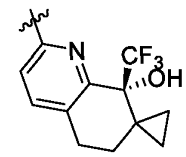 , 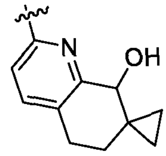 , 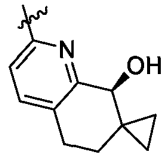 ,

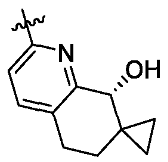 , 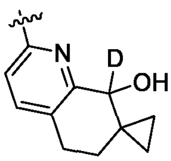 , 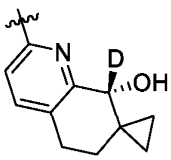 , 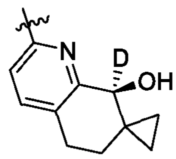 , 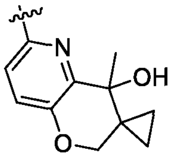 , 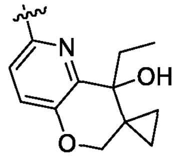 ,

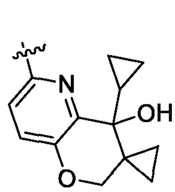 , 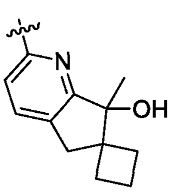 , 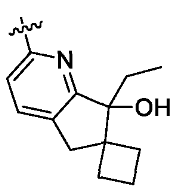 , 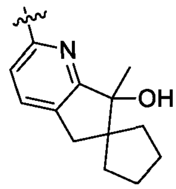 , , ,

, , , , , ,

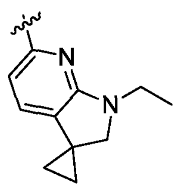 , 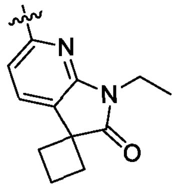 , 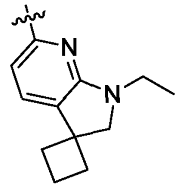 , 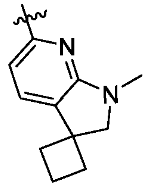 , 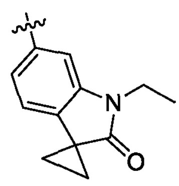 , 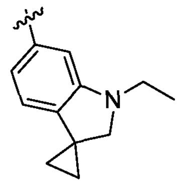 ,

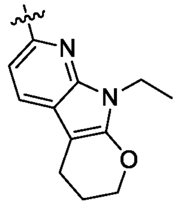 , 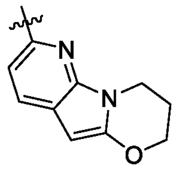 , 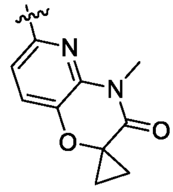 , 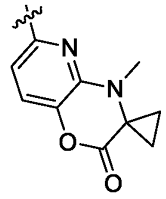 , 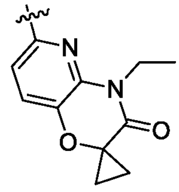 , 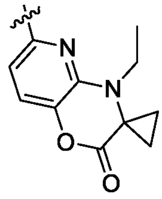 ,

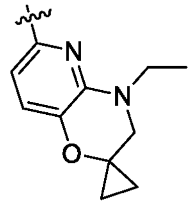 , 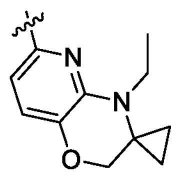 , 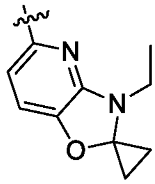 , 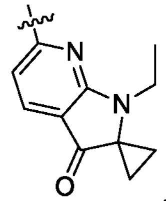 , 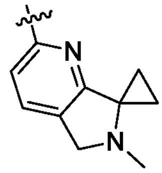 .

6. The compound, or a stereoisomer thereof, or a pharmaceutically acceptable salt thereof according to claim 1, wherein $R^3$ is selected from a group consisting of hydrogen, fluoro, methyl, $-CH_2OH$, methoxy.

7. The compound, or a stereoisomer thereof, or a pharmaceutically acceptable salt thereof according to claim 1, wherein $R^4$ is selected from a group consisting of nitrogen-containing 6-membered heterocyclyl, 7-membered nitrogen-containing bridged-ring, 8-membered nitrogen-containing bridged-ring, 9-membered nitrogen-containing heterospiro-ring, 11-membered nitrogen-containing heterospiro-ring.

8. The compound, or a stereoisomer thereof, or a pharmaceutically acceptable salt thereof according to claim 7, wherein $R^4$ is selected from a group consisting of

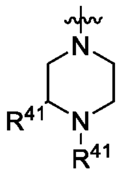 , 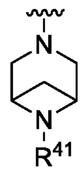 , 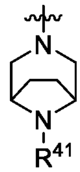 ,

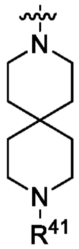 , 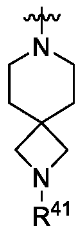 , 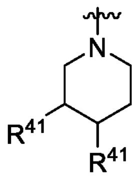 , 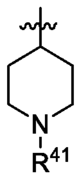 , 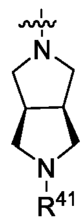 , 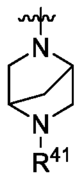 , 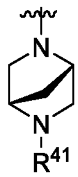 , 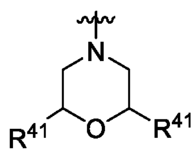 ,

wherein $R^{41}$ according to calim 1.

9. The compound, or a stereoisomer thereof, or a pharmaceutically acceptable salt thereof according to claim 8, wherein $R^4$ is selected from a group consisting of

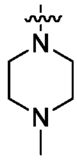, 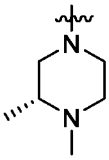, 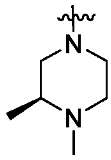,

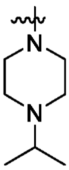, 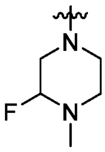, 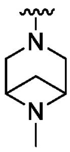, 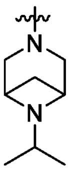, 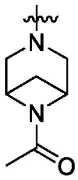, 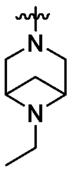, 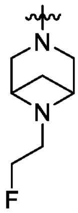, 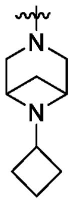, 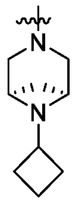, 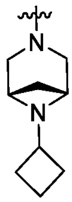, 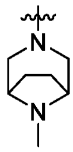, 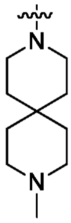,

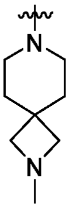, 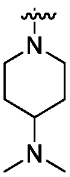, 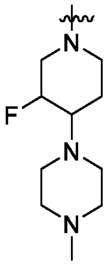, 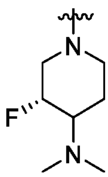, 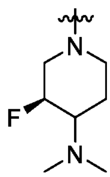, 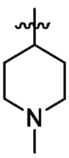, 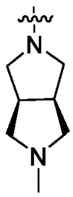, 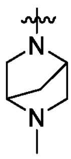, 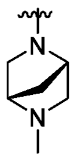, 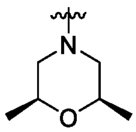, 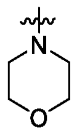.

**10.** The compound, or a stereoisomer thereof, or a pharmaceutically acceptable salt thereof according to claim 1, wherein $R^3$, $R^4$ together with the atom adjacent therewith form 6-membered nitrogen-containing heterocyclyl.

**11.** The compound, or a stereoisomer thereof, or a pharmaceutically acceptable salt thereof according to claim 10, wherein $R^3$, $R^4$ together with the atom adjacent therewith form

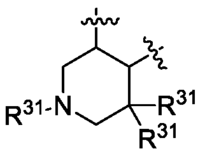, 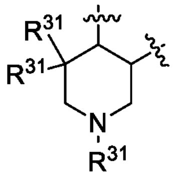

wherein $R^{31}$ according to calim 1.

**12.** The compound, or a stereoisomer thereof, or a pharmaceutically acceptable salt thereof according to claim 11, wherein $R^{31}$ is selected from a group consisting of methyl.

**13.** The compound, or a stereoisomer thereof, or a pharmaceutically acceptable salt thereof according to any one of claims 1, wherein:
the compound represented by Formula I is specifically:

| No. | Compound Structure | No. | Compound Structure |
|---|---|---|---|
| WEE1-001 | 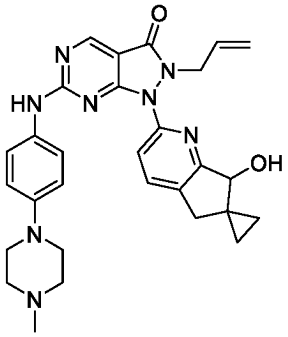 | WEE1-002 | 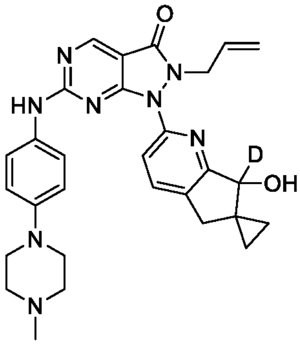 |
| WEE1-003 | 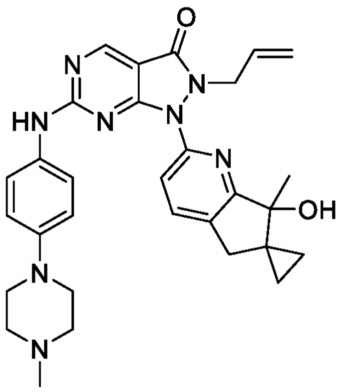 | WEE1-004 | 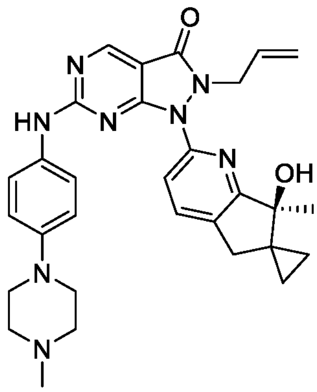 |
| WEE1-005 | 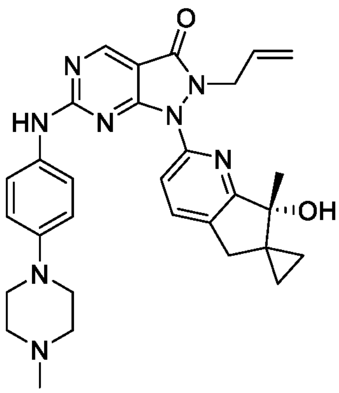 | WEE1-006 | 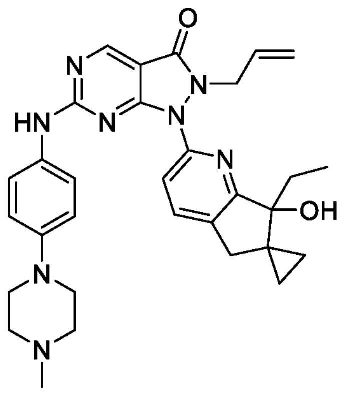 |
| WEE1-007 | 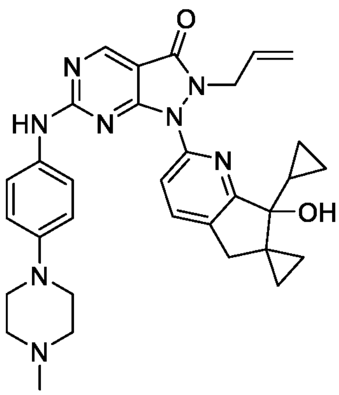 | WEE1-008 | 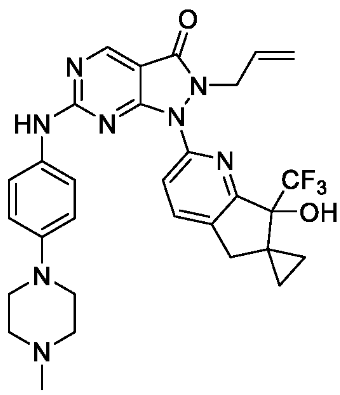 |

(continued)

| No. | Compound Structure | No. | Compound Structure |
|---|---|---|---|
| WEE1-009 | 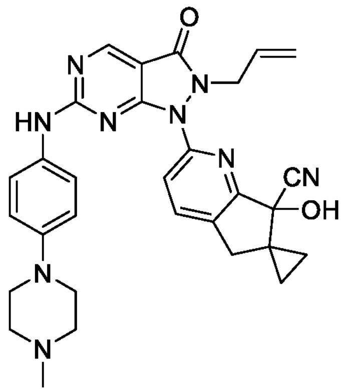 | WEE1-010 | 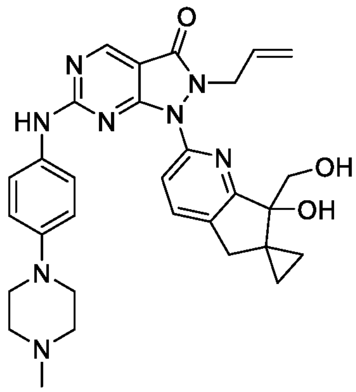 |
| WEE1-011 | 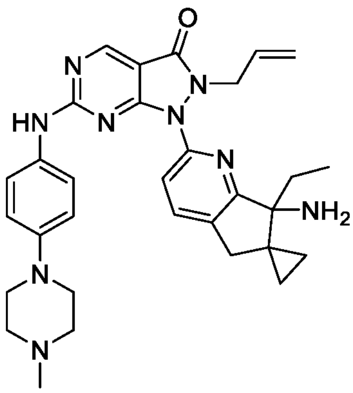 | WEE1-012 | 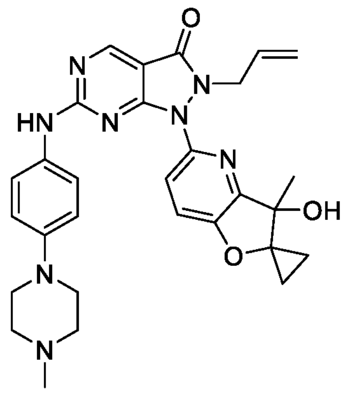 |
| WEE1-013 | 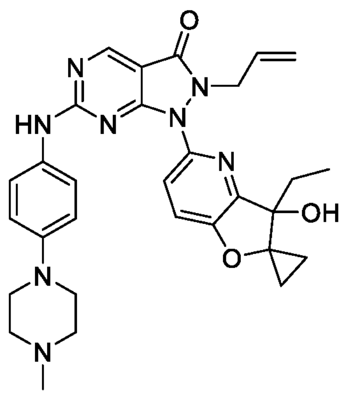 | WEE1-014 | 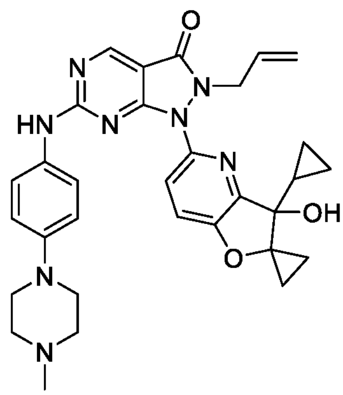 |
| WEE1-015 | 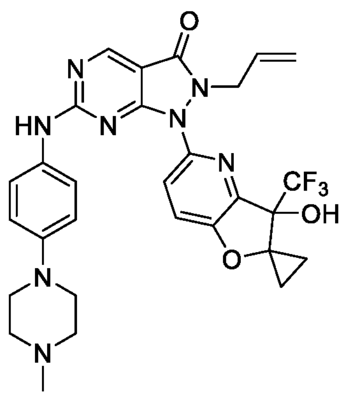 | WEE1-016 | 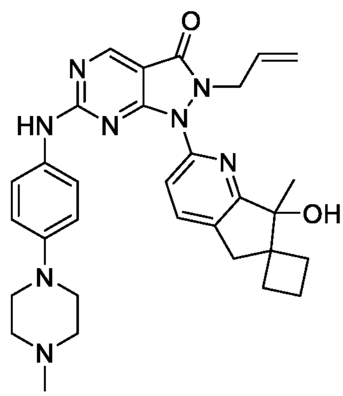 |

(continued)

| No. | Compound Structure | No. | Compound Structure |
|---|---|---|---|
| WEE1-017 | 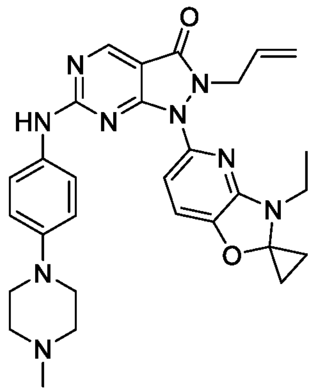 | WEE1-018 | 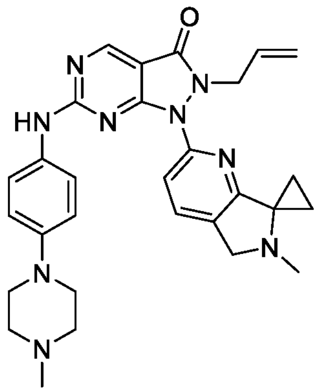 |
| WEE1-019 | 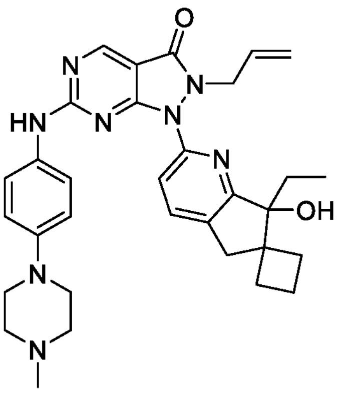 | WEE1-020 | 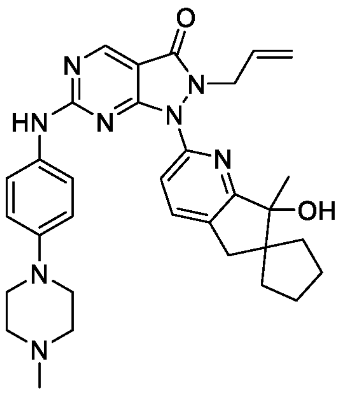 |
| WEE1-021 | 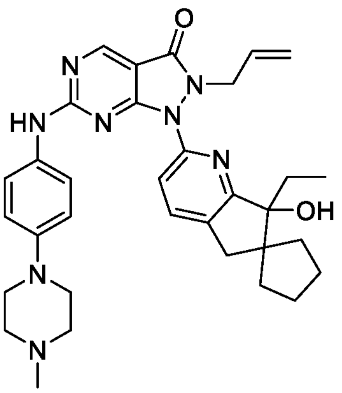 | WEE1-022 | 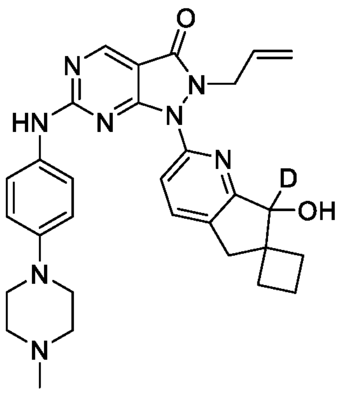 |
| WEE1-023 | 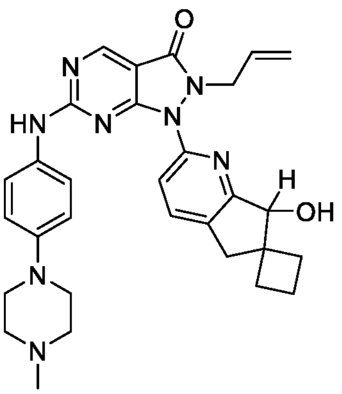 | WEE1-024 | 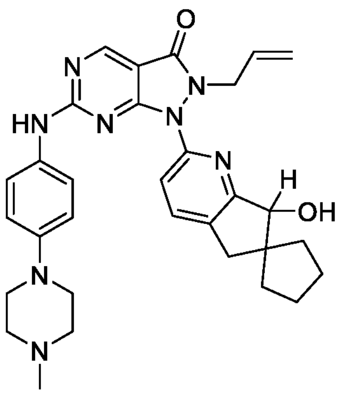 |

(continued)

| No. | Compound Structure | No. | Compound Structure |
|---|---|---|---|
| WEE1-025 | 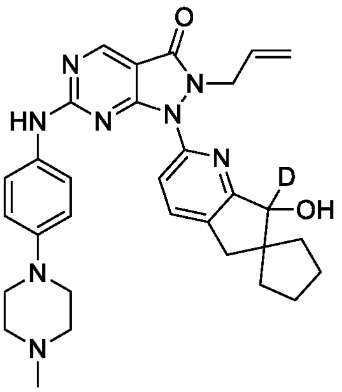 | WEE1-026 | 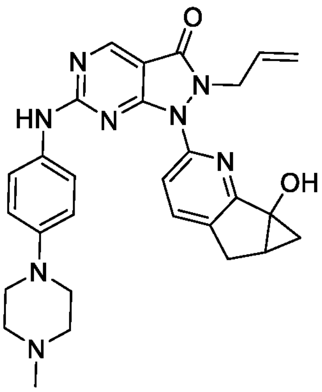 |
| WEE1-027 | 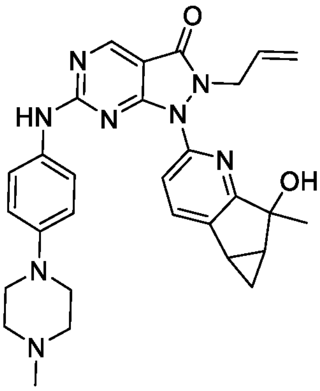 | WEE1-028 | 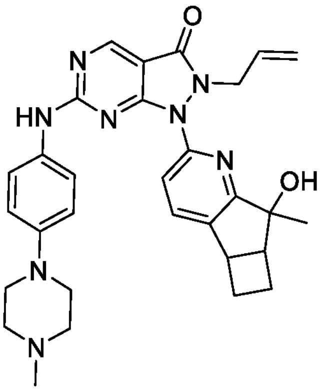 |
| WEE1-029 | 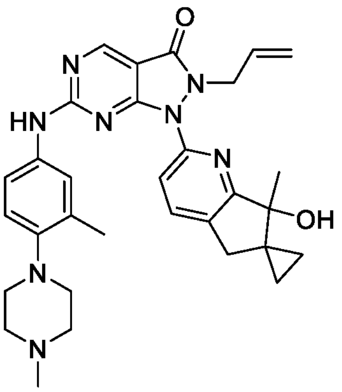 | WEE1-030 | 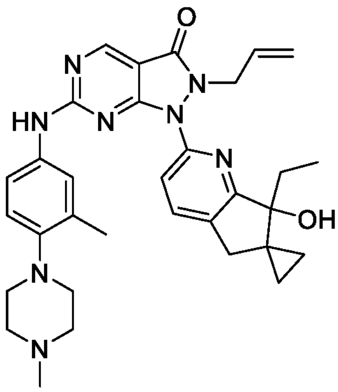 |
| WEE1-031 | 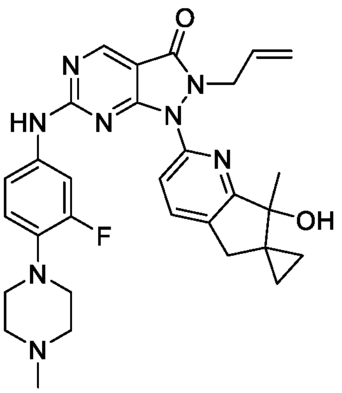 | WEE1-032 | 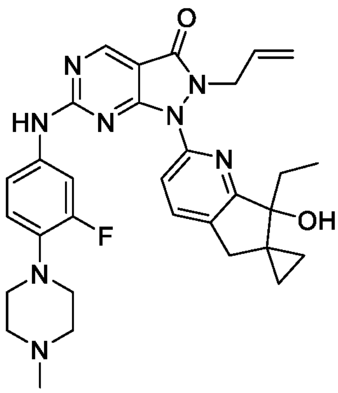 |

(continued)

| No. | Compound Structure | No. | Compound Structure |
|---|---|---|---|
| WEE1-033 | 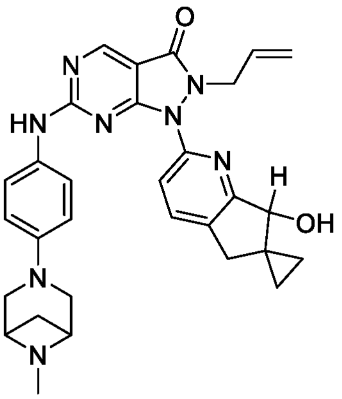 | WEE1-034 | 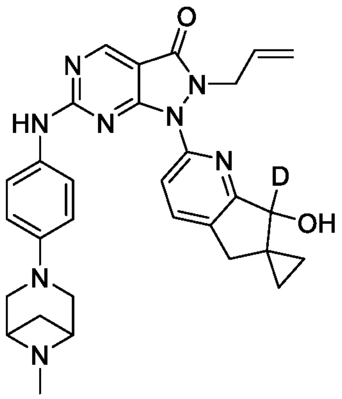 |
| WEE1-035 | 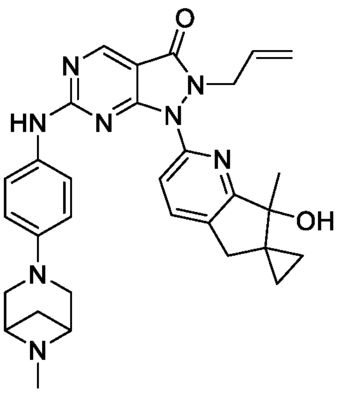 | WEE1-036 | 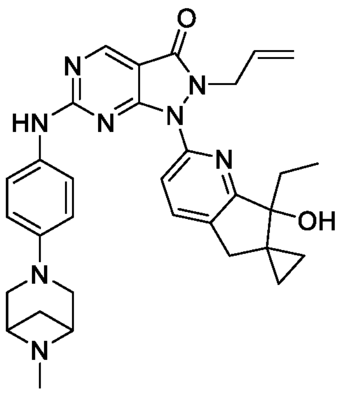 |
| WEE1-037 | 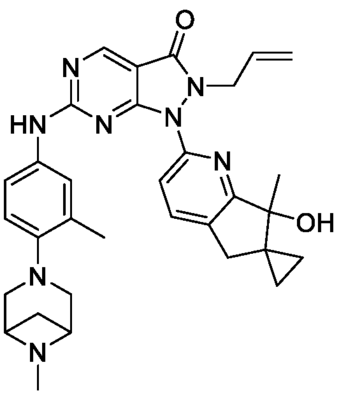 | WEE1-038 | 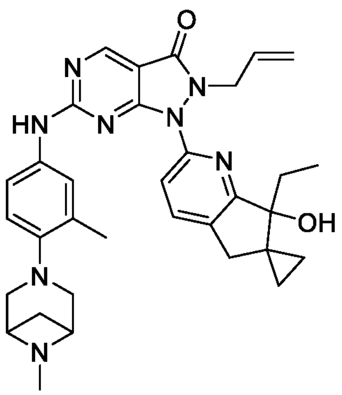 |
| WEE1-039 | 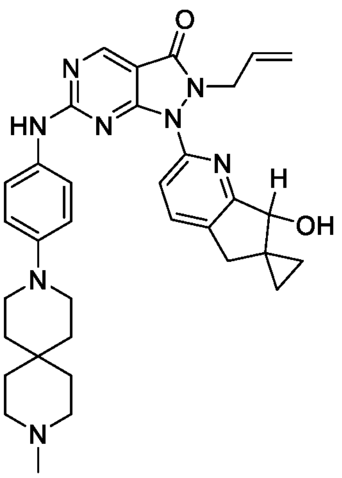 | WEE1-040 | 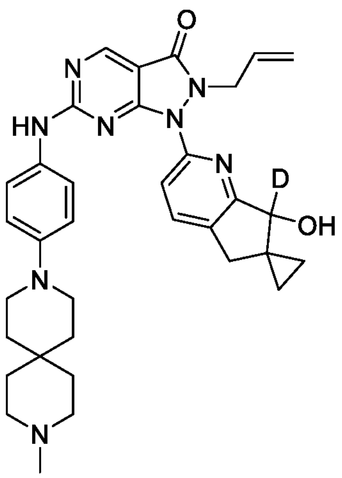 |

(continued)

| No. | Compound Structure | No. | Compound Structure |
|---|---|---|---|
| WEE1-041 | 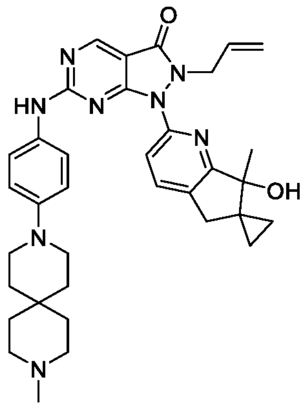 | WEE1-042 | 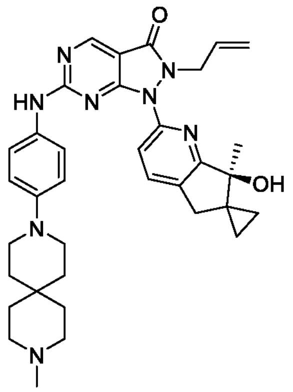 |
| WEE1-043 | 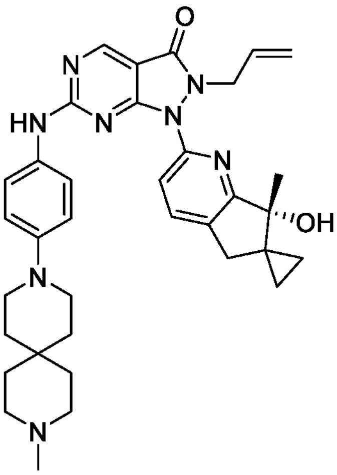 | WEE1-044 | 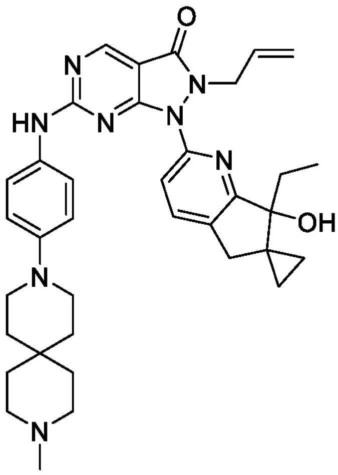 |
| WEE1-045 | 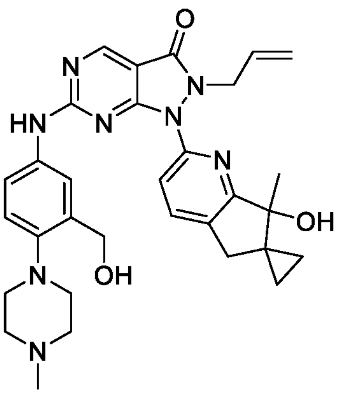 | WEE1-046 | 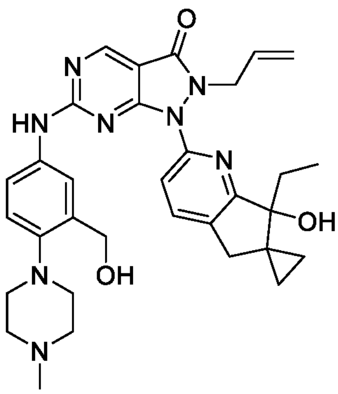 |

(continued)

| No. | Compound Structure | No. | Compound Structure |
|---|---|---|---|
| WEE1-047 | 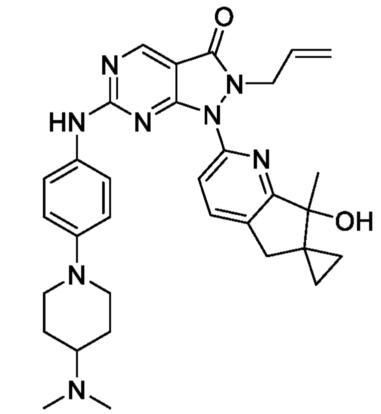 | WEE1-048 | 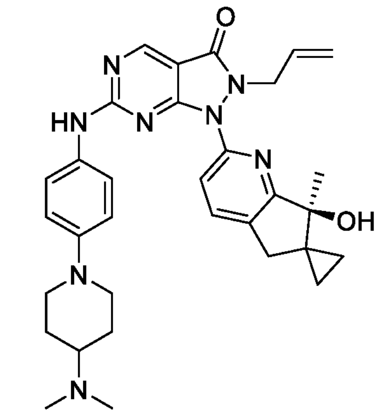 |
| WEE1-049 | 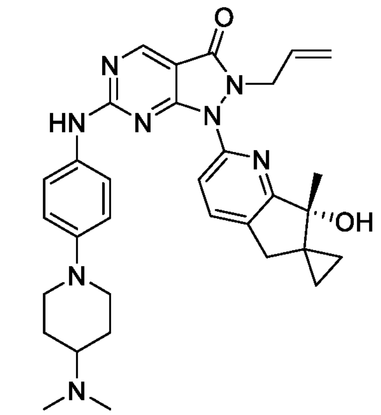 | WEE1-050 | 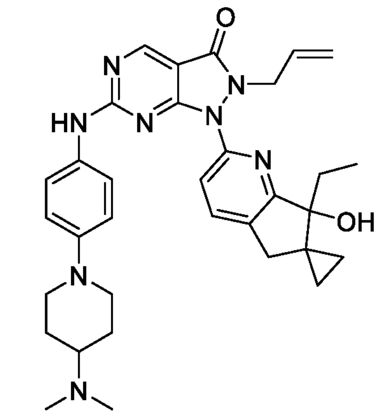 |
| WEE1-051 | 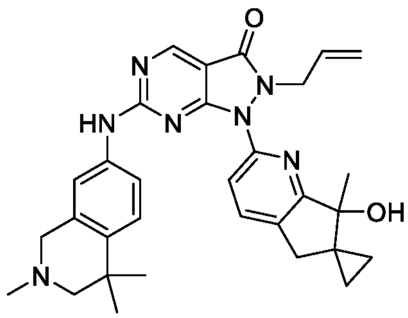 | WEE1-052 | 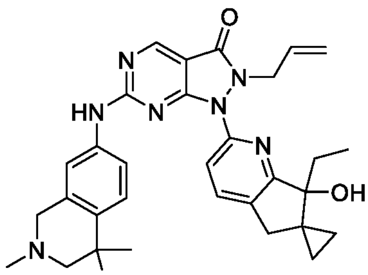 |
| WEE1-053 | 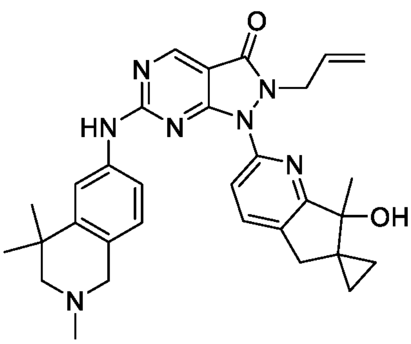 | WEE1-054 | 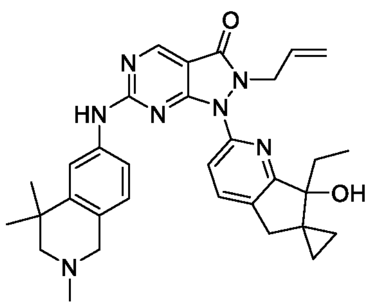 |

(continued)

| No. | Compound Structure | No. | Compound Structure |
|---|---|---|---|
| WEE1-055 | 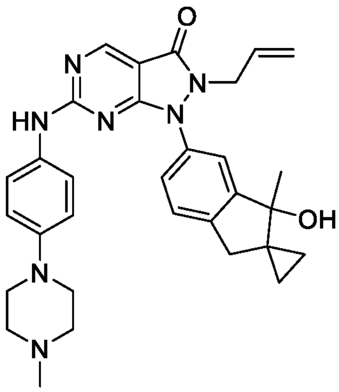 | WEE1-056 | 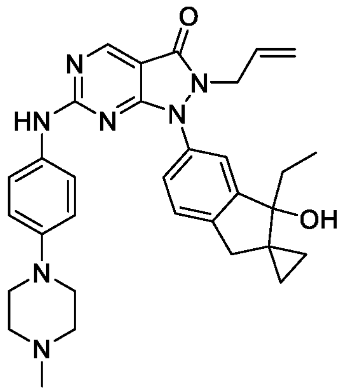 |
| WEE1-057 | 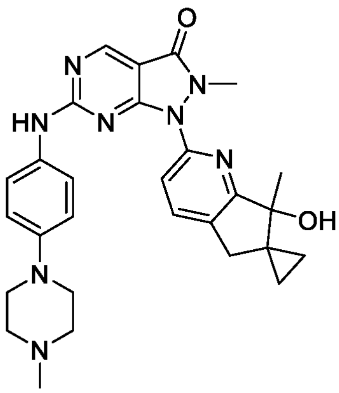 | WEE1-058 | 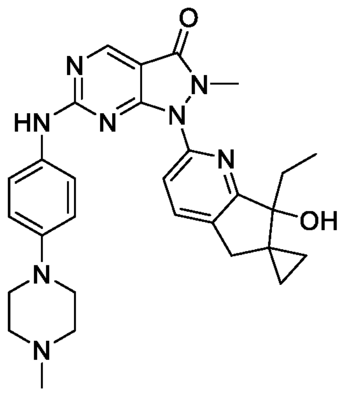 |
| WEE1-059 | 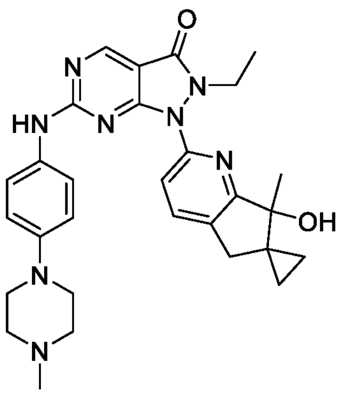 | WEE1-060 | 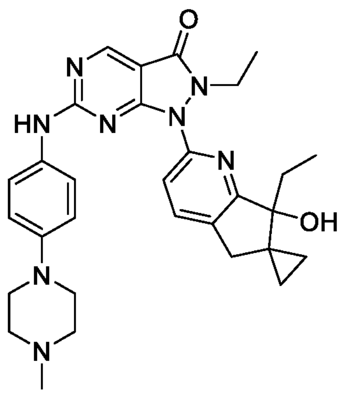 |
| WEE1-061 | 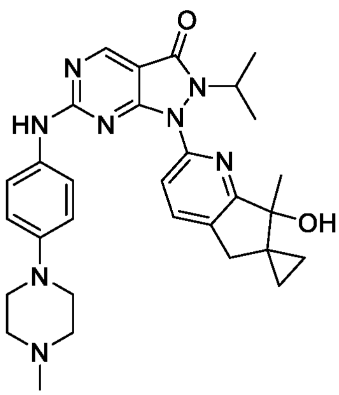 | WEE1-062 | 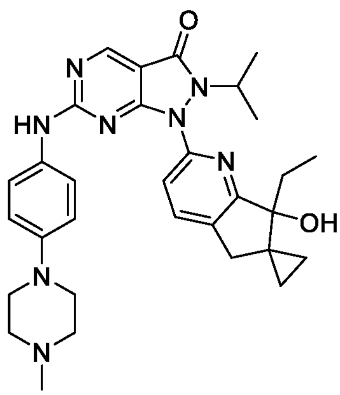 |

(continued)

| No. | Compound Structure | No. | Compound Structure |
|---|---|---|---|
| WEE1-063 | 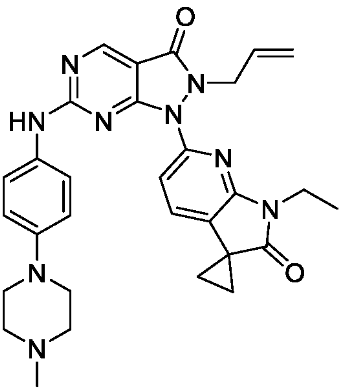 | WEE1-064 | 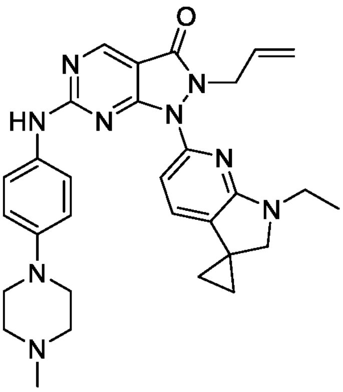 |
| WEE1-065 | 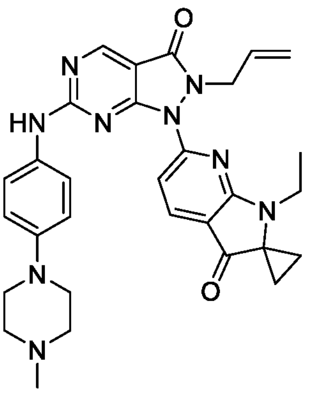 | WEE1-066 | 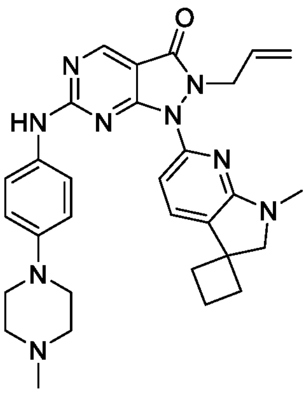 |
| WEE1-067 | 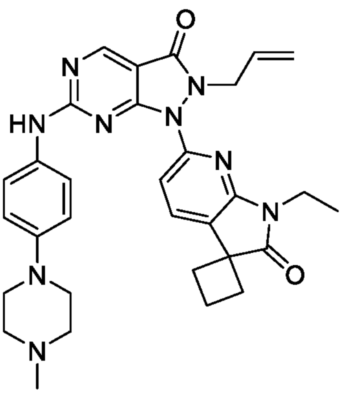 | WEE1-068 | 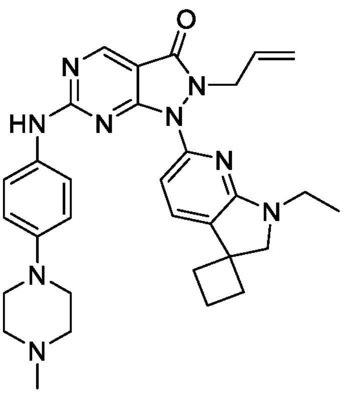 |
| WEE1-069 | 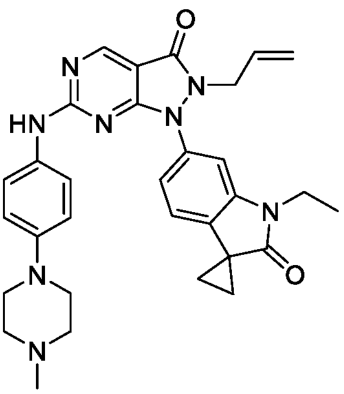 | WEE1-070 | 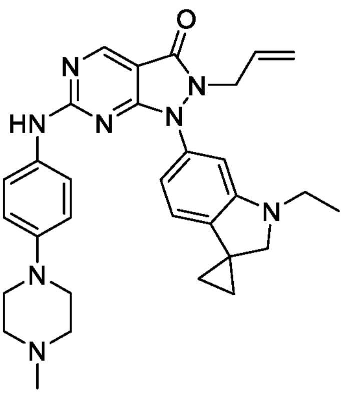 |

(continued)

| No. | Compound Structure | No. | Compound Structure |
|---|---|---|---|
| WEE1-071 | 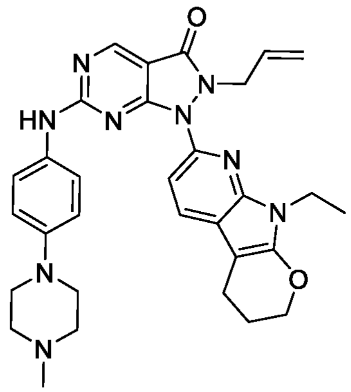 | WEE1-072 | 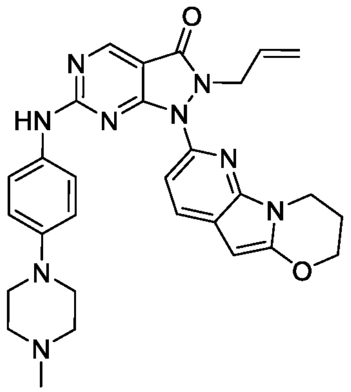 |
| WEE1-073 | 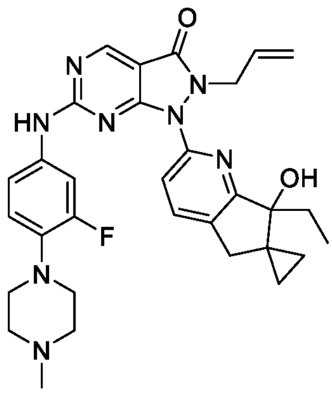 | WEE1-074 | 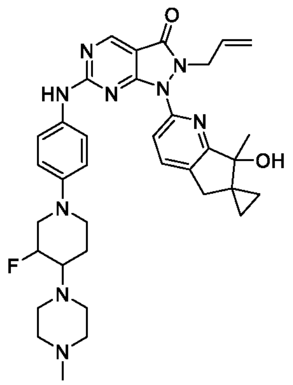 |
| WEE1-075 | 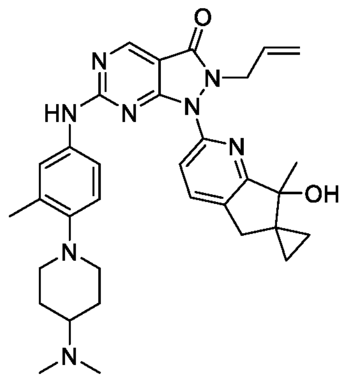 | WEE1-076 | 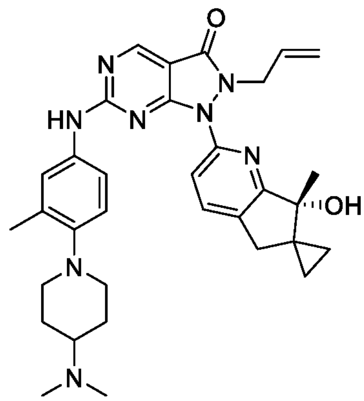 |
| WEE1-077 | 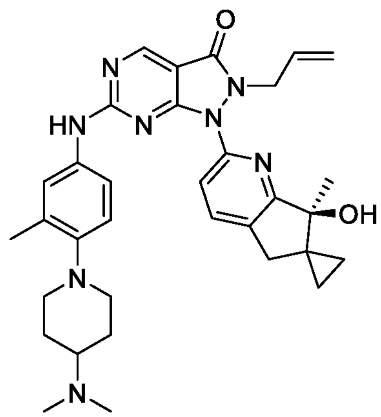 | WEE1-078 | 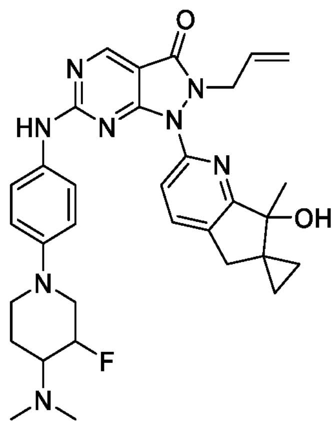 |

(continued)

| No. | Compound Structure | No. | Compound Structure |
|---|---|---|---|
| WEE1-079 | 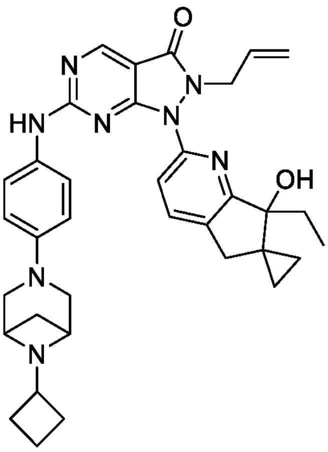 | WEE1-080 | 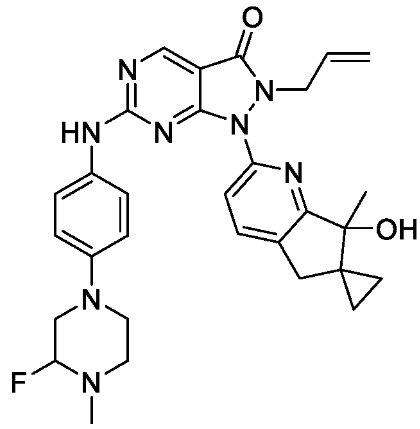 |
| WEE1-081 | 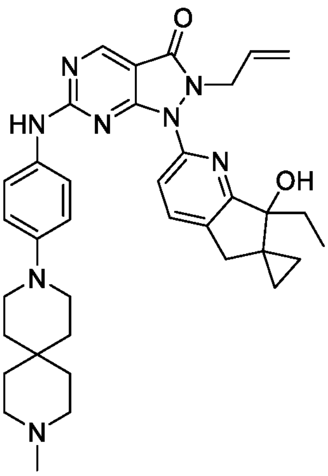 | WEE1-082 | 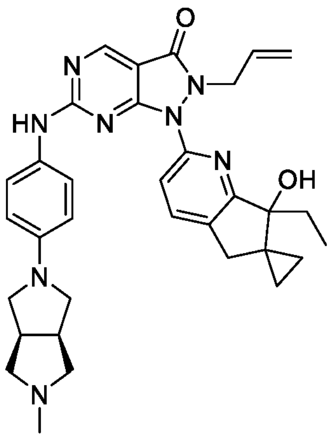 |
| WEE1-083 | 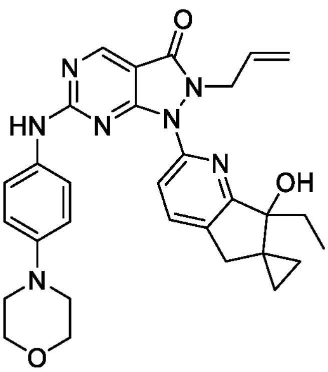 | WEE1-084 | 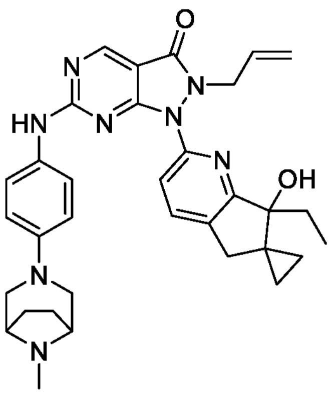 |

(continued)

| No. | Compound Structure | No. | Compound Structure |
|---|---|---|---|
| WEE1-085 | 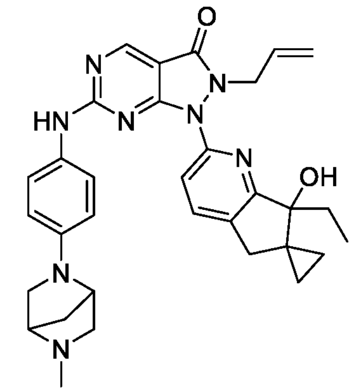 | WEE1-086 | 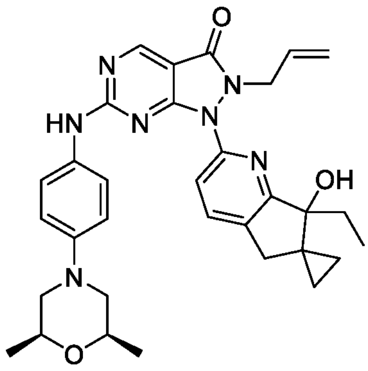 |
| WEE1-087 | 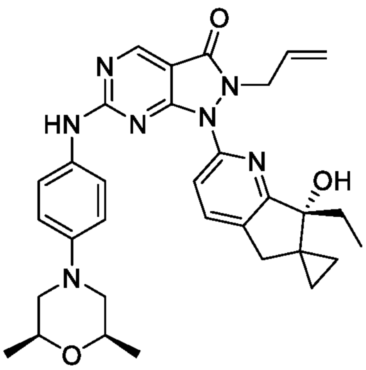 | WEE1-088 | 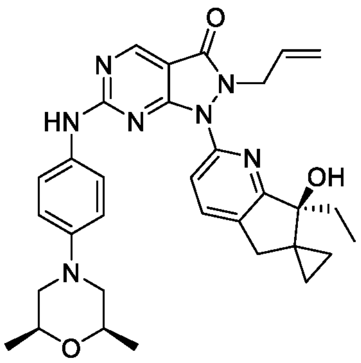 |
| WEE1-089 | 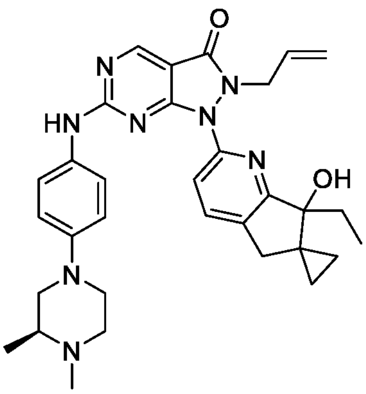 | WEE1-090 | 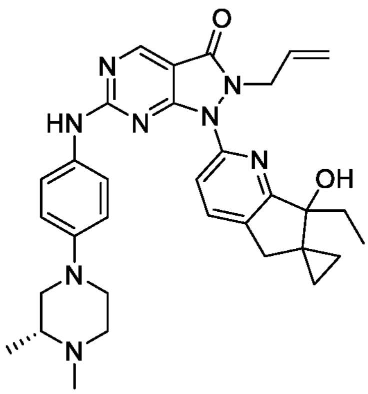 |
| WEE1-091 | 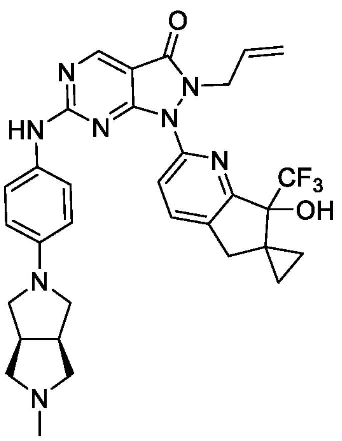 | WEE-092 | 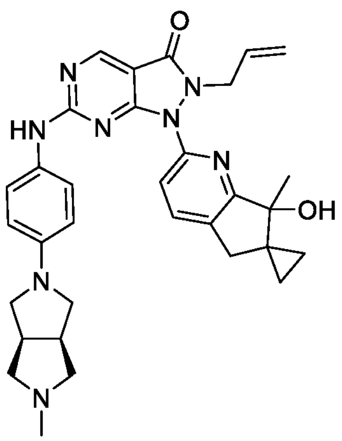 |

(continued)

| No. | Compound Structure | No. | Compound Structure |
|---|---|---|---|
| WEE1-093 | 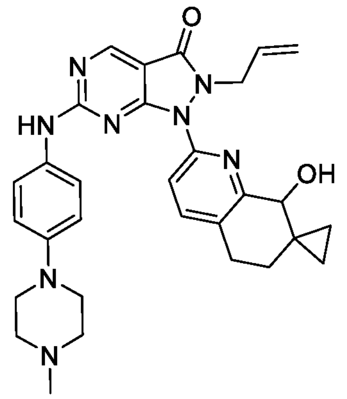 | WEE1-094 | 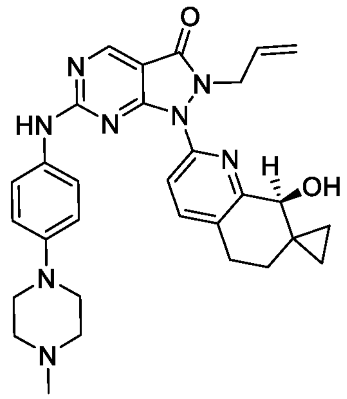 |
| WEE1-095 | 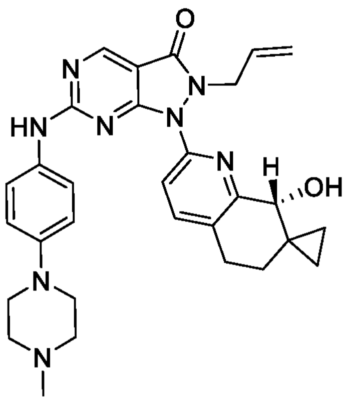 | WEE1-096 | 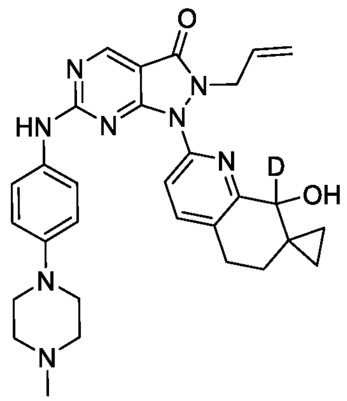 |
| WEE1-097 | 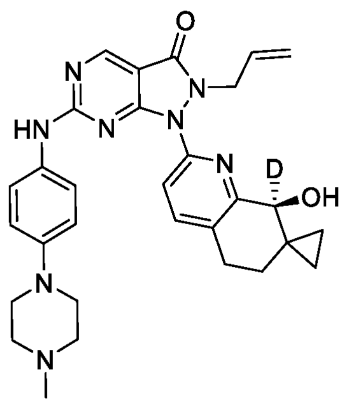 | WEE1-098 | 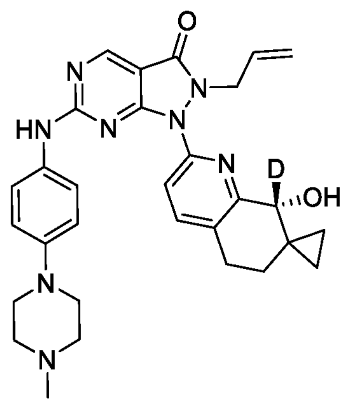 |
| WEE1-099 | 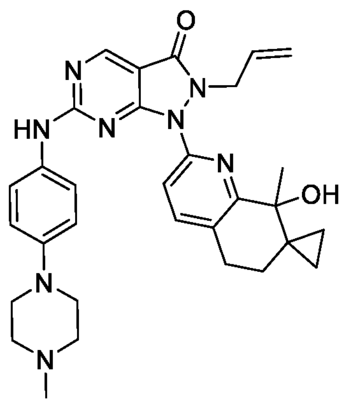 | WEE1-100 | 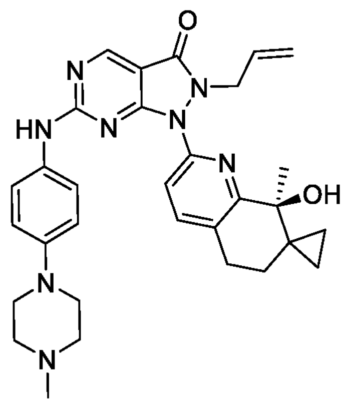 |

(continued)

| No. | Compound Structure | No. | Compound Structure |
|---|---|---|---|
| WEE1-101 | 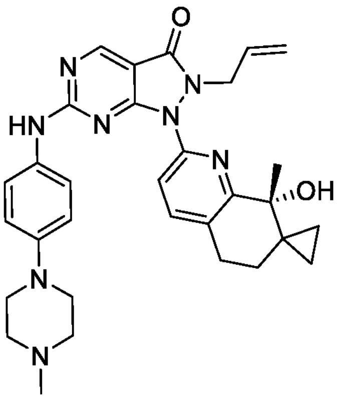 | WEE1-102 | 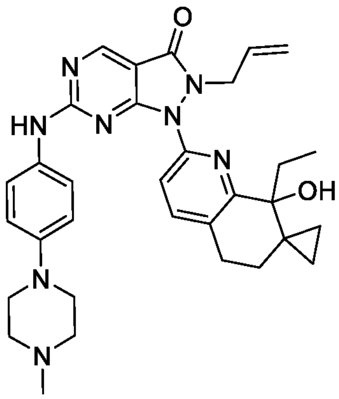 |
| WEE1-103 | 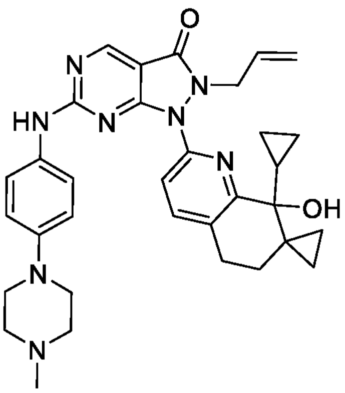 | WEE1-104 | 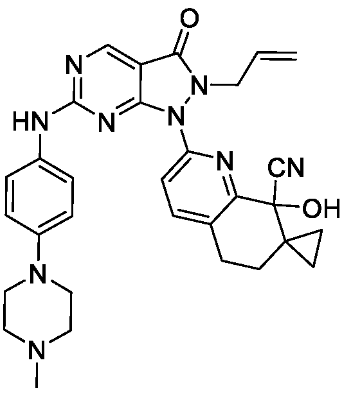 |
| WEE1-105 | 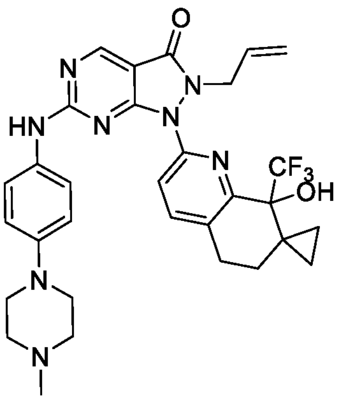 | WEE1-106 | 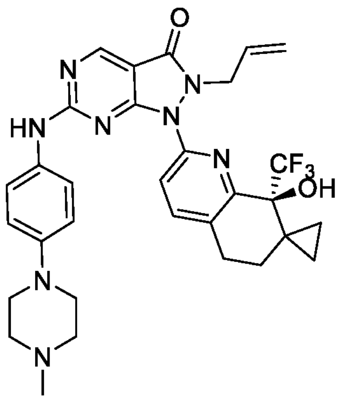 |
| WEE1-107 | 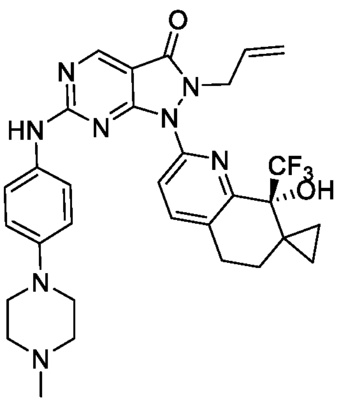 | WEE1-108 | 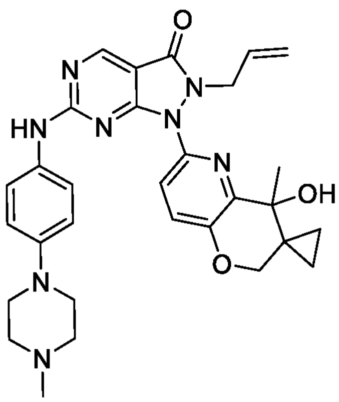 |

(continued)

| No. | Compound Structure | No. | Compound Structure |
|---|---|---|---|
| WEE1-109 | 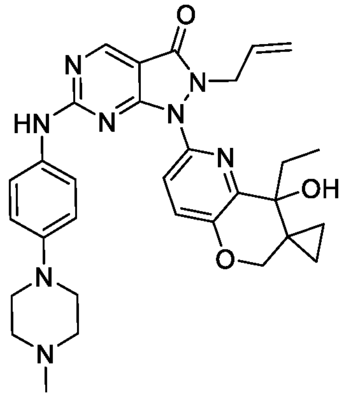 | WEE1-110 | 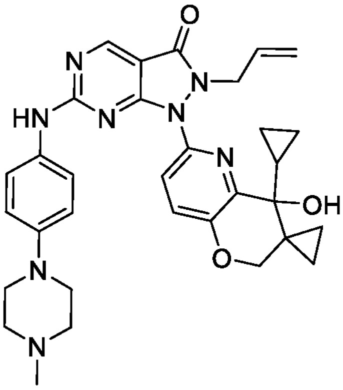 |
| WEE1-111 | 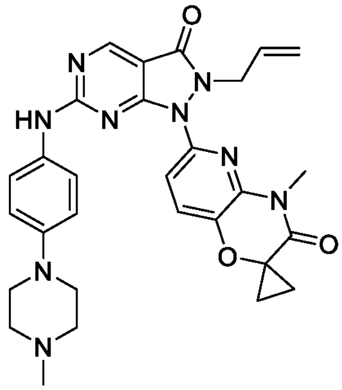 | WEE1-112 | 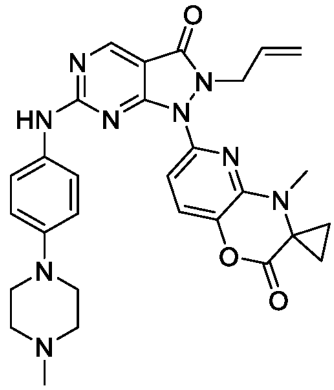 |
| WEE1-113 | 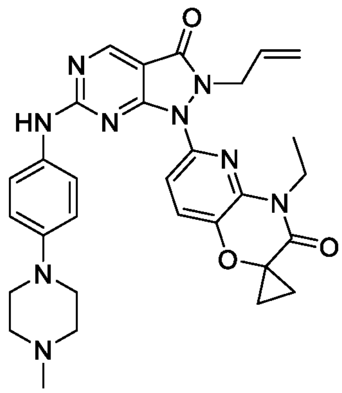 | WEE1-114 | 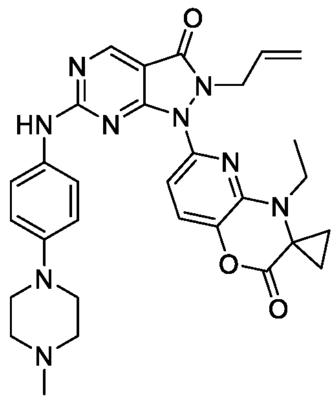 |
| WEE1-115 | 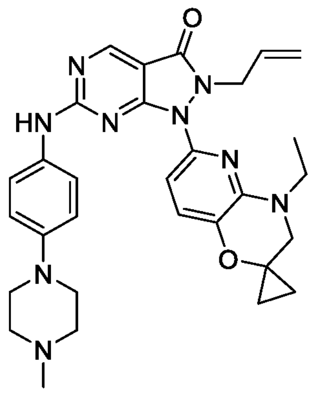 | WEE1-116 | 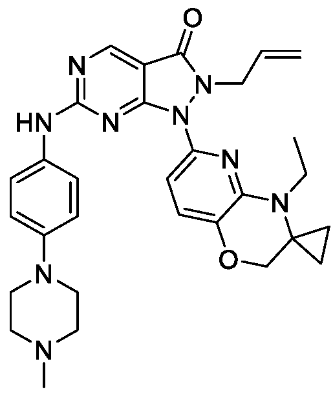 |

(continued)

| No. | Compound Structure | No. | Compound Structure |
|---|---|---|---|
| WEE1-117 | 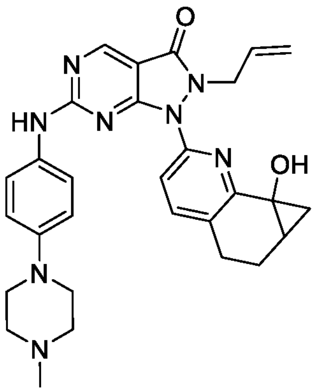 | WEE1-118 | 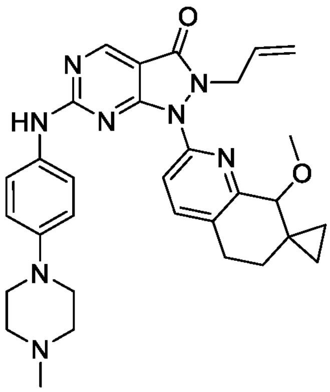 |
| WEE1-119 | 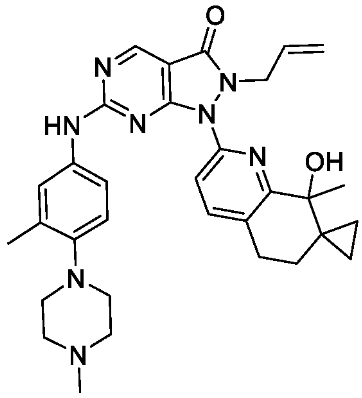 | WEE1-120 | 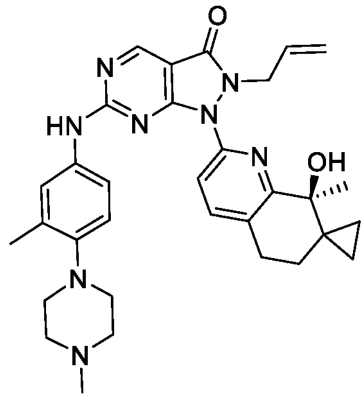 |
| WEE1-121 | 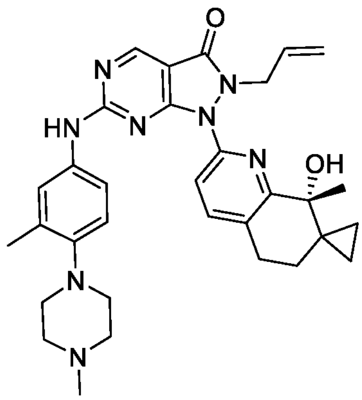 | WEE1-122 | 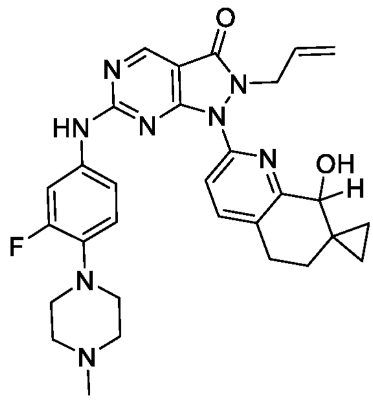 |
| WEE1-123 | 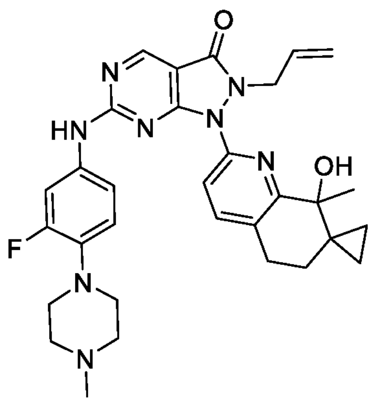 | WEE1-124 | 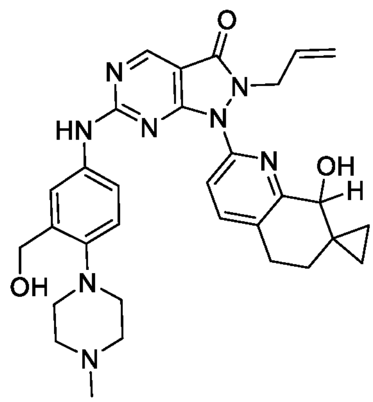 |

(continued)

| No. | Compound Structure | No. | Compound Structure |
|---|---|---|---|
| WEE1-125 | 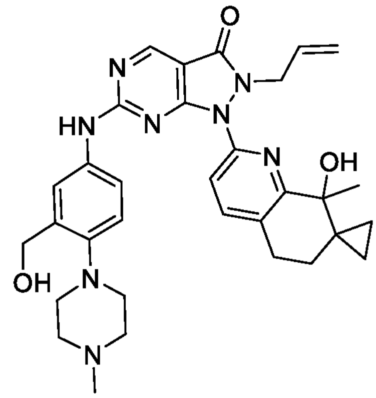 | WEE1-126 | 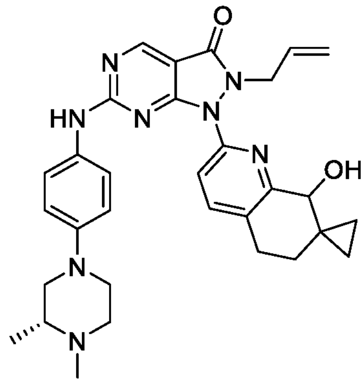 |
| WEE1-127 | 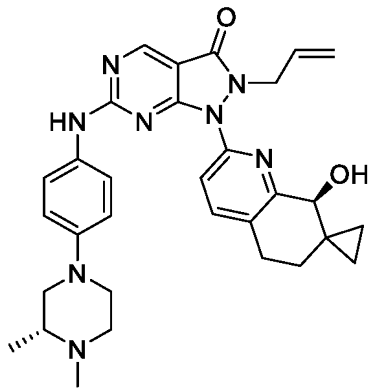 | WEE1-128 | 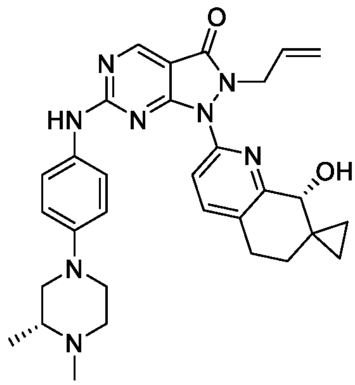 |
| WEE1-129 | 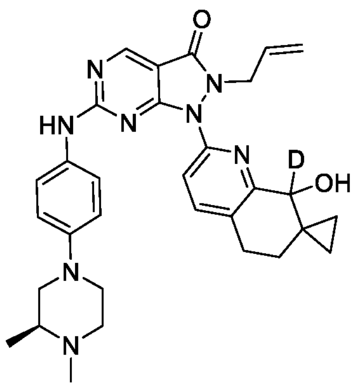 | WEE1-130 | 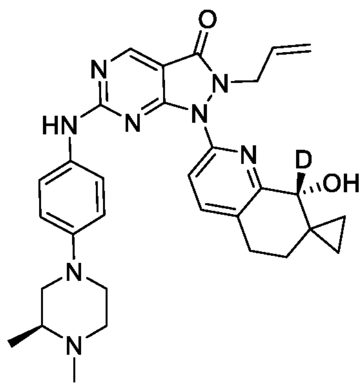 |
| WEE1-131 | 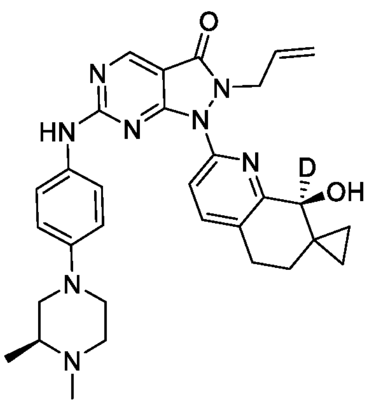 | WEE1-132 | 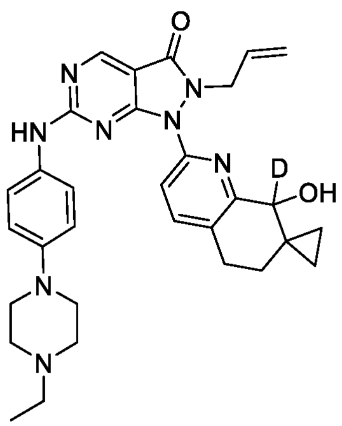 |

(continued)

| No. | Compound Structure | No. | Compound Structure |
|---|---|---|---|
| WEE1-133 | 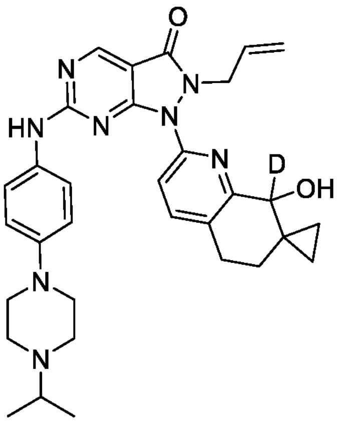 | WEE1-134 | 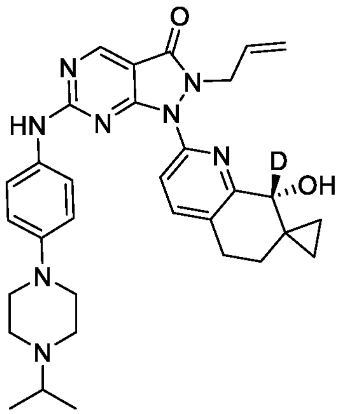 |
| WEE1-135 | 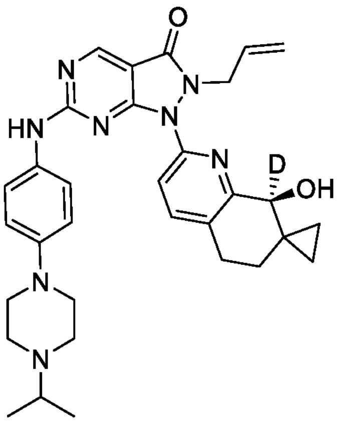 | WEE1-136 | 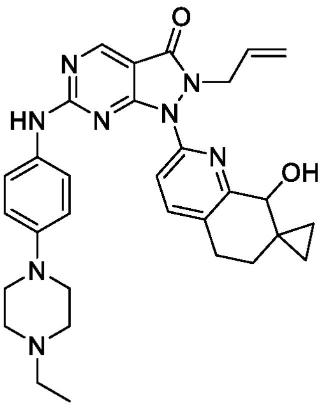 |
| WEE1-137 | 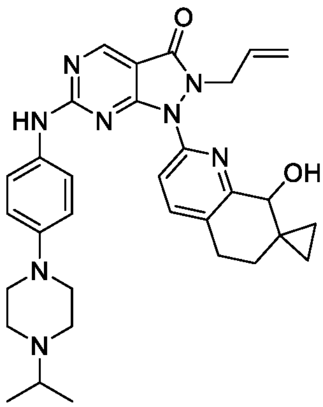 | WEE1-138 | 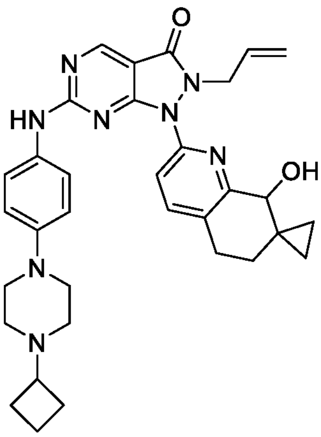 |

(continued)

| No. | Compound Structure | No. | Compound Structure |
|---|---|---|---|
| WEE1-139 | 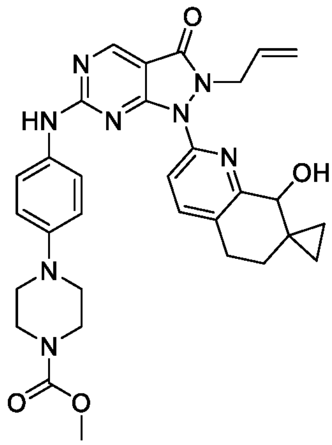 | WEE1-140 | 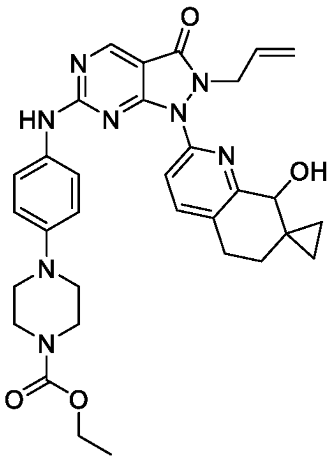 |
| WEE1-141 | 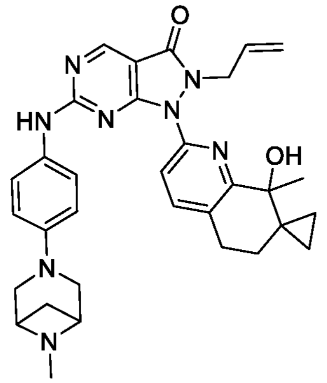 | WEE1-142 | 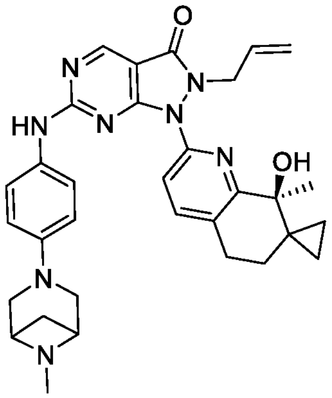 |
| WEE1-143 | 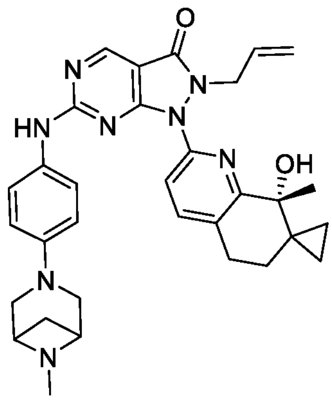 | WEE1-144 | 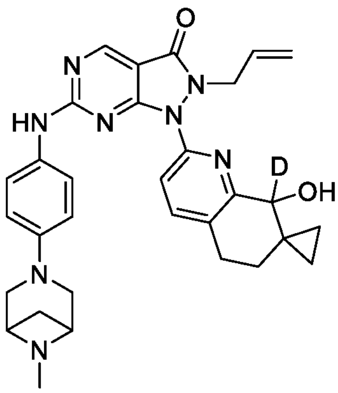 |
| WEE1-145 | 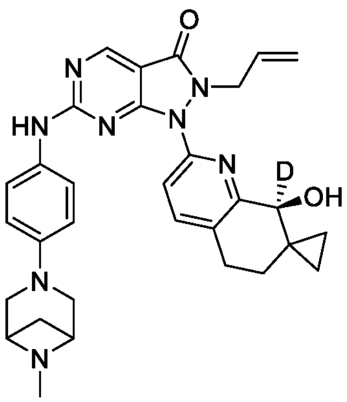 | WEE1-146 | 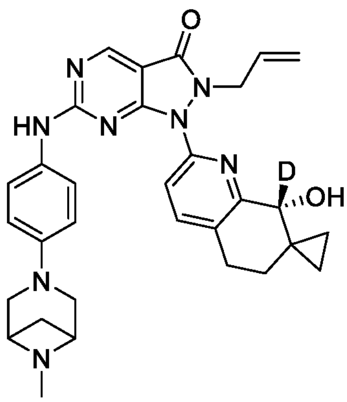 |

(continued)

| No. | Compound Structure | No. | Compound Structure |
|---|---|---|---|
| WEE1-147 | 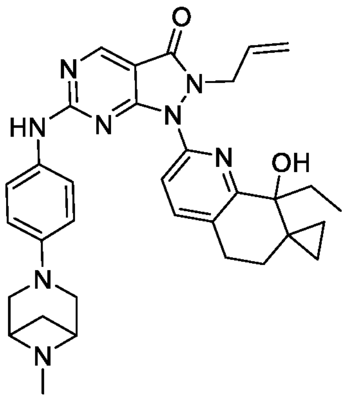 | WEE1-148 | 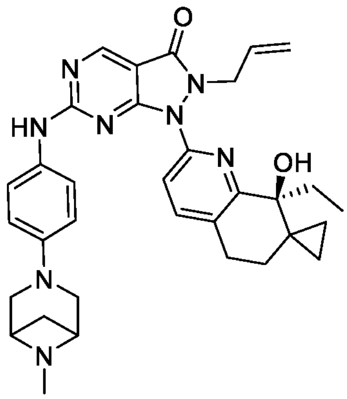 |
| WEE1-149 | 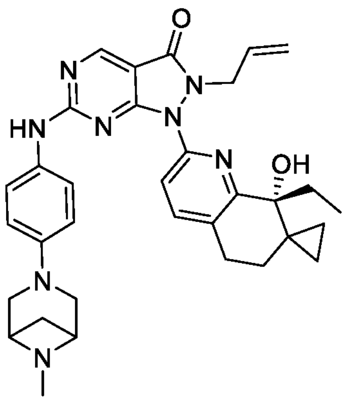 | WEE1-150 | 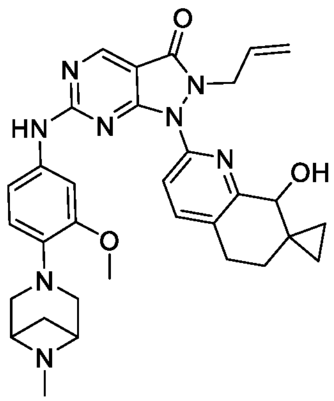 |
| WEE1-151 | 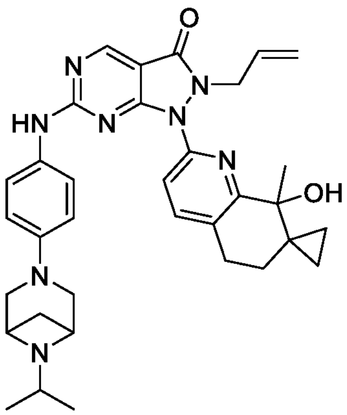 | WEE1-152 | 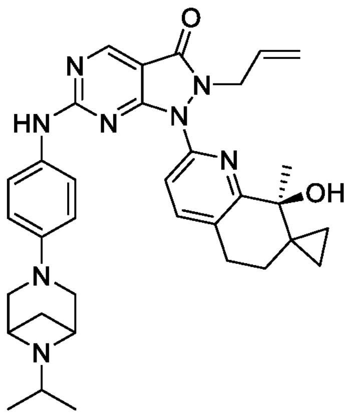 |
| WEE1-153 | 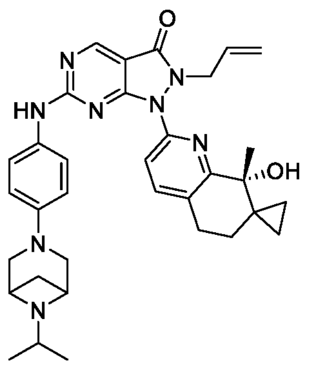 | WEE1-154 | 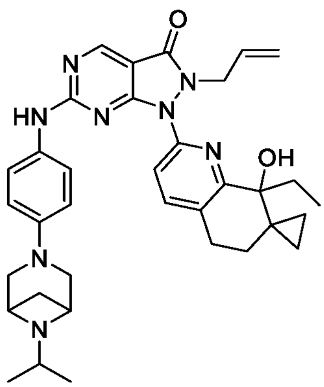 |

(continued)

| No. | Compound Structure | No. | Compound Structure |
|---|---|---|---|
| WEE1-155 | 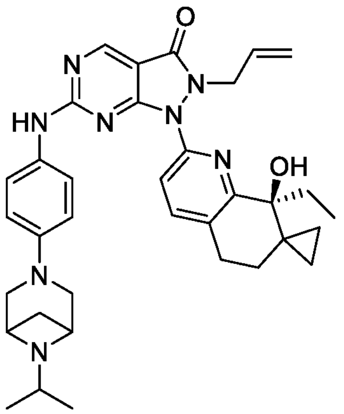 | WEE1-156 | 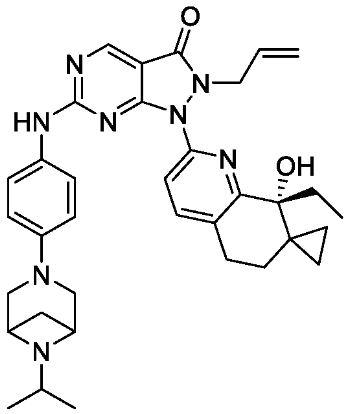 |
| WEE1-157 | 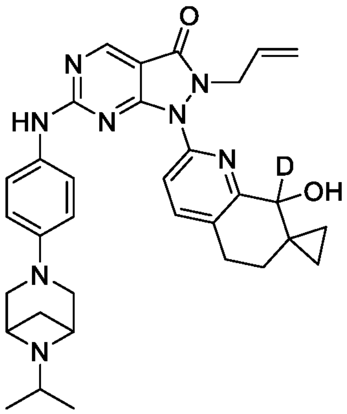 | WEE1-158 | 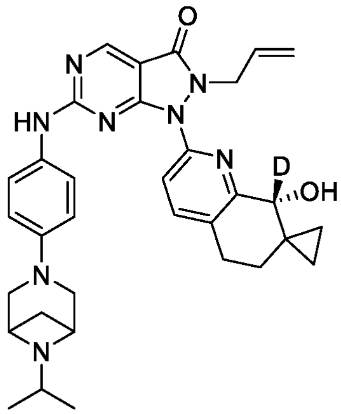 |
| WEE1-159 | 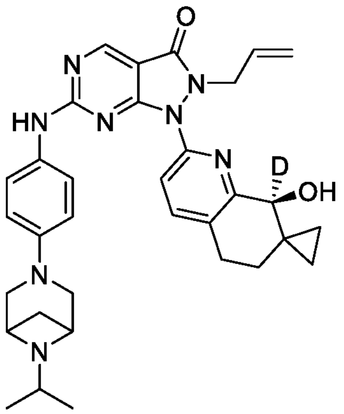 | WEE1-160 | 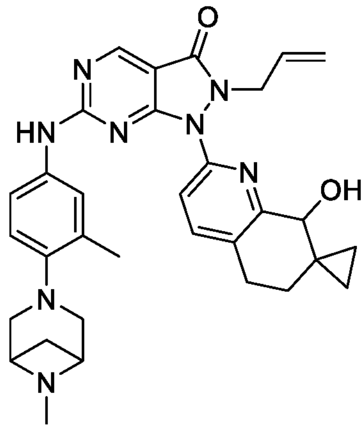 |

(continued)

| No. | Compound Structure | No. | Compound Structure |
|---|---|---|---|
| WEE1-161 | 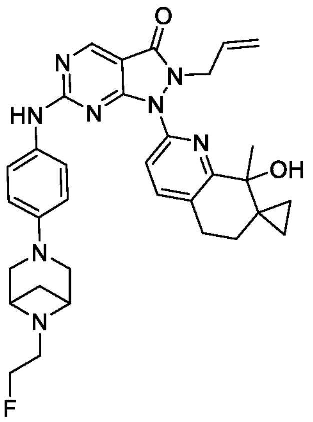 | WEE1-162 | 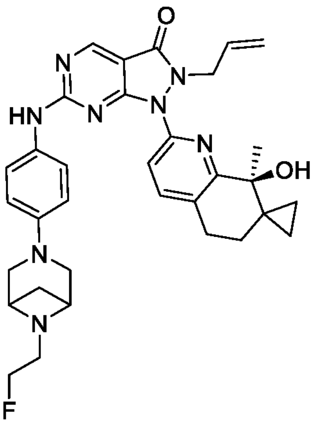 |
| WEE1-163 | 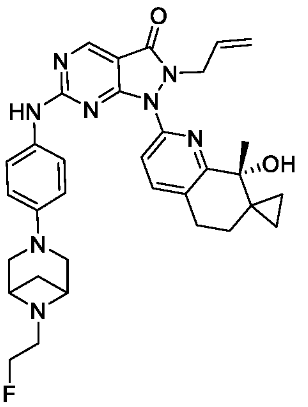 | WEE1-164 | 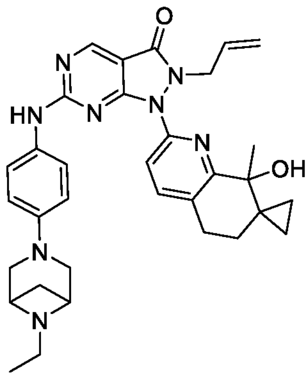 |
| WEE1-165 | 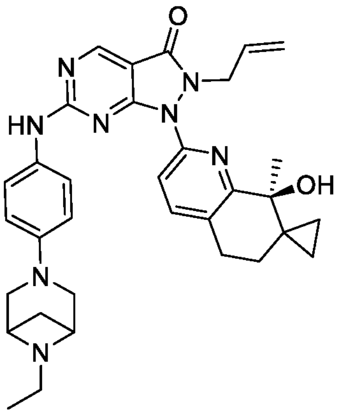 | WEE1-166 | 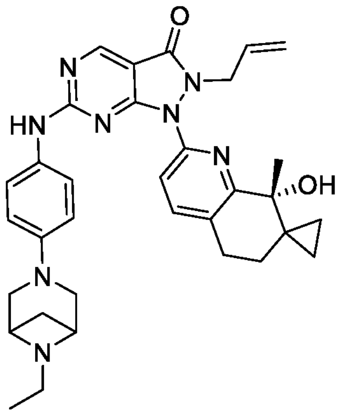 |

(continued)

| No. | Compound Structure | No. | Compound Structure |
|---|---|---|---|
| WEE1-167 | 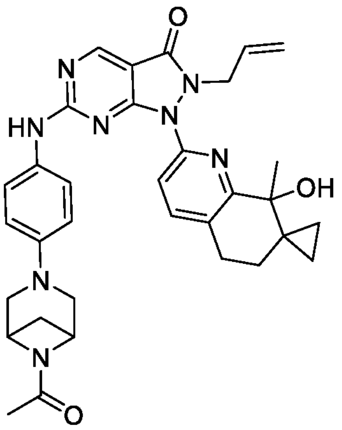 | WEE1-168 | 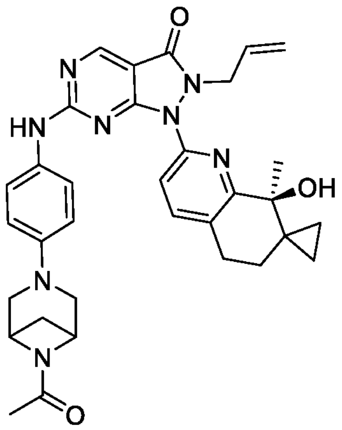 |
| WEE1-169 | 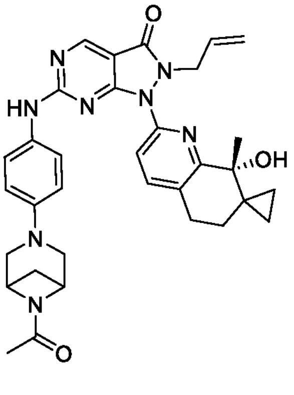 | WEE1-170 | 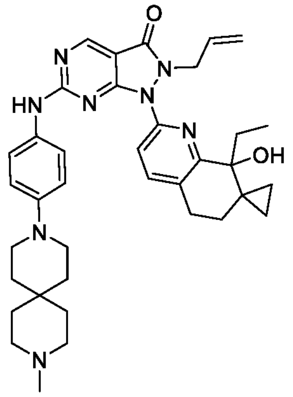 |
| WEE1-171 | 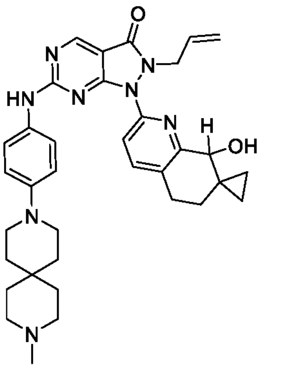 | WEE1-172 | 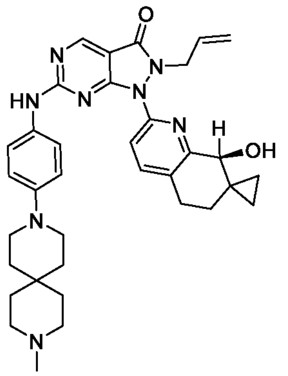 |
| WEE1-173 | 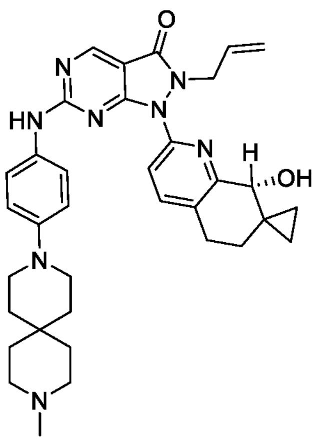 | WEE1-174 | 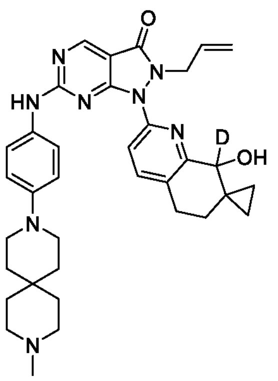 |

(continued)

| No. | Compound Structure | No. | Compound Structure |
|---|---|---|---|
| WEE1-175 | 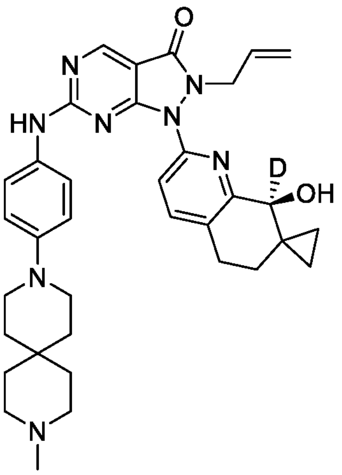 | WEE1-176 | 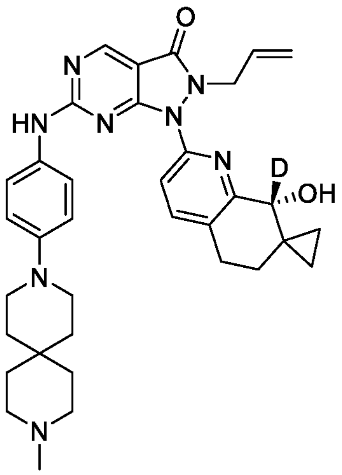 |
| WEE1-177 | 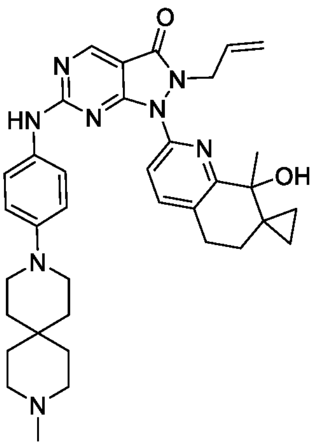 | WEE1-178 | 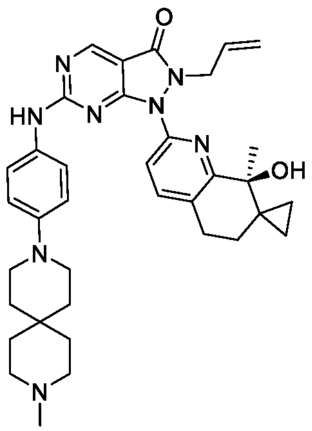 |
| WEE1-179 | 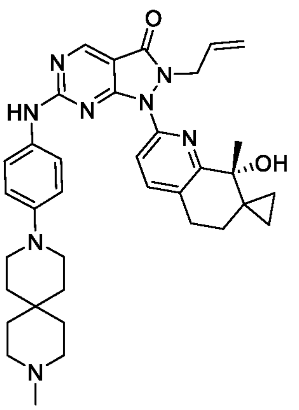 | WEE1-180 | 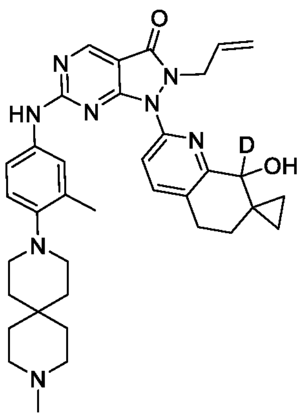 |

(continued)

| No. | Compound Structure | No. | Compound Structure |
|---|---|---|---|
| WEE1-181 | 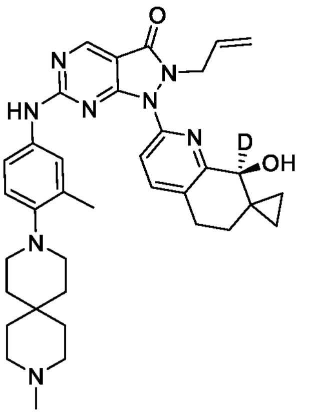 | WEE1-182 | 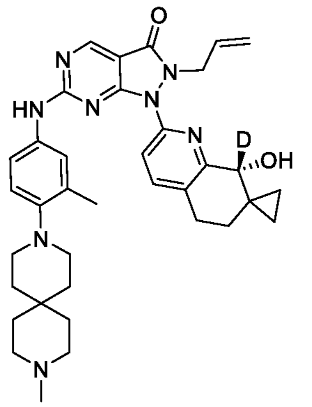 |
| WEE1-183 | 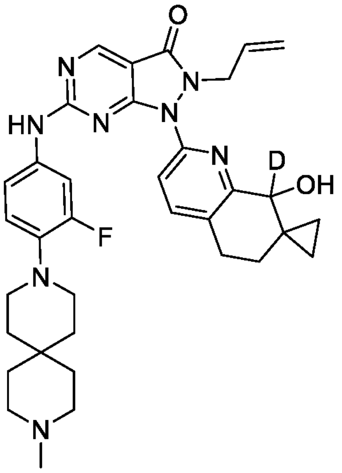 | WEE1-184 | 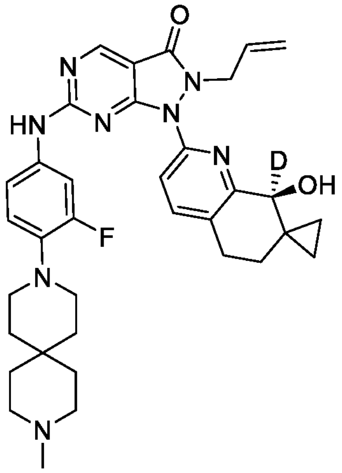 |
| WEE1-185 | 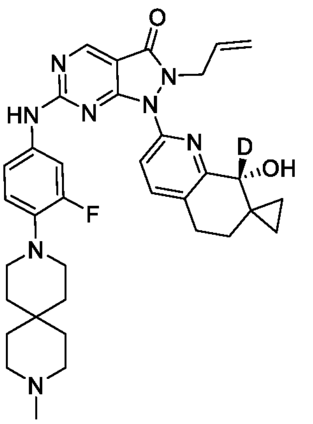 | WEE1-186 | 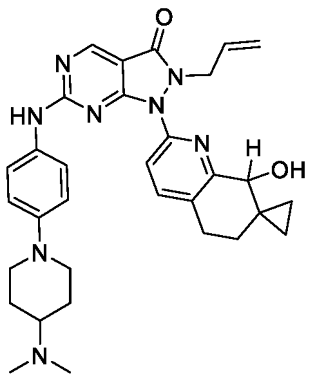 |

(continued)

| No. | Compound Structure | No. | Compound Structure |
|---|---|---|---|
| WEE1-187 | 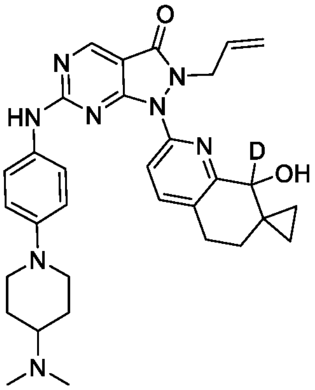 | WEE1-188 | 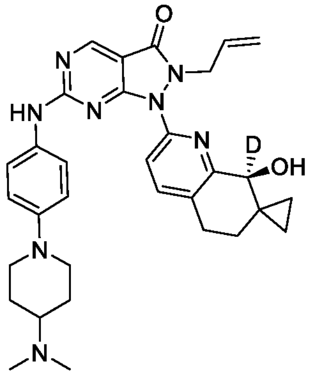 |
| WEE1-189 | 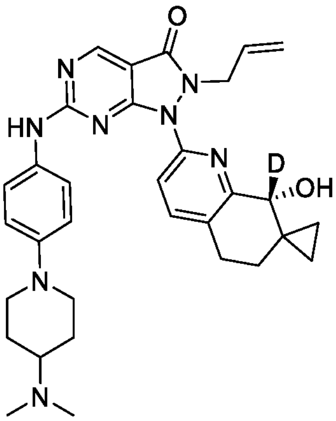 | WEE1-190 | 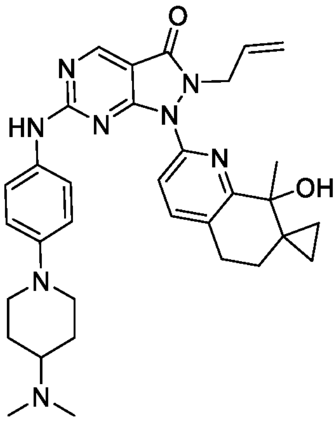 |
| WEE1-191 | 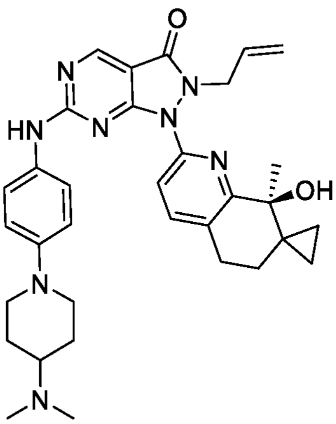 | WEE1-192 | 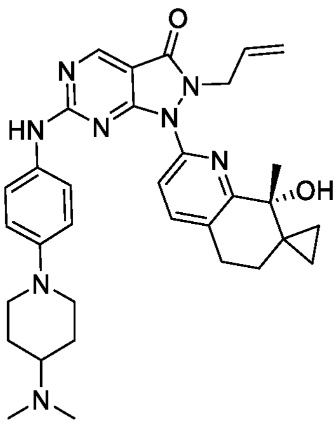 |
| WEE1-193 | 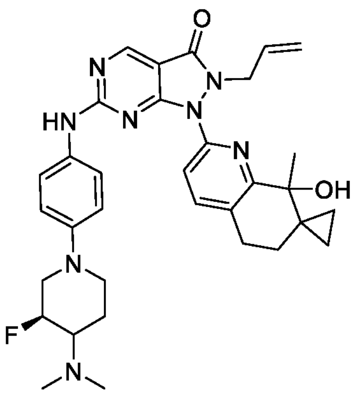 | WEE1-194 | 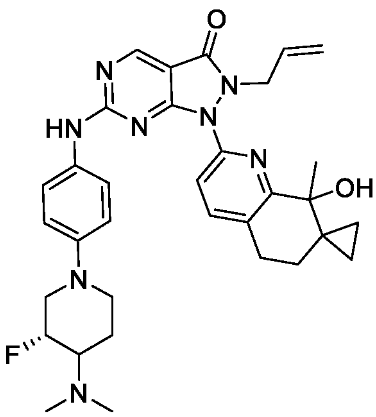 |

(continued)

| No. | Compound Structure | No. | Compound Structure |
|---|---|---|---|
| WEE1-195 | 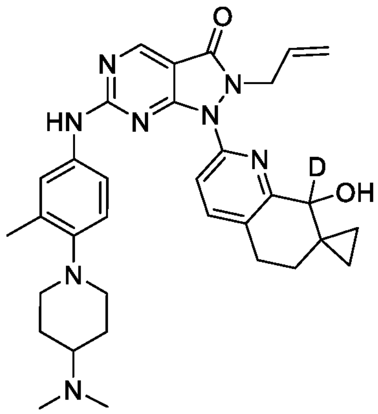 | WEE1-196 | 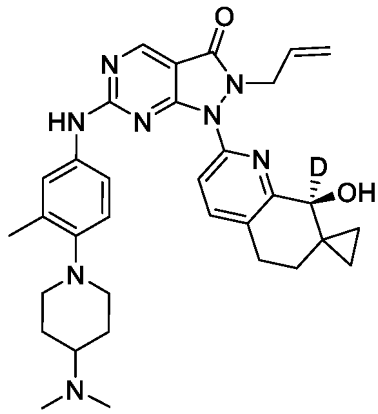 |
| WEE1-197 | 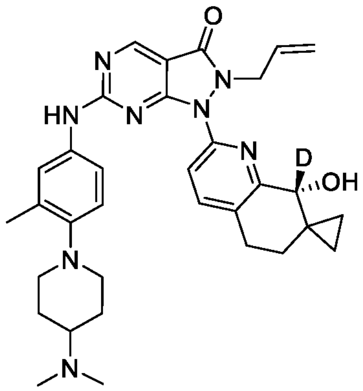 | WEE1-198 | 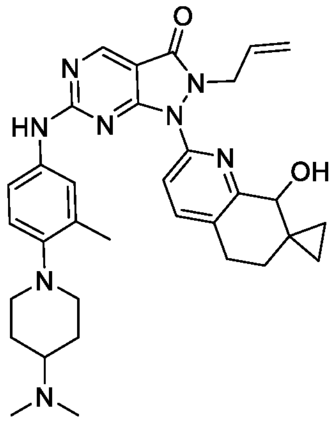 |
| WEE1-199 | 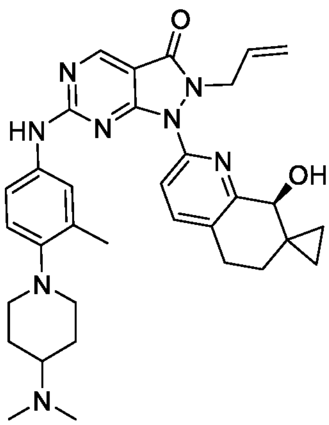 | WEE1-200 | 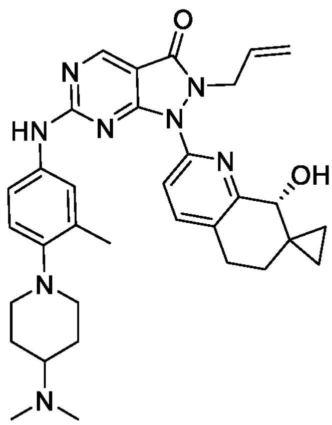 |

(continued)

| No. | Compound Structure | No. | Compound Structure |
|---|---|---|---|
| WEE1-201 | 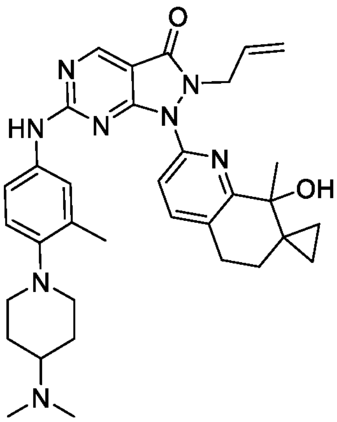 | WEE1-202 | 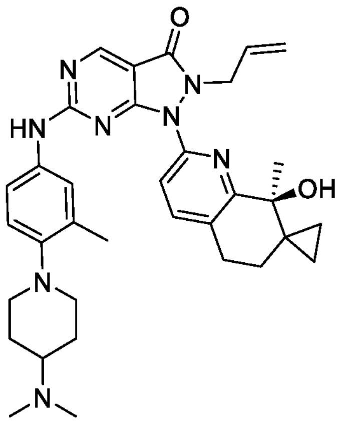 |
| WEE1-203 | 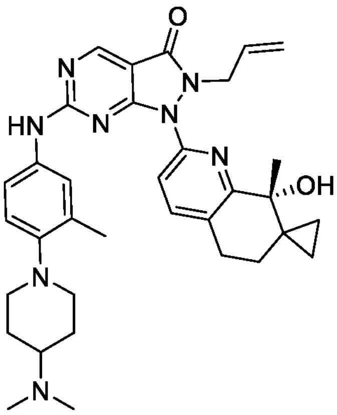 | WEE1-204 | 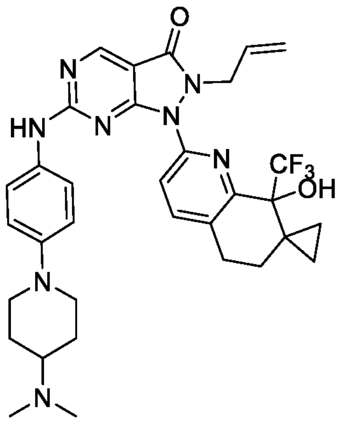 |
| WEE1-205 | 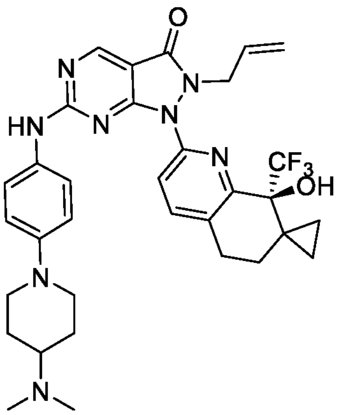 | WEE1-206 | 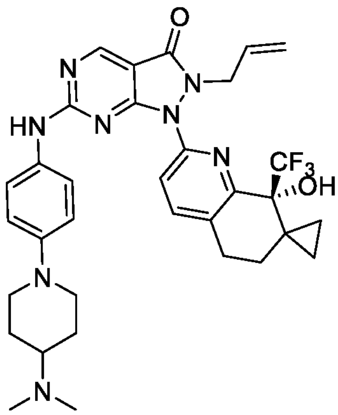 |
| WEE1-207 | 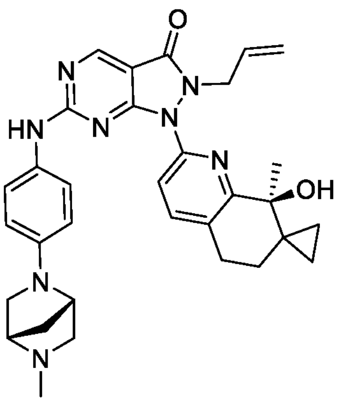 | WEE1-208 | 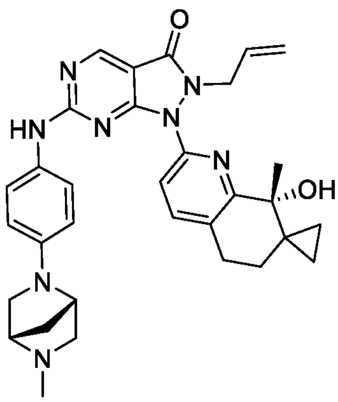 |

(continued)

| No. | Compound Structure | No. | Compound Structure |
|---|---|---|---|
| WEE1-209 | 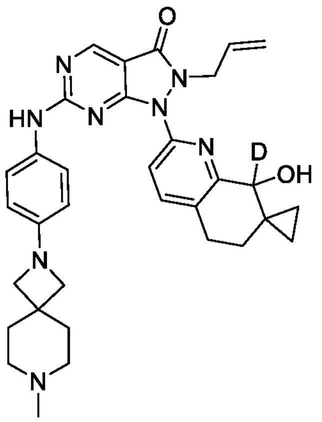 | WEE1-210 | 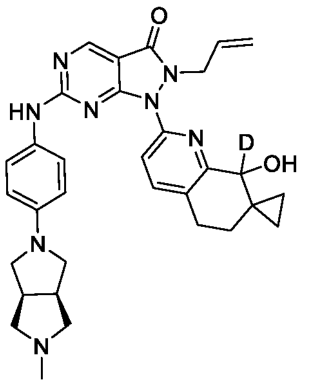 |
| WEE1-211 | 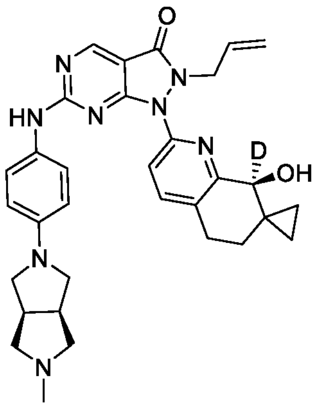 | WEE1-212 | 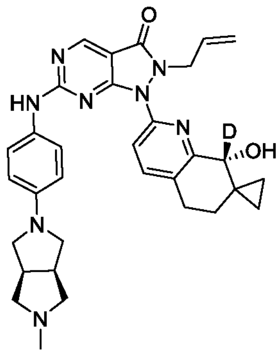 |
| WEE1-213 | 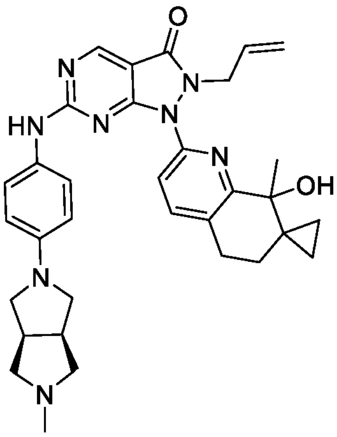 | WEE1-214 | 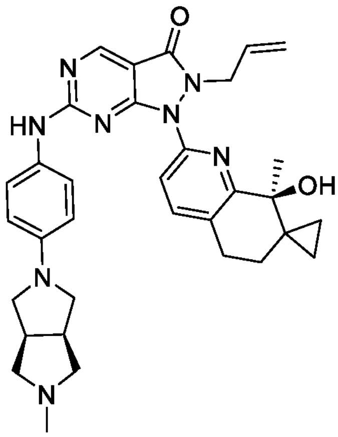 |

(continued)

| No. | Compound Structure | No. | Compound Structure |
|---|---|---|---|
| WEE1-215 | 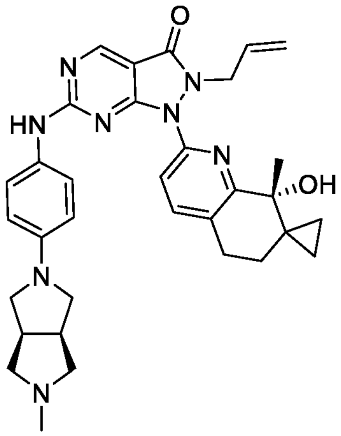 | WEE1-216 | 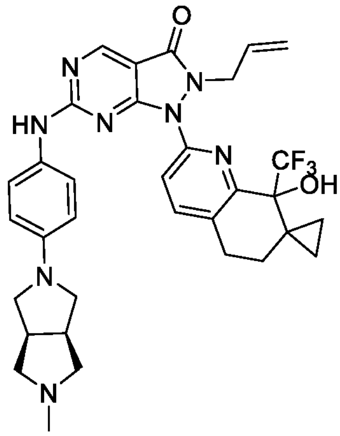 |
| WEE1-217 | 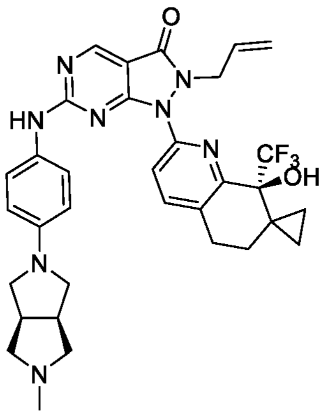 | WEE1-218 | 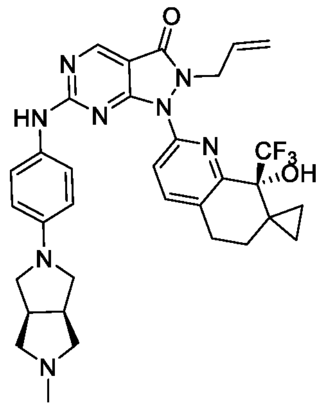 |
| WEE1-219 | 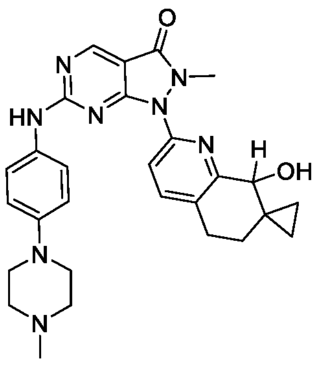 | WEE1-220 | 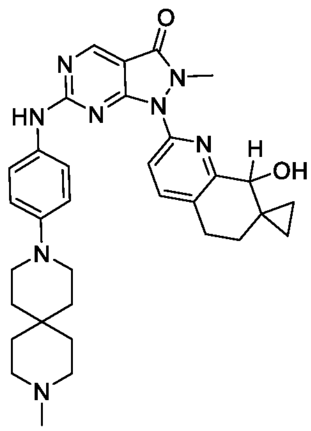 |

(continued)

| No. | Compound Structure | No. | Compound Structure |
|---|---|---|---|
| WEE1-221 | 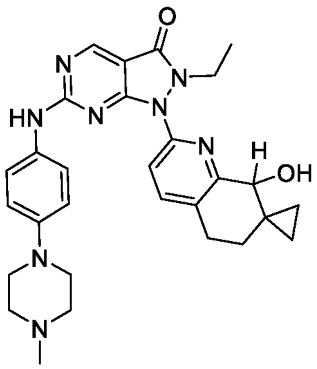 | WEE1-222 | 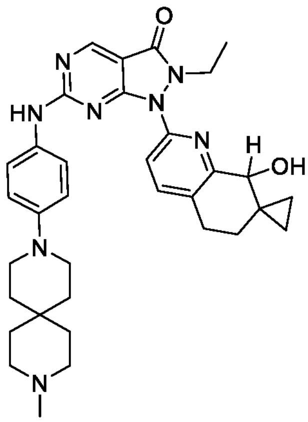 |
| WEE1-223 | 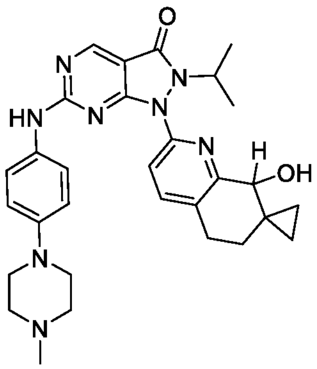 | WEE1-224 | 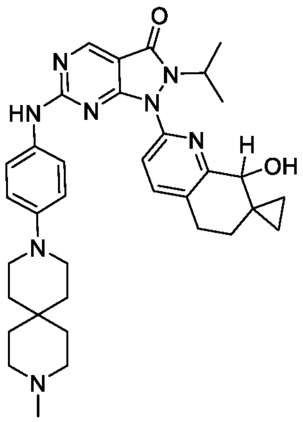 |
| WEE1-225 | 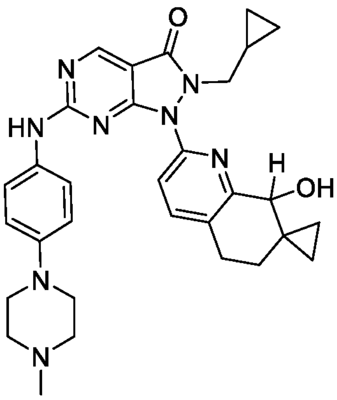 | WEE1-226 | 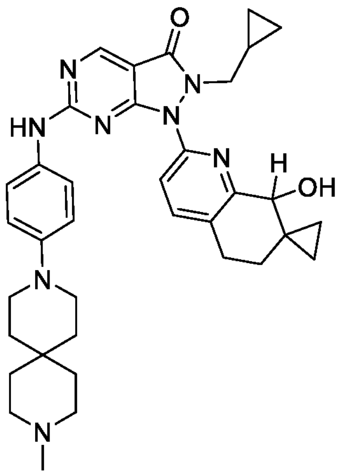 |

(continued)

| No. | Compound Structure | No. | Compound Structure |
|---|---|---|---|
| WEE1-227 | 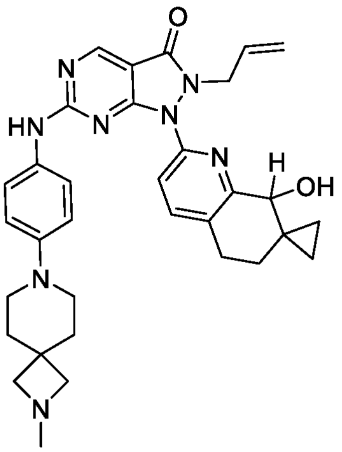 | WEE1-228 | 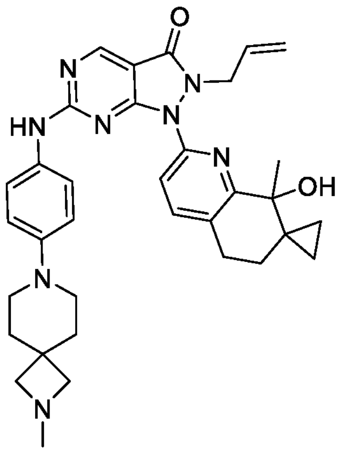 |
| WEE1-229 | 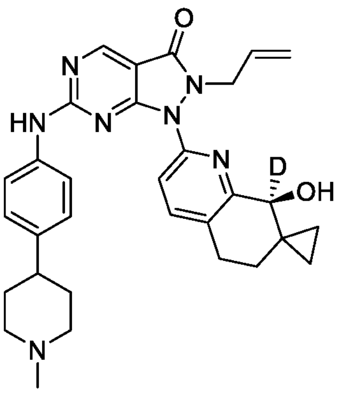 | WEE1-230 | 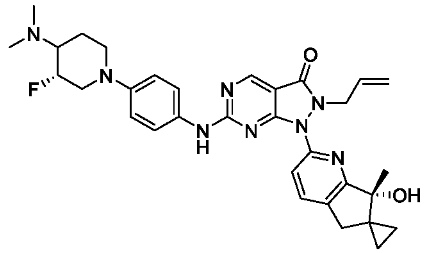 |
| WEE1-231 | 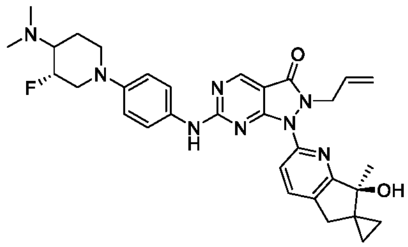 | WEE1-232 | 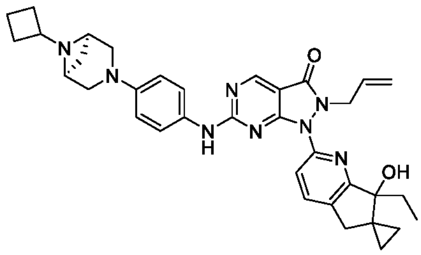 |
| WEE1-233 | 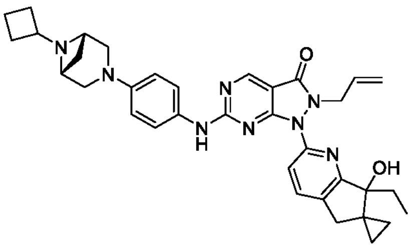 | WEE1-234 | 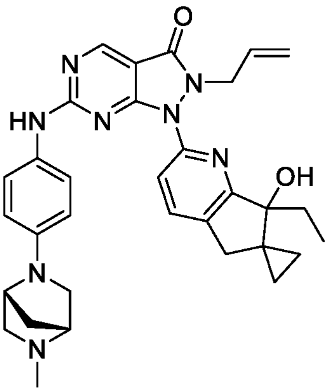 |

(continued)

| No. | Compound Structure | No. | Compound Structure |
|---|---|---|---|
| WEE1-235 | 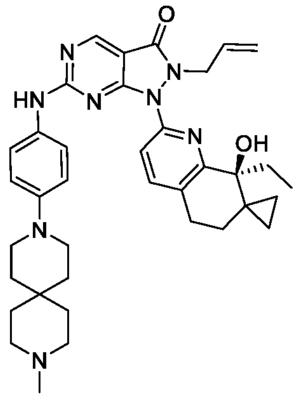 | WEE1-236 | 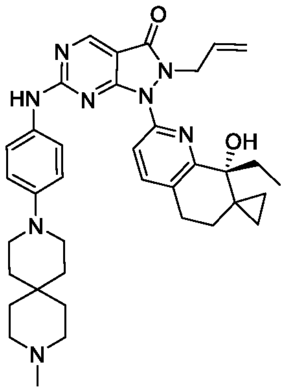 |
| WEE1-237 | 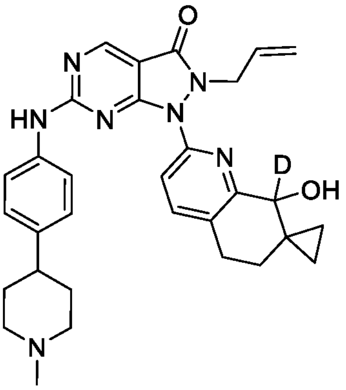 | | |

**14.** The compound, or a stereoisomer thereof, or a pharmaceutically acceptable salt thereof according to any one of claims 1, wherein the compound represented by Formula I is specifically

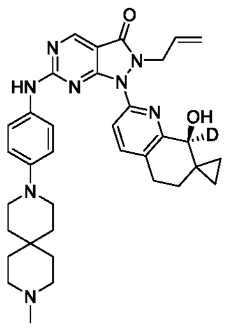 or 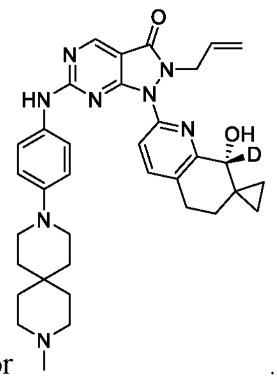 .

**15.** Use of the compound according to any one of claims 1 to 14, or a stereoisomer thereof, or a pharmaceutically acceptable salt thereof, in the preparation of a medicament for treatment of WEE1-mediated disease.

**16.** The use according to claim 15, wherein the WEE1-mediated disease is one or more selected from diseases related to inflammation, autoimmune disease, infectious disease, cancer, precancer syndrome.

**17.** A pharmaceutical composition, **characterized in that** it is a formulation prepared with the compound of any one of claims 1 to 14, or a stereoisomer thereof, or a pharmaceutically acceptable salt thereof, together with pharmaceutically acceptable excipients.

# INTERNATIONAL SEARCH REPORT

International application No.

**PCT/CN2023/072296**

**A. CLASSIFICATION OF SUBJECT MATTER**

C07D519/00(2006.01)i;C07D487/04(2006.01)i;C07D487/12(2006.01)i;A61K31/519(2006.01)i;A61P29/00(2006.01)i;A61P31/00(2006.01)i;A61P35/00(2006.01)i;A61P37/00(2006.01)i

According to International Patent Classification (IPC) or to both national classification and IPC

**B. FIELDS SEARCHED**

Minimum documentation searched (classification system followed by classification symbols)

IPC: C07D519/-; C07D487/-; A61K31/-; A61P29/-; A61P31/-; A61P35/-; A61P37/-

Documentation searched other than minimum documentation to the extent that such documents are included in the fields searched

Electronic data base consulted during the international search (name of data base and, where practicable, search terms used)

VEN, DWPI, CNTXT, CNKI, CAplus, REG: WEE1, 根据权利要求通式进行STN结构式检索, STN structural formula search is performed according to the general formula in the claims

**C. DOCUMENTS CONSIDERED TO BE RELEVANT**

| Category* | Citation of document, with indication, where appropriate, of the relevant passages | Relevant to claim No. |
|---|---|---|
| A | CN 113387962 A (SHANGHAI DE NOVO PHARMATECH CO., LTD.) 14 September 2021 (2021-09-14)<br>claims 1, 5, and 10-21, and description, embodiment compounds | 1-17 |
| A | CN 110198943 A (SHIJIAZHUANG SAGACITY NEW DRUG DEVELOPMENT CO, LTD.) 03 September 2019 (2019-09-03)<br>see entire document | 1-17 |
| A | WO 2017075629 A2 (THE REGENTS OF THE UNIVERSITY OF COLORADO, A BODY CORPORATE) 04 May 2017 (2017-05-04)<br>see entire document | 1-17 |
| A | WO 2018133829 A1 (MEDSHINE DISCOVERY INC.) 26 July 2018 (2018-07-26)<br>see entire document | 1-17 |
| PA | CN 115403582 A (JIANGSU TASLY DIYI PHARMACEUTICAL CO., LTD.) 29 November 2022 (2022-11-29)<br>see claim 1, and the last two compounds of claim 23 | 1-17 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **17 April 2023** | **23 April 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2023/072296**

| Patent document cited in search report | Publication date (day/month/year) | Patent family member(s) | Publication date (day/month/year) |
|---|---|---|---|
| CN 113387962 A | 14 September 2021 | None | |
| CN 110198943 A | 03 September 2019 | WO 2018133829 A1 | 26 July 2018 |
| | | JP 2020510630 A | 09 April 2020 |
| | | JP 6717457 B2 | 01 July 2020 |
| | | US 2020017528 A1 | 16 January 2020 |
| | | US 10954253 B2 | 23 March 2021 |
| | | EP 3572413 A1 | 27 November 2019 |
| | | EP 3572413 A4 | 19 August 2020 |
| | | EP 3572413 B1 | 03 November 2021 |
| | | ES 2902050 T3 | 24 March 2022 |
| WO 2017075629 A2 | 04 May 2017 | JP 2018536651 A | 13 December 2018 |
| | | JP 6692423 B2 | 13 May 2020 |
| | | US 2019084985 A1 | 21 March 2019 |
| | | US 10947238 B2 | 16 March 2021 |
| | | AU 2016344040 A1 | 17 May 2018 |
| | | AU 2016344040 B2 | 10 December 2020 |
| | | CA 3003737 A1 | 04 May 2017 |
| | | CA 3003737 C | 14 September 2021 |
| | | WO 2017075629 A3 | 08 June 2017 |
| | | EP 3368538 A2 | 05 September 2018 |
| | | EP 3368538 A4 | 12 June 2019 |
| | | EP 3368538 B1 | 01 September 2021 |
| WO 2018133829 A1 | 26 July 2018 | None | |
| CN 115403582 A | 29 November 2022 | WO 2022247641 A1 | 01 December 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007126122 A
- WO 2008133866 A
- WO 2013012681 A
- WO 2013126656 A
- WO 2014167347 A
- WO 2015092431 A
- WO 2018011569 A
- WO 2018011570 A
- WO 2018090939 A
- WO 2018133829 A
- WO 2018171633 A


### Non-patent literature cited in the description


- *Clinical Cancer Research*, 2011, vol. 17 (13), 4200-4207
- *Molecular & Cellular Biology*, 2012, vol. 32 (20), 4226
- *Proceedings of the National Academy of Sciences of the United States of America*, 2007, vol. 104 (10), 3753-3758
- *Oncotarget*, 2016, vol. 7 (31), 49902-49916
- *Molecular Cancer*, 2014, vol. 13 (1), 72
- *Drug News&Perspectives*, 2010, vol. 23 (7), 425
- *Molecular Cancer Therapeutics*, 2013, vol. 12 (12), 2675-2684
- *Cancer Biology &Therapy*, 2010, vol. 9 (7), 523-525
- *CHEMICAL ABSTRACTS*, 955365-80-7
- *CHEMICAL ABSTRACTS*, 2376146-48-2